(19) Europäisches Patentamt
European Patent Office
Office européen des brevets

(11) EP 4 497 754 A1

(12) **EUROPEAN PATENT APPLICATION**
published in accordance with Art. 153(4) EPC

(43) Date of publication:
**29.01.2025 Bulletin 2025/05**

(21) Application number: **23773906.5**

(22) Date of filing: **22.03.2023**

(51) International Patent Classification (IPC):
***C07K 5/10*** (2006.01) ***A61K 38/07*** (2006.01)
***A61P 25/04*** (2006.01) ***A61P 29/00*** (2006.01)

(52) Cooperative Patent Classification (CPC):
**A61K 38/07; A61P 25/04; A61P 29/00; C07K 5/10**

(86) International application number:
**PCT/CN2023/083063**

(87) International publication number:
**WO 2023/179659 (28.09.2023 Gazette 2023/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.03.2022 CN 202210291141**
**23.03.2022 CN 202210291100**
**23.03.2022 CN 202210291169**

(71) Applicant: **Jiangsu NHWA Pharmaceutical Co., Ltd**
**Xuzhou, Jiangsu 221000 (CN)**

(72) Inventors:
- **HOU, Yuanyuan**
**Xuzhou, Jiangsu 221000 (CN)**
- **XU, Xiangqing**
**Xuzhou, Jiangsu 221000 (CN)**
- **YU, Minquan**
**Xuzhou, Jiangsu 221000 (CN)**
- **ZHAO, Song**
**Xuzhou, Jiangsu 221000 (CN)**
- **GUO, Qiang**
**Xuzhou, Jiangsu 221000 (CN)**
- **WANG, Wei**
**Xuzhou, Jiangsu 221000 (CN)**
- **ZHANG, Datong**
**Xuzhou, Jiangsu 221000 (CN)**
- **FENG, Pengwen**
**Xuzhou, Jiangsu 221000 (CN)**
- **CHENG, Qidong**
**Xuzhou, Jiangsu 221000 (CN)**
- **WANG, Ao**
**Xuzhou, Jiangsu 221000 (CN)**
- **HE, Xiaojing**
**Xuzhou, Jiangsu 221000 (CN)**
- **XU, Changda**
**Xuzhou, Jiangsu 221000 (CN)**
- **DONG, Yingying**
**Xuzhou, Jiangsu 221000 (CN)**
- **QIU, Yinli**
**Xuzhou, Jiangsu 221000 (CN)**
- **LU, Aijun**
**Shanghai 201203 (CN)**
- **CHEN, Jianhui**
**Shanghai 201203 (CN)**

(74) Representative: **Lavoix**
**Bayerstraße 83**
**80335 München (DE)**

# (54) POLYAMIDE COMPOUNDS, METHOD FOR PREPARING SAME, AND MEDICAL USE THEREOF

(57) The present invention relates to the field of medicines, particularly to synthetic polyamide compounds represented by formulas IA, IB, and IC, or pharmaceutically acceptable salts and stereoisomers thereof, a composition containing same, a method for preparing same, and use thereof in the field of medicines.

EP 4 497 754 A1

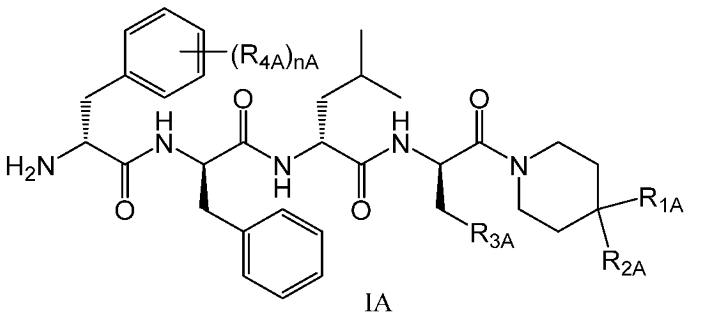

IA

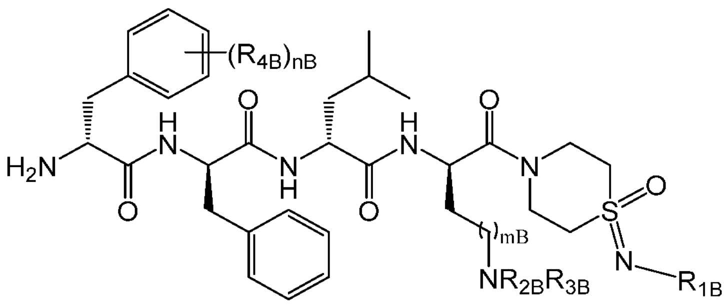

IB

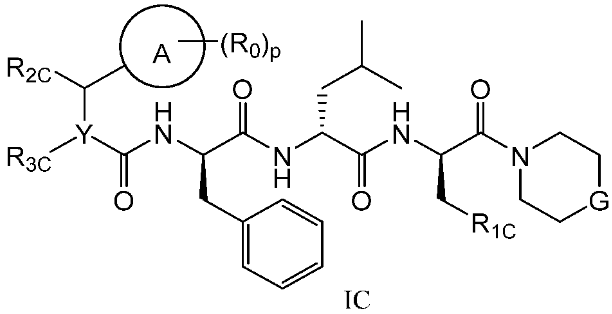

IC

## Description

### TECHNICAL FIELD

The present invention relates to the field of medicines, particularly to a synthetic polyamide compound, or a pharmaceutically acceptable salt and a stereoisomer thereof, a composition comprising same, and use thereof in the field of medicines.

### BACKGROUND

Opioid receptors are an important class of G protein-coupled receptors and are targets for binding to endogenous opioid peptides and opioid drugs. After being activated, opioid receptors play a regulatory role in nervous system immunity and endocrine system. Opioid drugs are the strongest and most commonly used central analgesics at present. Opioid receptors present in the central nervous system include $\mu$, $\delta$, $\kappa$ receptors and the like.

$\kappa$-opioid receptor (KOR) is composed of 380 amino acids. It is expressed in sensory neurons, dorsal root ganglion cells, and primary afferent neuron terminals, and is involved in important physiological activities such as pain perception, neuroendocrine, emotional behavior, and cognition.

KOR agonists, as medicaments, have good application prospects in the pharmaceutical industry. For example, CN108290926A discloses a class of phenylpropanamide derivatives as KOR agonists, and CN 101627049 A also discloses a class of synthetic peptide amides as KOR agonists, wherein the compound D-Phe-D-Phe-D-Leu-D-Lys-[$\omega$(4-aminopiperidine-4-carboxylic acid)]-OH (development code: CR845) has been clinically studied.

Although some KOR agonists already exist in the art, there is still a need for novel KOR agonists with improved activity and/or druggability.

### SUMMARY

The present invention provides a novel $\kappa$-opioid receptor (KOR receptor) agonist compound, which surprisingly exhibits excellent effects and functions. In particular, such novel amide bond-containing compounds not only have excellent KOR receptor agonistic potency (high affinity for the $\kappa$-opioid receptor), but also have very good hydrophilicity and thus less ability to penetrate the blood brain barrier and lower ability to enter the brain. In some embodiments, the compounds of the present invention also have higher selectivity for the $\kappa$-opioid receptor than for the $\mu$- and $\delta$-opioid receptors. In some embodiments, the compounds of the present invention also have lower addiction, better physicochemical properties (e.g., solubility, physical and/or chemical stability), improved pharmacokinetic properties (e.g., lower inhibition of cytochrome $P_{450}$ isoenzymes, improved bioavailability, suitable half-life and duration of action), improved safety (lower toxicity and/or fewer side effects (e.g., side effects on the central nervous system, respiratory depression, sedation, causing hallucinations, antidiuresis, nausea, constipation, dependence, etc.)), good patient compliance, and/or less susceptibility to developing tolerance, and other superior druggability properties. In some embodiments, the compounds of the present invention have improved safety, i.e., have lower acute toxicity and cardiotoxicity. In some embodiments, the compounds of the present invention have an improved safety window (e.g., have a greater safe dose range of administration, or are less likely to have side effects at the same dose). In some embodiments, the compounds of the present invention have improved pharmacokinetic properties (e.g., improved bioavailability and longer half-life, etc.).

One aspect of the present invention provides a compound of formula IA, a stereoisomer thereof, or a pharmaceutically acceptable salt thereof:

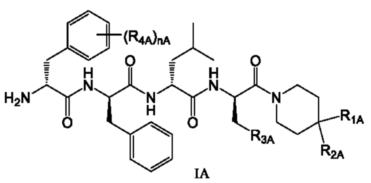

IA

wherein,

$R_{1A}$ is H or -COOH, preferably H; $R_{2A}$ is $-NR_{aA}C(=O)OR_{bA}$ or $-NR_{aA}S(=O)_2OR_{ba}$, preferably - $NR_{aA}C(=O)OR_{bA}$, wherein $R_{aA}$ is H or $C_{1-6}$ alkyl substituted with one or more groups selected from amino, $C_{1-6}$ alkylamino, $C_{1-6}$ alkoxy, halogen, hydroxyl, nitro, cyano, $NH_2C(=O)$-, and $C_{1-6}$ alkoxy, preferably H or $C_{1-6}$ alkyl substituted with amino, $C_{1-6}$ alkylamino, or $C_{1-6}$ alkoxy, and more preferably H or $C_{1-6}$ alkyl substituted with amino or $C_{1-6}$ alkylamino; $R_{bA}$ is selected from $C_{1-6}$ alkyl, $C_{6-14}$ aryl, 5- to 14-membered heteroaryl, $C_{3-8}$ cycloalkyl, and 3- to 8-membered heterocyclyl,

preferably from $C_{1-6}$ alkyl and 3- to 8-membered heterocyclyl, more preferably from $C_{1-6}$ alkyl and

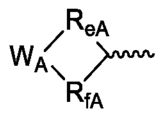

,

and even more preferably from $C_{1-6}$ alkyl and

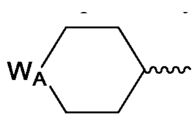

,

the group $R_{ba}$ described above is optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, wherein $R_{eA}$ and $R_{fA}$ are each independently $-(CH_2)_{n1A}$- and $-(CH_2)_{n1A'}$-, n1A and n1A' are each independently selected from 0, 1, 2, and 3, preferably 2, and n1A and n1A' are not both 0; $W_A$ is selected from -NH-C(=O)-, $-NH-S(=O)_2-$, $-NR_{5A}-$, - O-, -S-, and $-S(=O)_2-$, preferably from -O- and $-S(=O)_2-$, $R_{5A}$ is selected from H, $C_{1-6}$ alkyl, amidino, and $HOOC-(CH_2)_{n3A}-$, wherein n3A is selected from 1, 2, and 3;

or $R_{1A}$ and $R_{2A}$, together with the carbon atom to which they are both attached form an optionally substituted 9- to 10-membered bicyclic moiety, preferably, the bicyclic moiety, together with the piperidine ring attached thereto, forms a structure selected from:

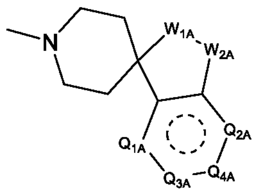

and

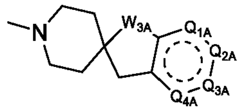

,

when any one of $Q_{1A}$-$Q_{4A}$ is N, the rest are C, or $Q_{1A}$-$Q_{4A}$ are all C;

$W_{1A}$ and $W_{2A}$ are each independently -C(=O)-NH-, -NH-C(=O)-, $-S(=O)_2-NH-$, $-NH-S(=O)_2-$, -S-, -O-, - $NR_{6A}$-, $-NR_{6A}-CH_2-$, $-(CH_2)_{n2A}-$ optionally substituted with one or more groups selected from $-NH_2$, -OH, halogen, nitro, cyano, $C_{1-6}$ haloalkyl, $NH_2C(=O)$-, and $C_{1-6}$ alkoxy, or absent, wherein $R_{6A}$ is selected from H, $C_{1-6}$ alkyl, amidino, and $HOOC-(CH_2)_{n3A}-$; preferably, $W_{1A}$ and $W_{2A}$ cannot be simultaneously absent;

$W_{3A}$ is $-(CH_2)_{n2A}-$ optionally substituted with one or more groups selected from $NH_2$, -OH, halogen, nitro, cyano, $C_{1-6}$ haloalkyl, $NH_2C(=O)$-, and $C_{1-6}$ alkoxy;

n2A is selected from 0, 1, 2, and 3;

more preferably, the bicyclic moiety, together with the piperidine ring attached thereto, forms a structure selected from:

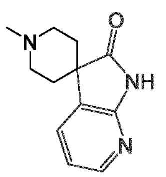 , 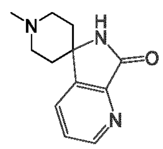 , 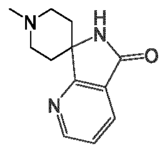 , 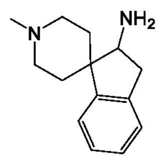 , 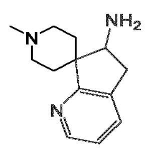 , 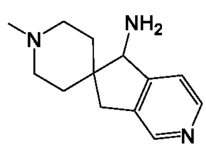 ,

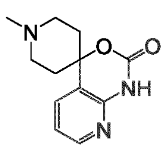 , 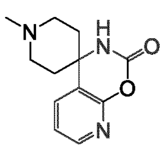 , 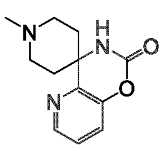 , 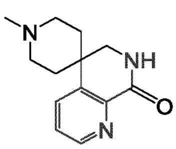 , 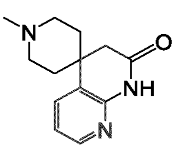 , 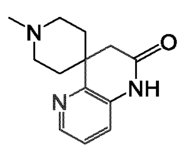 ,

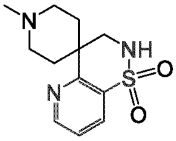, 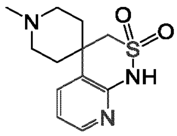, 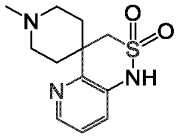, 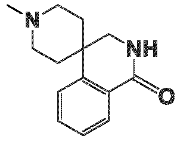, 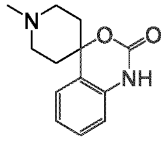, 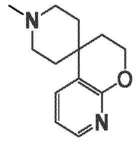,

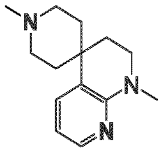, 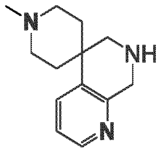, and 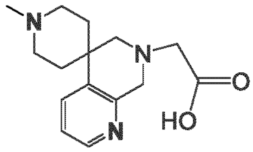,

more preferably from

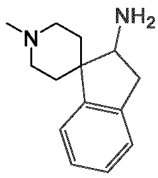,

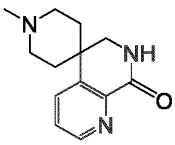, 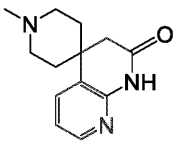, 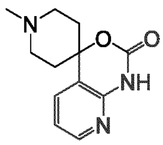, 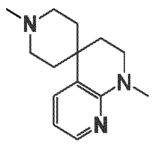, and 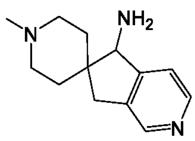, even more preferably from

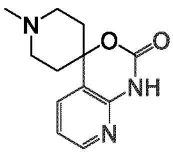 and 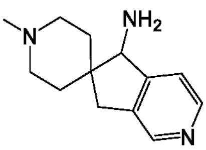;

$R_{3A}$ is selected from H or $-(CH_2)_{mA}NR_{cA}R_{dA}$;
$R_{cA}$ and $R_{dA}$ are each independently selected from H, $C_{1-6}$ alkyl, amidino, and $C_{1-6}$ alkoxycarbonyl;
$R_{4A}$ is selected from halogen, $NO_2$, $C_{1-6}$ alkyl, $C_{1-6}$ haloalkyl, cyano, $NH_2C(=O)$-, and $C_{1-6}$ alkoxy;
mA and nA are each independently 0, 1, 2, 3, 4, or 5.

Another aspect of the present invention provides a method for preparing the compound of the present invention, which is selected from the following methods:

method I:
method I comprises the following steps:

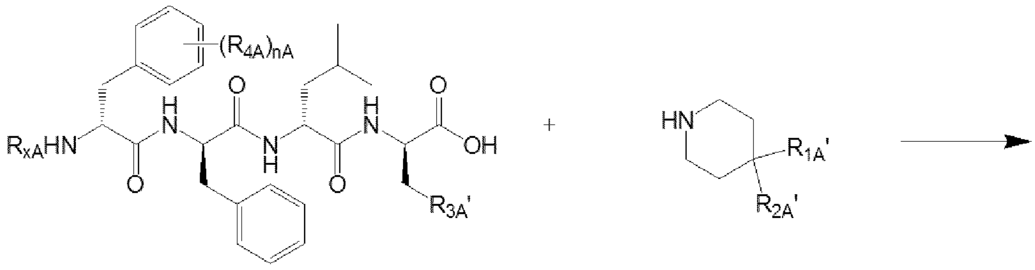

iA-2 iA-3

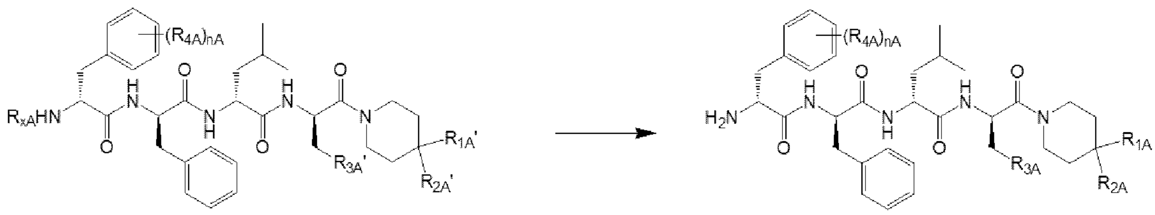

iA-1 iA

,

method II:

method II comprises the following steps:

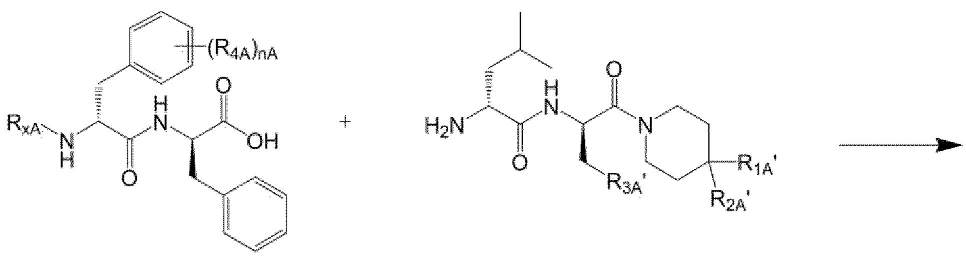

iiA-2 iiA-3

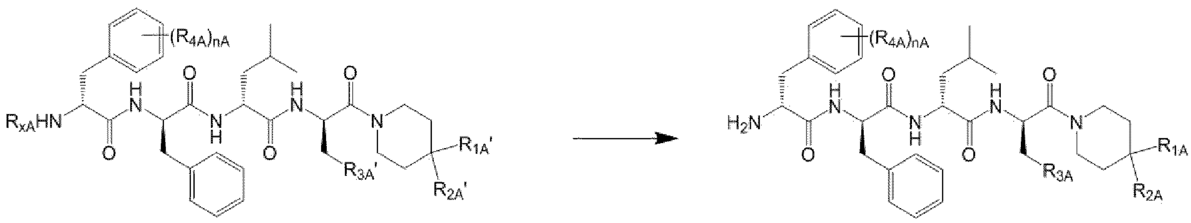

iA-1 iA

,

wherein $R_{1A}$' is $R_{1A}$ or $R_{1A}$ in which $NH_2$ is protected with an amino-protecting group, $R_{2A}$' is $R_{2A}$ or $R_{2A}$ in which $NH_2$ is protected with an amino-protecting group, $R_{3A}$' is $R_{3A}$ or $R_{3A}$ in which $NH_2$ is protected with an amino-protecting group, $R_{1A}$, $R_{2A}$, $R_{3A}$, $R_{4A}$, and nA are as as defined above, and $R_{xA}$ is an amino-protecting group;

the amino-protecting groups are preferably each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, *tert*-butylsulfinyl, or ethoxycarbonyl.

The present invention further provides a compound of the following general formula, a stereoisomer thereof, or a salt thereof,

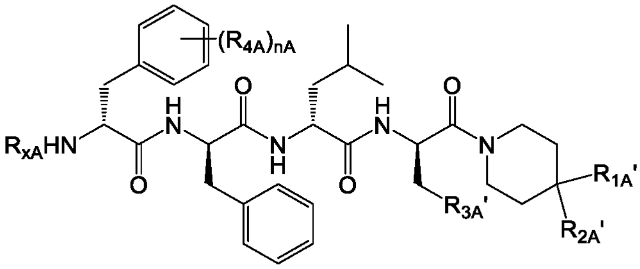

iA-1

wherein $R_{1A}$' is $R_{1A}$ or $R_{1A}$ in which $NH_2$ is protected with an amino-protecting group, $R_{2A}$' is $R_{2A}$ or $R_{2A}$ in which $NH_2$ is protected with an amino-protecting group, $R_{3A}$' is $R_{3A}$ or $R_{3A}$ in which $NH_2$ is protected with an amino-protecting group, $R_{1A}$, $R_{2A}$, $R_{3A}$, $R_{4a}$, and nA are as defined above, and $R_{xA}$ is an amino-protecting group;
the amino-protecting groups are preferably each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, *tert*-butylsulfinyl, or ethoxycarbonyl.

One aspect of the present invention provides a compound of formula IB, a stereoisomer thereof, or a pharmaceutically acceptable salt thereof:

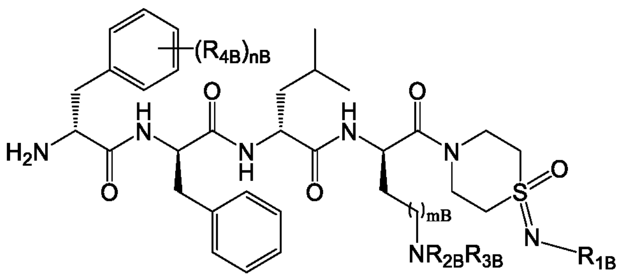

IB

wherein,

$R_{1B}$ is selected from H, $C_{1-6}$ alkyl (e.g., methyl), $C_{1-6}$ alkylcarbonyl (e.g., methylcarbonyl), $C_{1-6}$ alkoxycarbonyl (e.g., methoxycarbonyl), $C_{6-14}$ aryl, $C_{6-14}$ arylcarbonyl, $C_{6-14}$ aryloxycarbonyl, $C_{3-8}$ cycloalkyl, $C_{3-8}$ cycloalkylcarbonyl, $C_{3-8}$ cycloalkoxycarbonyl, 5- to 14-membered heteroaryl, 5- to 14-membered heteroarylcarbonyl, 5- to 14-membered heteroaryloxycarbonyl, 3- to 8-membered heterocyclyl, 3- to 8-membered heterocyclylcarbonyl, and 3- to 8-membered heterocyclyloxycarbonyl, preferably selected from H, $C_{1-6}$ alkyl (e.g., methyl), $C_{1-6}$ alkylcarbonyl (e.g., methylcarbonyl), and $C_{1-6}$ alkoxycarbonyl (e.g., methoxycarbonyl), and more preferably selected from $C_{1-6}$ alkyl (e.g., methyl) and $C_{1-6}$ alkylcarbonyl (e.g., methylcarbonyl), the above substituents are each optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;
$R_{2B}$ and $R_{3B}$ are each independently selected from H, $C_{1-6}$ alkyl (e.g., methyl and isopropyl), amidino, and $C_{1-6}$ alkoxycarbonyl (e.g., methoxycarbonyl);
$R_{4B}$ is selected from halogen, $NO_2$, $C_{1-6}$ alkyl, $C_{1-6}$ haloalkyl, cyano, $NH_2C(=O)$-, and $C_{1-6}$ alkoxy;
mB and nB are each independently 0, 1, 2, 3, 4, or 5, for example, mB is 3, and/or nB is 0.

Another aspect of the present invention provides a method for preparing the compound of the present invention, which comprises the following steps:

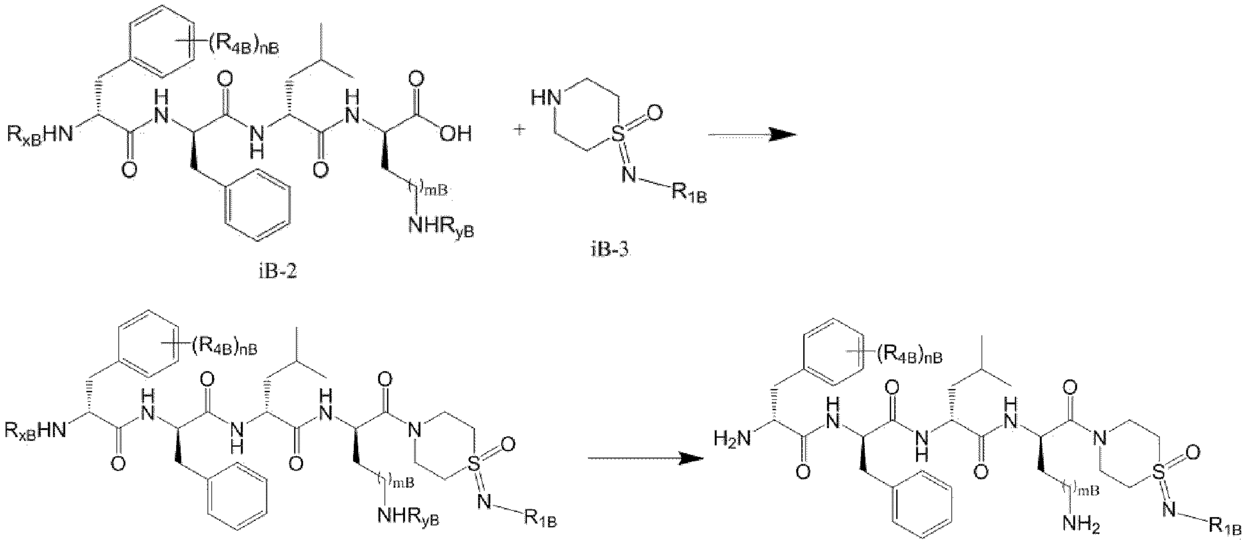

iB-2 iB-3

iB-1 iB

wherein, $R_{1B}$, $R_{4B}$, mB, and nB are as defined above, and $R_{xB}$ and $R_{yB}$ are amino-protecting groups, preferably independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, or ethoxycarbonyl.

Another aspect of the present invention provides a compound of formula iB-1, a stereoisomer thereof, or a salt thereof,

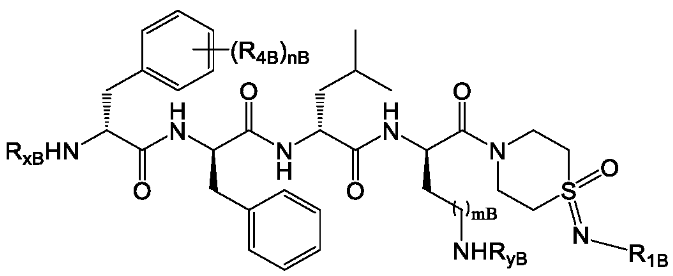

**iB-1**

wherein $R_{1B}$, $R_{4B}$, mB, and nB are as defined above, and $R_{xB}$ and $R_{yB}$ are amino-protecting groups, preferably independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, or ethoxycarbonyl.

One aspect of the present invention provides a compound of formula IC, a stereoisomer thereof, or a pharmaceutically acceptable salt thereof:

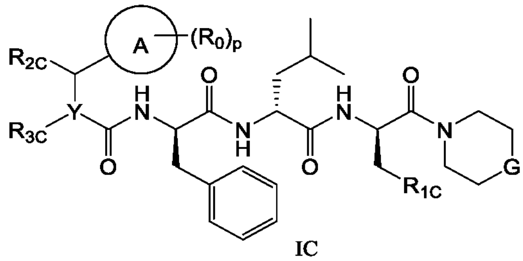

**IC**

wherein,

ring A is $C_{3-8}$ cycloalkyl, $C_{6-14}$ aryl, or 5- to 14-membered heteroaryl, preferably phenyl;
Y is selected from CH or N;

G is selected from -S-, -O-, $-CR_{4C}R_{5C}-$, $-NR_{6C}-$, $-S(=O)_2-$, $-S(=O)(=NR_{6C}')-$, and

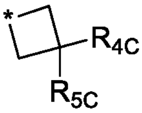

,

preferably -O-, - $CR_{4C}R_{5C}-$, $-NR_{6C}-$, $-S(=O)_2-$, $-S(=O)(=NR_{6C}')-$, and

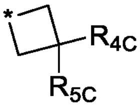

;

$R_{1C}$ is selected from H or $-(CH_2)_tNR_{aC}R_{bC}$;

$R_{2C}$ is selected from H, amino, hydroxyl, $C_{1-6}$ alkyl, $C_{1-6}$ alkylamino, and $C_{1-6}$ aminoalkyl, wherein the alkyl is optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy;

when Y is CH, $R_{3C}$ is selected from H, hydroxyl, $C_{1-6}$ alkyl, 3- to 8-membered heterocyclyl, and $C_{1-6}$ alkoxy, and when Y is N, $R_{3C}$ is selected from H, $C_{1-6}$ alkyl, $C_{3-8}$ cycloalkyl, $C_{3-8}$ cycloalkyl-$(CH_2)_{mC}-$, 3- to 8-membered heterocyclyl, 3- to 8-membered heterocyclyl-$(CH_2)_{mC}-$, and $-(CH_2)_{mC}NR_{10}R_{11}$, wherein the alkyl, cycloalkyl, heterocyclyl, and alkoxy are optionally substituted with one or more groups selected from halogen, hydroxyl, amino, nitro, cyano, $C_{1-6}$ alkyl, $C_{1-6}$ haloalkyl, $NH_2C(=O)-$, $C_{1-6}$ alkoxy, and $C_{1-6}$ alkylamino;

$R_{4C}$ and $R_{5C}$ are each independently selected from H, $C_{1-6}$ alkyl, $C_{1-6}$ alkyl-O-, hydroxyl, $-C(O)OR_7$, $-NR_8R_9$, $-NR_{cC}C(O)NR_8R_9$, $C_{1-6}$ alkylamino, 3- to 8-membered heterocyclyl-$(CH_2)_{mC}-$, halogen, cyano, - $NR_{cC}S(=O)_2NR_8R_9$, $-NR_{cC}C(O)OR_{dC}$, $-NR_{cC}S(=O)_2OR_{dC}$, $-NR_{cC}C(O)R_7'$, and $-NH(CH_2)_{mC}NR_8R_9$, wherein the alkyl and heterocyclyl are optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy;

or $CR_{4C}R_{5C}$ forms a 3- to 8-membered heterocyclic or 9- to 10-membered bicyclic moiety, wherein the 3- to 8-membered heterocyclic or 9- to 10-membered bicyclic moiety, together with the piperidine ring attached thereto, forms a structure selected from:

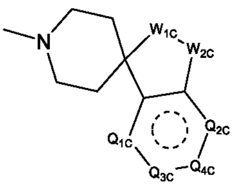

,

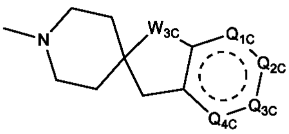

,

and

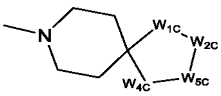

,

when any one of $Q_{1C}$-$Q_{4C}$ is N, the rest are C, or $Q_{1C}$-$Q_{4C}$ are all C;

$W_{1C}$ and $W_{2C}$ are each independently -C(=O)-NH-, -NH-C(=O)-, $-S(=O)_2-NH-$, $-NH-S(=O)_2-$, -S-, -O-, - $NR_{12}-$, $-NR_{12}-CH_2-$, $-(CH_2)_{n2C}-$ optionally substituted with one or more groups selected from $-NH_2$, -OH, halogen, nitro, cyano, $C_{1-6}$ haloalkyl, $NH_2C(=O)-$, and $C_{1-6}$ alkoxy, or absent, $R_{12}$ is selected from H, $C_{1-6}$ alkyl, amidino, and HOOC-$(CH_2)_{n3C}-$; preferably, $W_{1C}$ and $W_{2C}$ cannot be simultaneously absent;

$W_{3C}$ is $-(CH_2)_{n2C}-$ optionally substituted with one or more groups selected from $NH_2$, -OH, halogen, nitro, cyano, $C_{1-6}$ haloalkyl, $NH_2C(=O)-$, and $C_{1-6}$ alkoxy, or absent; preferably $-(CH_2)_{n2C}-$ optionally substituted with one or more groups selected from $NH_2$, -OH, halogen, nitro, cyano, $C_{1-6}$ haloalkyl, $NH_2C(=O)-$, and $C_{1-6}$ alkoxy;

$W_{4C}$ and $W_{5C}$ are $-(CH_2)_{n2C}-$ optionally substituted with one or more groups selected from $NH_2$, -OH, halogen, nitro, cyano, $C_{1-6}$ haloalkyl, $NH_2C(=O)-$, and $C_{1-6}$ alkoxy, or absent;

n2C is selected from 0, 1, 2, and 3;

more preferably, the 3- to 8-membered heterocyclic or 9- to 10-membered bicyclic moiety, together with the piperidine

ring attached thereto, forms a structure selected from:

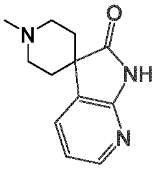 , 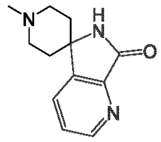 , 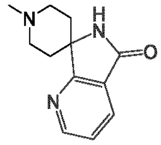 , 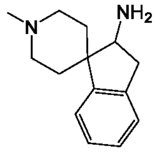 , 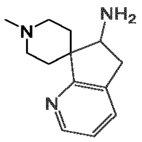 , 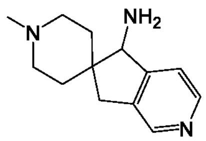 ,

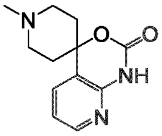 , 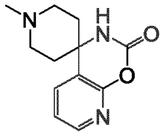 , 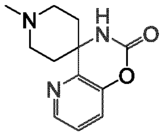 , 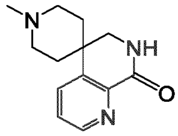 , 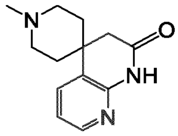 , 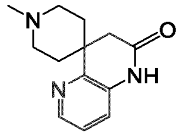 ,

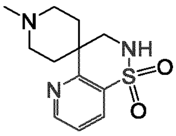 , 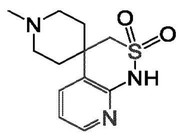 , 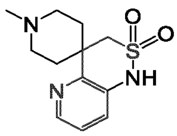 , 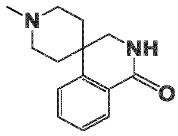 , 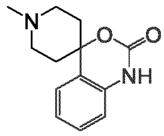 , 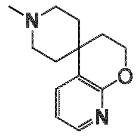 ,

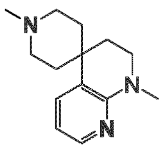 , 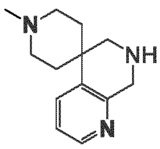 , 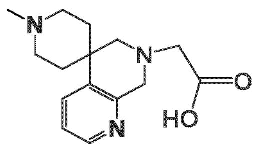 , 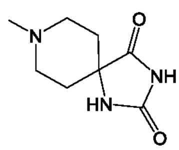 ,

and

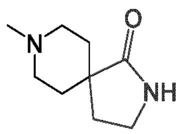 ,

more preferably from

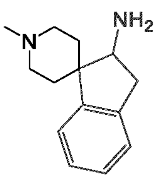 , 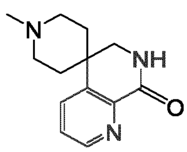 , 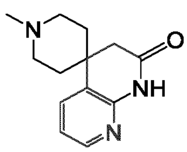 , 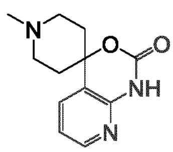 , 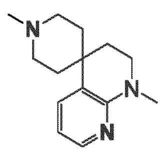 , 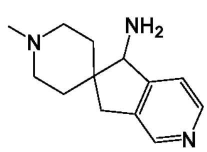 ,

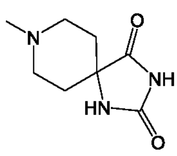 , and 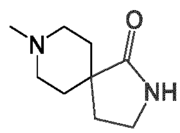 ;

$R_{aC}$ and $R_{bC}$ are each independently selected from H, $C_{1-6}$ alkyl, amidino, and $C_{1-6}$ alkoxycarbonyl;
$R_{cC}$ is H or $C_{1-6}$ alkyl substituted with one or more groups selected from amino, $C_{1-6}$ alkylamino, $C_{1-6}$ alkoxy, halogen, hydroxyl, nitro, cyano, $NH_2C(=O)$-, and $C_{1-6}$ alkoxy, preferably H or $C_{1-6}$ alkyl substituted with amino, $C_{1-6}$ alkylamino, or $C_{1-6}$ alkoxy, and more preferably H or $C_{1-6}$ alkyl substituted with amino or $C_{1-6}$ alkylamino;
$R_{dC}$ is selected from $C_{1-6}$ alkyl, $C_{6-14}$ aryl, 5- to 14-membered heteroaryl, $C_{3-8}$ cycloalkyl, and 3- to 8-membered heterocyclyl, preferably from $C_{1-6}$ alkyl and 3- to 8-membered heterocyclyl, more preferably from $C_{1-6}$ alkyl and

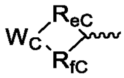

,

and even more preferably from $C_{1-6}$ alkyl and

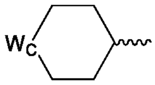

,

the group $R_{dC}$ described above is optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, wherein $R_{eC}$ and $R_{fC}$ are each independently $-(CH_2)_{n1C}-$ and - $(CH_2)_{n1C'}-$, wherein n1C and n1C' are each independently selected from 0, 1, 2, and 3, preferably 2, and n1C and n1C' are not both 0; $W_C$ is selected from -NH-C(=O)-, -NH-S(=O)$_2$-, -NR$_{12}$-, -O-, -S-, and -S(=O)$_2$-, preferably from -O- and -S(=O)$_2$-, wherein $R_{12}$ is selected from H, $C_{1-6}$ alkyl, amidino, and HOOC-$(CH_2)_{n3C}$-, wherein n3C is selected from 1, 2, and 3;

$R_{6C}$ and $R_{6C}$' are independently selected from H, $C_{1-6}$ alkyl, $C_{1-6}$ alkylcarbonyl, $C_{1-6}$ alkyl-S(=O)$_2$-, $C_{1-6}$ alkoxycarbonyl, $C_{6-14}$ aryl, $C_{6-14}$ arylcarbonyl, $C_{6-14}$ aryloxycarbonyl, $C_{3-8}$ cycloalkyl, $C_{3-8}$ cycloalkylcarbonyl, $C_{3-8}$ cycloalkoxycarbonyl, 5- to 14-membered heteroaryl, 5- to 14-membered heteroarylcarbonyl, 5- to 14-membered heteroaryloxycarbonyl, 3- to 8-membered heterocyclyl, 3- to 8-membered heterocyclylcarbonyl, and 3- to 8-membered heterocyclyloxycarbonyl, the above substituents are each optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;

$R_7$ and $R_7$' are each independently selected from H, $C_{1-6}$ alkyl, $C_{3-8}$ cycloalkyl, 3- to 8-membered heterocyclyl, $C_{6-14}$ aryl, and 5- to 14-membered heteroaryl, wherein the alkyl, cycloalkyl, heterocyclyl, aryl, and heteroaryl are optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;

$R_8$ and $R_9$ are each independently H or $C_{1-6}$ alkyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;

$R_{10}$ and $R_{11}$ are each independently H or $C_{1-6}$ alkyl, or $R_{10}$ and $R_{11}$, together with the nitrogen atom to which they are both attached form 3- to 8-membered heterocyclyl, wherein the alkyl and heterocyclyl are optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;

p and t are each independently selected from 0, 1, 2, 3, 4, or 5;

mC is independently selected from 1, 2, 3, and 4 at each occurrence;

$R_0$ is selected from H, halogen, $NO_2$, cyano, $NH_2C(=O)$-, $C_{1-6}$ alkoxy, and $C_{1-6}$ alkyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy.

Another aspect of the present invention provides a method for preparing the compound of the present invention, which is selected from the following methods:

method I:

method I comprises the following steps:

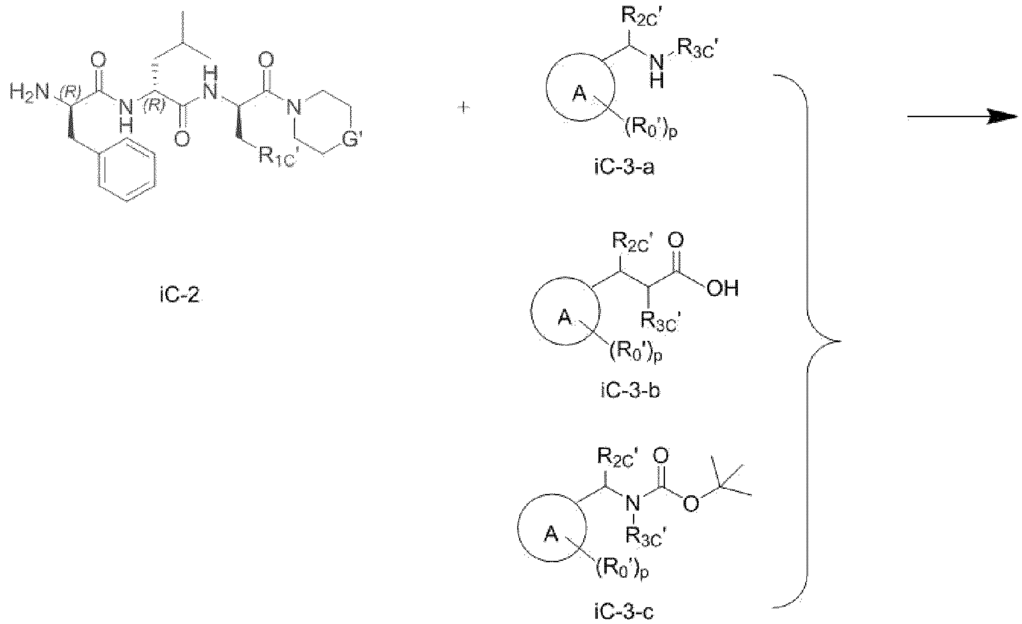

iC-2

iC-3-a

iC-3-b

iC-3-c

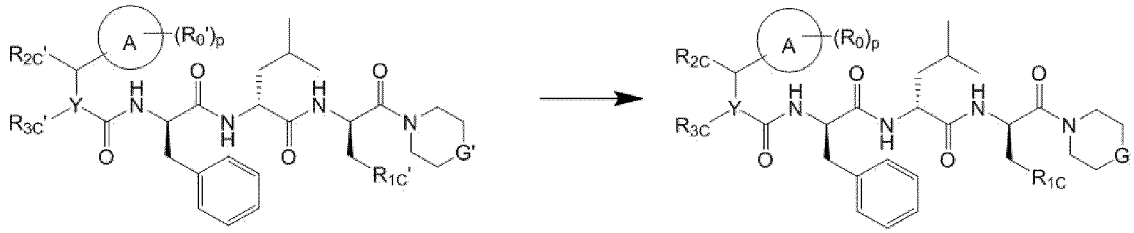

iC-1

IC

method II:

method II comprises the following steps:

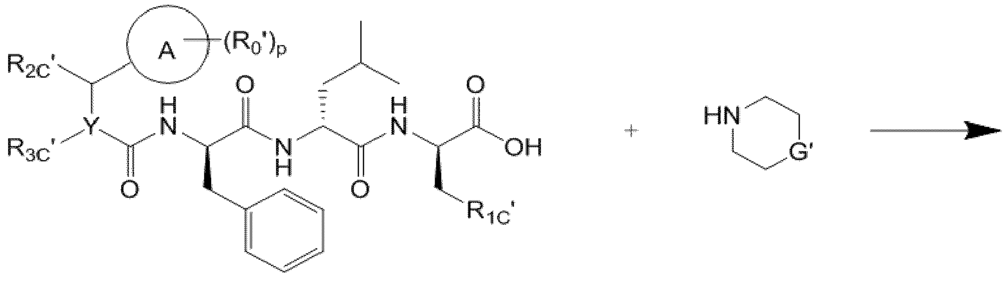

iiC-2

iiC-3

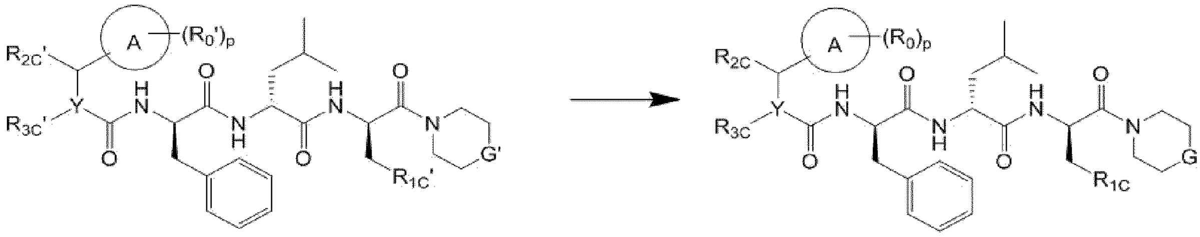

iiC-1

IC

Preferably, a compound of formula iiC-2 is prepared by a method selected from:

method III:

method III comprises the following steps: subjecting a compound of formula iiC-4-a and a compound of formula iiC-5-a to a condensation reaction, a hydrolysis reaction, an optional removal reaction of an amino-protecting group, and an amino protection reaction to give the compound of formula iiC-2,

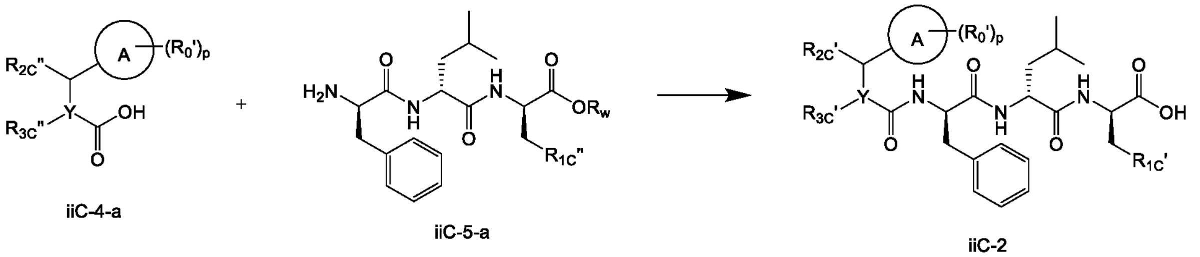

iiC-4-a iiC-5-a iiC-2

,

method IV:

method IV comprises the following steps: subjecting a compound of formula iiC-4-b and a compound of formula iiC-5-b to a condensation reaction, a hydrolysis reaction, an optional removal reaction of an amino-protecting group, and an amino protection reaction to give the compound of formula iiC-2,

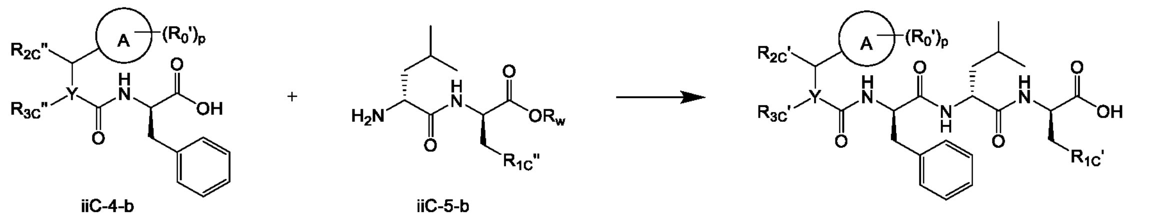

iiC-4-b iiC-5-b iiC-2

,

method V:

method V comprises the following steps: subjecting a compound of formula iiC-4-c and a compound of formula iiC-5-c to a condensation reaction, a hydrolysis reaction, an optional removal reaction of an amino-protecting group, and an amino protection reaction to give the compound of formula iiC-2,

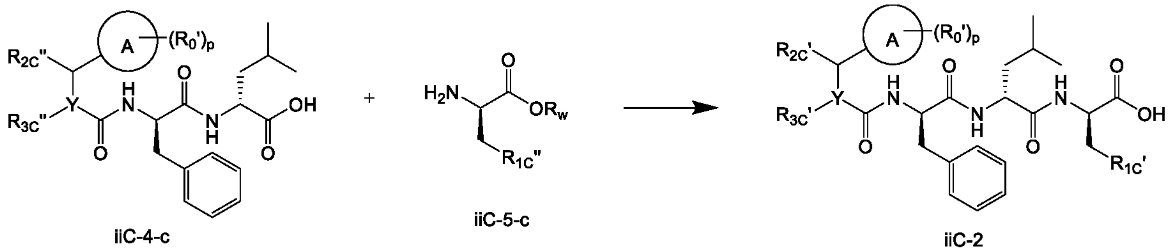

iiC-4-c iiC-5-c iiC-2

,

method VI:

method VI comprises the following steps: subjecting a compound of formula iiC-4-d and a compound of formula iiC-5-d to a condensation reaction, a hydrolysis reaction, an optional removal reaction of an amino-protecting group, and an amino protection reaction to give the compound of formula iiC-2,

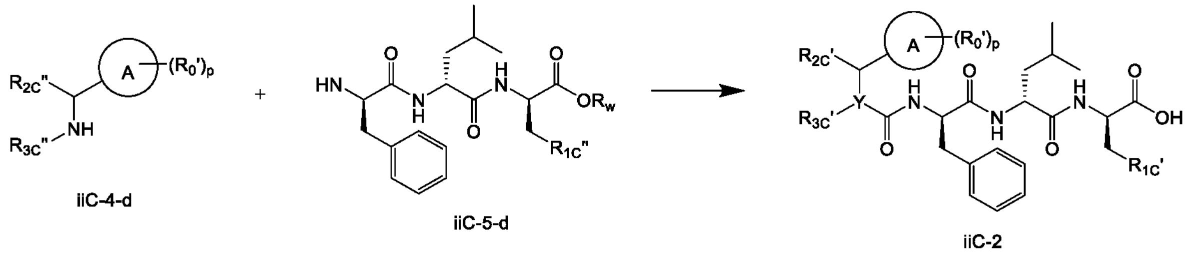

iiC-4-d iiC-5-d iiC-2

.

preferably, the formula iC-2 is

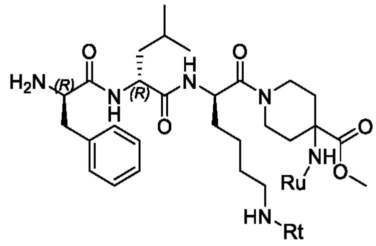

;

preferably, iiC-2 is selected from:

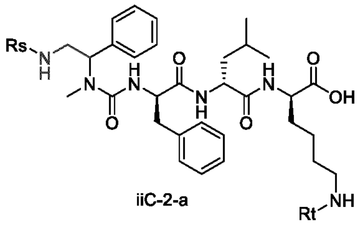

iiC-2-a ,

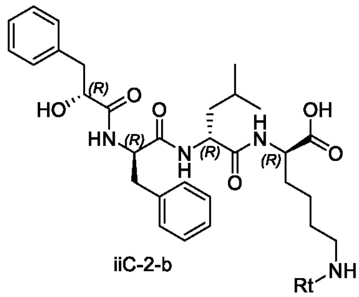

iiC-2-b ,

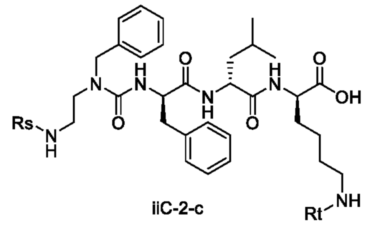

iiC-2-c ,

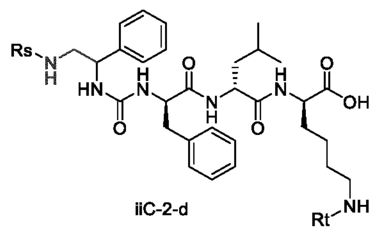

iiC-2-d ,

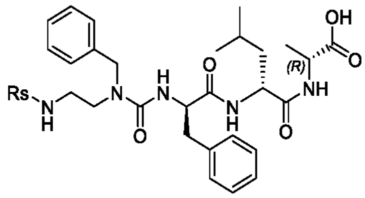

iiC-2-e , and

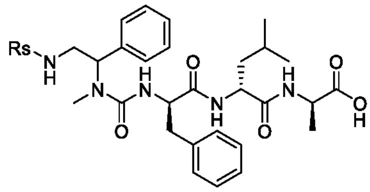

iiC-2-f ,

wherein $R_0$' is $R_0$ or $R_0$ in which $NH_2$ is protected with an amino-protecting group, $R_{1C}$' and $R_{1C}$" are $R_{1C}$ or $R_{1C}$ in which $NH_2$ is protected with an amino-protecting group, $R_{2C}$' and $R_{2C}$" are $R_{2C}$ or $R_{2C}$ in which $NH_2$ is protected with an amino-protecting group, $R_{3C}$' and $R_{3C}$" are $R_{3C}$ or $R_{3C}$ in which $NH_2$ or the cyclic imino is protected with an amino-protecting group, G' is G or G in which $NH_2$ and/or the cyclic imino is protected with an amino-protecting group and/or the carboxyl is protected with a carboxyl-protecting group, Rw is a carboxyl-protecting group, ring A, Y, $R_0$, $R_{1C}$, $R_{2C}$, $R_{3C}$, G, and p are as defined above, and Rs, Rt, and Ru are each independently selected from H and amino-protecting groups;

preferably, the amino-protecting groups are each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, *tert*-butylsulfinyl, 1-phenylethyl, or ethoxycarbonyl; the carboxyl-protecting groups are each independently selected from $C_{1-6}$ alkyl, allyl, benzyl, 2,4-dimethoxybenzyl, *p*-methoxybenzyl, methoxyethoxymethyl, pentafluorophenyl, and 4-*p*-methylbenzyloxybenzyl.

Another aspect of the present invention provides a compound of the following general formula, a stereoisomer thereof, or a salt thereof,

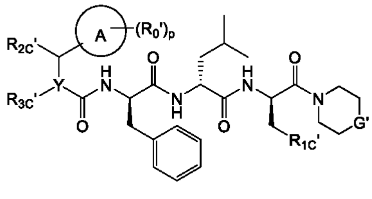

iC-1

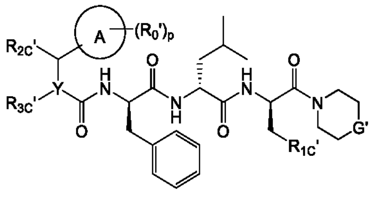

iiC-1

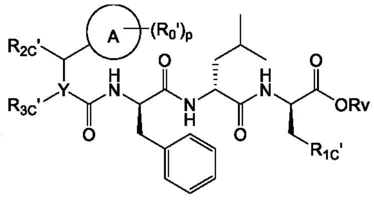

iiC-2-X

preferably, formula iiC-2-X is selected from:

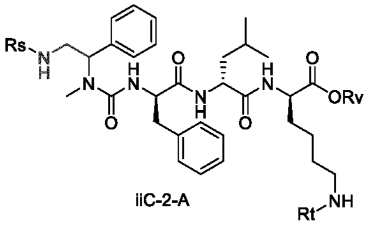
iiC-2-A ,

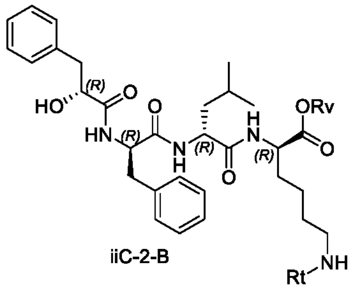
iiC-2-B ,

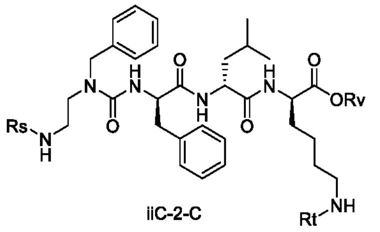
iiC-2-C ,

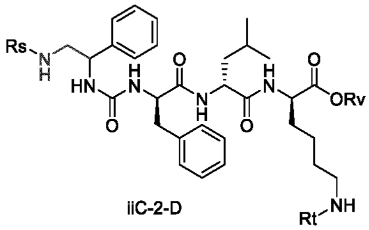
iiC-2-D ,

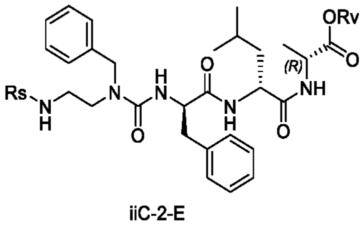
iiC-2-E , and

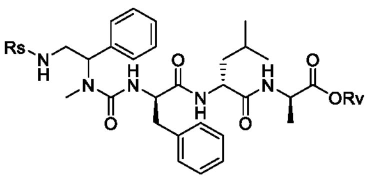
iiC-2-F ,

wherein $R_0$' is $R_0$ or $R_0$ in which $NH_2$ is protected with an amino-protecting group, $R_{1C}$' is $R_{1C}$ or $R_{1C}$ in which $NH_2$ is protected with an amino-protecting group, $R_{2C}$' is $R_{2C}$ or $R_{2C}$ in which $NH_2$ is protected with an amino-protecting group, $R_{3C}$' is $R_{3C}$ or $R_{3C}$ in which $NH_2$ or the cyclic imino is protected with an amino-protecting group, G' is G or G in which $NH_2$ and/or the cyclic imino is protected with an amino-protecting group and/or the carboxyl is protected with a carboxyl-protecting group, ring A, Y, $R_0$, $R_{1C}$, $R_{2C}$, $R_{3C}$, G, and p are as defined above, Rs and Rt are each independently selected from H and amino-protecting groups, and Rv is selected from H and carboxyl-protecting groups;

preferably, the amino-protecting groups are each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, *tert*-butylsulfinyl, 1-phenylethyl, or ethoxycarbonyl; the carboxyl-protecting groups are each independently selected from $C_{1-6}$ alkyl, allyl, benzyl, 2,4-dimethoxybenzyl, *p*-methoxybenzyl, methoxyethoxymethyl, pentafluorophenyl, and 4-*p*-methylbenzyloxybenzyl.

Another aspect of the present invention provides a pharmaceutical composition comprising a prophylactically or therapeutically effective amount of the compound of the present invention, the stereoisomer thereof, or the pharmaceutically acceptable salt thereof, and a pharmaceutically acceptable carrier or excipient, as well as optionally other therapeutic agents.

Another aspect of the present invention provides use of the compound of the present invention, the stereoisomer thereof, or the pharmaceutically acceptable salt thereof, or the pharmaceutical composition, in the preparation of a medicament for agonizing κ-opioid receptors.

Another aspect of the present invention provides a method for preventing and/or treating a related disease mediated by κ-opioid receptor, comprising administering an effective amount of the compound of the present invention, the stereoisomer thereof, or the pharmaceutically acceptable salt thereof, or the pharmaceutical composition.

Another aspect of the present invention provides the use of the compound of the present invention, the stereoisomer thereof, or the pharmaceutically acceptable salt thereof, or the pharmaceutical composition, in the preparation of a medicament, particularly, the medicament is used for preventing and/or treating a related disease mediated by κ-opioid receptor.

The related disease mediated by the κ-opioid receptor in the present disclosure is selected from pain, inflammation, pruritus, edema, hyponatremia, hypokalemia, ileus, cough, and glaucoma, preferably pain. The pain disclosed herein is selected from neuropathic pain, trunk pain, visceral pain, skin pain, arthritis pain, kidney stone pain, uterine cramp, dysmenorrhea, endometriosis, dyspepsia, post-surgical pain, post-medical treatment pain, ocular pain, otitis pain, breakthrough cancer pain, and pain associated with a GI disorder.

## DETAILED DESCRIPTION

Terminology

Unless otherwise defined, all technical and scientific terms used herein have the same meaning as commonly understood by those skilled in the art to which the claimed subject matter belongs.

Unless otherwise stated, the present invention employs conventional methods of mass spectrometry, NMR, HPLC, protein chemistry, biochemistry, recombinant DNA techniques, pharmacology, etc. within the skill of the art. Unless specific definitions are provided, nomenclature and laboratory operations and techniques related to the chemistry, such as analytical chemistry, synthetic organic chemistry, and medicinal and pharmaceutical chemistry, described herein are known to those skilled in the art. In general, the foregoing techniques and procedures can be performed by conventional methods that are well known in the art and described in various general and more specific documents, and these documents are cited and discussed in the present specification.

The term "alkyl" refers to an aliphatic hydrocarbon group, which may be branched or linear alkyl. According to the structure, the alkyl may be a monovalent group or a divalent group (i.e., alkylene). In the present invention, the alkyl is preferably alkyl having 1 to 8 carbon atoms, more preferably "lower alkyl" having 1 to 6 carbon atoms, and even more preferably alkyl having 1 to 4 carbon atoms. Typical alkyl groups include, but are not limited to, methyl, ethyl, propyl, butyl, pentyl, hexyl, and the like. It should be understood that the "alkyl" mentioned herein includes all possible configurations and conformations of the alkyl. For example, the "propyl" mentioned herein includes *n*-propyl and isopropyl, "butyl" includes *n*-butyl, isobutyl, and *tert*-butyl, "pentyl" includes *n*-pentyl, isopropyl, neopentyl, *tert*-pentyl, and pent-3-yl, and the like. In some embodiments, the alkyl is optionally substituted with one or more (such as 1 to 3) suitable substituents.

The term "aryl" refers to an all-carbon monocyclic or polycyclic aromatic group having a conjugated $\pi$-electron system. For example, the term "$C_{6-14}$ aryl" as used herein refers to an aromatic group containing 6 to 14 carbon atoms. In some embodiments, the $C_{6-14}$ aryl is preferably $C_{6-10}$ aryl. Examples of aryl groups may include phenyl, naphthyl, anthryl, and the like. In some embodiments, the aryl is optionally substituted with one or more (such as 1 to 3) suitable substituents.

The term "heteroaryl" refers to a monocyclic or polycyclic aromatic group containing one or more identical or different heteroatoms, such as oxygen, nitrogen, or sulfur. For example, the term "5- to 14-membered heteroaryl" as used herein refers to heteroaryl having 5 to 14 ring atoms. Further, the heteroaryl refers to a monocyclic, bicyclic, or tricyclic aromatic group, which has 5, 6, 8, 9, 10, 11, 12, 13, or 14 ring atoms, in particular 1 or 2 or 3 or 4 or 5 or 6 or 9 or 10 carbon atoms, and contains at least one heteroatom that may be identical or different, such as oxygen, nitrogen, or sulfur. In addition, the heteroaryl may be a benzo-fused group. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, etc., and benzo derivatives thereof; or pyridinyl, pyridazinyl, pyrimidinyl, pyrazinyl, triazinyl, etc., and benzo derivatives thereof. In some embodiments, the heteroaryl is optionally substituted with one or more (such as 1 to 3) suitable substituents.

The term "cycloalkyl" refers to a saturated or unsaturated non-aromatic monocyclic or polycyclic (such as bicyclic) hydrocarbon ring group (e.g., monocyclic rings such as cyclopropyl, cyclobutyl, cyclopentyl, cyclohexyl, cycloheptyl, cyclooctyl, and cyclononyl, or bicyclic rings, including spiro, fused, or bridged systems, such as bicyclo [1.1.1]pentyl, bicyclo[2.2.1]heptyl, bicyclo[3.2.1]octyl, or bicyclo[5.2.0]nonyl, decahydronaphthyl, etc.). Non-limiting examples of cycloalkyl include, but are not limited to, cyclopropyl, cyclobutyl, cyclopentyl, cyclohexyl, etc. In some embodiments, the cycloalkyl is optionally substituted with one or more (such as 1 to 3) suitable substituents.

The term "heterocyclyl" refers to a saturated or unsaturated monocyclic or polycyclic group, which has, for example, 3 to 12 ring atoms or 3 to 8 ring atoms in the ring, the ring atoms including carbon atoms and one or more (e.g., one, two, three, or four) heteroatoms selected from nitrogen, oxygen, and sulfur; the heterocyclyl may be linked to the rest of a molecule via any one of the carbon atoms or the nitrogen atom (if present). The carbon atom in the heterocyclyl may be optionally substituted with oxo (=O). The sulfur atom of the ring may be optionally oxidized to an S-oxide, such as SO or $SO_2$. The nitrogen atom of the ring may be optionally oxidized to an N-oxide. For example, 3- to 8-membered heterocyclyl is a group having 3 to 8 ring atoms including carbon atoms and heteroatoms, including, but not limited to, oxiranyl, aziridinyl, azetidinyl, oxetanyl, thietanyl, tetrahydrofuranyl, dioxolyl, pyrrolidinyl, pyrrolidonyl, imidazolidinyl, pyrazolidinyl, pyrrolinyl, tetrahydropyranyl, piperidinyl, morpholinyl, thiomorpholinyl, piperazinyl, pyrimidindionyl, 2-oxopyrrolidinyl, 3,5-dioxopiperidinyl, sulfolanyl, 1,1-dioxothiomorpholinyl, and 1,1-dioxotetrahydrothiopyranyl. In some embodiments, the heterocyclyl is optionally substituted with one or more (such as 1 to 3) suitable substituents. In some embodiments, the 3- to 8-membered heterocyclyl is 3- to 8-membered heterocyclyl containing one heteroatom selected from oxygen, nitrogen, and sulfur; in some embodiments, the 3- to 8-membered heterocyclyl is

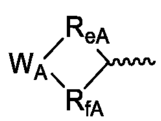

or

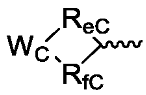

,

wherein $W_A$, $R_{eA}$, $R_{fA}$, $W_C$, $R_{eC}$, and $R_{fC}$ are as defined in the present specification.

The term "alkoxy" refers to alkyl-O-, wherein the alkyl is as defined herein. Typical alkoxy groups include, but are not limited to, methoxy, ethoxy, propoxy, butoxy, pentyloxy, hexyloxy, and the like.

The term "aryloxy" refers to aryl-O-, wherein the aryl is as defined herein.

The term "heteroaryloxy" refers to heteroaryl-O-, wherein the heteroaryl is as defined herein.

The term "cycloalkoxy" refers to cycloalkyl-O-, wherein the cycloalkyl is as defined herein.

The term "heterocyclyloxy" refers to heterocyclyl-O-, wherein the heterocyclyl is as defined herein.

The term "carbonyl" refers to an organic functional group, -C(=O)-, which is formed by linking two atoms, carbon and oxygen, via a double bond. The term "alkoxycarbonyl" refers to alkoxy-C(=O)-, i.e., the group is linked to the rest of a compound via carbonyl. The term "alkylcarbonyl" refers to alkyl-C(=O)-, i.e., the group is linked to the rest of a compound via carbonyl. Similarly, the term "arylcarbonyl" refers to aryl-C(=O)-, i.e., the group is linked to the rest of a compound via carbonyl; the term "aryloxycarbonyl" refers to aryloxy-C(=O)-, i.e., the group is linked to the rest of a compound via carbonyl; and so on.

The term "cyano" refers to a group, -CN, where a carbon atom and a nitrogen atom are linked via a triple bond.

The term "amidino" refers to

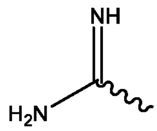

.

The term "halo-" or "halogen" refers to fluorine, chlorine, bromine, and iodine.

The term "haloalkyl" refers to alkyl in which at least one hydrogen is replaced by a halogen atom. In certain embodiments, if two or more hydrogen atoms are replaced by halogen atoms, the halogen atoms are identical or different from each other. Examples of the haloalkyl include, but are not limited to, monofluoromethyl, difluoromethyl, trifluoromethyl, monofluoroethyl, difluoroethyl, trifluoroethyl, and the like.

The term "aminoalkyl" refers to alkyl substituted with amino.

The term "alkylamino" refers to amino substituted with alkyl.

The expression "$C_1$-$C_6$" or "$C_{1-6}$" encompasses a range of 1 to 6 carbon atoms and should be understood as also encompassing any sub-range therein as well as each point value, including, for example, $C_1$-$C_6$, $C_2$-$C_6$, $C_3$-$C_6$, $C_4$-$C_6$, $C_5$-$C_6$, $C_1$-$C_5$, $C_2$-$C_5$, $C_3$-$C_5$, $C_4$-$C_5$, $C_1$-$C_4$, $C_2$-$C_4$, $C_3$-$C_4$, $C_1$-$C_3$, $C_2$-$C_3$, $C_1$-$C_2$, etc., as well as $C_1$, $C_2$, $C_3$, $C_4$, $C_5$, $C_6$, etc. Similarly, the expression "$C_3$-$C_8$" or "$C_{3-8}$" encompasses a range of 3 to 8 carbon atoms and should be understood as also encompassing any sub-range therein as well as each point value, including, for example, $C_3$-$C_6$, etc., as well as $C_3$, $C_4$, $C_5$, $C_6$, $C_7$, $C_8$, etc. The expression "3- to 8-membered" should be understood as encompassing any sub-range therein as well as each point value, for example, 3- to 7-membered, 3- to 6-membered, 3- to 5-membered, and 4- to 8-membered, as well as 3-membered, 4-membered, 5-membered, 6-membered, 7-membered, 8-membered, etc. Similarly, the expression "5- to 14-membered" should be understood as encompassing any sub-range therein as well as each point value, for example, 5-membered, 6-membered, 7-membered, 8-membered, 9-membered, 10-membered, 11-membered, 12-membered, 13-membered, 14-membered, etc. Other similar expressions herein should also be understood in a similar manner.

The term "optionally substituted" or "optionally substituted with..." means that the group mentioned may or may not be substituted.

The term "optionally substituted" or "substituted" means that the group mentioned may be substituted with one or more additional groups, and the additional groups are each independently selected from, for example, alkyl, alkenyl, alkynyl, haloalkyl, alkoxy, alkylthio, halogen, sulfhydryl, hydroxyl, nitro, amino, cyano, carboxyl, oxo, cycloalkyl, heterocyclyl, aryl, heteroaryl, $NH_2C(=O)$-, alkylamino, and the like.

The expression "one or more" in "substituted with one or more groups selected from..." or "optionally substituted with one or more groups selected from..." refers to 1 to 5, or 1 to 4, or 1 to 3, or 1 to 2 group substitutions, provided that the valence requirements are met.

In addition, it should be understood that the terms "$C_{6-14}$ arylcarbonyl" and "$C_{6-14}$ aryloxycarbonyl" refer to a group in which the aryl is $C_{6-14}$ aryl; the terms "$C_{3-8}$ cycloalkylcarbonyl" and "$C_{3-8}$ cycloalkyloxycarbonyl" refer to a group in which the cycloalkyl is $C_{3-8}$ cycloalkyl; the terms "5- to 14-membered heteroarylcarbonyl" and "5- to 14-membered

heteroaryloxycarbonyl" refer to a group in which the heteroaryl is 5- to 14-membered heteroaryl; the terms "3- to 8-membered heterocyclylcarbonyl" and "3- to 8-membered heterocyclyloxycarbonyl" refer to a group in which the heterocyclyl is 3- to 8-membered heterocyclyl; and the term "$C_{1-6}$ alkoxycarbonyl" refers to a group in which the alkyl is $C_{1-6}$ alkyl. Similarly, "$C_{1-6}$ aminoalkyl" refers to a group in which the alkyl is $C_{1-6}$ alkyl.

The term "pharmaceutically acceptable carrier" refers to those substances that do not have a significant irritating effect on organisms and do not impair the biological activity and properties of the active compound. The "pharmaceutically acceptable carrier" includes, but is not limited to, a glidant, a sweetener, a diluent, a preservative, a dye colorant, a flavoring agent, a surfactant, a wetting agent, a dispersant, a disintegrant, a stabilizer, a solvent, or an emulsifier.

The names of the compounds of the present disclosure, "compound *-0" and "compound *-100" (wherein * is the compound number), are stereoisomers of "compound *", respectively. For example, compound 3 is a racemate, and compounds 3-0 and 3-100 are stereoisomers thereof, respectively.

"*" in the present invention refers to a linking site. For example, when G of the present disclosure is

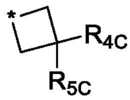
,

it indicates that

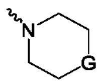

is

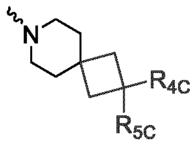
.

In addition, it should be understood that the expression "G is selected from -$CR_{4C}R_{5C}$-" means that the C atom is a ring atom, i.e.,

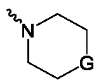

is

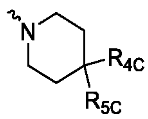
;

similarly, "G is selected from -S(=O)(=$NR_{6C}$')-" means that the S atom is a ring atom, i.e.,

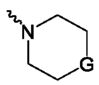

is

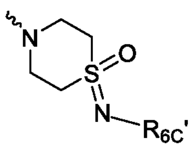
.

It should also be understood that the structure

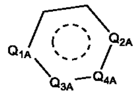

or

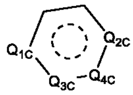

mentioned in the present disclosure refers to an aromatic ring, for example, when any one of $Q_{1A}/Q_{1C}$-$Q_{4A}/Q_{4C}$ is N and the rest are C, the structure is a pyridine ring, and when $Q_{1A}/Q_{1C}$-$Q_{4A}/Q_{4C}$ are all C, the structure is a benzene ring. It should also be understood that the expression "the rest are C" in the description described above means that the ring atoms are C, and in practice, means that "the rest are CH" according to valence bond theory.

The compounds of the present invention or salts thereof may contain one or more stereogenic centers, and each stereogenic center is independently present in an R or S configuration, and thus, enantiomers, diastereomers, and other stereoisomeric forms may be generated. These forms may be defined, in terms of absolute stereochemistry, as (R)- or (S)-, or as (D)- or (L)- for amino acids. The present invention is intended to encompass all such possible isomers or mixtures thereof, e.g., substantially pure enantiomers, diastereomers, racemates, or mixtures thereof. In certain embodiments, preferred compounds are those isomer compounds that exhibit superior biological activity. Purified or partially purified isomers and stereoisomers, or racemic or diastereomeric mixtures of the compounds of the present invention are also encompassed within the scope of the present invention. Purification and separation of such materials may be accomplished by standard techniques known in the art.

In some embodiments, the compounds of the present disclosure are racemic. In some embodiments, the compounds of the present disclosure are single enantiomers. In some embodiments, the compounds of the present disclosure are substantially free of other isomers. In some embodiments, the compounds of the present disclosure are single isomers substantially free of other isomers. In some embodiments, the compounds of the present disclosure comprise 25% or less of other isomers, or comprise 20% or less of other isomers, or comprise 15% or less of other isomers, or comprise 10% or less of other isomers, or comprise 5% or less of other isomers, or comprise 1% or less of other isomers.

In some embodiments, the compounds of the present disclosure have a stereochemical purity of at least 75%, or have a stereochemical purity of at least 80%, or have a stereochemical purity of at least 85%, or have a stereochemical purity of at least 90%, or have a stereochemical purity of at least 95%, or have a stereochemical purity of at least 96%, or have a stereochemical purity of at least 97%, or have a stereochemical purity of at least 98%, or have a stereochemical purity of at least 99%.

In some embodiments, asymmetric carbon atoms in the compounds of the present disclosure may all be present in racemic or enantiomerically enriched forms, such as an (R)-, (S)-, or (R,S)- configuration. In certain embodiments, the asymmetric carbon atom in the (S)- or (R)- configuration in the compound of the present disclosure has at least 50% enantiomeric excess, at least 60% enantiomeric excess, at least 70% enantiomeric excess, at least 80% enantiomeric excess, at least 90% enantiomeric excess, at least 95% enantiomeric excess, or at least 99% enantiomeric excess.

As used herein, "substantially pure" or "substantially free of other isomers" means that the product contains, by weight, less than 10%, preferably less than 5%, preferably less than 4%, preferably less than 3%, preferably less than 2%, or preferably less than 1% of other isomers relative to the preferred isomer. It should be understood that when the present disclosure mentions or indicates that the configuration of a compound is an absolute configuration, it means that the compound is in the substantially pure absolute configuration; similarly, when the present disclosure mentions or indicates that a compound is a single enantiomer, it means that the compound is in the substantially pure enantiomer form.

The following detailed description is intended to illustrate non-limiting embodiments and to enable others skilled in the art to more fully understand the technical schemes of the present invention, its principles, and its practical applications.

<u>Compound</u>

The present disclosure provides a compound of formula IA, a stereoisomer thereof, or a pharmaceutically acceptable salt thereof:

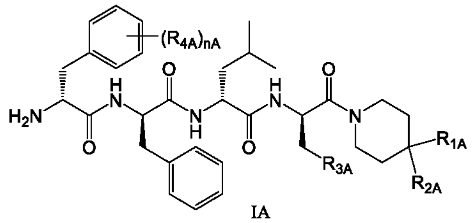

IA

In some embodiments, $R_{1A}$ is H or -COOH, preferably H; $R_{2A}$ is $-NR_{aA}C(=O)OR_{bA}$ or $-NR_{aA}S(=O)_2OR_{bA}$, preferably $-NR_{aA}C(=O)OR_{bA}$, wherein $R_{aA}$ is H or $C_{1-6}$ alkyl substituted with one or more groups selected from amino, $C_{1-6}$ alkylamino, $C_{1-6}$ alkoxy, halogen, hydroxyl, nitro, cyano, $NH_2C(=O)$-, and $C_{1-6}$ alkoxy, preferably H or $C_{1-6}$ alkyl substituted with amino, $C_{1-6}$ alkylamino, or $C_{1-6}$ alkoxy, and more preferably H or $C_{1-6}$ aminoalkyl; $R_{bA}$ is selected from $C_{1-6}$ alkyl, $C_{6-14}$ aryl, 5- to 14-membered heteroaryl, $C_{3-8}$ cycloalkyl, and 3- to 8-membered heterocyclyl (preferably

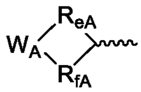

),

preferably from $C_{1-6}$ alkyl and 3- to 8-membered heterocyclyl, more preferably from $C_{1-6}$ alkyl and

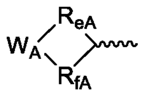

,

more preferably from $C_{1-6}$ alkyl and

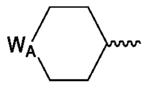

,

and even more preferably from $C_{1-6}$ alkyl and

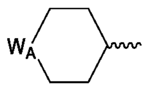

,

the group $R_{bA}$ described above is optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, wherein $R_{eA}$ and $R_{fA}$ are each independently $-(CH_2)_{n1A}$- and $-(CH_2)_{n1A'}$-, and in some embodiments, are - $(CH_2)_{n1A}$- and $-(CH_2)_{n1A'}$-, respectively, wherein n1A and n1A' are each independently selected from 0, 1, 2, and 3, preferably 2, and n1A and n1A' are not both 0; $W_A$ is selected from -NH-C(=O)-, -NH-S(=O)$_2$-, $-NR_{5A}$-, -O-, -S-, and -S(=O)$_2$-, preferably from -O- and -S(=O)$_2$-, wherein $R_{5A}$ is selected from H, $C_{1-6}$ alkyl, amidino, and HOOC-$(CH_2)_{n3A}$-, wherein n3A is selected from 1, 2, and 3. In some embodiments, $R_{aA}$ is H. In some embodiments, $R_{aA}$ is $C_{1-6}$ alkyl substituted with amino, $C_{1-6}$ alkylamino, or $C_{1-6}$ alkoxy, such as methoxyethyl, methylaminoethyl, aminopropyl, aminoethyl, or the like. In some embodiments, $R_{aA}$ is H or $C_{1-6}$ aminoalkyl. In some embodiments, $R_{bA}$ is $C_{1-6}$ alkyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, such as methyl, ethyl, or propyl, preferably methyl. In some embodiments, $R_{bA}$ is

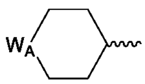

optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, wherein $W_A$ is selected from -NH-C(=O)-, -NH-S(=O)$_2$-, $-NR_{5A}$-, -O-, -S-, and -S(=O)$_2$-, preferably from -O- and -S(=O)$_2$-; $R_{5A}$ is selected from H, $C_{1-6}$ alkyl, amidino, and HOOC-$(CH_2)_{n3A}$-, wherein n3A is selected from 1, 2, and 3.

In some embodiments, $R_{1A}$ and $R_{2A}$, together with the carbon atom to which they are both attached form an

optionally substituted 9- to 10-membered bicyclic moiety (specifically a 9- to 10-membered fused ring). Preferably, the bicyclic moiety, together with the piperidine ring attached thereto, forms a structure selected from:

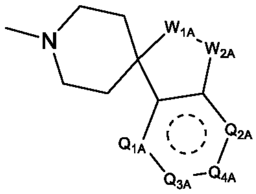

and

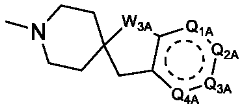
,

when any one of $Q_{1A}$-$Q_{4A}$ is N, the rest are CH, or $Q_{1A}$-$Q_{4A}$ are all CH;

$W_{1A}$ and $W_{2A}$ are each independently -C(=O)-NH-, -NH-C(=O)-, $-S(=O)_2-NH-$, $-NH-S(=O)_2-$, -S-, -O-, - $NR_{6A}$-, $-NR_{6A}-CH_2-$, $-(CH_2)_{n2A}-$ optionally substituted with one or more groups selected from $-NH_2$, -OH, halogen, nitro, cyano, $C_{1-6}$ haloalkyl, $NH_2C(=O)-$, and $C_{1-6}$ alkoxy, or absent, wherein $R_{6A}$ is selected from H, $C_{1-6}$ alkyl, amidino, and $HOOC-(CH_2)_{n3A}-$, n3A is selected from 1, 2, and 3, preferably 1; preferably, $W_{1A}$ and $W_{2A}$ cannot be simultaneously absent;

$W_{3A}$ is $-(CH_2)_{n2A}-$ optionally substituted with one or more groups selected from $NH_2$, -OH, halogen, nitro, cyano, $C_{1-6}$ haloalkyl, $NH_2C(=O)-$, and $C_{1-6}$ alkoxy, wherein n2A is selected from 0, 1, 2, and 3.

More preferably, the bicyclic moiety, together with the piperidine ring attached thereto, forms a structure selected from:

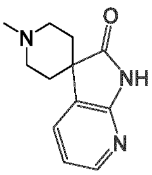
,
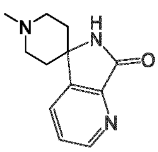
,
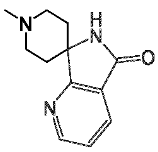
,
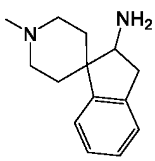
,
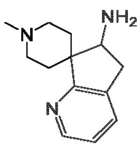
,
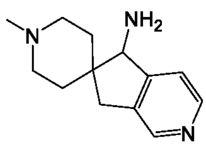
,
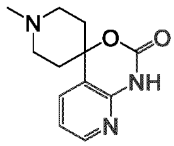
,
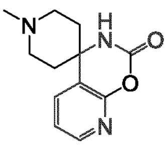
,
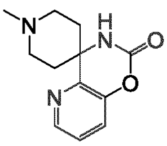
,
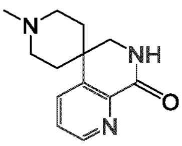
,
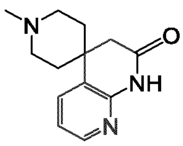
,
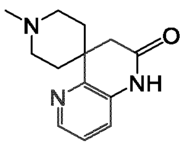
,
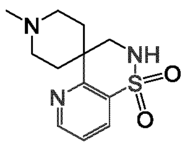
,
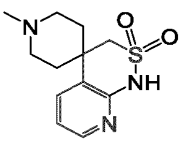
,
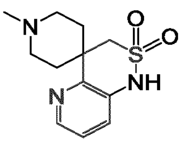
,
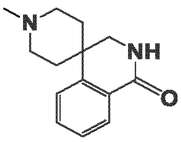
,
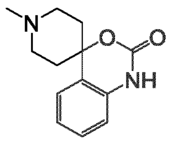
,
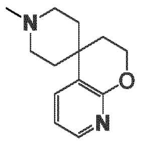
,
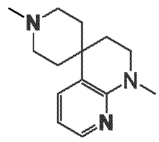
,
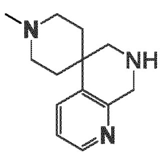
,

and

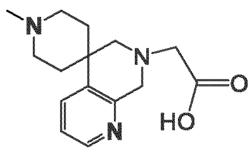,

more preferably from

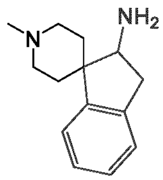, 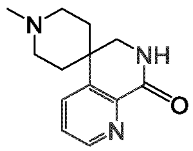,

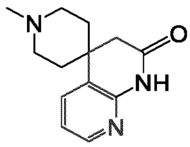, 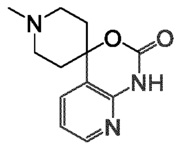, 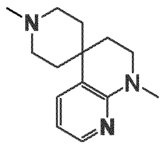,

and

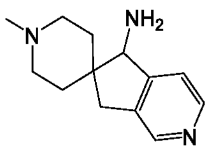,

and even more preferably from

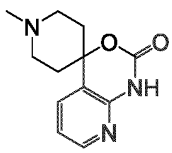

and

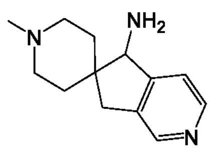.

In some embodiments, $R_{3A}$ is selected from H or $-(CH_2)_{mA}NR_{cA}R_{dA}$, wherein $R_{cA}$ and $R_{dA}$ are each independently selected from H, $C_{1-6}$ alkyl (e.g., methyl, isopropyl, etc.), amidino, and $C_{1-6}$ alkoxycarbonyl (e.g., methoxycarbonyl, etc.), and mA is selected from 0, 1, 2, 3, 4, or 5. In some embodiments, $R_{3A}$ is H. In some embodiments, $R_{cA}$ and $R_{dA}$ are each independently selected from H and $C_{1-6}$ alkoxycarbonyl (e.g., methoxycarbonyl, etc.). In some embodiments, $R_{cA}$ and $R_{dA}$ are each H. In some embodiments, $R_{cA}$ is H, and $R_{dA}$ is $C_{1-6}$ alkoxycarbonyl, preferably methoxycarbonyl. In some embodiments, $_{mA}$ is selected from 0, 1, 2, or 3, preferably 3.

In some embodiments, $R_{4A}$ is selected from halogen, $NO_2$, $C_{1-6}$ alkyl, $C_{1-6}$ haloalkyl, cyano, $NH_2C(=O)$-, and $C_{1-6}$ alkoxy, preferably from F, Cl, $NO_2$, $CH_3$, $CF_3$, cyano, and $NH_2C(=O)$-.

In some embodiments, nA is 0, 1, 2, 3, 4, or 5, preferably 0, 1, 2, or 3, and more preferably 0.

In some embodiments, formula IA is as shown in formula IIA, preferably formula IIIA, even more preferably formula IVA, and still more preferably formula VA:

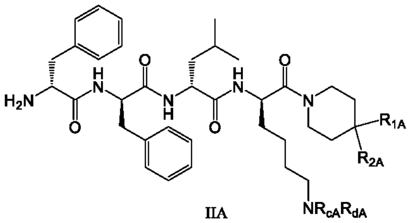

IIA

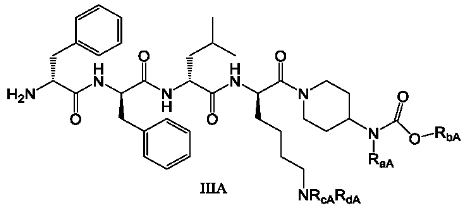

IIIA

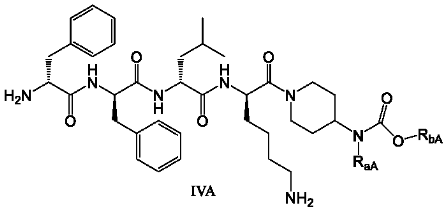

IVA

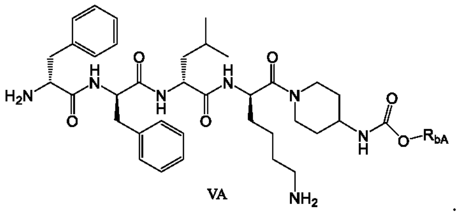

VA

.

Preferably, $R_{bA}$ in formula IA, formula IIA, formula IIIA, formula IVA, or formula VA is selected from $C_{1-6}$ alkyl and

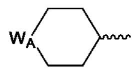

,

preferably

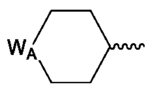

,

wherein $W_A$ is selected from -O- and $-S(=O)_2-$.

In some embodiments, formula IA is as shown in formula VIA:

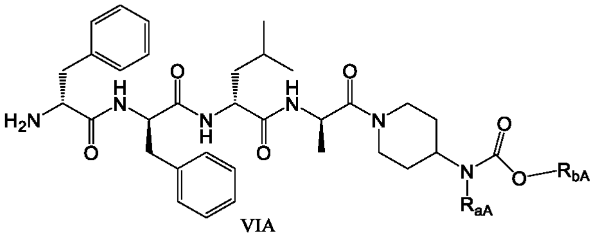

VIA

.

Preferably, $R_{aA}$ in formula VIA is H or $C_{1-6}$ alkyl substituted with amino, $C_{1-6}$ alkylamino, or $C_{1-6}$ alkoxy, more preferably H or $C_{1-6}$ aminoalkyl.

In some embodiments, the compound of the present invention is a compound of formula IIIA, a stereoisomer thereof, or a pharmaceutically acceptable salt thereof, wherein $R_{aA}$ is H; $R_{bA}$ is selected from $C_{1-6}$ alkyl,

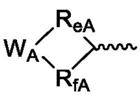

,

$R_{eA}$ and $R_{fa}$ are $-(CH_2)_{n1A}-$ and $-(CH_2)_{n1A'}-$, respectively, n1A and n1A' are each independently selected from 0, 1, 2, and 3, preferably 2, and n1A and n1A' are not both 0; $W_A$ is selected from -NH-C(=O)-, - NH-$S(=O)_2$-, $-NR_{5A}-$, -O-, -S-, and $-S(=O)_2-$, wherein $R_{5A}$ is selected from H, $C_{1-6}$ alkyl, amidino, and HOOC-$(CH_2)_{n3A}$-, wherein n3A is selected from 1, 2, and 3; $R_{cA}$ and $R_{dA}$ are each independently selected from H, $C_{1-6}$ alkyl, amidino, and $C_{1-6}$ alkoxycarbonyl.

In some embodiments, the compound of the present invention is a compound of formula IIIA, a stereoisomer thereof, or a pharmaceutically acceptable salt thereof, wherein $R_{aA}$ is H; $R_{bA}$ is selected from $C_{1-6}$ alkyl and

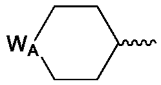

,

wherein $W_A$ is selected from -O- and -S(=O)$_2$-; $R_{cA}$ and $R_{dA}$ are each independently selected from H, $C_{1-6}$ alkyl, amidino, and $C_{1-6}$ alkoxycarbonyl.

In some embodiments, the compound of the present invention is a compound of formula IVA, a stereoisomer thereof, or a pharmaceutically acceptable salt thereof, wherein $R_{aA}$ is H; $R_{bA}$ is selected from $C_{1-6}$ alkyl and

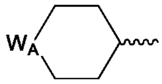

,

wherein $W_A$ is selected from -O- and -S(=O)$_2$-.

In some embodiments, the compound of the present invention is selected from compounds shown below, stereoisomers thereof, or pharmaceutically acceptable salts thereof:

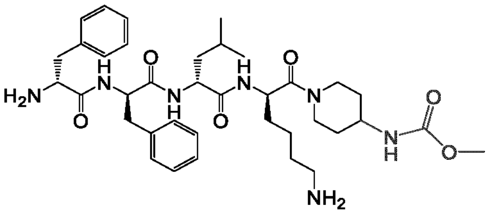

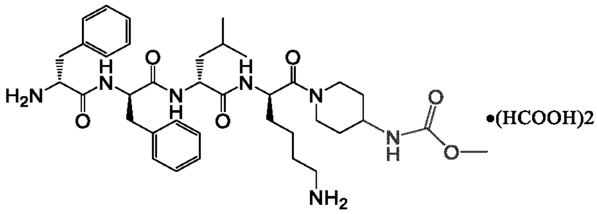

•(HCOOH)2

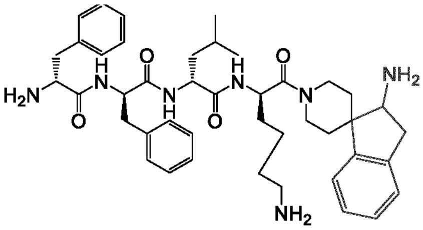

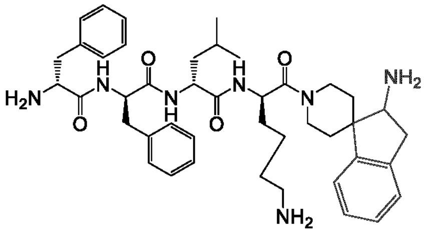

•(HCOOH)3

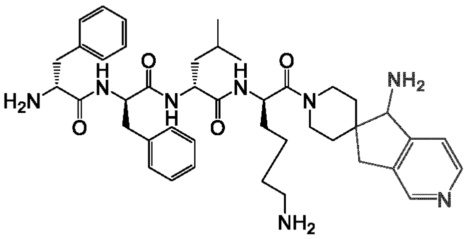

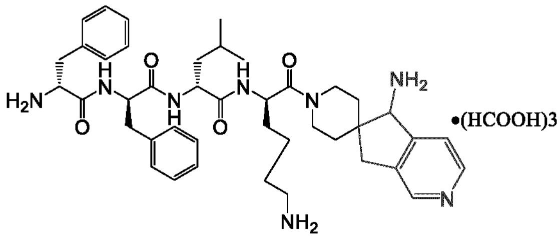

•(HCOOH)3

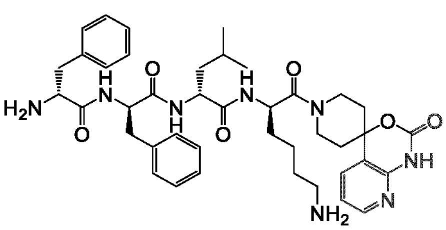

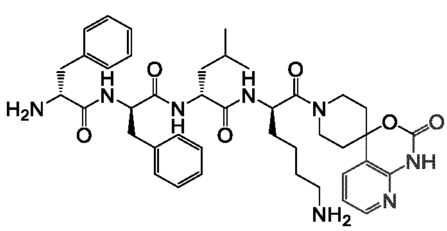

•(HCOOH)2

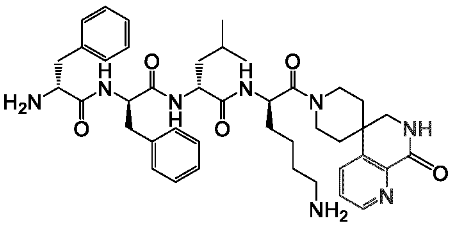

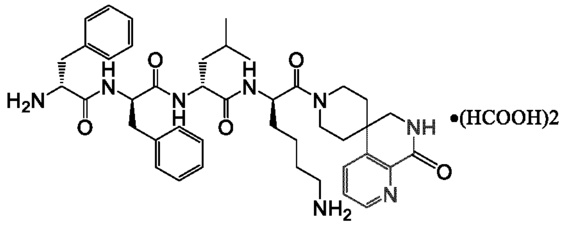

•(HCOOH)2

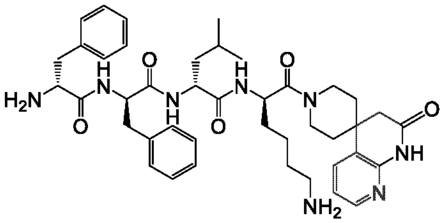

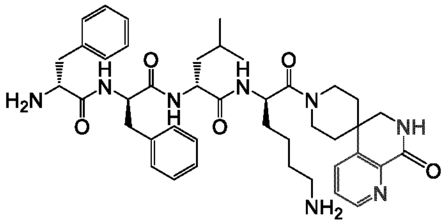

•(HCOOH)2

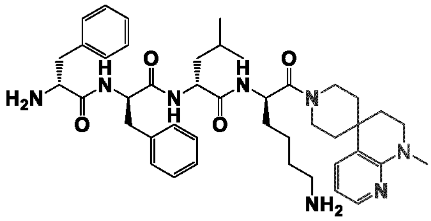

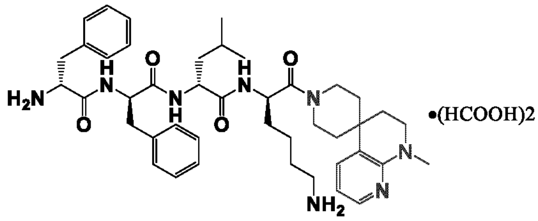

•(HCOOH)2

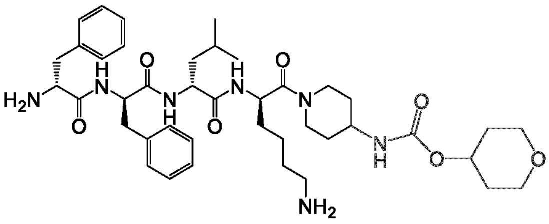

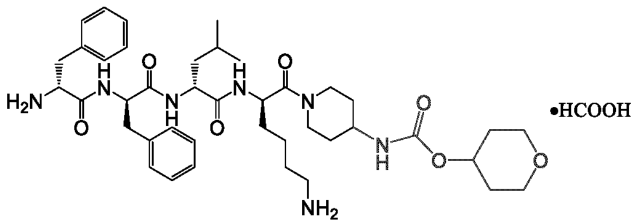

•HCOOH

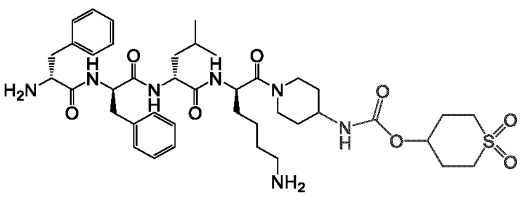

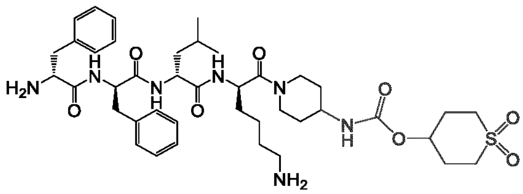

•(HCOOH)2

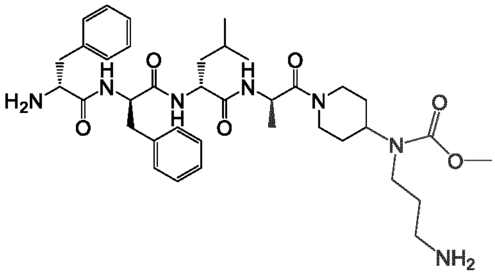

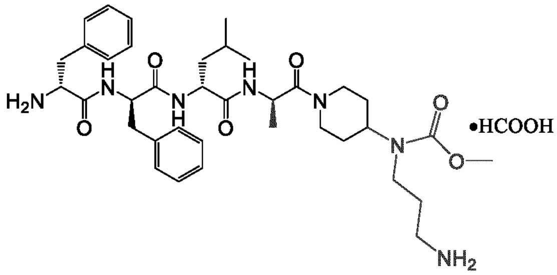

•HCOOH

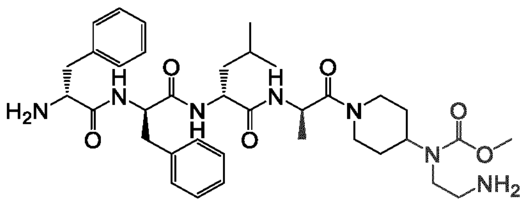

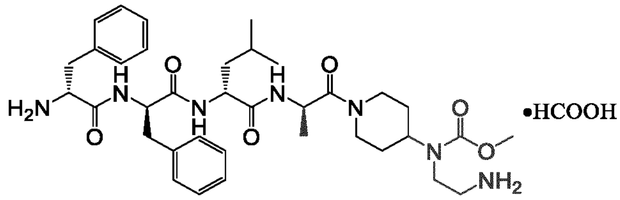

•HCOOH

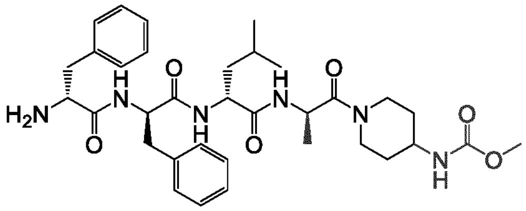

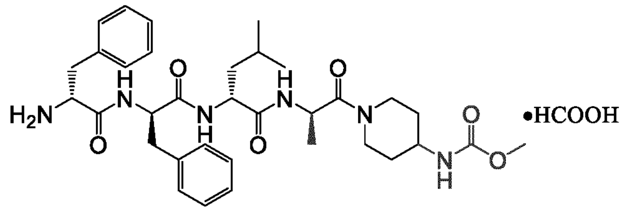

•HCOOH

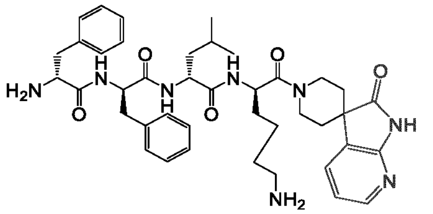

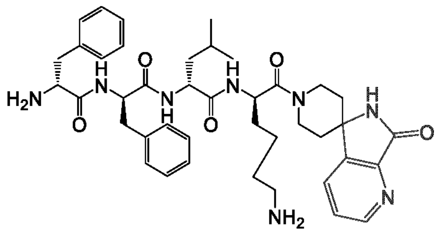

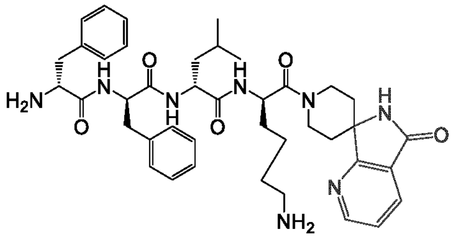

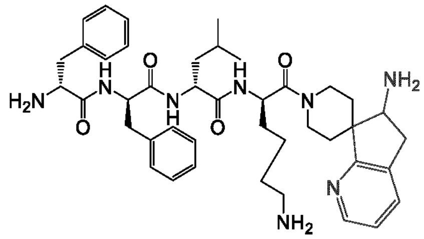

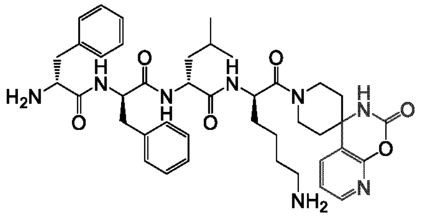

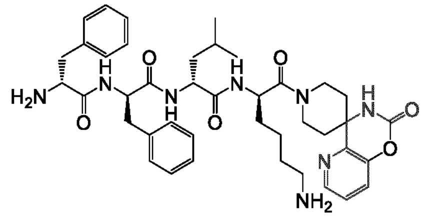

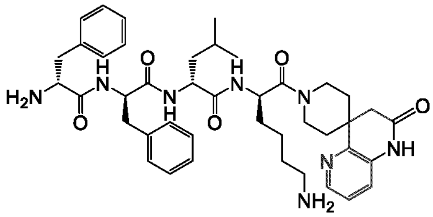

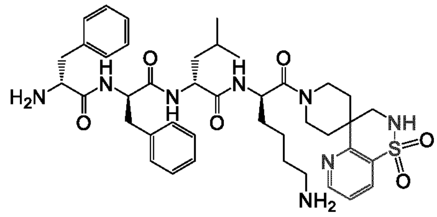

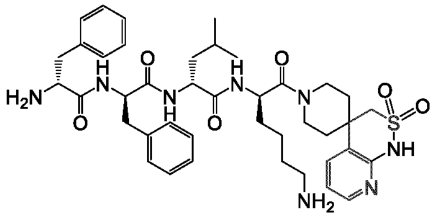

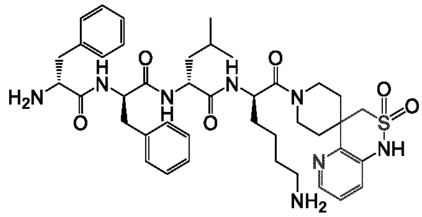

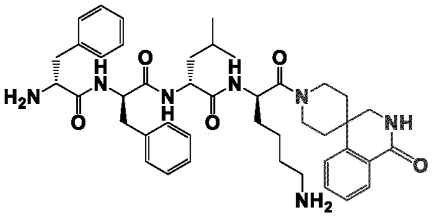

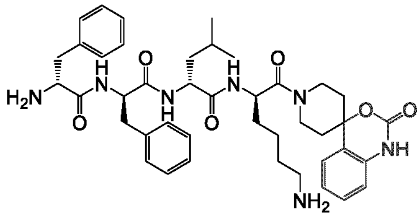

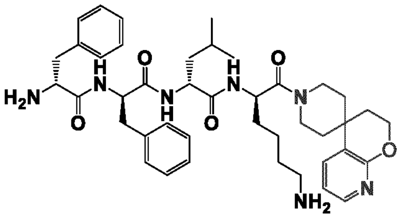

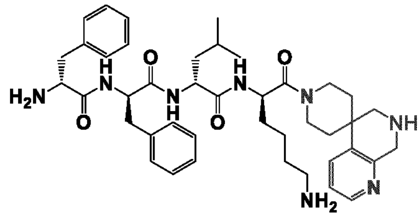

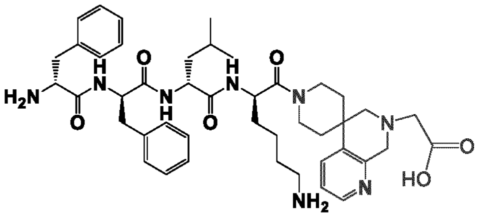

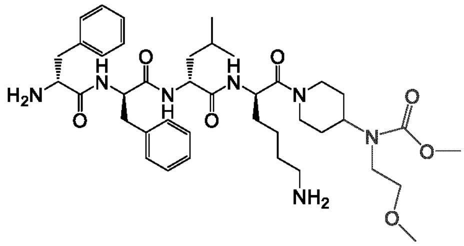

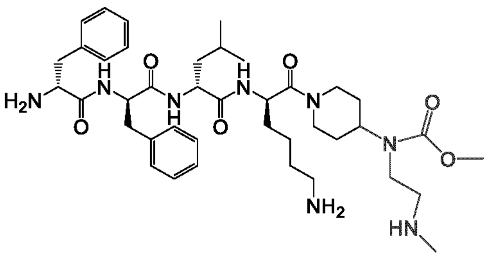

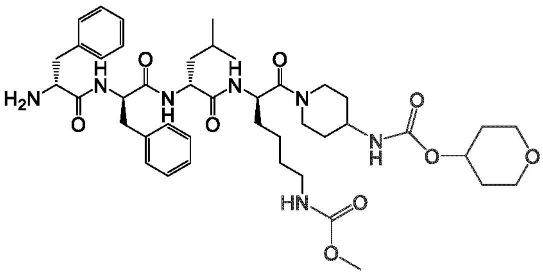

.

The present disclosure further provides a compound of the following general formula, a stereoisomer thereof, or a salt thereof, which is an intermediate for preparing the compounds of the present disclosure described above,

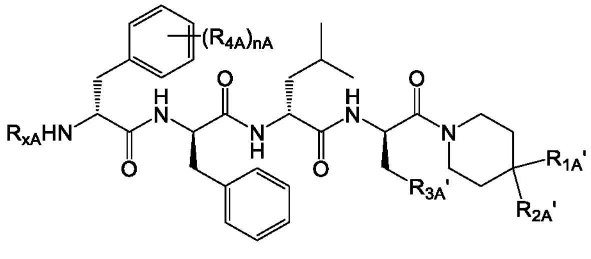

iA-1

wherein, $R_{1A}$' is $R_{1A}$ or $R_{1A}$ in which $NH_2$ is protected with an amino-protecting group, $R_{2A}$' is $R_{2A}$ or $R_{2A}$ in which $NH_2$ is protected with an amino-protecting group, $R_{3A}$' is $R_{3A}$ or $R_{3A}$ in which $NH_2$ is protected with an amino-protecting group, $R_{1A}$, $R_{2A}$, $R_{3A}$, $R_{4a}$, and nA are as defined above, and $R_{xA}$ is an amino-protecting group;

the amino-protecting groups are preferably each independently selected from *tert*-butoxycarbonyl (Boc), 9-fluorenylmethoxycarbonyl (Fmoc), allyloxycarbonyl (Alloc), trichloroethoxycarbonyl (Troc), trimethylsilylethoxycarbonyl (Teoc), benzyloxycarbonyl (CBz), *p*-toluenesulfonyl (Tosyl), *p*-nitrobenzenesulfonyl (Nosyl), *tert*-butyl (*t*-Bu), trifluoroacetyl (Tfa), methoxycarbonyl, *tert*-butylsulfinyl, or ethoxycarbonyl.

Preparation method

In some embodiments, the compound of the present invention may be prepared by a method selected from:

method I:
method I comprises the following steps:

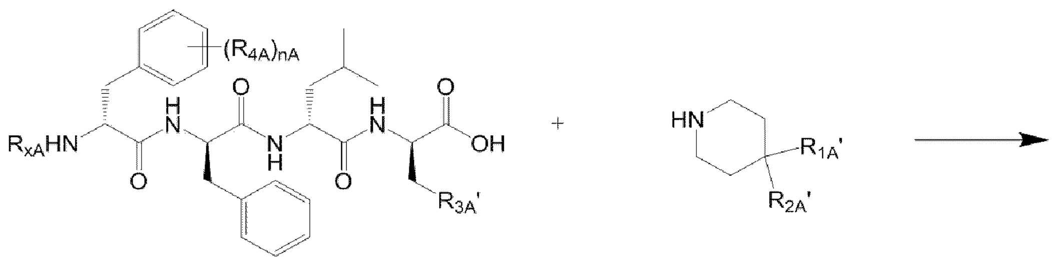

iA-2 iA-3

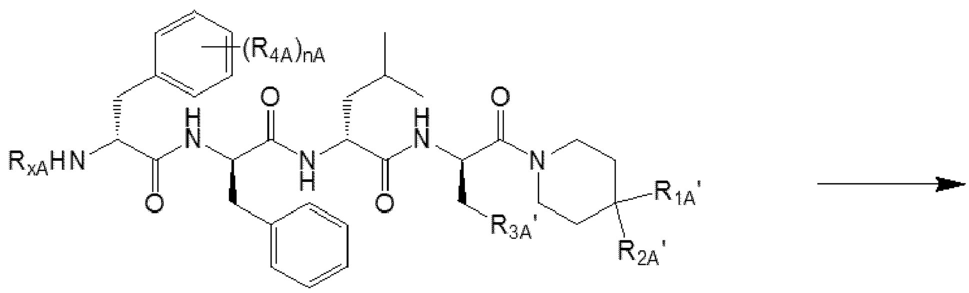

iA-1

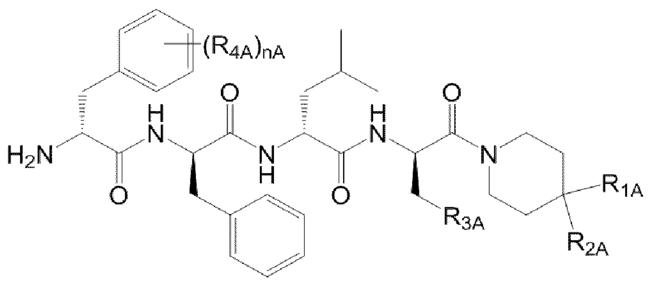

iA

method II:
method II comprises the following steps:

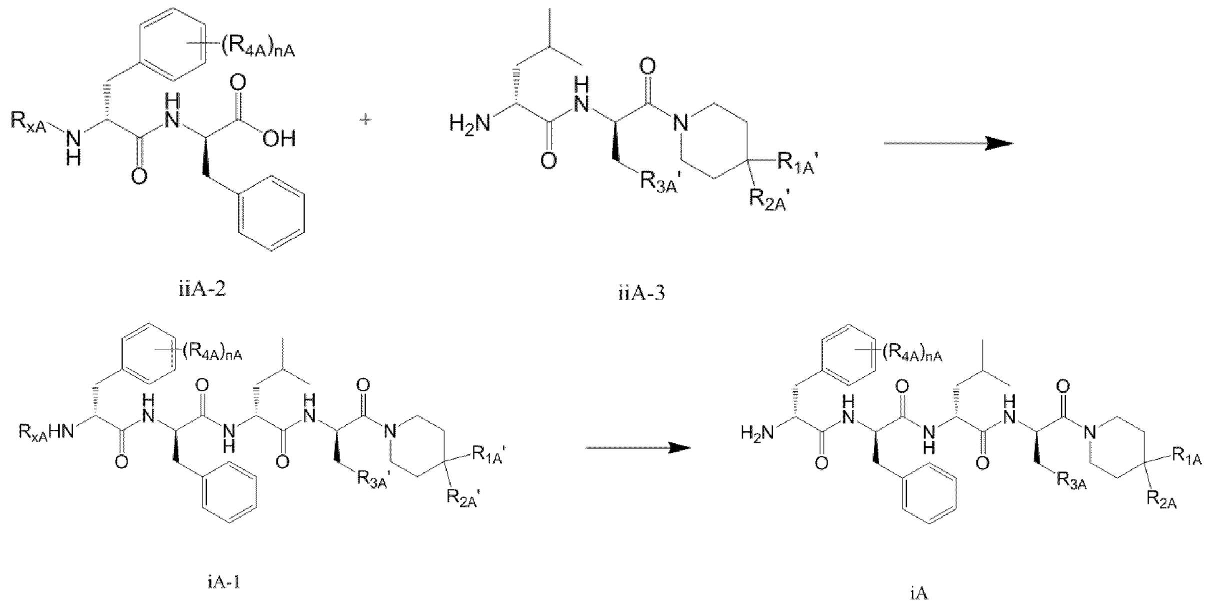

iiA-2 iiA-3

iA-1 iA

wherein, $R_{1A}$' is $R_{1A}$ or $R_{1A}$ in which $NH_2$ is protected with an amino-protecting group, $R_{2A}$' is $R_{2A}$ or $R_{2A}$ in which $NH_2$ is protected with an amino-protecting group, $R_{3A}$' is $R_{3A}$ or $R_{3A}$ in which $NH_2$ is protected with an amino-protecting group, $R_{1A}$, $R_{2A}$, $R_{3A}$, $R_{4a}$, and nA are as defined above, and $R_{xA}$ is an amino-protecting group;

the amino-protecting groups are preferably each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, *tert*-butylsulfinyl, or ethoxycarbonyl.

Compound

The present disclosure provides a compound of formula IB, a stereoisomer thereof, or a pharmaceutically acceptable salt thereof:

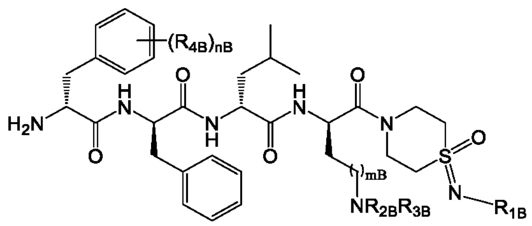

IB

$R_{1B}$ is selected from H, $C_{1-6}$ alkyl (e.g., methyl), $C_{1-6}$ alkylcarbonyl (e.g., methylcarbonyl), $C_{1-6}$ alkoxycarbonyl (e.g., methoxycarbonyl), $C_{6-14}$ aryl, $C_{6-14}$ arylcarbonyl, $C_{6-14}$ aryloxycarbonyl, $C_{3-8}$ cycloalkyl, $C_{3-8}$ cycloalkylcarbonyl, $C_{3-8}$ cycloalkoxycarbonyl, 5- to 14-membered heteroaryl, 5- to 14-membered heteroarylcarbonyl, 5- to 14-membered heteroaryloxycarbonyl, 3- to 8-membered heterocyclyl, 3- to 8-membered heterocyclylcarbonyl, and 3- to 8-membered heterocyclyloxycarbonyl, preferably from H, $C_{1-6}$ alkyl (e.g., methyl), $C_{1-6}$ alkylcarbonyl (e.g., methylcarbonyl), and $C_{1-6}$ alkoxycarbonyl (e.g., methoxycarbonyl), and more preferably from $C_{1-6}$ alkyl (e.g., methyl) and $C_{1-6}$ alkylcarbonyl (e.g., methylcarbonyl), the above substituents are each optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;

$R_{2B}$ and $R_{3B}$ are each independently selected from H, $C_{1-6}$ alkyl (e.g., methyl and isopropyl), amidino, and $C_{1-6}$ alkoxycarbonyl (e.g., methoxycarbonyl);

$R_{4B}$ is selected from halogen, $NO_2$, $C_{1-6}$ alkyl, $C_{1-6}$ haloalkyl, cyano, $NH_2C(=O)$-, and $C_{1-6}$ alkoxy, preferably from F, Cl, $NO_2$, $CH_3$, $CF_3$, cyano, and $NH_2C(=O)$-;

mB and nB are each independently 0, 1, 2, 3, 4, or 5, for example, mB is 3, and/or nB is 0.

In some embodiments, $R_{1B}$ is selected from H, $C_{1-6}$ alkyl, $C_{1-6}$ alkylcarbonyl, $C_{1-6}$ alkoxycarbonyl, $C_{6-14}$ aryl,

$C_{6-14}$ arylcarbonyl, $C_{6-14}$ aryloxycarbonyl, $C_{3-8}$ cycloalkyl, $C_{3-8}$ cycloalkylcarbonyl, $C_{3-8}$ cycloalkoxycarbonyl, 5- to 14-membered heteroaryl, 5- to 14-membered heteroarylcarbonyl, 5- to 14-membered heteroaryloxycarbonyl, 3- to 8-membered heterocyclyl, 3- to 8-membered heterocyclylcarbonyl, and 3- to 8-membered heterocyclyloxycarbonyl, preferably from $C_{1-6}$ alkyl and $C_{1-6}$ alkylcarbonyl, preferably from H, $C_{1-6}$ alkyl, $C_{1-6}$ alkylcarbonyl, and $C_{1-6}$ alkoxycarbonyl, and more preferably from $C_{1-6}$ alkyl and $C_{1-6}$ alkylcarbonyl, the above substituents are each optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy. In some embodiments, $R_{1B}$ is H. In some embodiments, $R_{1B}$ is $C_{1-6}$ alkyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, preferably methyl, ethyl, propyl, or butyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, and more preferably methyl. In some embodiments, $R_{1B}$ is $C_{1-6}$ alkylcarbonyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, preferably methylcarbonyl, ethylcarbonyl, propylcarbonyl, or butylcarbonyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, and more preferably methylcarbonyl.

In some embodiments, $R_{2B}$ and $R_{3B}$ are each independently selected from H, $C_{1-6}$ alkyl, amidino, and $C_{1-6}$ alkoxycarbonyl. In some embodiments, $R_{2B}$ and $R_{3B}$ are each independently selected from H and $C_{1-6}$ alkoxycarbonyl. In some embodiments, $R_{2B}$ and $R_{3B}$ are both H. In some embodiments, $R_{2B}$ is H, and $R_{3B}$ is $C_{1-6}$ alkoxycarbonyl, preferably methoxycarbonyl.

In some embodiments, $R_{4B}$ is selected from halogen, $NO_2$, $C_{1-6}$ alkyl, $C_{1-6}$ haloalkyl, cyano, $NH_2C(=O)$-, and $C_{1-6}$ alkoxy, preferably from F, Cl, $NO_2$, $CH_3$, $CF_3$, cyano, and $NH_2C(=O)$-.

In some embodiments, mB and nB are each independently 0, 1, 2, 3, 4, or 5. In some embodiments, mB is 0, 1, 2, 3, 4, or 5, preferably 3. In some embodiments, nB is 0, 1, 2, or 3, preferably 0.

In some embodiments, formula IB is preferably as shown in formula IIB,

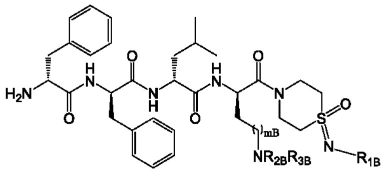

IIB

further preferably formula IIIB,

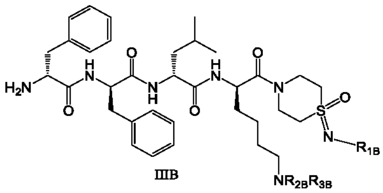

IIIB

and still further preferably formula IVB,

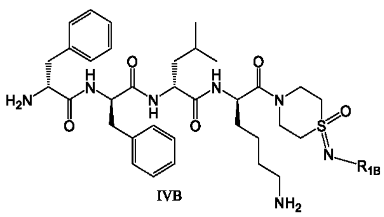

IVB

.

In some embodiments, in the general formulas described above, $R_{1B}$ is selected from $C_{1-6}$ alkyl and $C_{1-6}$ alkylcarbonyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, preferably $C_{1-6}$ alkyl or $C_{1-6}$ alkylcarbonyl, and more preferably methyl or methylcarbonyl.

In some embodiments, the compound of the present invention is selected from compounds shown below, stereoisomers thereof, or pharmaceutically acceptable salts thereof:

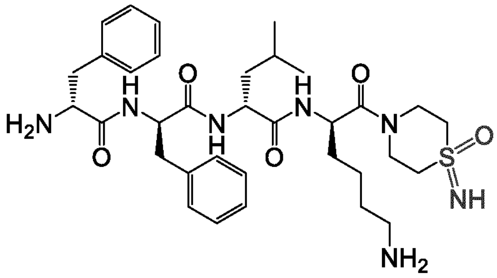

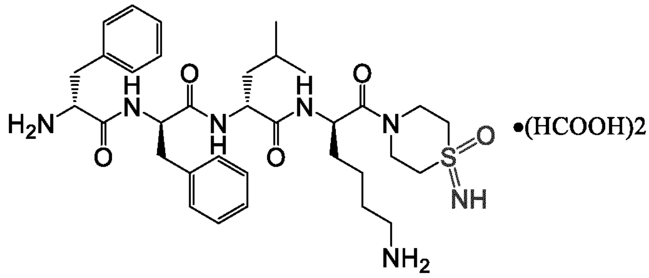

•(HCOOH)2

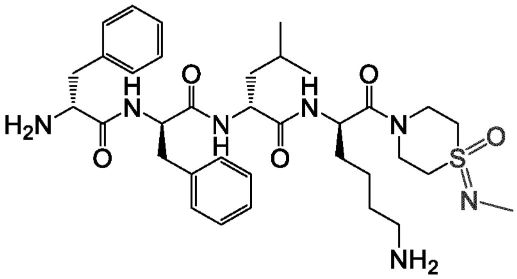

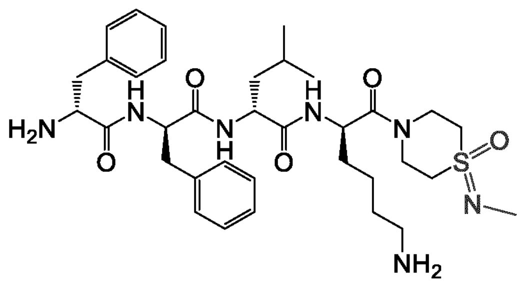

•HCOOH

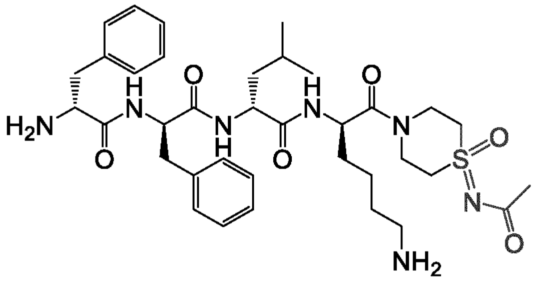

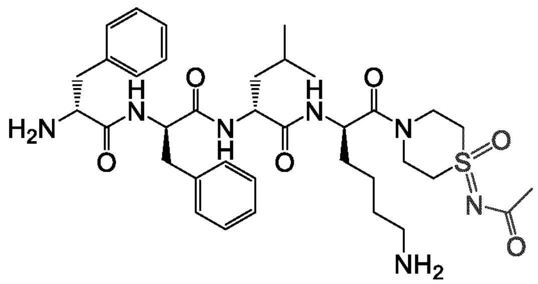

•HCOOH

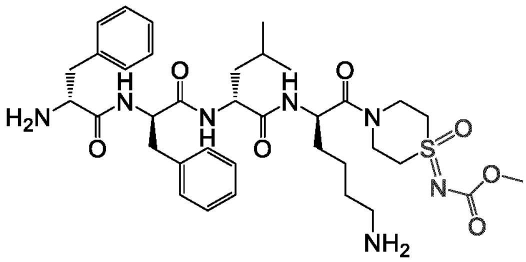

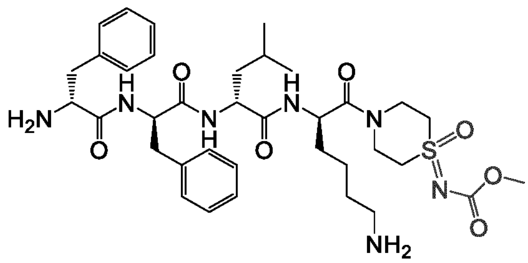

•(HCOOH)2

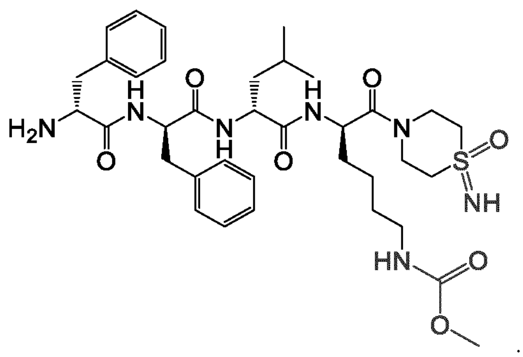

.

The present disclosure further provides a compound of formula iB-1, a stereoisomer thereof, or a salt thereof, which is an intermediate for preparing the compound of the present invention,

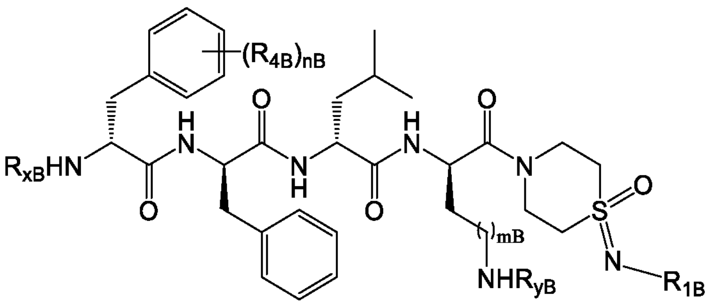

iB-1

wherein $R_{1B}$, $R_{4B}$, mB, and nB are as defined above, and $R_{xB}$ and $R_{yB}$ are amino-protecting groups, which are preferably each independently selected from *tert*-butoxycarbonyl (Boc), 9-fluorenylmethoxycarbonyl (Fmoc), allyloxycarbonyl (Alloc), trichloroethoxycarbonyl (Troc), trimethylsilylethoxycarbonyl (Teoc), benzyloxycarbonyl (CBz), *p*-toluenesulfonyl (Tosyl), *p*-nitrobenzenesulfonyl (Nosyl), *tert*-butyl (*t*-Bu), trifluoroacetyl (Tfa), methoxycarbonyl, or ethoxycarbonyl.

Similarly, the present disclosure further provides a compound of formula iB-4, a stereoisomer thereof, or a salt thereof,

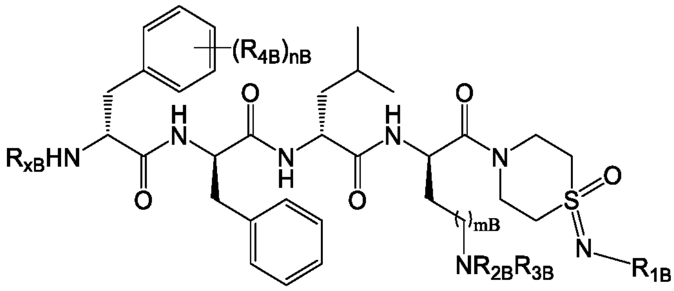

iB-4

,

wherein $R_{1B}$, $R_{2B}$, $R_{3B}$, $R_{4B}$, mB, nB, and $R_{xB}$ are as defined above.

Preparation method

In some embodiments, the compound of the present invention may be prepared by the following method, which comprises the following steps:

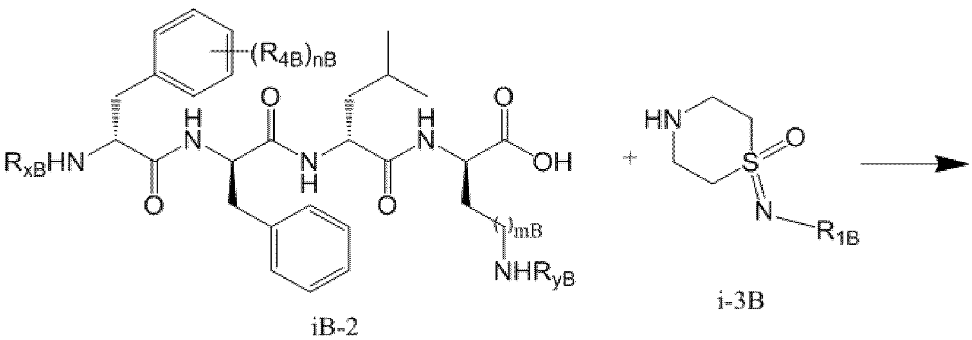

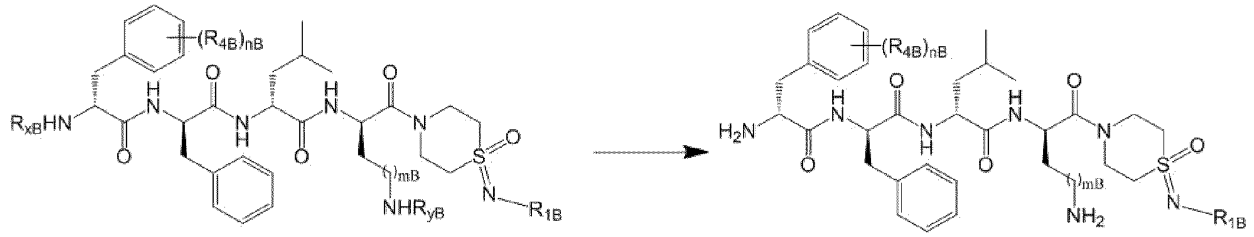

iB-1 iB

wherein, $R_{1B}$, $R_{4B}$, mB, and nB are as defined above, and $R_{xB}$ and $R_{yB}$ are amino-protecting groups, which are preferably each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, or ethoxycarbonyl.

Compound

The present disclosure provides a compound of formula IC, a stereoisomer thereof, or a pharmaceutically acceptable salt thereof:

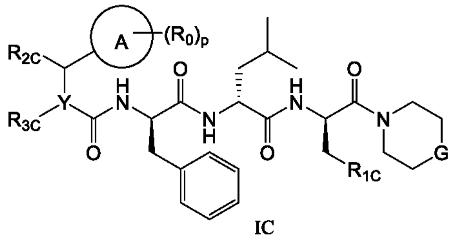

IC

.

In some embodiments, ring A is $C_{3-8}$ cycloalkyl, $C_{6-14}$ aryl (preferably monocyclic aryl), or 5- to 14-membered heteroaryl (preferably monocyclic heteroaryl), such as cyclopropyl, cyclopentyl, phenyl, pyridinyl, and pyrimidinyl. In some embodiments, ring A is phenyl.

In some embodiments, Y is selected from CH or N. In some embodiments, Y is N.

In some embodiments, G is selected from -S-, -O-, $-CR_{4C}R_{5C}-$, $-NR_{6C}-$, $-S(=O)_2-$, $-S(=O)(=NR_{6C}')-$, and

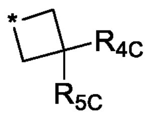

.

In some embodiments, G is selected from -O-, $-CR_{4C}R_{5C}-$, $-NR_{6C}-$, $-S(=O)_2-$, $-S(=O)(=NR_{6C}')-$, and

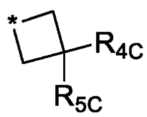

.

In some embodiments, G is -O-. In some embodiments, G is $-S(=O)_2-$.

In some embodiments, G is $-S(=O)(=NR_{6C}')-$, wherein $R_{6C}'$ is selected from H, $C_{1-6}$ alkyl (e.g., methyl), $C_{1-6}$ alkylcarbonyl (e.g., methylcarbonyl), $C_{1-6}$ alkoxycarbonyl (e.g., methoxycarbonyl), $C_{6-14}$ aryl, $C_{6-14}$ arylcarbonyl, $C_{6-14}$ aryloxycarbonyl, $C_{3-8}$ cycloalkyl, $C_{3-8}$ cycloalkylcarbonyl, $C_{3-8}$ cycloalkoxycarbonyl, 5- to 14-membered heteroaryl, 5- to 14-membered heteroarylcarbonyl, 5- to 14-membered heteroaryloxycarbonyl, 3- to 8-membered heterocyclyl, 3- to 8-membered heterocyclylcarbonyl, and 3- to 8-membered heterocyclyloxycarbonyl, preferably from H, $C_{1-6}$ alkyl (e.g., methyl), $C_{1-6}$ alkylcarbonyl (e.g., methylcarbonyl), and $C_{1-6}$ alkoxycarbonyl (e.g., methoxycarbonyl), and more preferably from $C_{1-6}$ alkyl (e.g., methyl) and $C_{1-6}$ alkylcarbonyl (e.g., methylcarbonyl), the above substituents are each optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy;

In some embodiments, G is $-CR_{4C}R_{5C}-$, wherein $R_{4C}$ and $R_{5C}$ are each independently selected from H, $C_{1-6}$ alkyl, $C_{1-6}$ alkyl-O-, hydroxyl, $-C(O)OR_7$, $-NR_8R_9$, $-NR_{cC}C(O)NR_8R_9$, $C_{1-6}$ alkylamino, 3- to 8-membered heterocy-

clyl-$(CH_2)_{mC}$-, halogen, cyano, -$NR_{cC}S(=O)_2NR_8R_9$, -$NR_{cC}C(O)OR_{dC}$, -$NR_{cC}S(=O)_2OR_{dC}$, -$NR_{cC}C(O)R_7$', and -$NH(CH_2)_{mC}NR_8R_9$, wherein the alkyl and heterocyclyl are optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy; $R_7$ and $R_7$' are each independently selected from H, $C_{1-6}$ alkyl, $C_{3-8}$ cycloalkyl, 3- to 8-membered heterocyclyl, $C_{6-14}$ aryl, and 5- to 14-membered heteroaryl, wherein the alkyl, cycloalkyl, heterocyclyl, aryl, and heteroaryl are optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy; $R_8$ and $R_9$ are each independently H or $C_{1-6}$ alkyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy; $R_{cC}$ is H or $C_{1-6}$ alkyl substituted with one or more groups selected from amino, $C_{1-6}$ alkylamino, $C_{1-6}$ alkoxy, halogen, hydroxyl, nitro, cyano, $NH_2C(=O)$-, and $C_{1-6}$ alkoxy, preferably H or $C_{1-6}$ alkyl substituted with amino, $C_{1-6}$ alkylamino, or $C_{1-6}$ alkoxy, and more preferably H or $C_{1-6}$ alkyl substituted with amino or $C_{1-6}$ alkylamino; $R_{dC}$ is selected from $C_{1-6}$ alkyl, $C_{6-14}$ aryl, 5- to 14-membered heteroaryl, $C_{3-8}$ cycloalkyl, and 3- to 8-membered heterocyclyl, preferably from $C_{1-6}$ alkyl and 3- to 8-membered heterocyclyl, more preferably from $C_{1-6}$ alkyl and

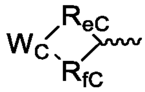,

and even more preferably from $C_{1-6}$ alkyl and

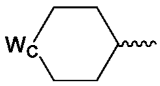,

the group $R_{dC}$ described above is optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, wherein $R_{eC}$ and $R_{fC}$ are each independently -$(CH_2)_{n1C}$- and -$(CH_2)_{n1C'}$-, and in some embodiments, are -$(CH_2)_{n1C}$- and -$(CH_2)_{n1C'}$-, respectively, wherein n1C and n1C' are each independently selected from 0, 1, 2, and 3, preferably 2, and n1C and n1C' are not both 0; $W_C$ is selected from -NH-C(=O)-, -NH-$S(=O)_2$-, -$NR_{12}$-, -O-, -S-, - and -$S(=O)_2$-, preferably from -O- and -$S(=O)_2$-, wherein $R_{12}$ is selected from H, $C_{1-6}$ alkyl, amidino, and HOOC-$(CH_2)_{n3C}$-, wherein n3C is selected from 1, 2, and 3; mC is selected from 1, 2, 3, and 4;

or $CR_{4C}R_{5C}$ forms a 3- to 8-membered heterocyclic or 9- to 10-membered bicyclic moiety (particularly a 9-to 10-membered fused ring), wherein the 3- to 8-membered heterocyclic or 9- to 10-membered bicyclic moiety, together with the piperidine ring attached thereto, forms a structure selected from:

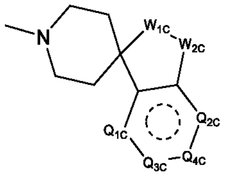, 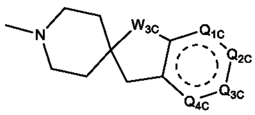,

and

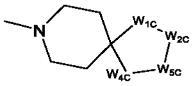,

when any one of $Q_{1C}$-$Q_{4C}$ is N, the rest are CH, or $Q_{1C}$-$Q_{4C}$ are all CH;

$W_{1C}$ and $W_{2C}$ are each independently -C(=O)-NH-, -NH-C(=O)-, -$S(=O)_2$-NH-, -NH-$S(=O)_2$-, -S-, -O-, - $NR_{12}$-, -$NR_{12}$-$CH_2$-, -$(CH_2)_{n2C}$- optionally substituted with one or more groups selected from -$NH_2$, -OH, halogen, nitro, cyano, $C_{1-6}$ haloalkyl, $NH_2C(=O)$-, and $C_{1-6}$ alkoxy, or absent, wherein $R_{12}$ is selected from H, $C_{1-6}$ alkyl, amidino, and HOOC-$(CH_2)_{n3C}$-; preferably, $W_{1C}$ and $W_{2C}$ cannot be simultaneously absent;

$W_{3C}$ is -$(CH_2)_{n2C}$- optionally substituted with one or more groups selected from $NH_2$, -OH, halogen, nitro, cyano, $C_{1-6}$ haloalkyl, $NH_2C(=O)$-, and $C_{1-6}$ alkoxy, or absent; preferably -$(CH_2)_{n2C}$- optionally substituted with one or more groups selected from $NH_2$, -OH, halogen, nitro, cyano, $C_{1-6}$ haloalkyl, $NH_2C(=O)$-, and $C_{1-6}$ alkoxy;

$W_{4C}$ and $W_{5C}$ are $-(CH_2)_{n2C}-$ optionally substituted with one or more groups selected from $NH_2$, -OH, halogen, nitro, cyano, $C_{1-6}$ haloalkyl, $NH_2C(=O)-$, and $C_{1-6}$ alkoxy, or absent;
n2C is selected from 0, 1, 2, and 3;
more preferably, the 3- to 8-membered heterocyclic or 9- to 10-membered bicyclic moiety, together with the piperidine ring attached thereto, forms a structure selected from:

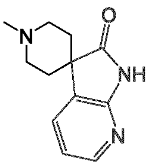, 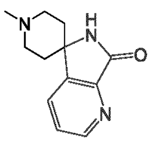, 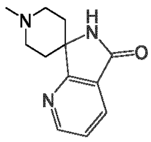, 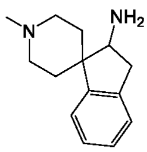, 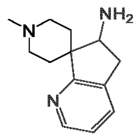, 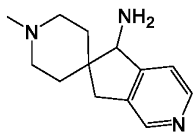, 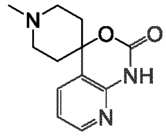,

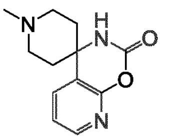, 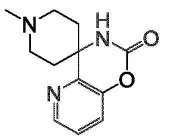, 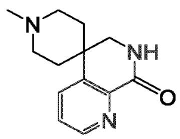, 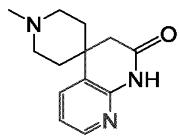, 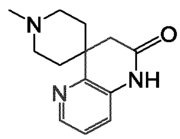, 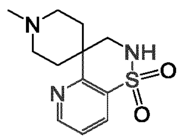,

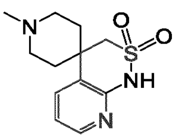, 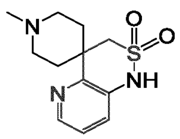, 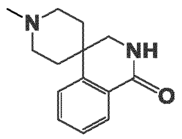, 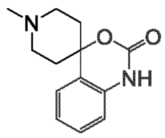, 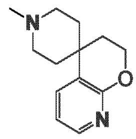, 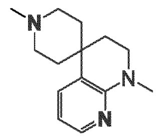,

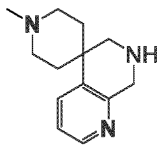, 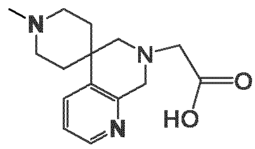, 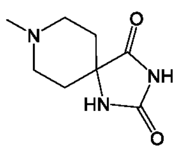,

and

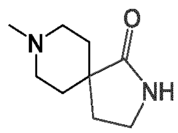,

more preferably from

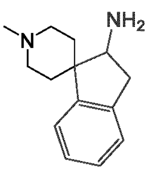, 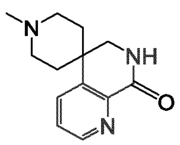, 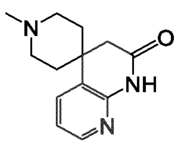, 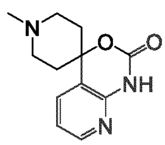, 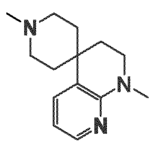, 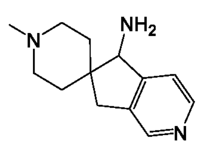,

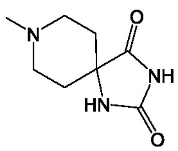,

and

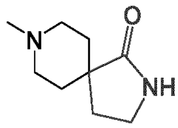

In some embodiments, G is $-CR_{4C}R_{5C}-$, wherein $R_{4C}$ is H, and $R_{5C}$ is $-NR_{cC}C(O)OR_{dC}$, wherein $R_{cC}$ and $R_{dC}$ are as defined above.
In some embodiments, G is $-CR_{4C}R_{5C}-$, wherein $R_{4C}$ is $-NR_8R_9$, and $R_{5C}$ is $-C(O)OR_7$, wherein $R_7$, $R_8$, and $R_9$ are as defined above. In some embodiments, $R_7$ is H or $C_{1-6}$ alkyl optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy, e.g., H or $C_{1-6}$ alkyl, such as H or methyl. In some embodiments, $R_8$ and $R_9$ are both H.

In some embodiments, G is $-S(=O)(=NR_{6C}')-$, wherein $R_{6C}'$ is as defined above.

In some embodiments, $R_{1C}$ is selected from H or $-(CH_2)_tNR_{aC}R_{bC}$, wherein $R_{aC}$ and $R_{bC}$ are each independently selected from H, $C_{1-6}$ alkyl (e.g., methyl, isopropyl, etc.), amidino, and $C_{1-6}$ alkoxycarbonyl (e.g., methoxycarbonyl, etc.), and t is selected from 0, 1, 2, 3, 4, or 5. In some embodiments, $R_{1C}$ is H. In some embodiments, $R_{aC}$ and $R_{bC}$ are each independently selected from H and $C_{1-6}$ alkoxycarbonyl (e.g., methoxycarbonyl, etc.). In some embodiments, $R_{aC}$ and $R_{bC}$ are each H. In some embodiments, t is 0, 1, 2, or 3, preferably 3.

In some embodiments, $R_{2C}$ is selected from H, amino, hydroxyl, $C_{1-6}$ alkyl, $C_{1-6}$ alkylamino, and $C_{1-6}$ aminoalkyl, wherein the alkyl is optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy.

In some embodiments, when Y is CH, $R_{3C}$ is selected from H, hydroxyl, $C_{1-6}$ alkyl, 3- to 8-membered heterocyclyl, and $C_{1-6}$ alkoxy, and when Y is N, $R_{3C}$ is selected from H, $C_{1-6}$ alkyl, $C_{3-8}$ cycloalkyl, $C_{3-8}$ cycloalkyl-$(CH_2)_{mC}$-, 3- to 8-membered heterocyclyl, 3- to 8-membered heterocyclyl-$(CH_2)_{mC}$-, and $-(CH_2)_{mC}NR_{10}R_{11}$, wherein the alkyl, cycloalkyl, heterocyclyl, and alkoxy are optionally substituted with one or more groups selected from halogen, hydroxyl, amino, nitro, cyano, $C_{1-6}$ alkyl, $C_{1-6}$ haloalkyl, $NH_2C(=O)-$, $C_{1-6}$ alkoxy, and $C_{1-6}$ alkylamino; $R_{10}$ and $R_{11}$ are each independently H or $C_{1-6}$ alkyl, or $R_{10}$ and $R_{11}$, together with the nitrogen atom to which they are both attached form 3- to 8-membered heterocyclyl (e.g.,

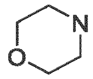

or

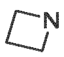

), wherein the alkyl and heterocyclyl are optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy; mC is independently selected from 1, 2, 3, and 4 at each occurrence.

In some embodiments, $R_0$ is selected from H, halogen, $NO_2$, cyano, $NH_2C(=O)-$, $C_{1-6}$ alkoxy, and $C_{1-6}$ alkyl optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy, preferably from H, F, Cl, $NO_2$, $CH_3$, $CF_3$, cyano, $NH_2C(=O)-$, and $C_{1-6}$ aminoalkyl. In some embodiments, $R_{4C}$ is H.

In some embodiments, p is 0, 1, 2, 3, 4, or 5, preferably 0, 1, 2, or 3, and more preferably 0.

In some embodiments, formula IC is as shown in formula IIC-1 or IIC-2:

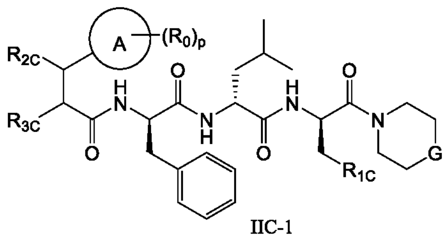

IIC-1

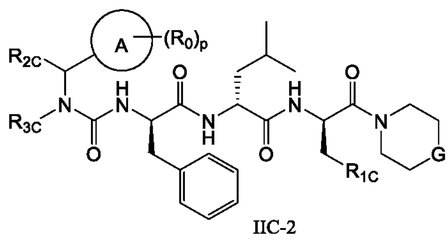

IIC-2

In some embodiments, in IIC-1, $R_{2C}$ is $C_{1-6}$ aminoalkyl or amino, and $R_{3C}$ is H or $C_{1-6}$ alkyl; in some embodiments, $R_{2C}$ is amino, and $R_{3C}$ is H.

In some embodiments, in formula IIC-2, $R_{2C}$ is $C_{1-6}$ aminoalkyl or amino, and $R_{3C}$ is H or $C_{1-6}$ alkyl, or $R_{2C}$ is H or $C_{1-6}$ alkyl, and $R_{3C}$ is $C_{1-6}$ aminoalkyl or 3- to 8-membered heterocyclyl containing nitrogen.

In some embodiments, formula IC is as shown in formula IIIC or formula IVC:

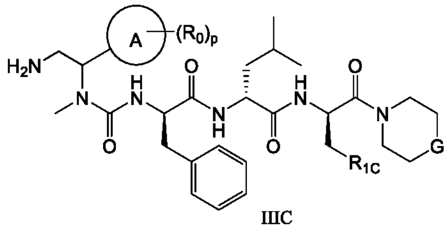

IIIC

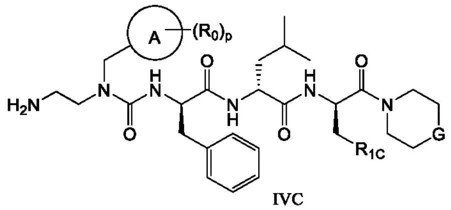

IVC

In some embodiments, in the general formulas described above, one or more of the following criteria are satisfied:

(1) $R_{1C}$ is $-(CH_2)_tNR_{aC}R_{bC}$, preferably $-(CH_2)_3NH_2$;
(2) ring A is phenyl; and
(3) p is 0.

In some embodiments, in the general formulas, G is selected from one of the following:

(1) G is $-S(=O)(=NR_{6C}')-$;
(2) G is $-CR_{4C}R_{5C}-$, wherein $R_{4C}$ is H, and $R_{5C}$ is $-NR_{cC}C(O)OR_{dC}$; and
(3) G is $-CR_{4C}R_{5C}-$, wherein $R_{4C}$ is $-NR_8R_9$, and $R_{5C}$ is $-C(O)OR_7$, wherein $R_7$ is selected from H, $C_{1-6}$ alkyl, $C_{3-8}$ cycloalkyl, 3- to 8-membered heterocyclyl, $C_{6-14}$ aryl, and 5- to 14-membered heteroaryl, wherein the alkyl, cycloalkyl, heterocyclyl, aryl, and heteroaryl are optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy; and $R_7$ is preferably H or $C_{1-6}$ alkyl optionally substituted with one or more groups selected from halogen, hydroxyl, amino, nitro, cyano, $C_{1-6}$ alkyl, $C_{16}$ haloalkyl, $NH_2C(=O)-$, and $C_{1-6}$ alkoxy.

In some embodiments, formula IC is as shown in formula IVC-1, IVC-2, or IVC-3:

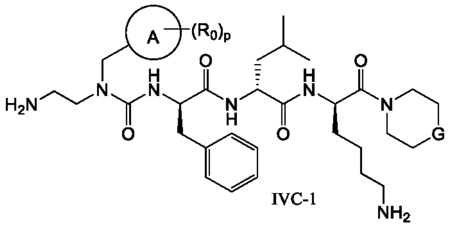

IVC-1

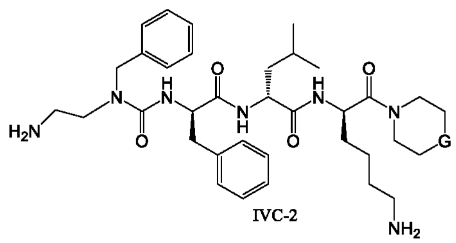

IVC-2

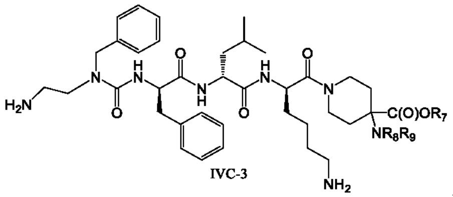

IVC-3

In some embodiments, the compound of the present disclosure is selected from compounds shown below, stereoisomers thereof, or pharmaceutically acceptable salts thereof:

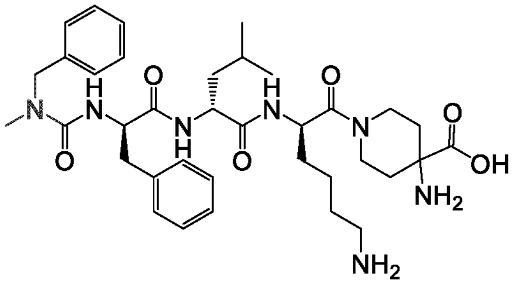

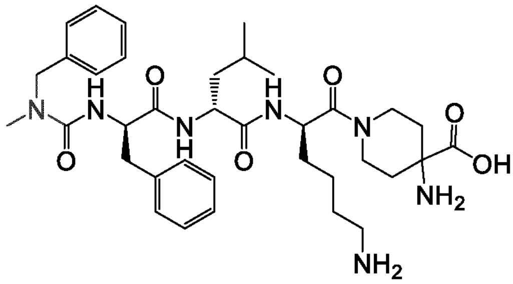
•HCOOH

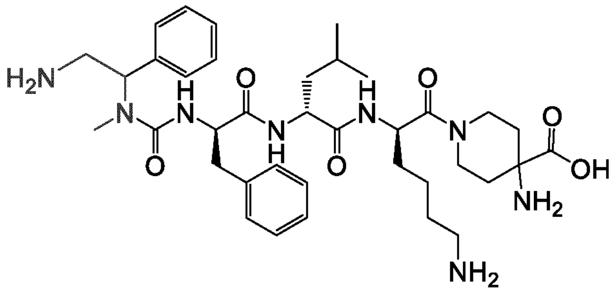

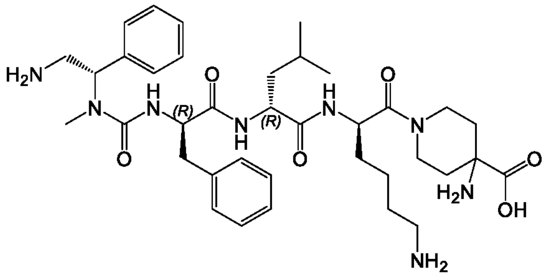

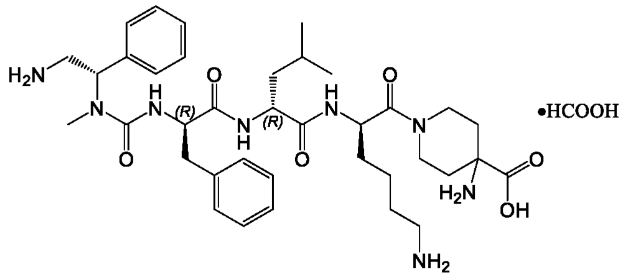
•HCOOH

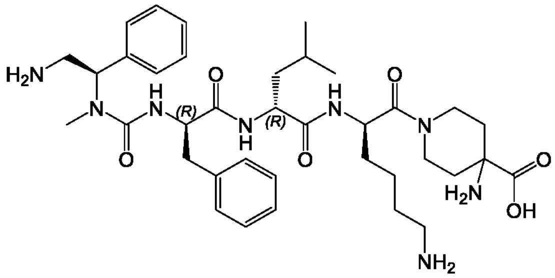

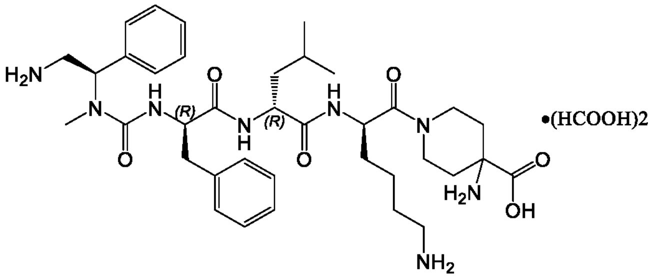
•(HCOOH)2

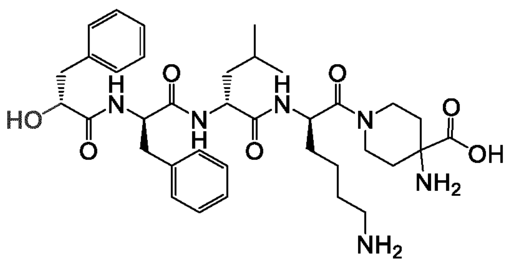

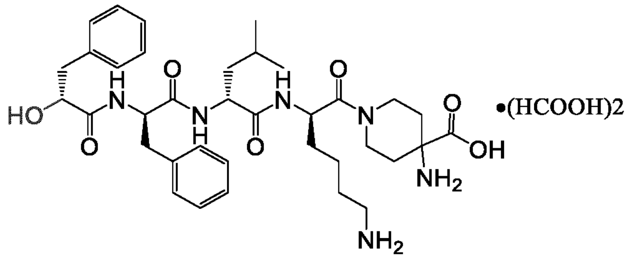
•(HCOOH)2

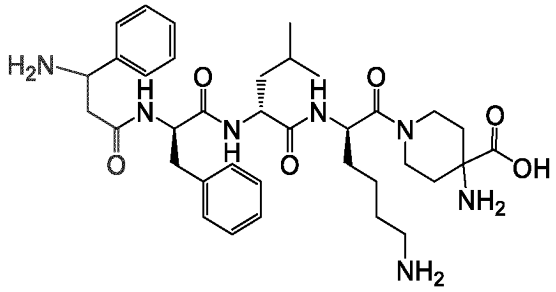

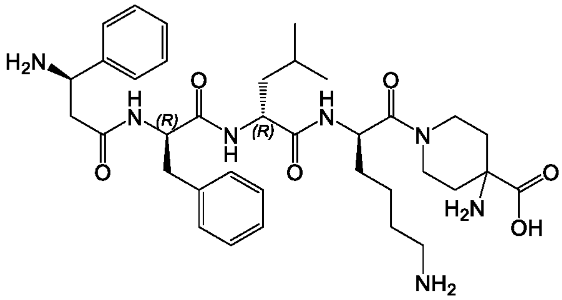

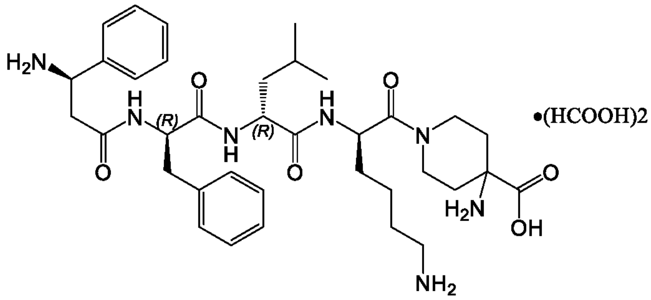
•(HCOOH)2

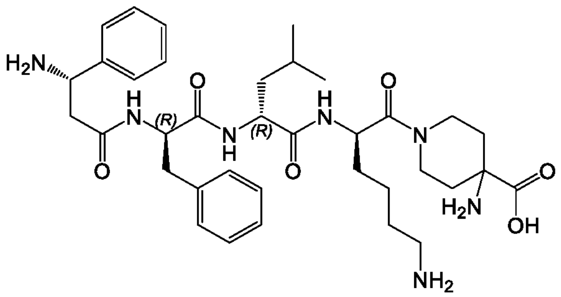

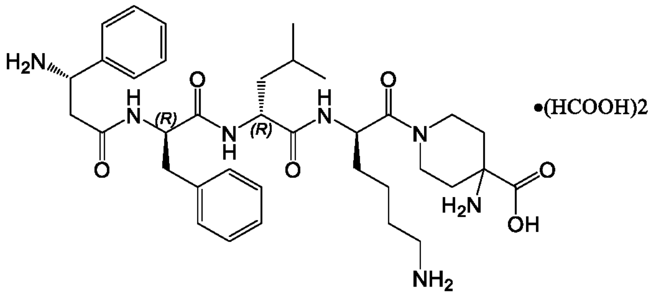
•(HCOOH)2

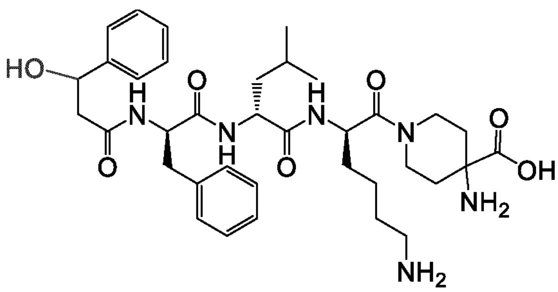

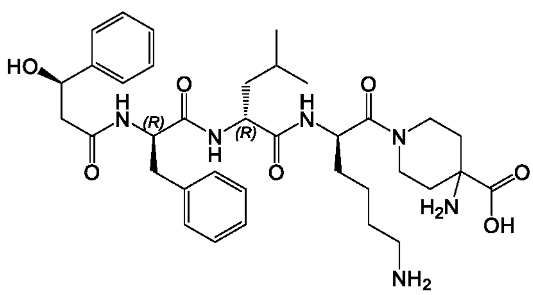

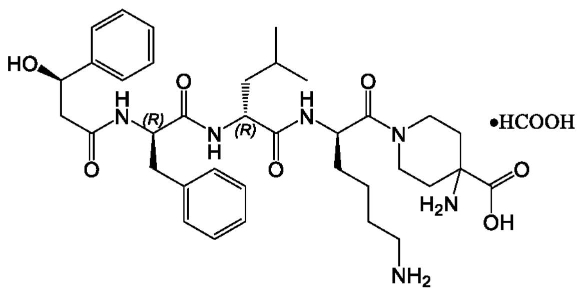
•HCOOH

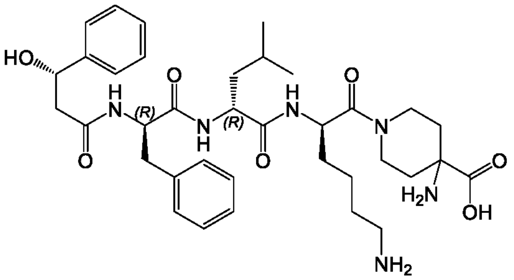

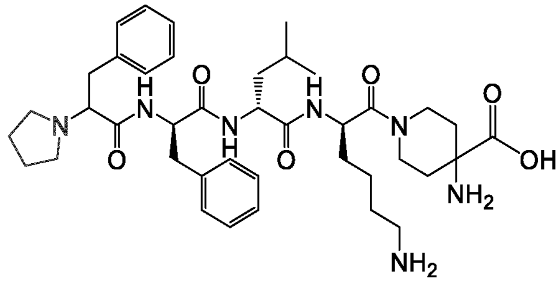

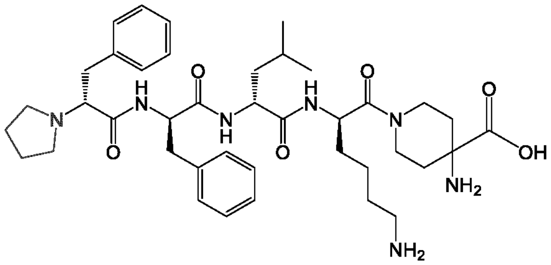

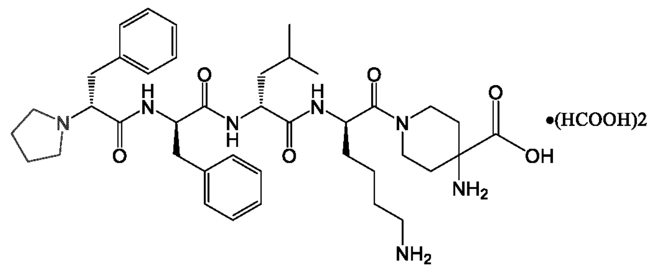

•(HCOOH)2

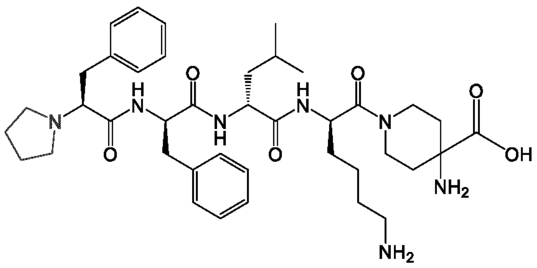

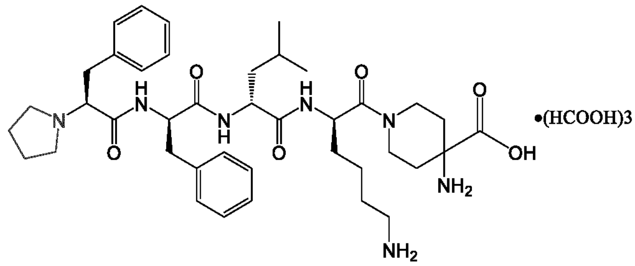

•(HCOOH)3

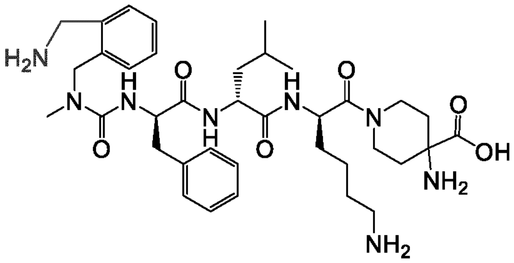

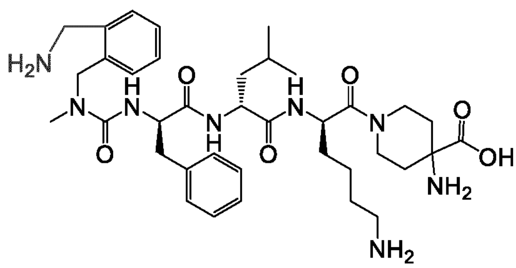

•(HCOOH)2

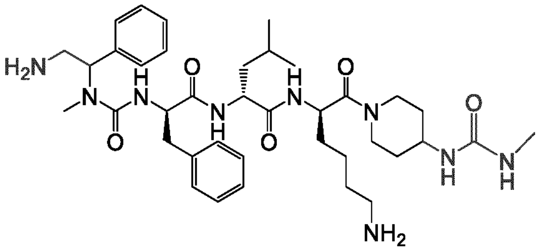

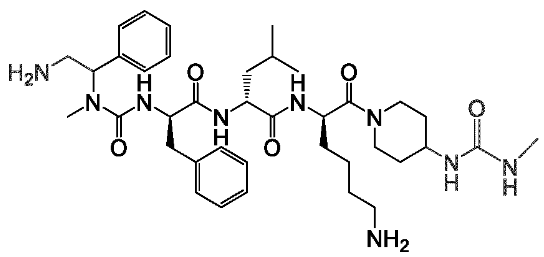

•(HCOOH)2

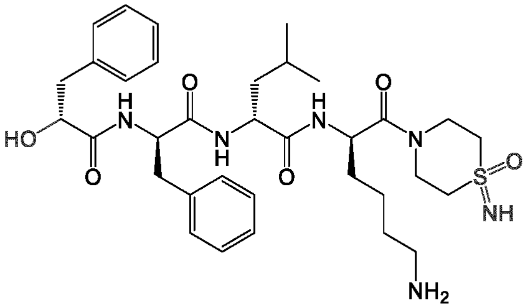

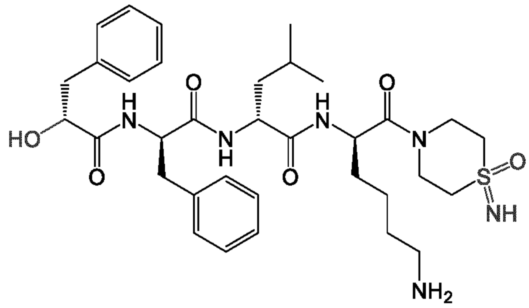

•HCOOH

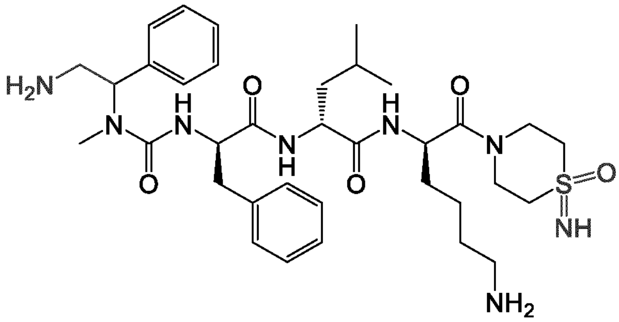

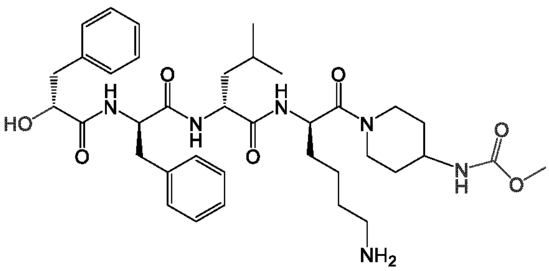

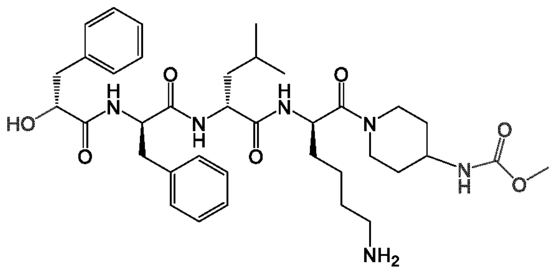

•HCOOH

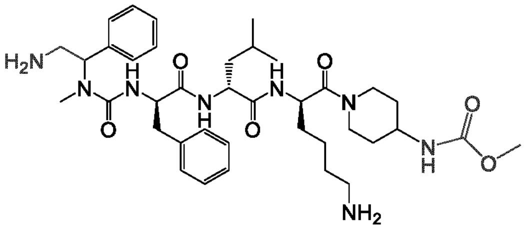

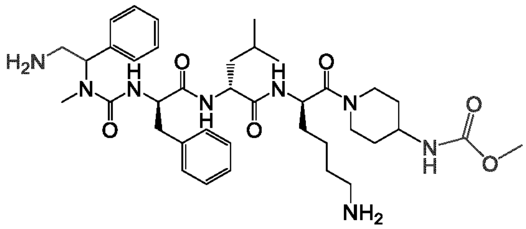

•(HCOOH)2

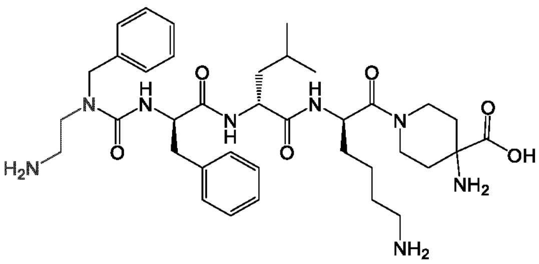

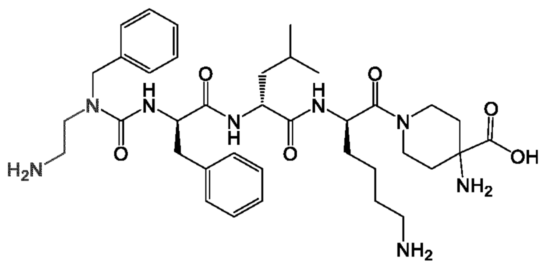

•(HCOOH)2

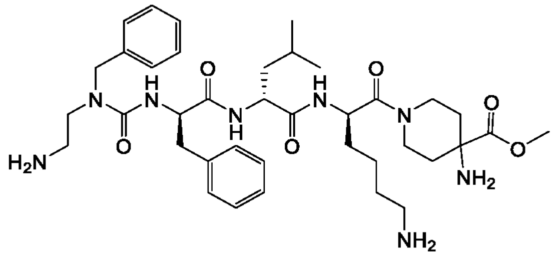

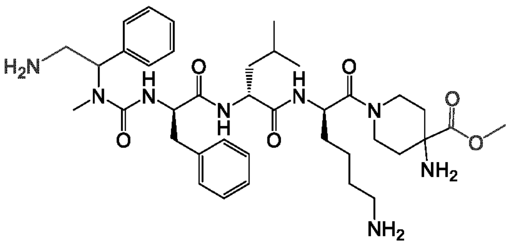

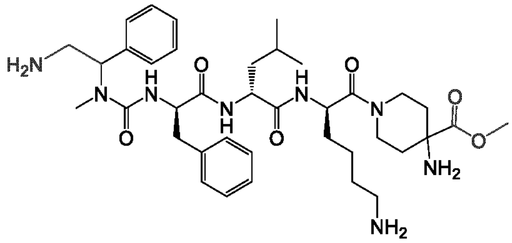

•(HCOOH)3

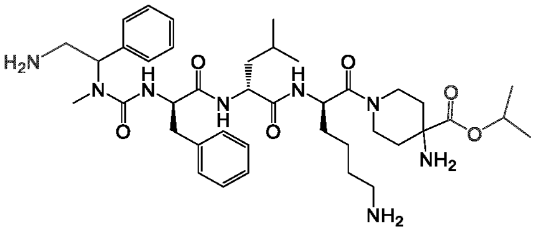

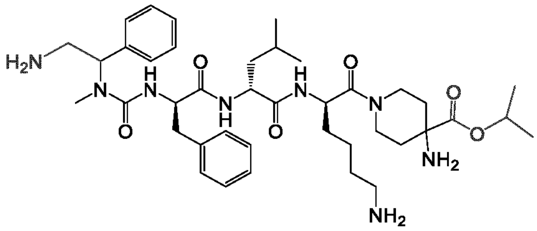

•(HCOOH)2

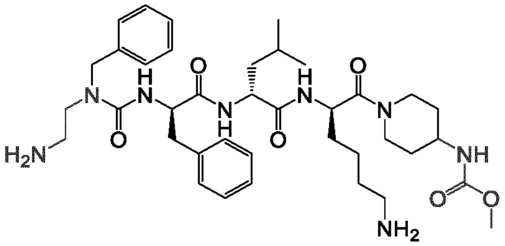

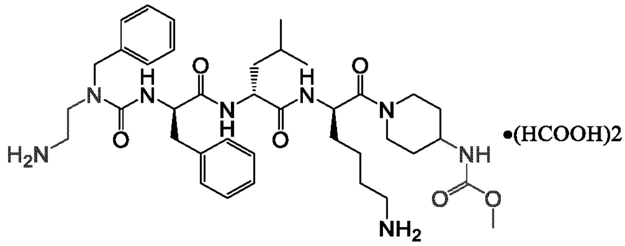

•(HCOOH)2

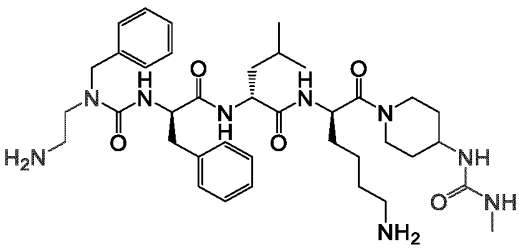

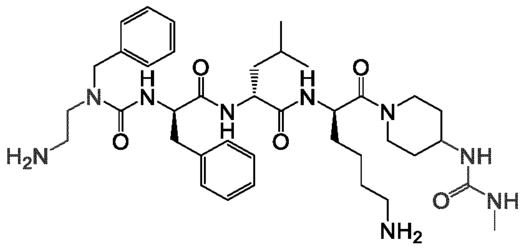

•(HCOOH)2

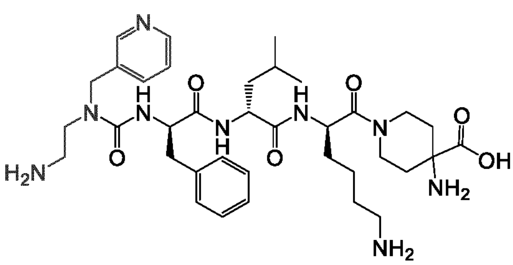

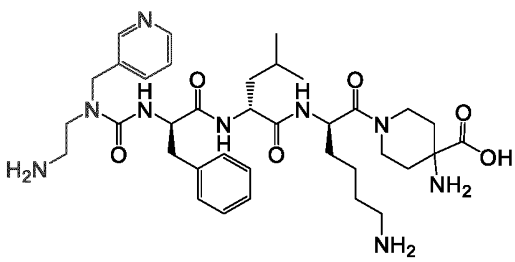

•HCOOH

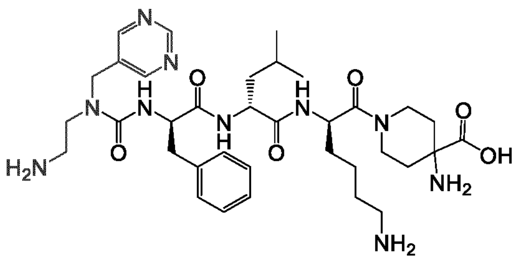

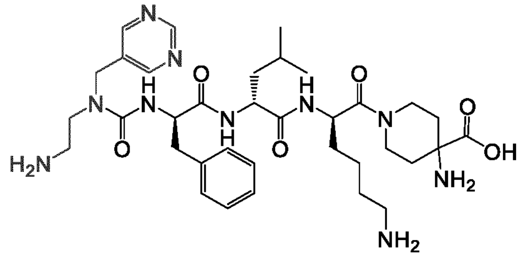

•HCOOH

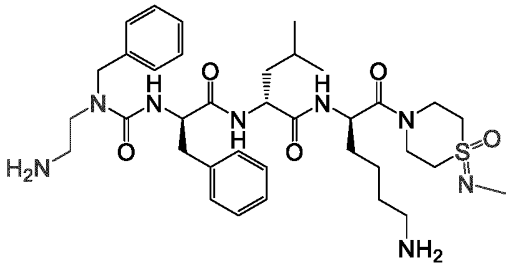

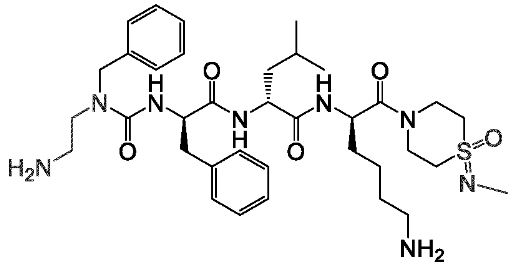

•(HCOOH)2

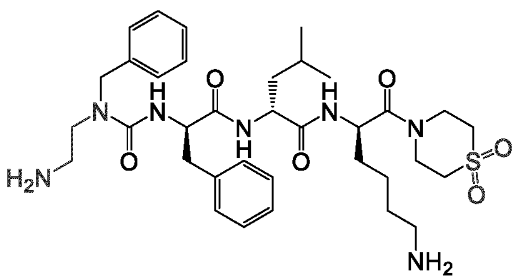

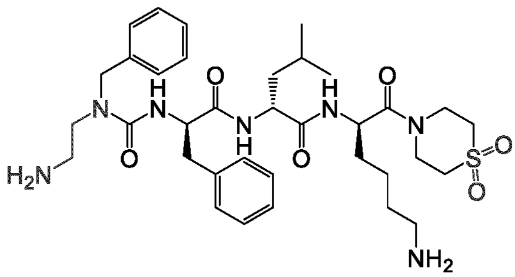

•(HCOOH)2

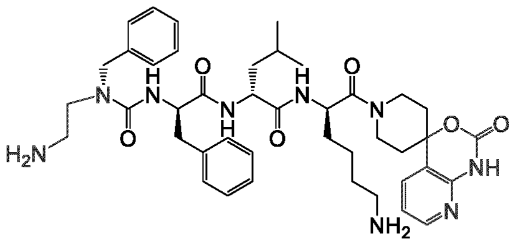

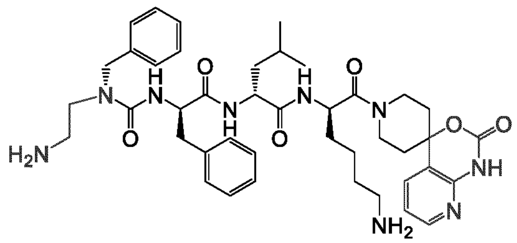

•HCOOH

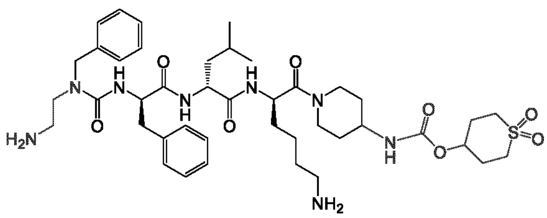

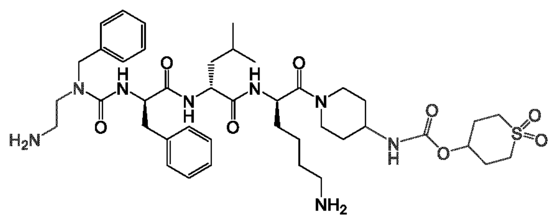

•(HCOOH)2

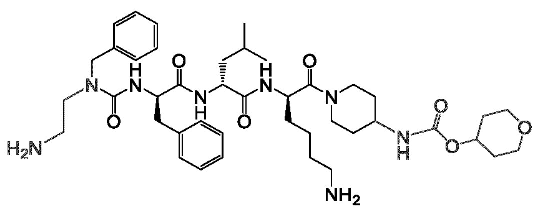

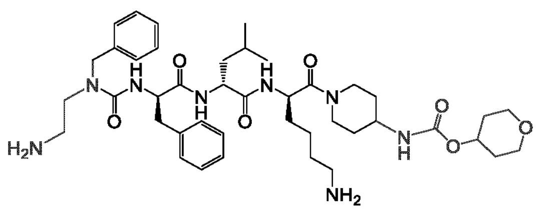

•(HCOOH)2

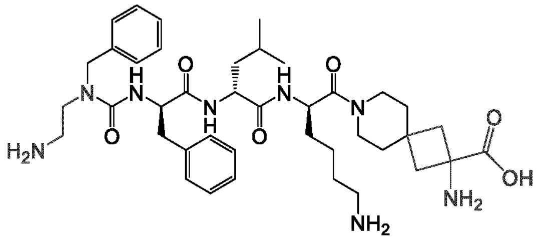

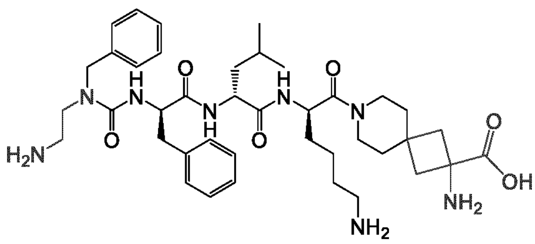

•(HCOOH)2

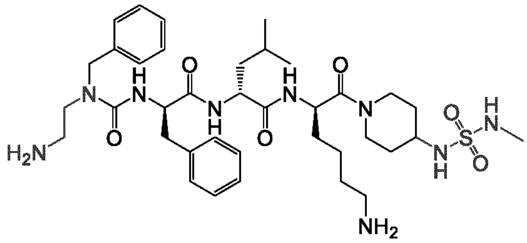

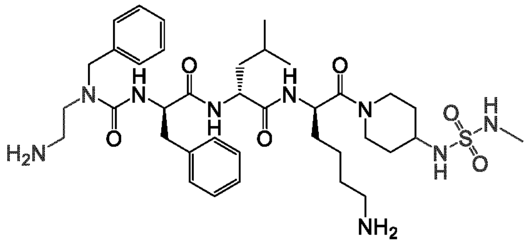

•(HCOOH)2

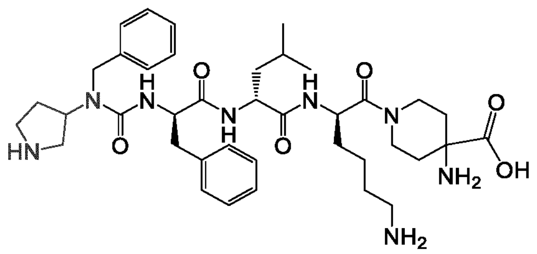

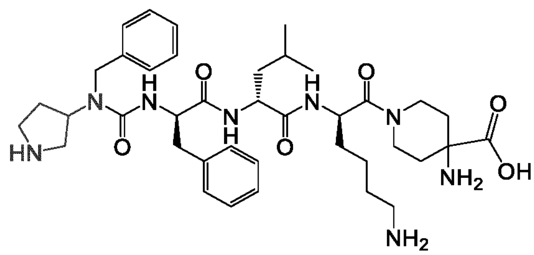

•HCOOH

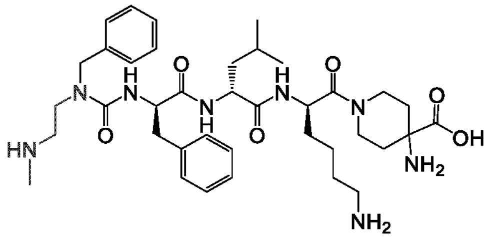

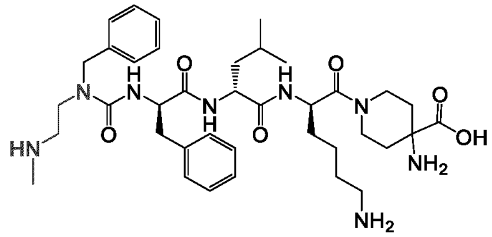

•(HCOOH)2

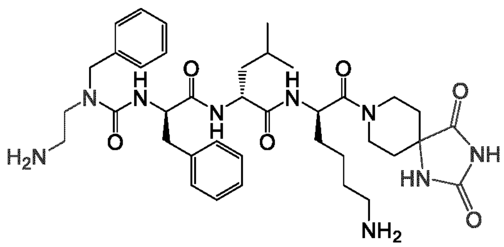

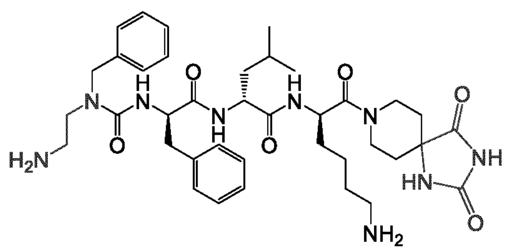

•(HCOOH)2

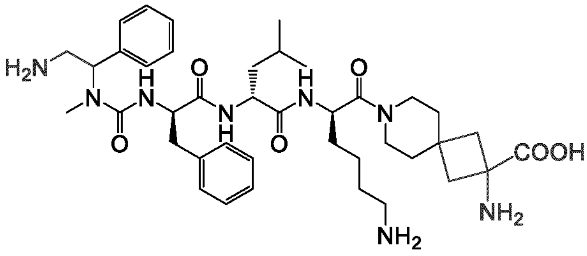

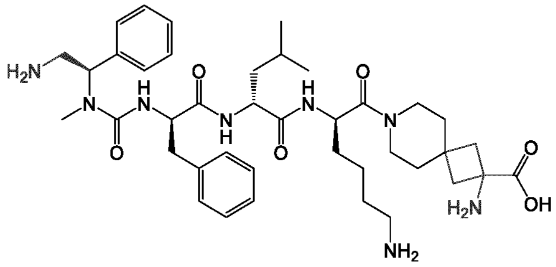

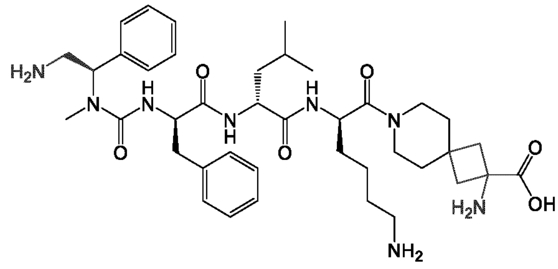

•HCOOH

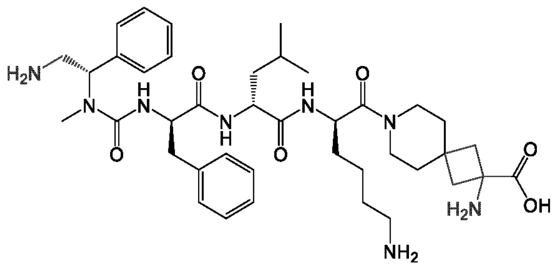

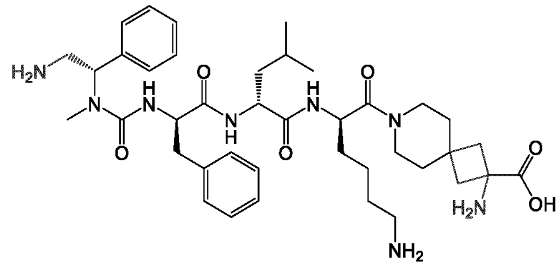

•HCOOH

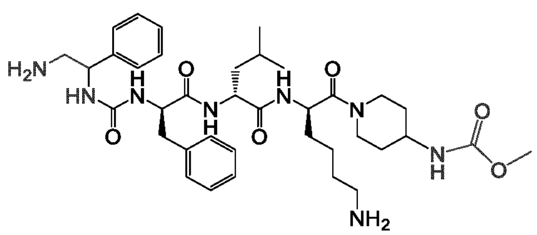

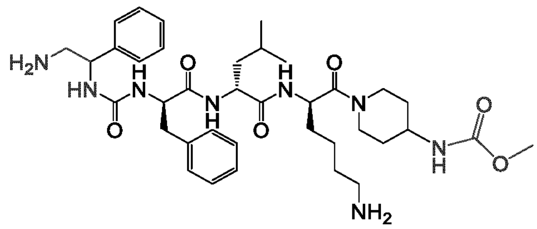

•(HCOOH)2

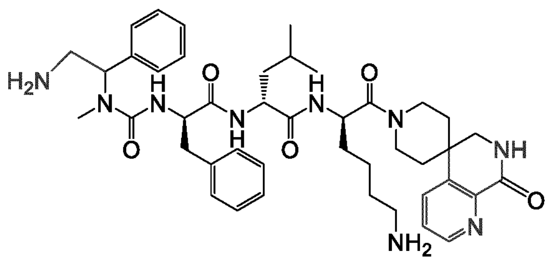

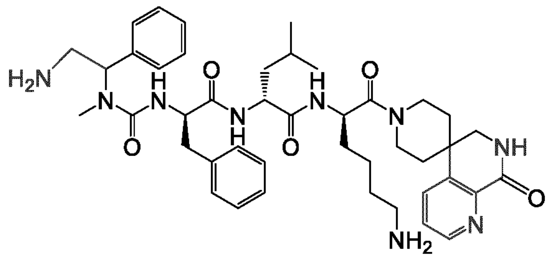

•HCOOH

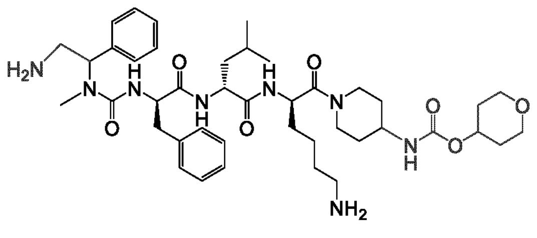

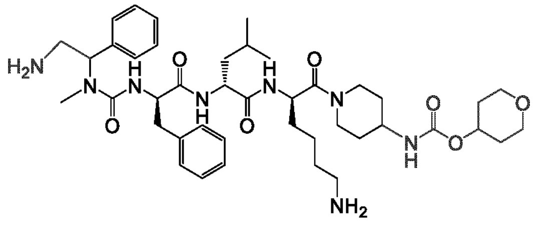

•(HCOOH)2

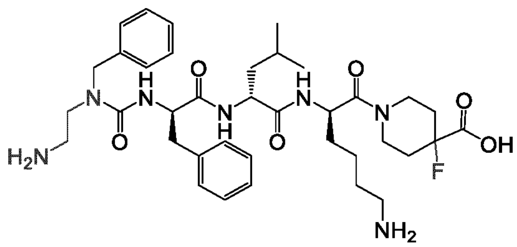

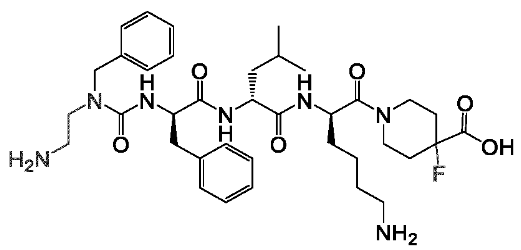

•HCOOH

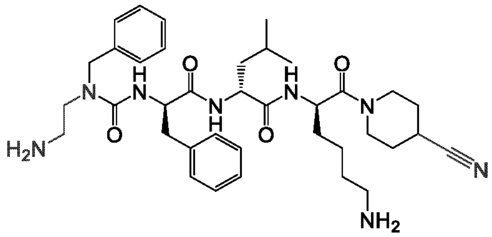

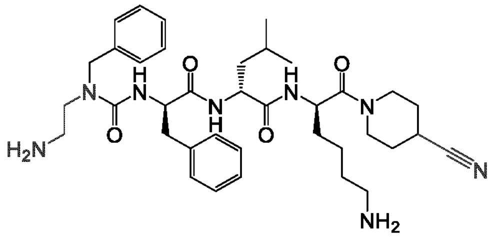

•(HCOOH)2

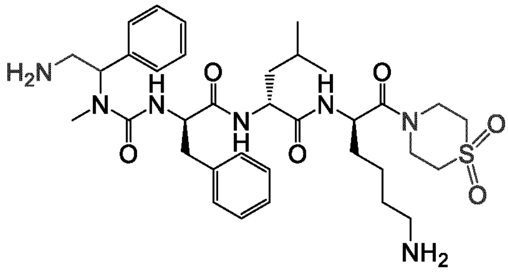

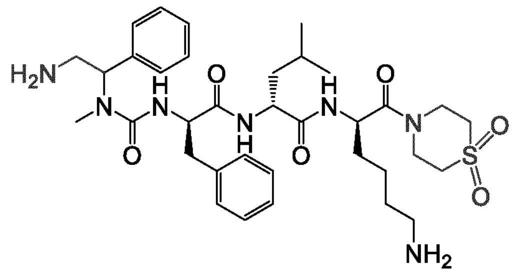

•HCOOH

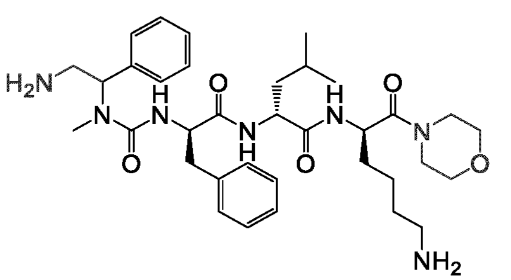

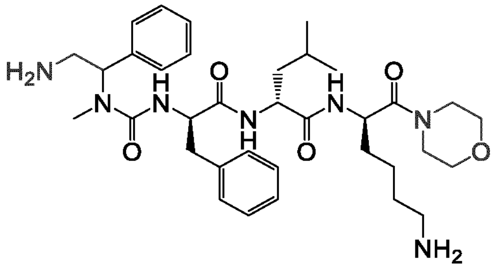

•(HCOOH)2

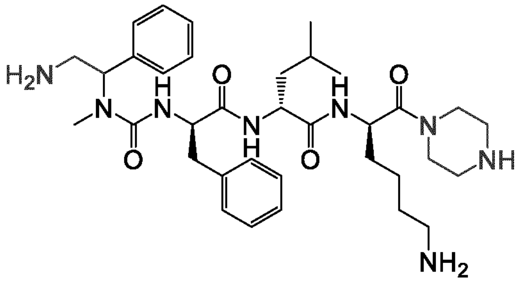

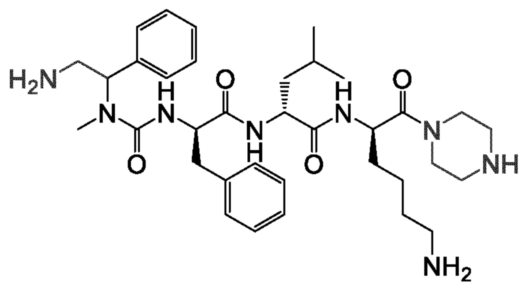

•HCOOH

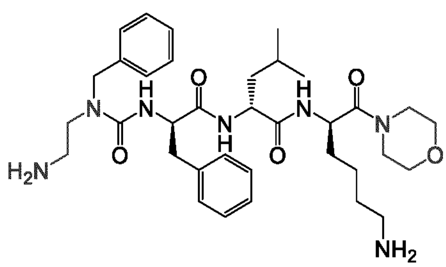

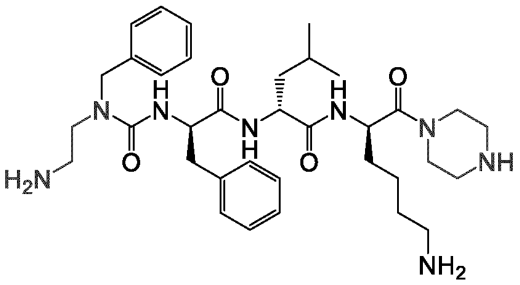

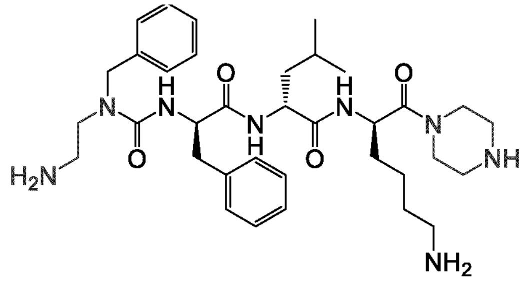

•HCOOH

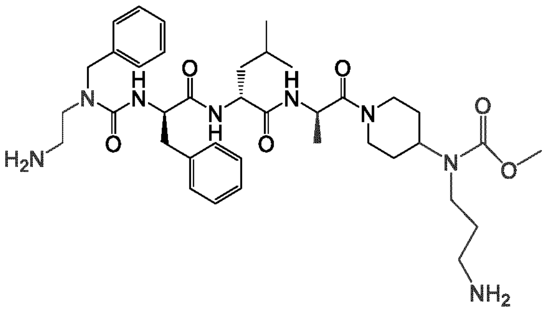

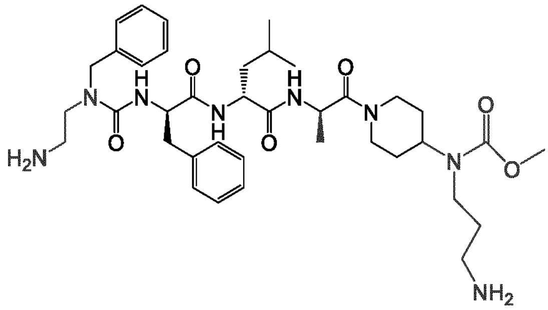

•(HCOOH)2

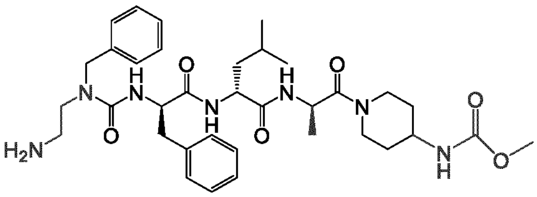

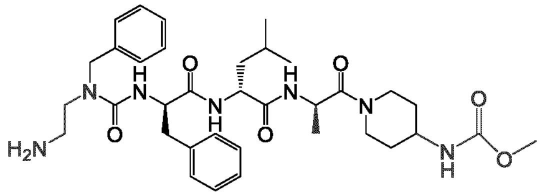

•HCOOH

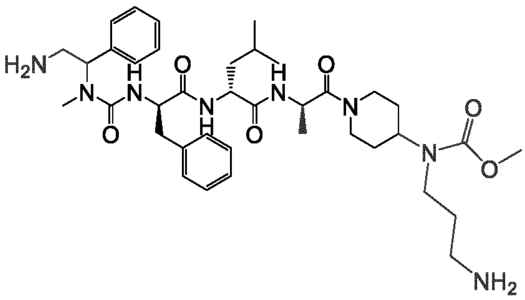

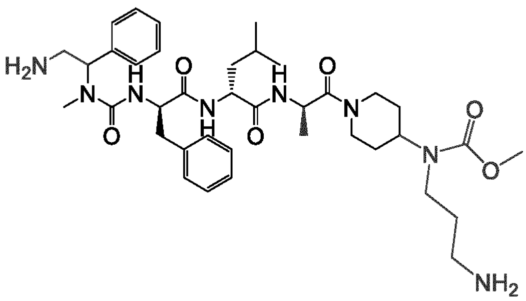

•(HCOOH)2

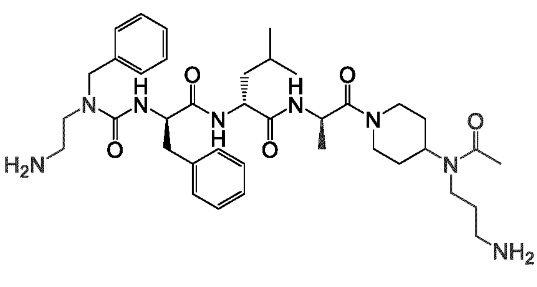

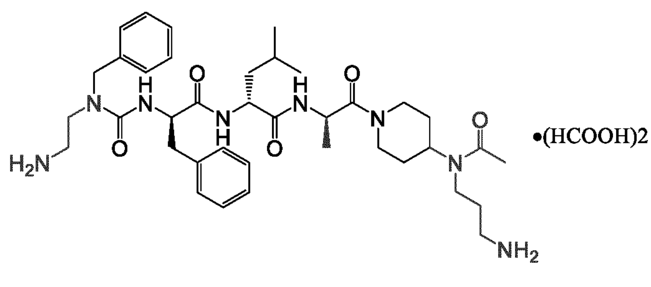

•(HCOOH)2

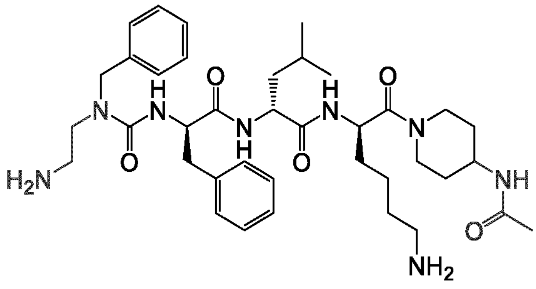

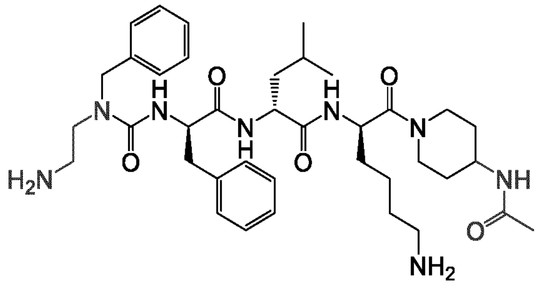

•HCOOH

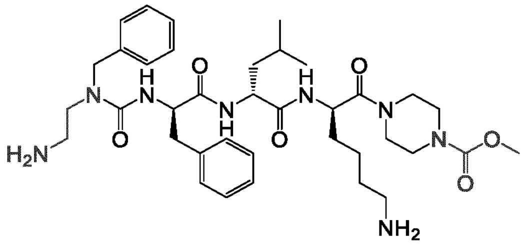

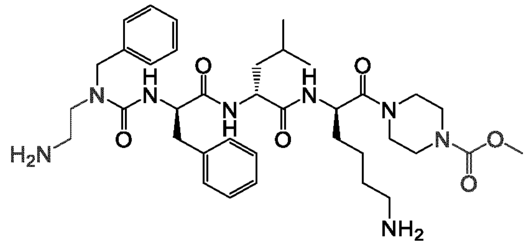

•(HCOOH)2

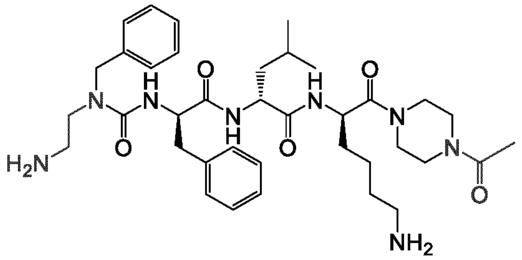

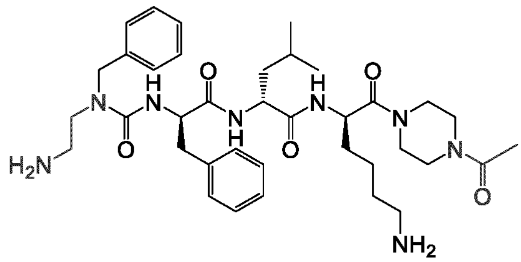

•(HCOOH)2

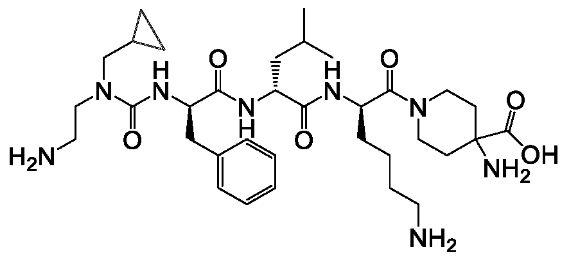

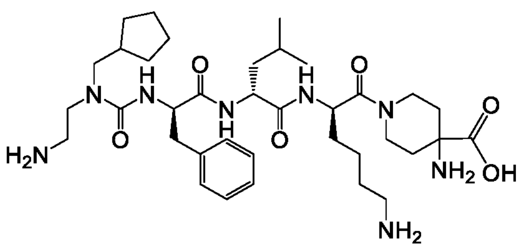

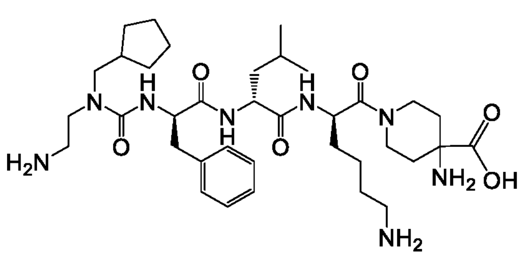

•(HCOOH)2

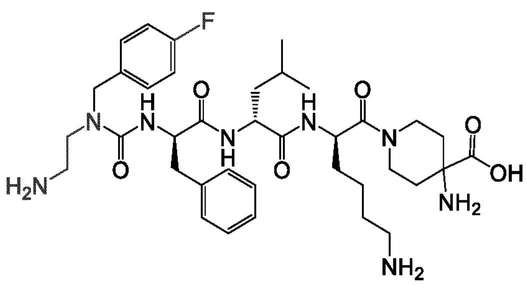

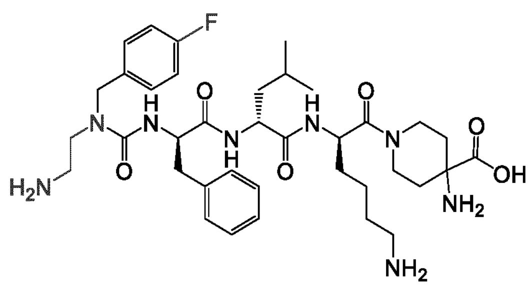

•(HCOOH)2

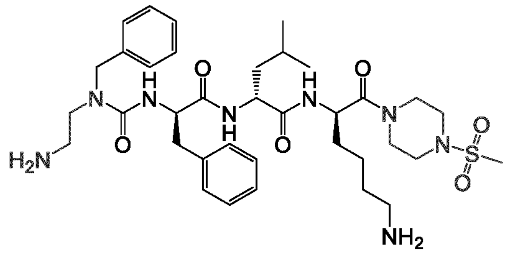

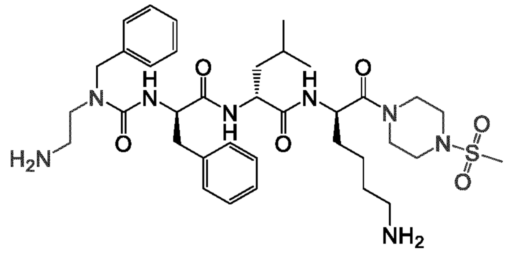

•(HCOOH)2

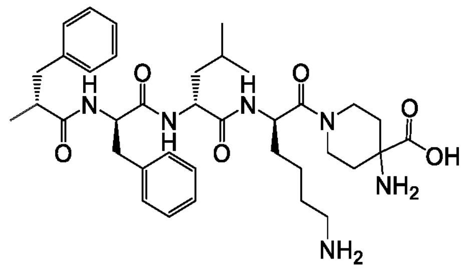

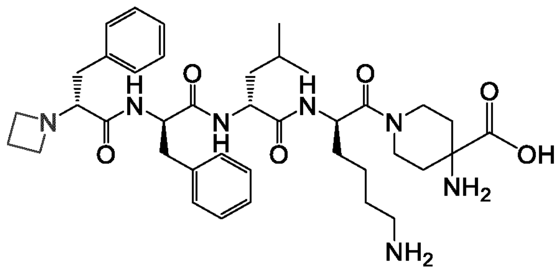

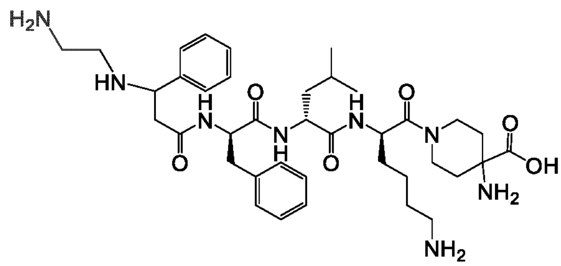

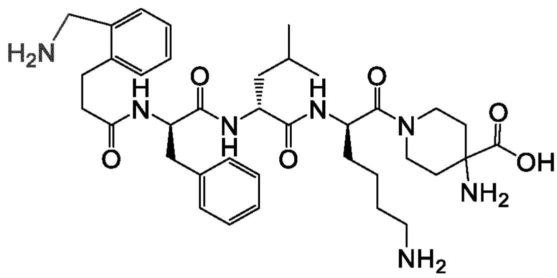

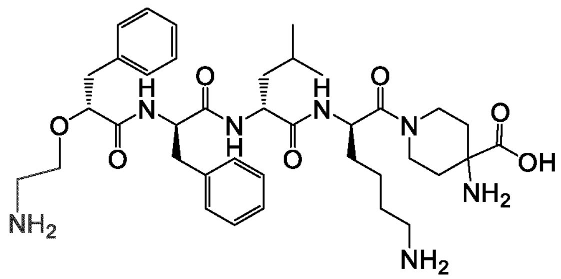

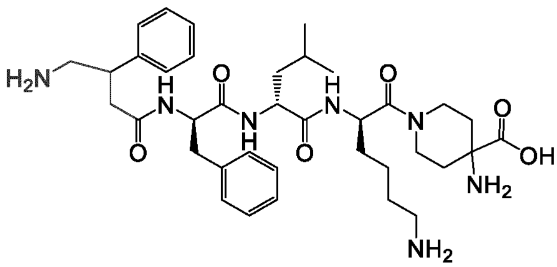

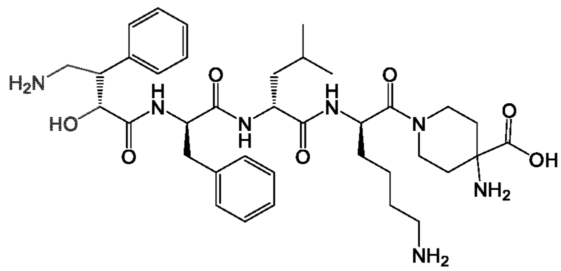

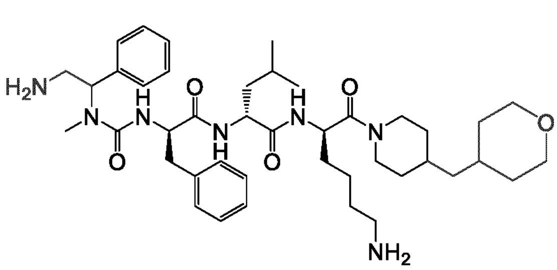

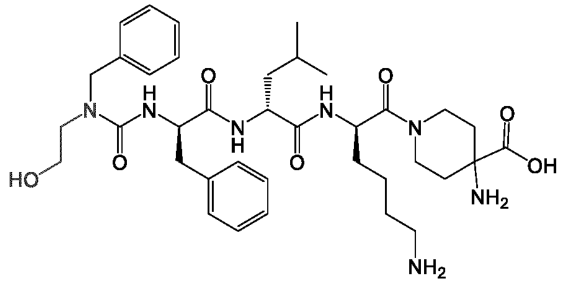

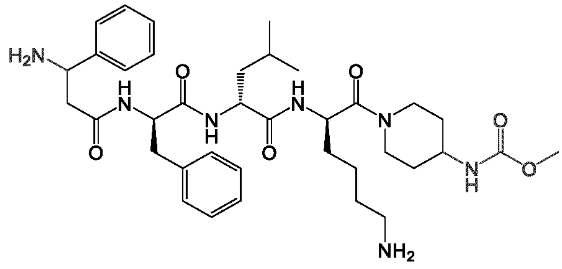

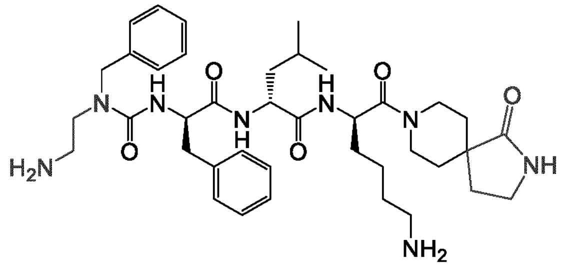

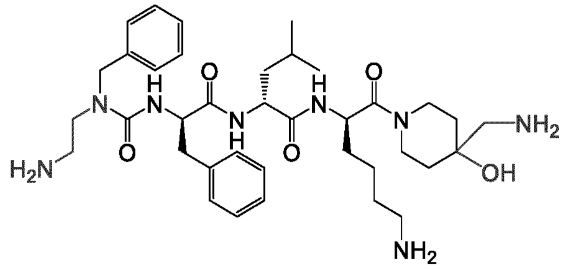

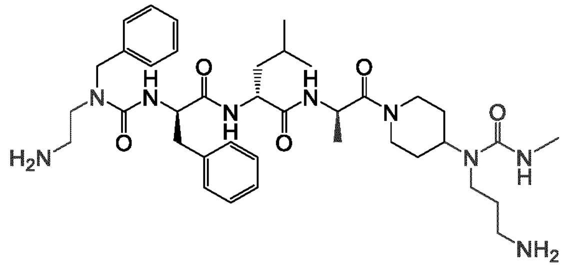

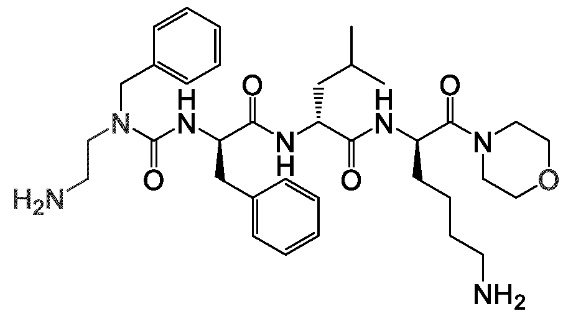

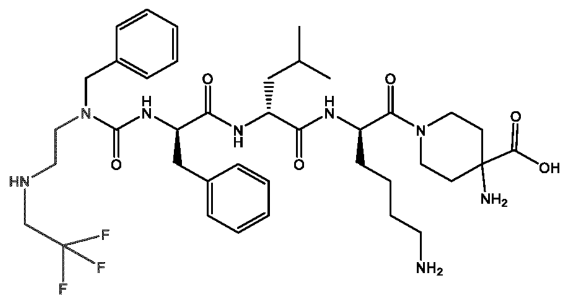

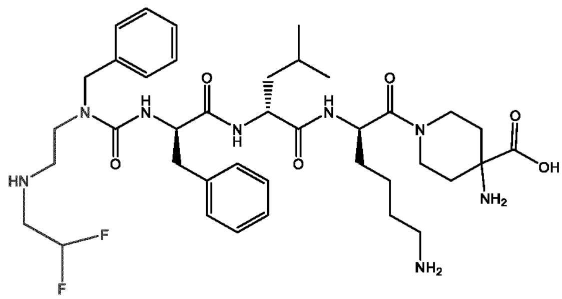

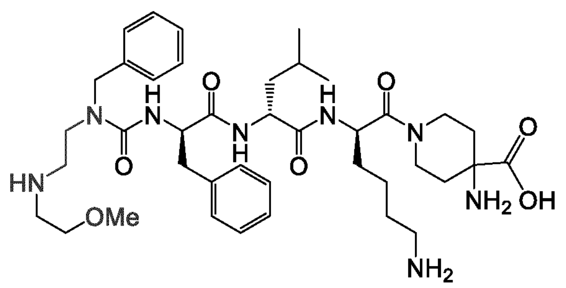

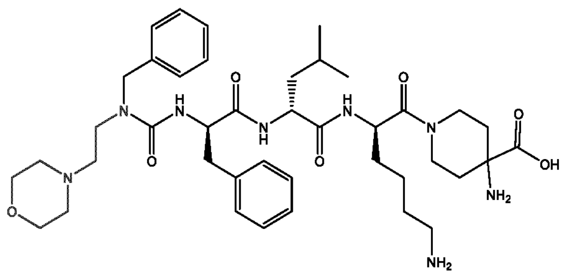

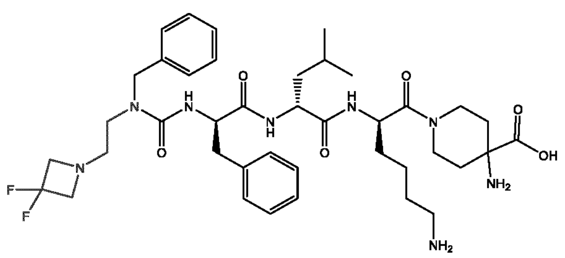

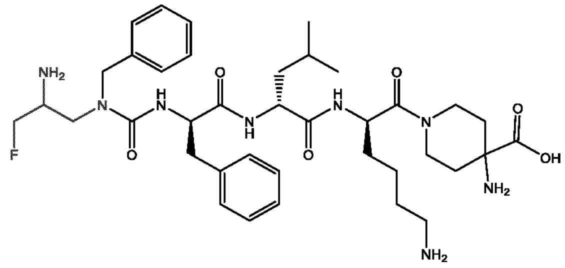

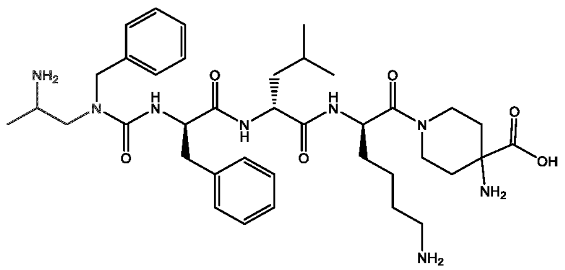

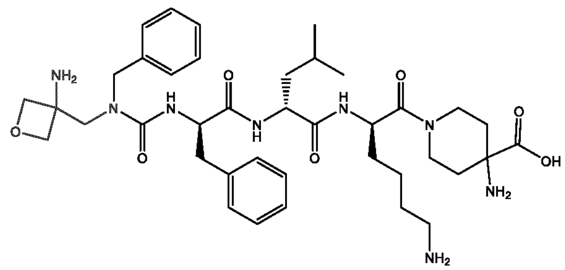

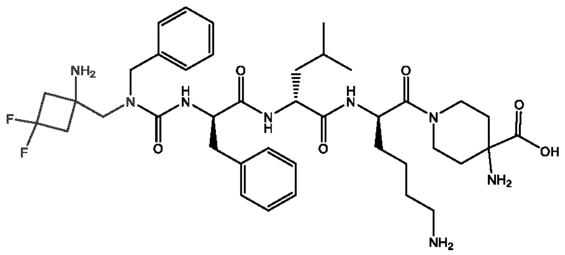

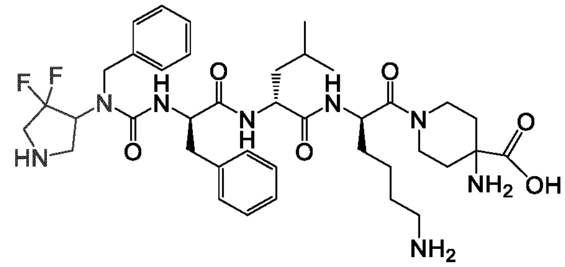

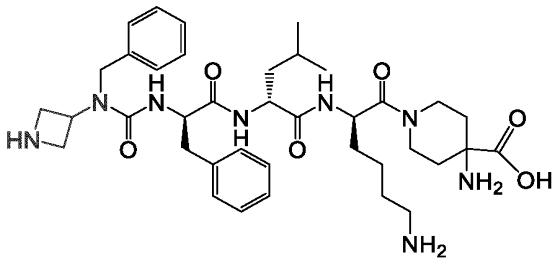

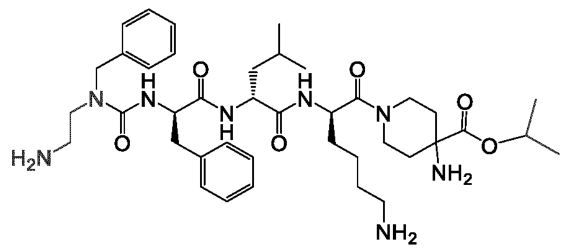

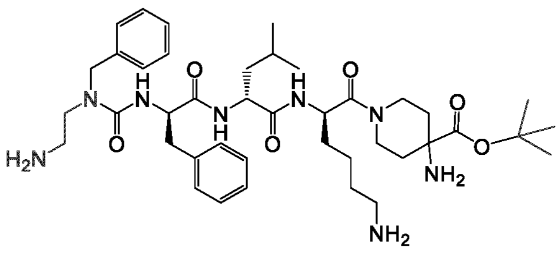

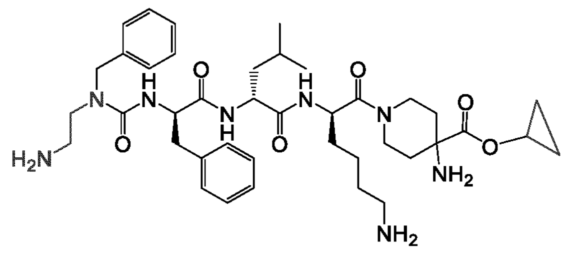

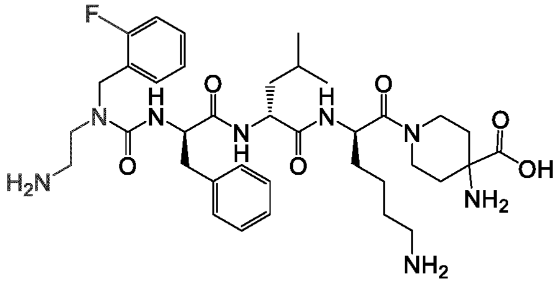

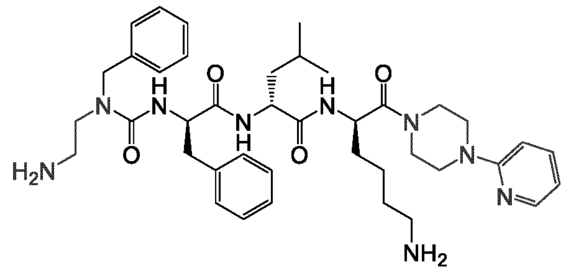

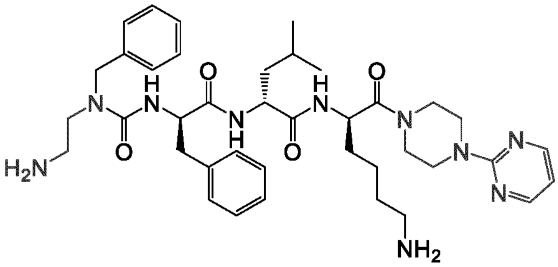

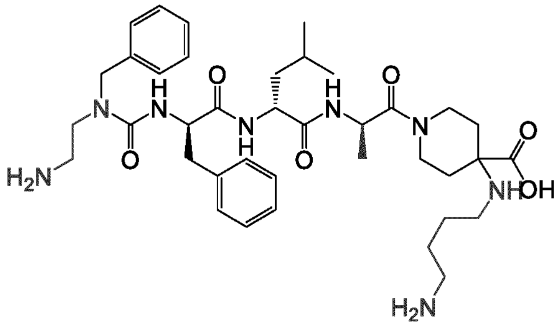

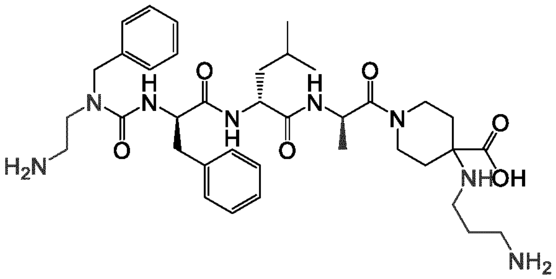

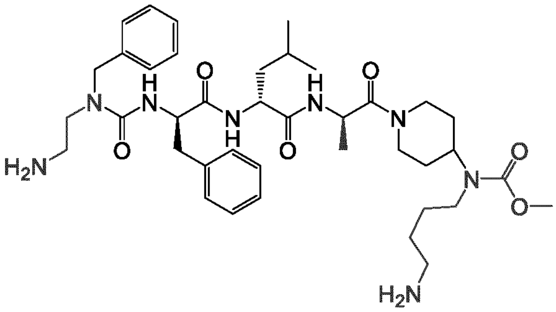

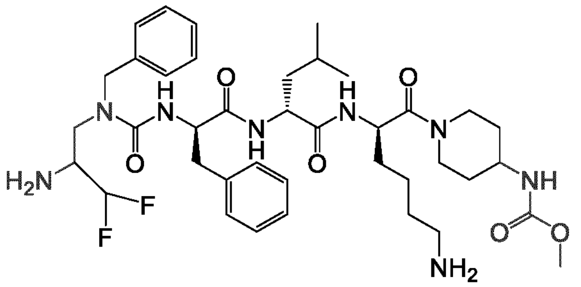

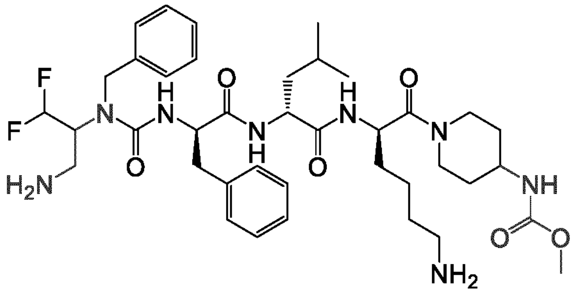

.

The present disclosure further provides a compound of the following general formula, a stereoisomer thereof, or a salt thereof, which is an intermediate for preparing the compounds of the present disclosure described above:

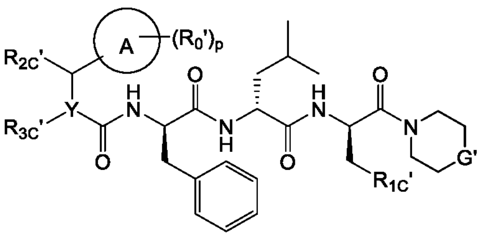

iC-1

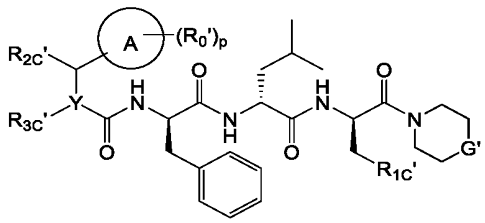

iiC-1

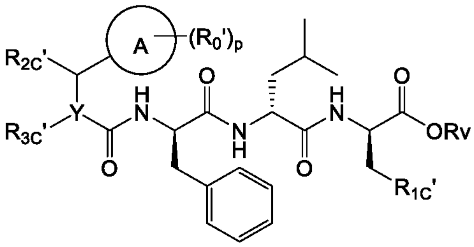

iiC-2-X

wherein, $R_0$' is $R_0$ or $R_0$ in which $NH_2$ is protected with an amino-protecting group, $R_{1C}$' is $R_{1C}$ or $R_{1C}$ in which $NH_2$ is protected with an amino-protecting group, $R_{2C}$' is $R_{2C}$ or $R_{2C}$ in which $NH_2$ is protected with an amino-protecting group, $R_{3C}$' is $R_{3C}$ or $R_{3C}$ in which $NH_2$ or the cyclic imino is protected with an amino-protecting group, G' is G or G in which $NH_2$ and/or the cyclic imino is protected with an amino-protecting group and/or the carboxyl is protected with a carboxyl-protecting group, ring A, Y, $R_0$, $R_{1C}$, $R_{2C}$, $R_{3C}$, G, and p are as defined above, and Rv is selected from H and carboxyl-protecting groups.

In some embodiments, formula iiC-2-X is selected from:

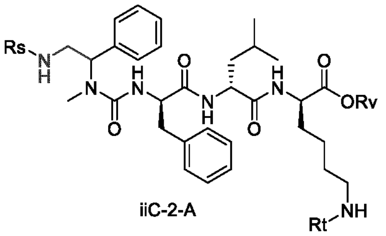
iiC-2-A ,

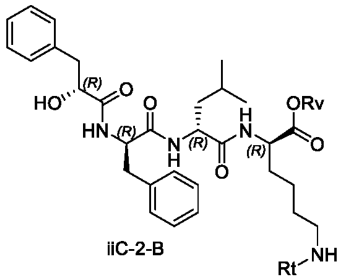
iiC-2-B ,

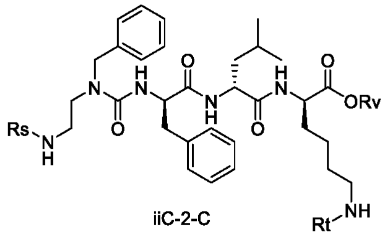
iiC-2-C ,

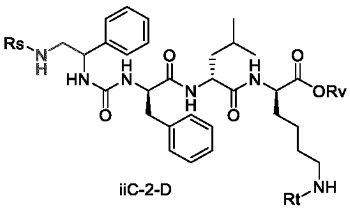
iiC-2-D ,

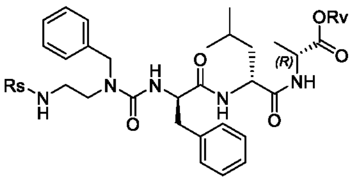
iiC-2-E , and

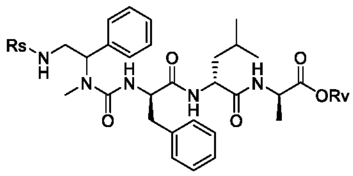
iiC-2-F ,

wherein Rs and Rt are each independently selected from H and amino-protecting groups.

In some embodiments, the amino-protecting groups are each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, *tert*-butylsulfinyl, 1-phenylethyl, or ethoxycarbonyl.

In some embodiments, the carboxyl-protecting groups are each independently selected from $C_{1-6}$ alkyl, allyl, benzyl, 2,4-dimethoxybenzyl, *p*-methoxybenzyl, methoxyethoxymethyl, pentafluorophenyl, and 4-*p*-methylbenzyloxybenzyl.

Preparation method

In some embodiments, the compound of the present invention may be prepared by a method selected from: method I:

method I comprises the following steps: subjecting a compound of formula iC-2 and a compound of formula iC-3-a or iC-3-b or iC-3-c to a condensation reaction to generate a compound of formula iC-1, and then subjecting the compound of formula iC-1 to a protecting group removal reaction to generate a compound of formula IC,

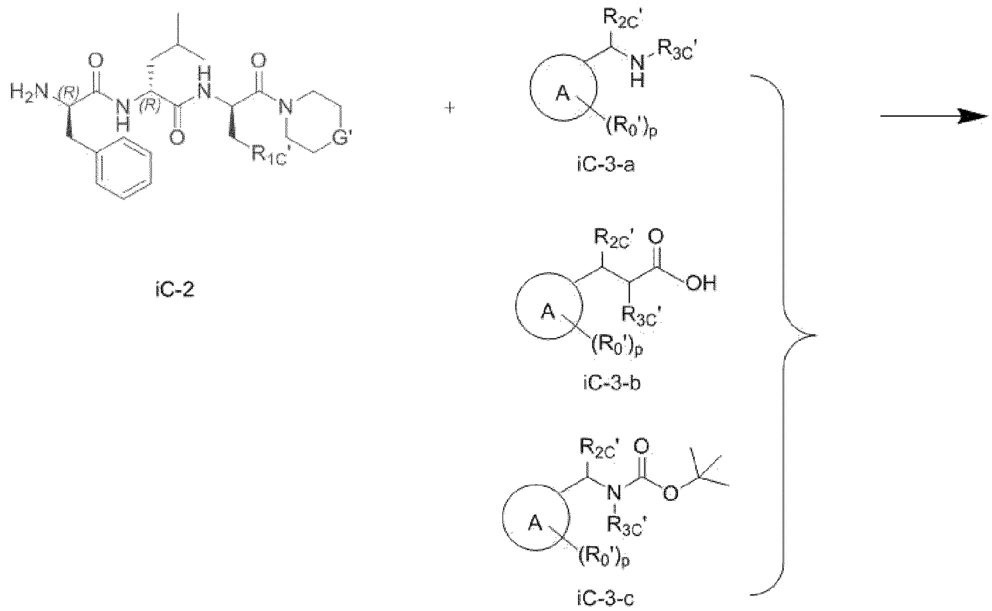

iC-2

iC-3-a

iC-3-b

iC-3-c

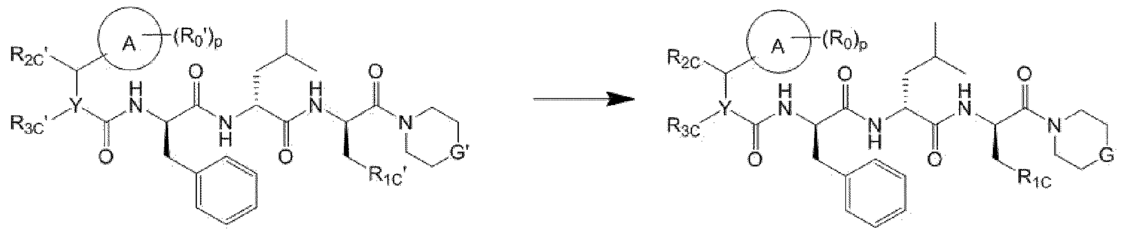

iC-1

IC

,

method II:

method II comprises the following steps: subjecting a compound of formula iiC-2 and a compound of formula iiC-3 to a condensation reaction to generate a compound of formula iiC-1, and then subjecting the compound of iiC-1 to a protecting group removal reaction to generate the compound of formula IC,

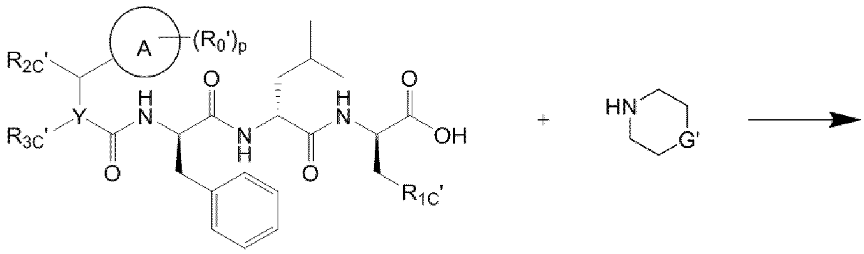

iiC-2

iiC-3

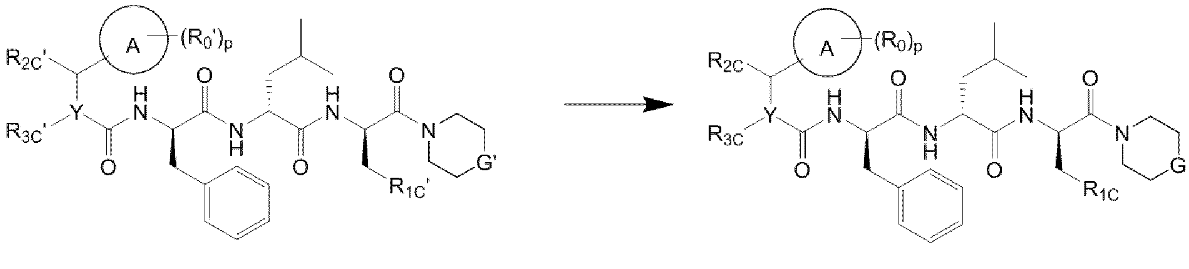

iiC-1

IC

.

Preferably, a compound of formula iiC-2 is prepared by a method selected from:

method III:

method III comprises the following steps: subjecting a compound of formula iiC-4-a and a compound of formula iiC-5-a to a condensation reaction, a hydrolysis reaction, an optional removal reaction of an amino-protecting group, and an amino protection reaction to give the compound of formula iiC-2,

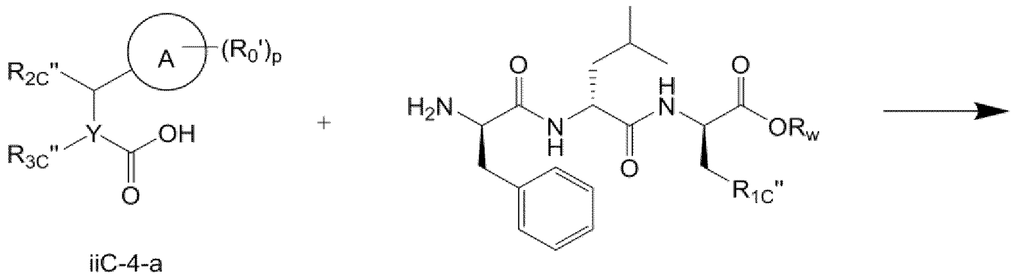

iiC-4-a

iiC-5-a

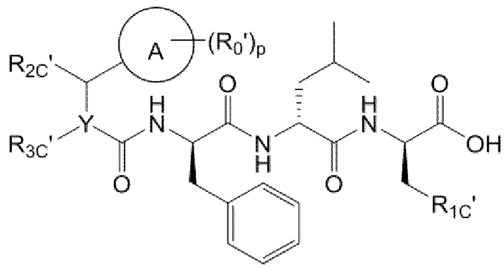

iiC-2

,

method IV:

method IV comprises the following steps: subjecting a compound of formula iiC-4-b and a compound of formula iiC-5-b to a condensation reaction, a hydrolysis reaction, an optional removal reaction of an amino-protecting group, and an amino protection reaction to give the compound of formula iiC-2,

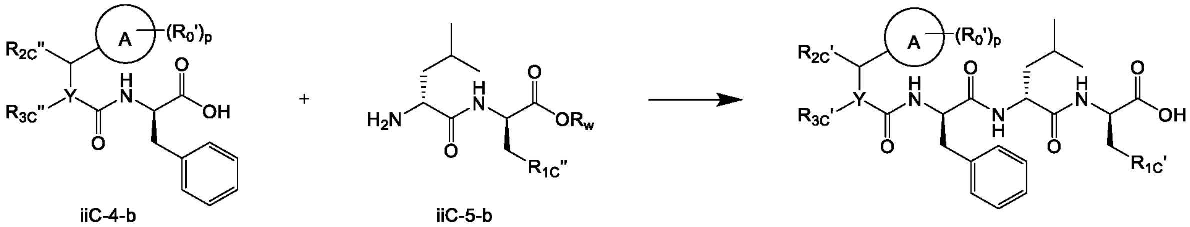

iiC-4-b

iiC-5-b

iiC-2

.

method V:

method V comprises the following steps: subjecting a compound of formula iiC-4-c and a compound of formula iiC-5-c to a condensation reaction, a hydrolysis reaction, an optional removal reaction of an amino-protecting group, and an amino protection reaction to give the compound of formula iiC-2,

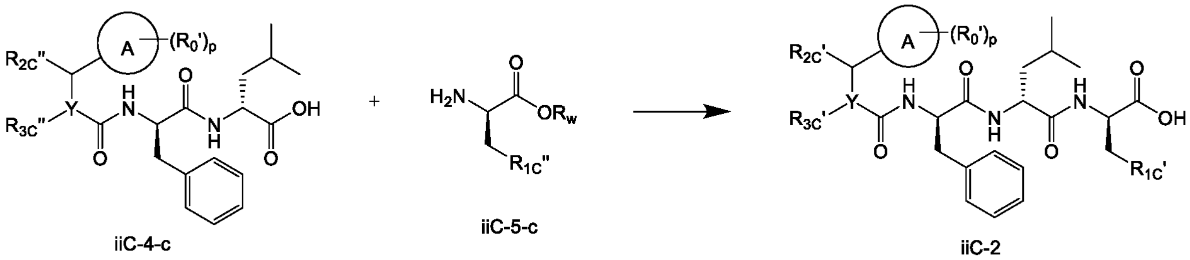

iiC-4-c

iiC-5-c

iiC-2

,

method VI:

method VI comprises the following steps: subjecting a compound of formula iiC-4-d and a compound of formula iiC-5-d to a condensation reaction, a hydrolysis reaction, an optional removal reaction of an amino-protecting group, and an amino protection reaction to give the compound of formula iiC-2,

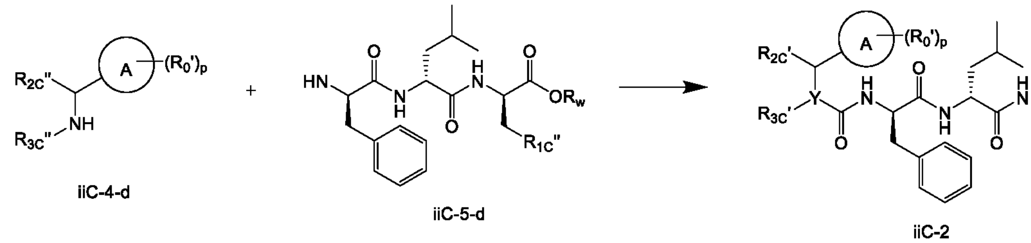

iiC-4-d iiC-5-d iiC-2

,

preferably, the formula iC-2 is

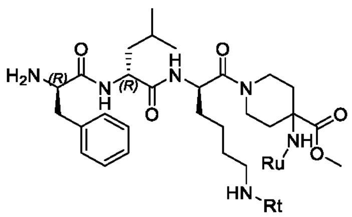

;

preferably, iiC-2 is selected from:

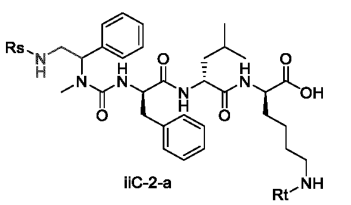

iiC-2-a ,

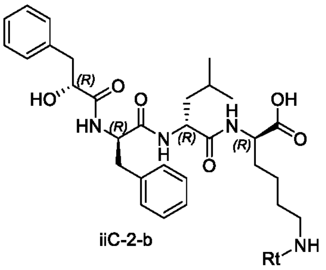

iiC-2-b ,

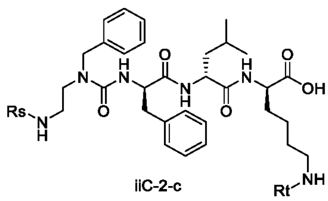

iiC-2-c ,

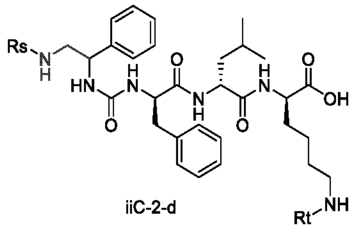

iiC-2-d ,

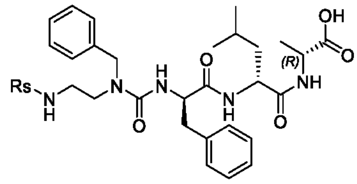

iiC-2-e , and

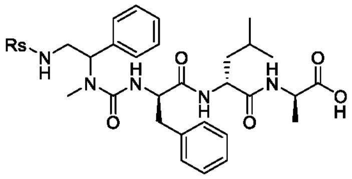

iiC-2-f ,

wherein, $R_0$' is $R_0$ or $R_0$ in which $NH_2$ is protected with an amino-protecting group, $R_{1C}$' and $R_{1C}$" are $R_{1C}$ or $R_{1C}$ in which $NH_2$ is protected with an amino-protecting group, $R_{2C}$' and $R_{2C}$" are $R_{2C}$ or $R_{2C}$ in which $NH_2$ is protected with an amino-protecting group, $R_{3C}$' and $R_{3C}$" are $R_{3C}$ or $R_{3C}$ in which $NH_2$ or the cyclic imino is protected with an amino-protecting group, G' is G or G in which $NH_2$ and/or the cyclic imino is protected with an amino-protecting group and/or the carboxyl is protected with a carboxyl-protecting group, Rw is a carboxyl-protecting group, ring A, Y, $R_0$, $R_{1C}$, $R_{2C}$, $R_{3C}$, G, and p are as defined above, and Rs, Rt, and Ru are each independently selected from H and amino-protecting groups;

preferably, the amino-protecting groups are each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, *tert*-butylsulfinyl, 1-phenylethyl, or ethoxycarbonyl; the carboxyl-protecting groups are each independently selected from $C_{1-6}$ alkyl, allyl, benzyl, 2,4-dimethoxybenzyl, *p*-methoxybenzyl, methoxyethoxymethyl, pentafluorophenyl, and 4-p-methylbenzyloxybenzyl.

In method III, method IV, method V, and method VI described above, the condensation reaction refers to a

condensation reaction of iiC-4 (specifically iiC-4-a, iiC-4-b, iiC-4-c, and iiC-4-d) with iiC-5 (specifically iiC-5-a, iiC-5-b, iiC-5-c, and iiC-5-d), respectively to form a condensation product; the hydrolysis reaction is intended to remove the carboxyl-protecting group Rw in the condensation product, but according to different hydrolysis conditions, the amino-protecting group (if present) in the condensation product may be partially or completely removed while the carboxyl-protecting group Rw in the condensation product is removed, and the product after the hydrolysis reaction is referred to as a hydrolysis product. In some embodiments, the hydrolysis product is the compound of iiC-2. In some embodiments, the hydrolysis product is further subjected to an amino protection reaction to give the compound of formula iiC-2. In some embodiments, the hydrolysis product is further subjected to a removal reaction of an amino-protecting group and an amino protection reaction to give the compound of formula iiC-2.

## EXAMPLES

Examples of the present invention are described in detail below. The following examples are exemplary and illustrative only, and should not be construed as limiting the present invention. Unless otherwise indicated, the proportions, percentages, and the like referred to herein are calculated by weight.

Reagents or instruments without specified manufacturers used herein are conventional products that are commercially available. Specifically, intermediate 2 (the structure is shown below) was purchased from HANGZHOU TAIJIA BIOTECH CO., LTD.

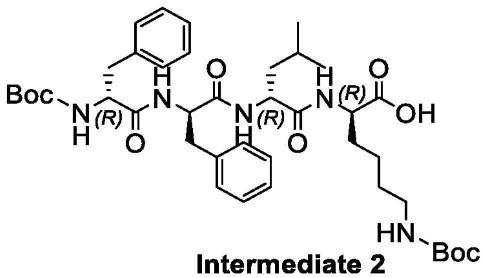

**Intermediate 2**

In addition, with respect to the purification of the final product, in each example, it is mentioned as "purified by reversed-phase flash chromatography", "purified by preparative HPLC", "purified by reversed-phase column chromatography", "purified by high-performance liquid chromatography", and "purified by a reversed-phase column". In some examples, the mobile phase conditions used for the purification are specifically indicated as "solvent ACN and solvent $H_2O$ (FA, 0.1%)", but it should be understood that the same or similar mobile phase conditions are also used in other examples. Under such chromatographic conditions, the compounds in certain examples are formed as formate salts. However, it should be understood by those skilled in the art that the free compounds may be formed by conventional neutralization reaction conditions in the art.

Abbreviations:

ACN: acetonitrile
BTC: (trichloromethyl)carbonate
CDI: N,N-carbonyl diimidazole
DCM: dichloromethane
DIEA: diethylamine
DIPEA: N,N-diisopropylethylamine
DMAP: 4-dimethylaminopyridine
DMF: dimethylformamide
HATU: 2-(7-azabenzotriazole)-N,N,N',N'-tetramethyluronium hexafluorophosphate
HOBT: 1-hydroxybenzotriazole
TBTU: O-benzotriazole-N,N,N',N'-tetramethyluronium tetrafluoroborate
TEA: triethylamine
TFA: trifluoroacetic acid
TFE: 2,2,2-trifluoroethanol
THF: tetrahydrofuran

Example 1. Synthesis of Compound 1

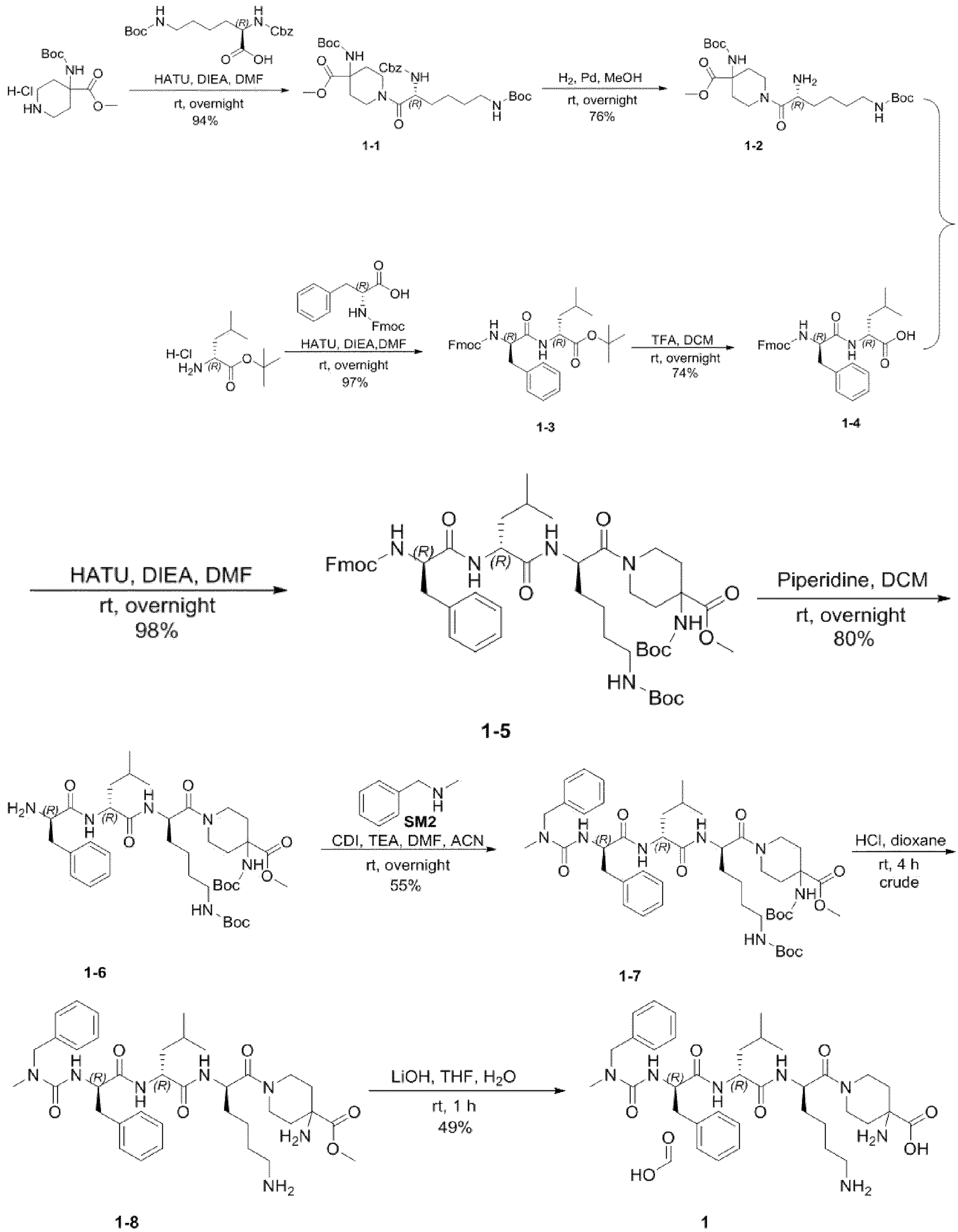

1.1 Synthesis of compound 1-1

Methyl 4-[(tert-butoxycarbonyl)amino]piperidine-4-carboxylate hydrochloride (3.7 g, 12.552 mmol, 1.00 equiv) was dissolved in DMF (37 mL) at room temperature, and DIEA (4.87 g, 37.656 mmol, 3 equiv), (2R)-2-{[(benzyloxy) carbonyl]amino}-6-[(tert-butoxycarbonyl)amino]hexanoic acid (5.73 g, 15.062 mmol, 1.2 equiv) and HATU (5.73 g, 15.062 mmol, 1.2 equiv) were separately added. The resulting mixture was stirred at room temperature overnight. The resulting mixture was extracted with dichloromethane (2 × 200 mL). The combined organic layer was washed with brine (2 × 300 mL), dried over anhydrous sodium sulfate, and filtered. The filtrate was concentrated under reduced pressure. The residue was purified by silica gel column chromatography and eluted with dichloromethane and methanol (20:1) to give methyl 1-[(2R)-2-{ [(benzyloxy)carbonyl] amino }-6-[(*tert*-butoxycarbonyl)amino]hexanoyl]-4-[(*tert*-butoxycarbonyl)amino]piperidine-4-carboxylate (8.97 g, 94.63%, purity: 82.0%) as an off-white solid.
LC-MS-1-1 (ES, m/z): [M+1] = 621

1.2 Synthesis of compound 1-3

*tert-Butyl* (2R)-2-amino-4-methylpentanoate hydrochloride (5 g, 22.347 mmol, 1.00 equiv) and DIEA (8.66 g, 67.041 mmol, 3 equiv) were dissolved in DMF (50.00 mL, 646.052 mmol, 28.91 equiv), and (2R)-2-{[(9H-fluoren-9-ylmethoxy)carbonyl]amino }-3-phenylpropionic acid (10.39 g, 26.816 mmol, 1.2 equiv) and HATU (10.20 g, 26.816 mmol, 1.2 equiv) were added. The resulting mixture was stirred at room temperature overnight. The resulting mixture was extracted with ethyl acetate (1 × 500 mL). The combined organic layer was washed with brine (5 × 300 mL), dried over anhydrous sodium sulfate, and filtered. The filtrate was concentrated under reduced pressure. The residue was purified by silica gel column chromatography and eluted with petroleum ether/ethyl acetate (4:1) to give *tert*-butyl (2R)-2-r(2R)-2-{[(9H-fluoren-9-ylmethoxy)carbonyl]amino}-3-phenylpropionylamino]-4-methylpentanoate (13.94 g, 97.60%, purity: 87.0%) as a white solid.
LC-MS-1-3 (ES, m/z): [M+1] = 557

1.3 Synthesis of compound 1-2

Methyl 1-[(2R)-2-{[(benzyloxy)carbonyl]amino}-6-[(*tert*-butoxycarbonyl)amino]hexanoyl]-4-[(*tert*-butoxycarbonyl)amino]piperidine-4-carboxylate (8.97 g, 14.450 mmol, 1.00 equiv) was dissolved in methanol (162.35 mL), and Pd/C (922.69 mg, w/t, 10%) was added. The resulting mixture was stirred at room temperature overnight under hydrogen atmosphere. The resulting mixture was filtered, and the filter cake was washed with methanol (3 × 20 mL). The filtrate was concentrated under reduced pressure. The residue was purified by reverse flash chromatography to give methyl 1-[(2R)-2-amino-6-[(*tert*-butoxycarbonyl)amino]hexanoyl]-4-[(*tert*-butoxycarbonyl)amino]piperidine-4-carboxylate (5.65 g, 76.53%, purity: 95.0%).
LC-MS-1-2 (ES, m/z): [M+1] = 487

1.4 Synthesis of compound 1-4

*tert*-Butyl (2R)-2-[(2R)-2-{[(9H-fluoren-9-ylmethoxy)carbonyl]amino}-3-phenylpropionylamino]-4-methylpentanoate (13.94 g, 25.040 mmol) was dissolved in DCM (100 mL, 1573.005 mmol, 62.82 equiv.), and TFA (1.00 mL, 1346.304 mmol, 53.77 equiv) was added. The resulting mixture was stirred at room temperature overnight. The resulting mixture was concentrated under reduced pressure. The crude product was recrystallized from dichloromethane/1 M hydrochloric acid in water, washed with water (1 × 400 mL), and filtered. The solid was collected to give (2R)-2-[(2R)-2-{[(9H-fluoren-9-ylmethoxy)carbonyl]amino}-3-phenylpropionylamino]-4-methylpentanoic acid (9.43 g, 74.48%, purity: 98.8%).
LC-MS-1-4 (ES, m/z): [M+1] = 501

1.5 Synthesis of compound 1-5

(2R)-2-[(2R)-2-{[(9H-Fluoren-9-ylmethoxy)carbonyl]amino}-3-phenylproplonylamino]-4-methylpentanoic acid (4.78 g, 9.539 mmol, 1.1 equiv) and DIEA (3.36 g, 26.016 mmol, 3 equiv) were dissolved in DMF (40 mL, 516.870 mmol, 59.60 equiv), and methyl 1-[(2R)-2-amino-6-[(*tert*-butoxycarbonyl)amino]hexanoyl]-4-[(*tert*-butoxycarbonyl)amino]piperidine-4-carboxylate (4.22 g, 8.672 mmol, 1.00 equiv) and HATU (3.96 g, 10.406 mmol, 1.2 equiv) were added. The resulting mixture was stirred at room temperature overnight. The resulting mixture was extracted with ethyl acetate (1 × 400 mL). The combined organic layer was washed with brine (3 × 200 mL), dried over anhydrous sodium sulfate, and filtered. The filtrate was concentrated under reduced pressure. The residue was purified by silica gel column chromatography and eluted with petroleum ether/ethyl acetate (1:2), and the fractions were concentrated to dryness by rotary

evaporation to give methyl 4-[(*tert*-butoxycarbonyl)amino]-1-[(2R)-6-[(*tert*-butoxycarbonyl)amino]]-2-[(2R)-2-[(2R)-2-{[(9H-fluoren-9-ylmethoxy) carbonyl]amino}-3-phenylpropionylamino]-4-methylpentanoylamino] hexanoyl]piperidine-4-carboxylate (8.39 g, 98.12%, purity: 98.3%).
LC-MS-1-5 (ES, m/z): [M+1] = 969

1.6 Synthesis of compound 1-6

Methyl 4-[(*tert*-butoxycarbonyl)amino]-1-[(2R)-6-[(*tert*-butoxycarbonyl)amino]-2-[(2R)-2-[(2R)-2]-{[(9H-fluoren-9-ylmethoxy)carbonyl]amino}-3-phenylpropionylamino]-4-methylpentanoylamino]hexanoyl]piperidine-4-carboxylate (8.93 g, 9.214 mmol, 1.00 equiv) was dissolved in DCM (80 mL), and piperidine (8.90 mL, 104.487 mmol, 11.34 equiv) was added portionwise. The resulting mixture was stirred at room temperature overnight. The resulting mixture was concentrated under reduced pressure. The residue was purified by reversed-phase flash chromatography to give methyl 1-[(2R)-2-[(2R)-2-[(2R)-2-amino-3-phenylpropionylamino]-4-methylpentanoylamino]-6-[(*tert*-butoxycarbonyl)amino] hexanoyl]-4-[(*tert*-butoxycarbonyl)amino]piperidine-4-carboxylate (5.74 g, 80.98%, purity: purity: 97.0%).
LC-MS-1-6 (ES, m/z): [M+1] = 747 1.7 Synthesis of compound 1-7
Carbonylimidazole (47.76 mg, 0.295 mmol, 1.1 equiv) was dissolved in N,N-dimethylformamide (1.60 mL), and methyl 1-[(2R)-2-[(2R)-2-[(2R)-2-amino-3-phenylpropionylamino]-4-methylpentanoylamino]-6-[(*tert*-butoxycarbonyl) amino]hexanoyl]-4-[(*tert*-butoxycarbonyl)amino]piperidine-4-carboxylate (200 mg, 0.268 mmol, 1.00 equiv), N-methylbenzylamine (32.45 mg, 0.268 mmol, 1 equiv) and triethylamine (54.19 mg, 0.536 mmol, 2 equiv) were added under stirring. The resulting mixture was stirred at room temperature overnight and concentrated under reduced pressure. The residue was purified by reversed-phase flash chromatography, and the fractions were concentrated to dryness by rotary evaporation to give methyl 1-[(2R)-2-[(2R)-2-[(2R)-2-{[benzyl(methyl)carbamoyl]amino}-3-phenylpropionylamino]-4-methylpentanoylamino]-6-[(*tert*-butoxycarbonyl)amino]hexanoyl]-4-[(*tert*-butoxycarbonyl)amino]piperidine-4-carboxylate (136 mg, 55.67%, purity: 66.7%).
LC-MS-1-7 (ES, m/z): [M+1] = 894

1.8 Synthesis of compound 1-8

Methyl 1-[(2R)-2-[(2R)-2-[(2R)-2-{[benzyl(methyl)carbamoyl]amino}-3-phenylpropionylamino]-4-methylpentanoylamino]-6-[(*tert*-butoxycarbonyl)amino]hexanoyl]-4-[(*tert*-butoxycarbonyl)amino]piperidine-4-carboxylate (136 mg, 0.152 mmol, 1.00 equiv) was dissolved in dioxane (10.00 mL), and a 4 M solution of HCl (gas) in 1,4-dioxane (15.0 mL) was added portionwise at room temperature. The resulting mixture was stirred at room temperature for 4 h. The reaction solution was concentrated under reduced pressure to give methyl 4-amino-1-[(2R)-6-amino-2-[(2R)-2-[(2R)-2-{[benzyl(methyl)carbamoyl]amino}-3-phenylpropionylamino]-4-methylpentanoylamino]hexanoyl] piperidine-4-carboxylate (115 mg, crude).
LC-MS-1-8 (ES, m/z): [M+1] = 694

1.9 Synthesis of compound 1

Methyl 4-amino-1-[(2R)-6-amino-2-[(2R)-2-[(2R)-2-{[benzyl(methyl)carbamoyl]amino}-3-phenylpropionylamino]-4-methylpentanoylamino]hexanoyl]piperidine-4-carboxylate (115 mg, 0.166 mmol, 1.00 equiv) was dissolved in a mixed solvent THF (10 mL)/water (2 mL), and $LiOH.H_2O$ (13.933 mg, 0.332 mmol, 2.00 equiv) was added. The resulting mixture was stirred at room temperature for 1 h. The mixture was acidified with 1 M HCl (aq.) to pH = 7. The resulting mixture was purified by reversed-phase flash chromatography to give 4-amino-1-[(2R)-6-amino-2-[(2R)-2-[(2R)-2-{[benzyl(methyl)carbamoyl]amino}-3-phenylpropionylamino]-4-methylpentanoylamino]hexanoyl]piperidine-4-carboxylic acid (60.0 mg, 49.23%, purity: 95.2%).
LC-MS-1 (ES, m/z): [M+1] = 680
$^1$H NMR-1 (400 MHz, Deuterium Oxide) δ 8.38(s, 1H), 7.36 - 7.22 (m, 6H), 7.18 (dd, *J* = 7.6, 2.0 Hz, 2H), 7.03 - 6.95 (m, 2H), 4.76 - 4.72 (m, 1H), 4.50 (ddd, *J* = 9.0, 5.7, 3.0 Hz, 1H), 4.43 (d, *J* = 16.5 Hz, 1H), 4.38 - 4.25 (m, 2H), 3.88 - 3.47 (m, 4H), 3.11 (dd, *J* = 13.9, 5.8 Hz, 1H), 2.97 - 2.85 (m, 3H), 2.77 (d, *J* = 2.9 Hz, 3H), 2.27 - 2.06 (m, 2H), 1.93 - 1.42 (m, 9H), 1.42 - 1.23 (m, 2H), 0.92 - 0.77 (m, 6H).

Example 2. Synthesis of Compound 2

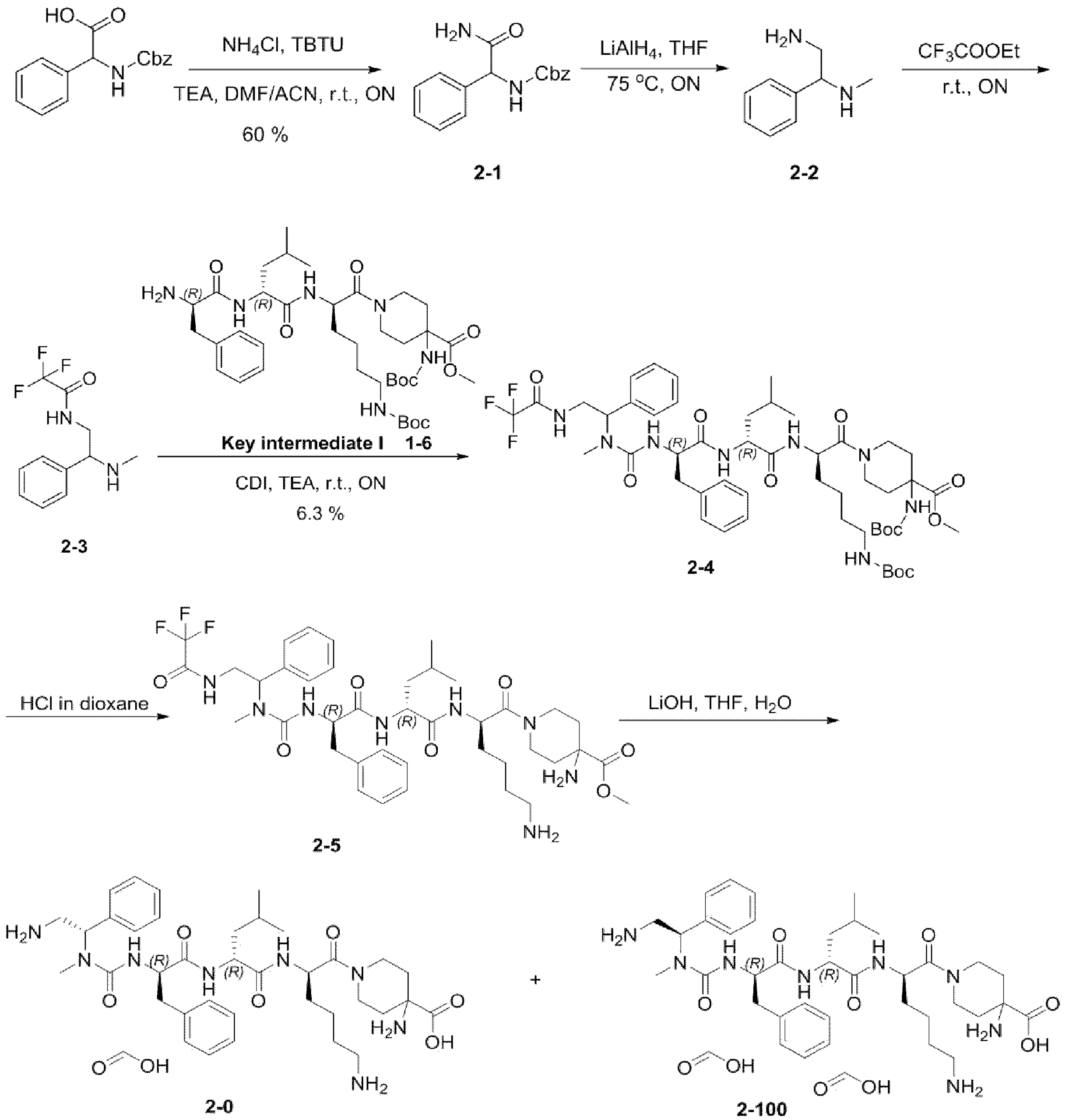


2-1 2-2 2-3 2-4 2-5 2-0 2-100

**LC-MS-2-0** (ES, m/z): [M+1] = 710

$^1$H NMR (400 MHz, Deuterium Oxide) δ 8.30 (s, 1H), 7.51 - 7.20 (m, 8H), 7.18 - 6.96 (m, 2H), 5.52 (ddd, *J* = 19.0, 10.7, 4.7 Hz, 1H), 4.45 (dt, *J* = 8.9, 6.2 Hz, 1H), 4.30 (p, *J* = 4.7 Hz, 1H), 3.75 (dp, *J* = 18.4, 6.9 Hz, 2H), 3.68 - 3.38 (m, 4H), 3.10 (ddd, *J* = 14.6, 9.2, 6.3 Hz, 1H), 2.97 (dt, *J* = 13.9, 10.1 Hz, 1H), 2.87 (q, *J* = 7.8 Hz, 2H), 2.53 (d, *J* = 5.4 Hz, 3H), 2.30 - 2.05 (m, 2H), 1.96 - 1.43 (m, 9H), 1.32 (d, *J* = 20.6 Hz, 2H), 0.85 (dd, *J* = 16.8, 5.6 Hz, 6H).

**LC-MS-2-100** (ES, m/z): [M+1] = 710

$^1$H NMR (400 MHz, Deuterium Oxide) δ 8.35 (s, 2H), 7.50 - 7.10 (m, 8H), 7.03 - 6.72 (m, 2H), 5.53 (dd, *J* = 11.1, 4.2 Hz, 1H), 4.86 - 4.74 (m, 1H), 4.72 - 4.59 (m, 1H), 4.35 (dt, *J* = 9.3, 5.2 Hz, 1H), 4.06 - 3.66 (m, 3H), 3.68 - 3.37 (m, 3H), 3.34 - 3.14 (m, 1H), 3.07 - 2.73 (m, 3H), 2.43 (d, *J* = 6.2 Hz, 3H), 2.31 - 2.01 (m, 2H), 1.97 - 1.21 (m, 11H), 0.85 (dd, *J* = 16.8, 5.6 Hz, 6H).

Example 3. Synthesis of Compound 3

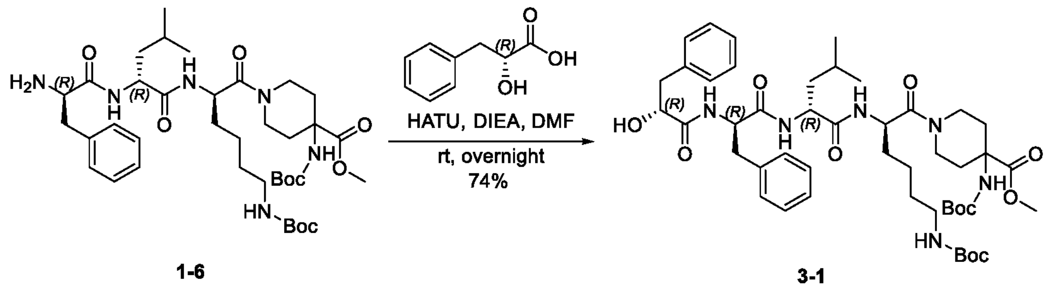


1-6

3-1

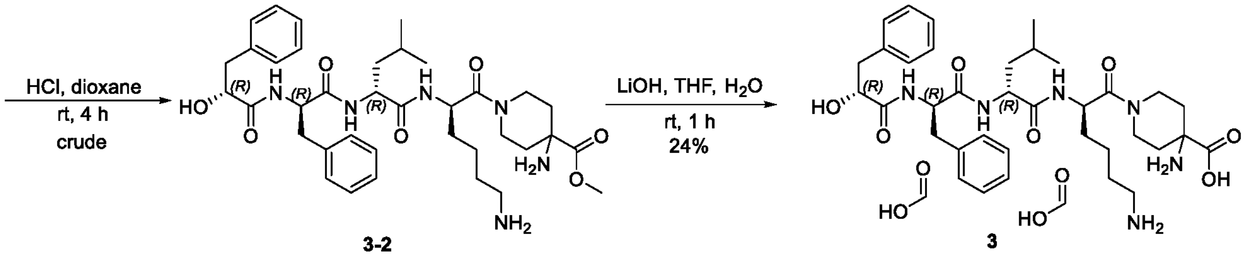


3-2

3

LC-MS-3 (ES, m/z): [M+1] = 681

$^1$H NMR-3 (400 MHz, Deuterium Oxide) δ 8.34 (s, 2H), 7.34 - 7.19 (m, 6H), 7.11 (dt, *J* = 7.3, 2.2 Hz, 2H), 7.09 - 7.01 (m, 2H), 4.53 (td, *J* = 6.9, 6.1, 3.1 Hz, 1H), 4.33 (ddd, *J* = 7.0, 4.9, 1.9 Hz, 1H), 4.30 - 4.21 (m, 1H), 3.88 - 3.46 (m, 4H), 3.01 - 2.82 (m, 5H), 2.75 (ddd, *J* = 14.1, 7.1, 4.5 Hz, 1H), 2.28 - 2.04 (m, 2H), 1.95 - 1.55 (m, 6H), 1.57 - 1.26 (m, 5H), 0.82 (dd, *J* = 19.0, 5.2 Hz, 6H).

Example 4. Synthesis of Compound 4

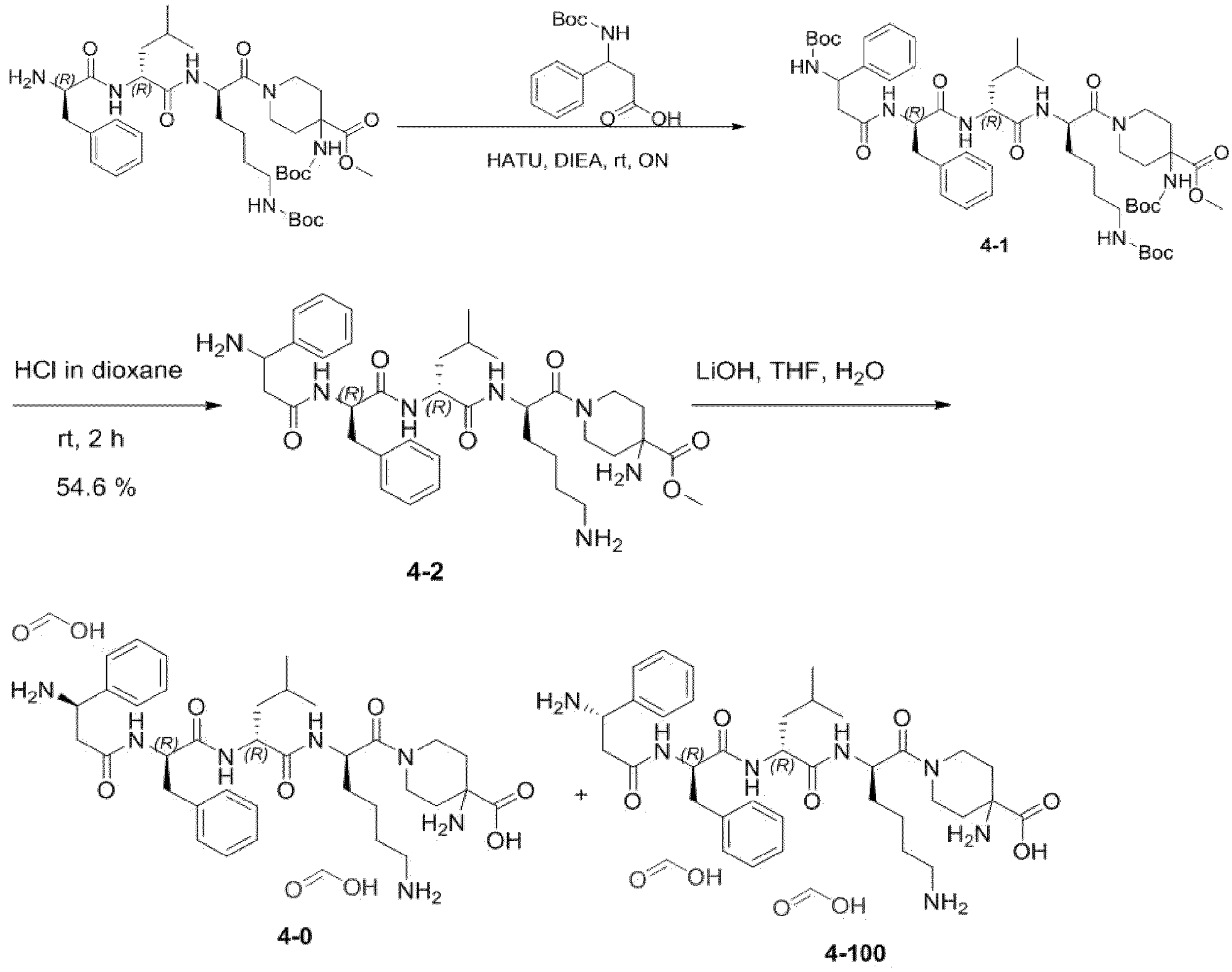


4.1 Synthesis of compound 4-1

Methyl 1-[(2R)-2-[(2R)-2-[(2R)-2-amino-3-phenylpropionylamino]-4-methylpentanoylamino]-6-[(*tert*-butoxycarbonyl)amino]hexanoyl]-4-[(*tert*-butoxycarbonyl)amino]piperidine-4-carboxylate (120 mg, 0.161 mmol, 1.00 equiv) was dissolved in DMF (7 mL), and 3-[(*tert*-butoxycarbonyl)amino]-3-phenylpropionic acid (51.15 mg, 0.193 mmol, 1.2 equiv), HATU (73.30 mg, 0.193 mmol, 1.2 equiv) and DIEA (62.29 mg, 0.483 mmol, 3 equiv) were added. The resulting mixture was stirred overnight, diluted with water (30 mL), and extracted with EA (3 × 20 mL). The combined organic layer was dried over anhydrous sodium sulfate and concentrated under reduced pressure to give methyl 4-[(tert-butoxycarbonyl)amino]-1-[(2R)-6-[(*tert*-butoxycarbonyl)amino]-2-[(2R)-2-[(2R)-2-{3-[(*tert*-butoxycarbonyl)amino]-3-phenylpropionylamino}-3-phenylpropionylamino]-4-methylpentanoylamino]hexanoyl]piperidine-4-carboxylate (256 mg, crude).

LC-MS-4-1 (ES, m/z): [M+1] = 994

4.2 Synthesis of compound 4-2

Methyl 4-[(*tert*-butoxycarbonyl)amino]-1-[(2R)-6-[(*tert*-butoxycarbonyl)amino]-2-[(2R)-2-[(2R)-2-{3-[(*tert*-butoxycarbonyl)amino]-3-phenylpropionylamino}-3-phenylpropionylamino]-4-methylpentanoylamino]hexanoyl]piperidine-4-carboxylate (265 mg, 0.267 mmol, 1.00 equiv) was dissolved in dioxane (5.3 mL), and a 4 M solution of HCl (gas) in 1,4-dioxane (8 mL) was added at room temperature. The resulting mixture was stirred at room temperature for 2 h and concentrated under reduced pressure to give methyl 4-amino-1-[(2R)-6-amino-2-[(2R)-2-[(2R)-2-(3-amino-3-phenylpropionylamino)-3-phenylpropionylamino]-4-methylpentanoylamino]hexanoyl]piperidine-4-carboxylate (101 mg, crude).

LC-MS-4-2 (ES, m/z): [M+1] = 694

4.3 Synthesis of compounds 4-0/100

Methyl 4-amino-1-[(2R)-6-amino-2-[(2R)-2-[(2R)-2-(3-amino-3-phenylpropionylamino)-3-phenylpropionylamino]-4-methylpentanoylamino]hexanoyl]piperidine-4-carboxylate (80 mg, 0.115 mmol, 1.00 equiv) was dissolved in THF (3 mL), and $H_2O$ (0.8 mL, 44.4075.15 mmol) and LiOH (5.52 mg, 0.230 mmol, 2 equiv) were added. The resulting mixture was stirred at room temperature for 4 h. The mixture was acidified with HCl (1 N) to pH 7. The resulting mixture was diluted with water (100 mL). The resulting mixture was extracted with ethyl acetate (3 × 100 mL). The combined organic layer was dried over anhydrous sodium sulfate and filtered. The filtrate was concentrated under reduced pressure. The residue was purified by reversed-phase flash chromatography. The fractions were concentrated to dryness by rotary evaporation to give 4-amino-1-[(2R)-6-amino-2-[(2R)-2-[(2R)-2-((3R)-3-amino-3-phenylpropionylamino)-3-phenylpropionylamino]-4-methylpentanoylamino]hexanoyl]piperidine-4-carboxylic acid (11 mg, 13.43%, purity: 95.3%) and 4-amino-1-[(2R)-6-amino-2-[(2R)-2-[(2R)-2-[(3S)-3-amino-3-phenylpropionylamino]-3-phenylpropionylamino]-4-methylpentanoylamino] hexanoyl]piperidine-4-carboxylic acid (11 mg, 13.74%, purity: 97.5%).

LC-MS-4-0 (ES, m/z): [M+1] = 680

**Compound 4-0:** $^1$H NMR (300 MHz, Deuterium Oxide) δ 8.34 (s, 2H), 7.22 (t, *J* = 41.9 Hz, 10H), 4.27 (d, *J* = 80.8 Hz, 4H), 3.64 (d, *J* = 47.2 Hz, 4H), 2.89 (s, 6H), 2.13 (s, 2H), 1.90 - 1.48 (m, 6H), 1.37 (s, 5H), 0.76 (d, *J* = 20.6 Hz, 6H).

LC-MS-4-100 (ES, m/z): [M+1] = 680

**Compound 4-100:** $^1$H NMR (300 MHz, Deuterium Oxide) δ 8.34 (s, 2H), 7.50 - 6.77 (m, 10H), 4.59 - 4.16 (m, 4H), 3.67 (d, *J* = 40.1 Hz, 4H), 3.05 - 2.62 (m, 6H), 2.12 (d, *J* = 27.4 Hz, 2H), 1.96 - 1.52 (m, 6H), 1.54 - 1.16 (m, 5H), 0.93 - 0.66 (m, 6H).

Example 5. Synthesis of Compound 5

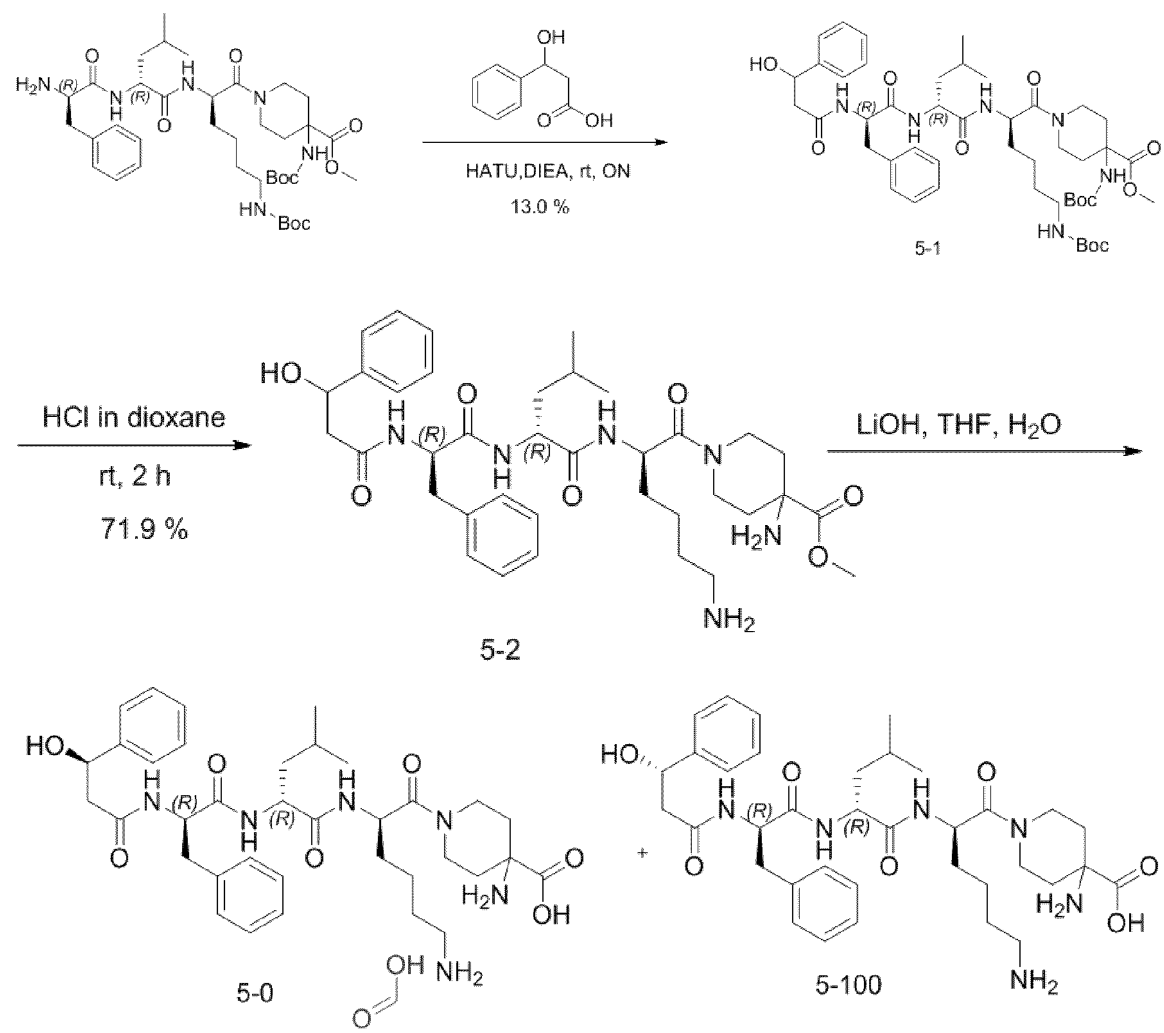


5-1

5-2

5-0

+

5-100

LC-MS-5-0 (ES, m/z): [M+1] = 680

**Compound 5-0:** $^1$H NMR (300 MHz, Deuterium Oxide) δ 8.37 (s, 1H), 7.37 - 7.08 (m, 10H), 4.90 (t, *J* = 7.1 Hz, 1H),

4.48 - 4.39 (m, 2H), 4.20 (t, *J* = 7.3 Hz, 1H), 3.86 - 3.45 (m, 4H), 3.06 - 2.78 (m, 4H), 2.78 - 2.50 (m, 2H), 2.26 - 2.02 (m, 2H), 1.93 - 1.53 (m, 6H), 1.51 - 1.19 (m, 5H), 0.80 (dd, *J* = 17.5, 5.1 Hz, 6H).

LC-MS-5-100 (ES, m/z): [M+1] = 680

**Compound 5-100:** $^1$H NMR (300 MHz, Deuterium Oxide) δ 7.39 - 7.01 (m, 10H), 4.94 (t, *J* = 7.1 Hz, 1H), 4.44 (t, *J* = 7.4 Hz, 2H), 4.24 (d, *J* = 8.8 Hz, 1H), 3.66 (d, *J* = 58.6 Hz, 4H), 2.99 - 2.56 (m, 6H), 2.14 (d, *J* = 20.9 Hz, 2H), 1.92 - 1.55 (m, 6H), 1.40 (d, *J* = 33.6 Hz, 5H), 0.81 (dd, *J* = 15.7, 4.9 Hz, 6H).

Example 6. Synthesis of Compound 6

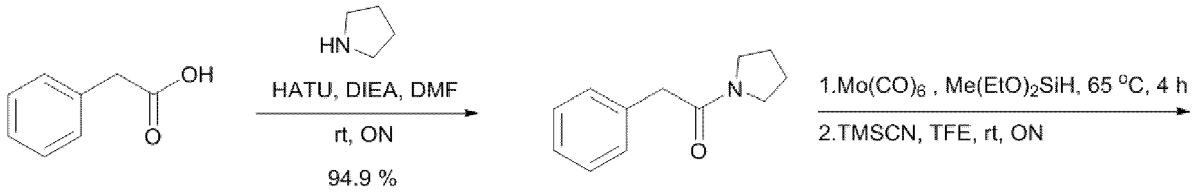


6-1

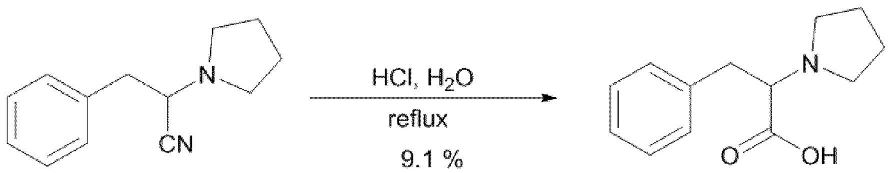


6-2

6-3

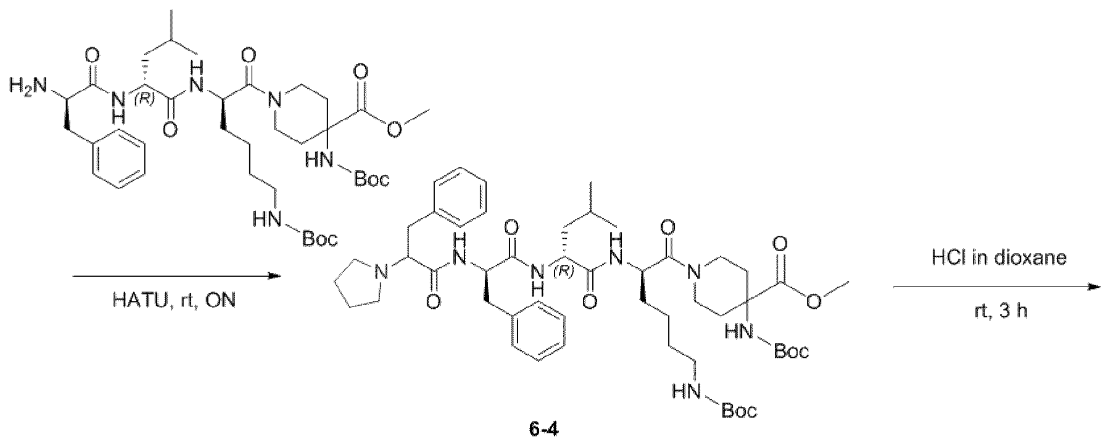


6-4

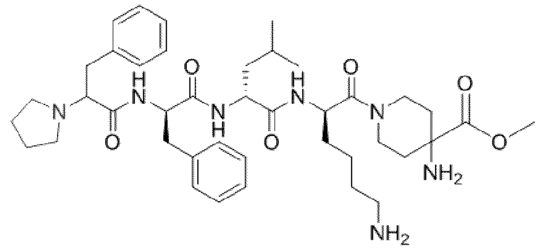

6-5

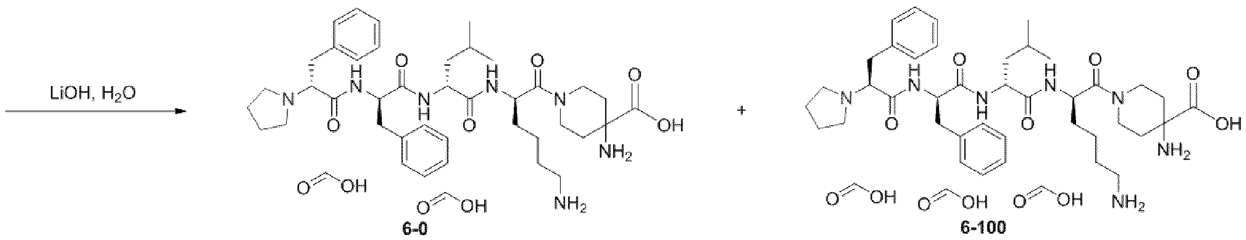


6-0

6-100

**LC-MS-6-0** (ES, m/z): [M+1] = 734

$^1$H NMR (400 MHz, Deuterium Oxide) δ 8.37 (s, 2H), 7.48 - 6.81 (m, 10H), 4.66 - 4.48 (m, 1H), 4.06 (q, *J* = 6.6, 5.5 Hz, 1H), 3.91 - 3.44 (m, 6H), 3.25 (dd, *J* = 13.1, 4.9 Hz, 1H), 3.16 - 2.48 (m, 9H), 2.35 - 1.97 (m, 2H), 1.98 - 1.18 (m, 15H), 0.91 (dd, *J* = 18.5, 5.3 Hz, 6H).

**LC-MS-6-100** (ES, m/z): [M+1] = 734

$^1$H NMR (400 MHz, Deuterium Oxide) δ 8.38 (s, 3H), 7.45 - 6.86 (m, 10H), 4.68 - 4.57 (m, 1H), 4.10 (d, *J* = 2.8 Hz, 1H), 3.96 - 3.42 (m, 5H), 3.42 - 3.17 (m, 1H), 3.02 (dt, *J* = 12.5, 5.7 Hz, 1H), 2.97 - 2.77 (m, 4H), 2.71 - 2.48 (m, 1H), 2.14 (dd, *J* = 27.5, 13.5 Hz, 2H), 2.02 - 1.49 (m, 13H), 1.39 - 1.23 (m, 2H), 1.08 - 0.79 (m, 6H).

Example 7. Synthesis of Compound 7

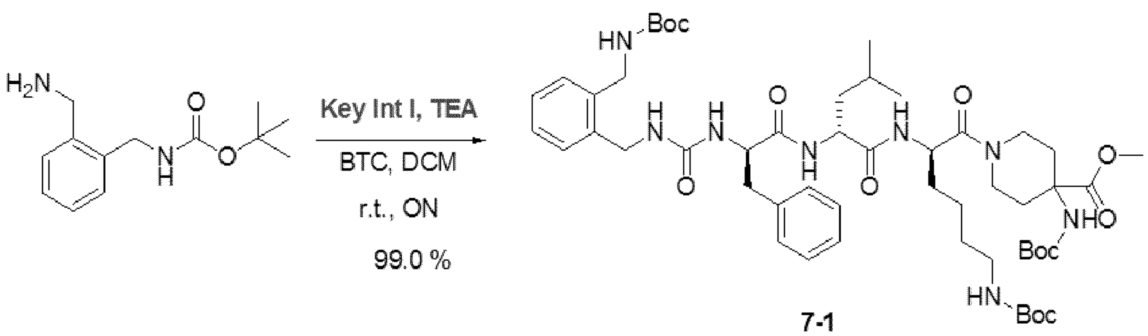


7-1

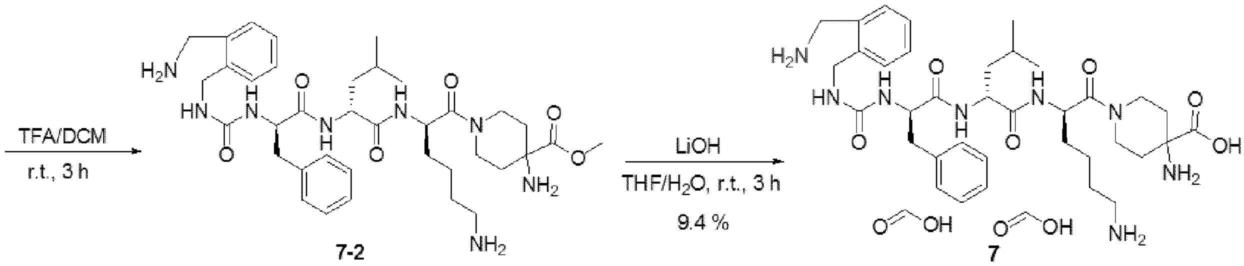


7-2

7

LC-MS-7 (ES, m/z): [M+1] = 695

$^1$H NMR (400 MHz, Deuterium Oxide) δ 8.35 (s, 2H), 7.53 - 6.99 (m, 9H), 4.39 (ddd, *J* = 8.8, 5.6, 3.0 Hz, 1H), 4.32 - 3.97 (m, 5H), 3.94 - 3.63 (m, 2H), 3.58 - 3.29 (m, 2H), 3.04 (dt, *J* = 13.9, 5.1 Hz, 1H), 2.93 - 2.63 (m, 3H), 2.39 - 1.92 (m, 2H), 1.97 - 1.18 (m, 11H), 0.79 (dd, *J* = 19.1, 5.4 Hz, 6H).

Example 8. Synthesis of Compound 8

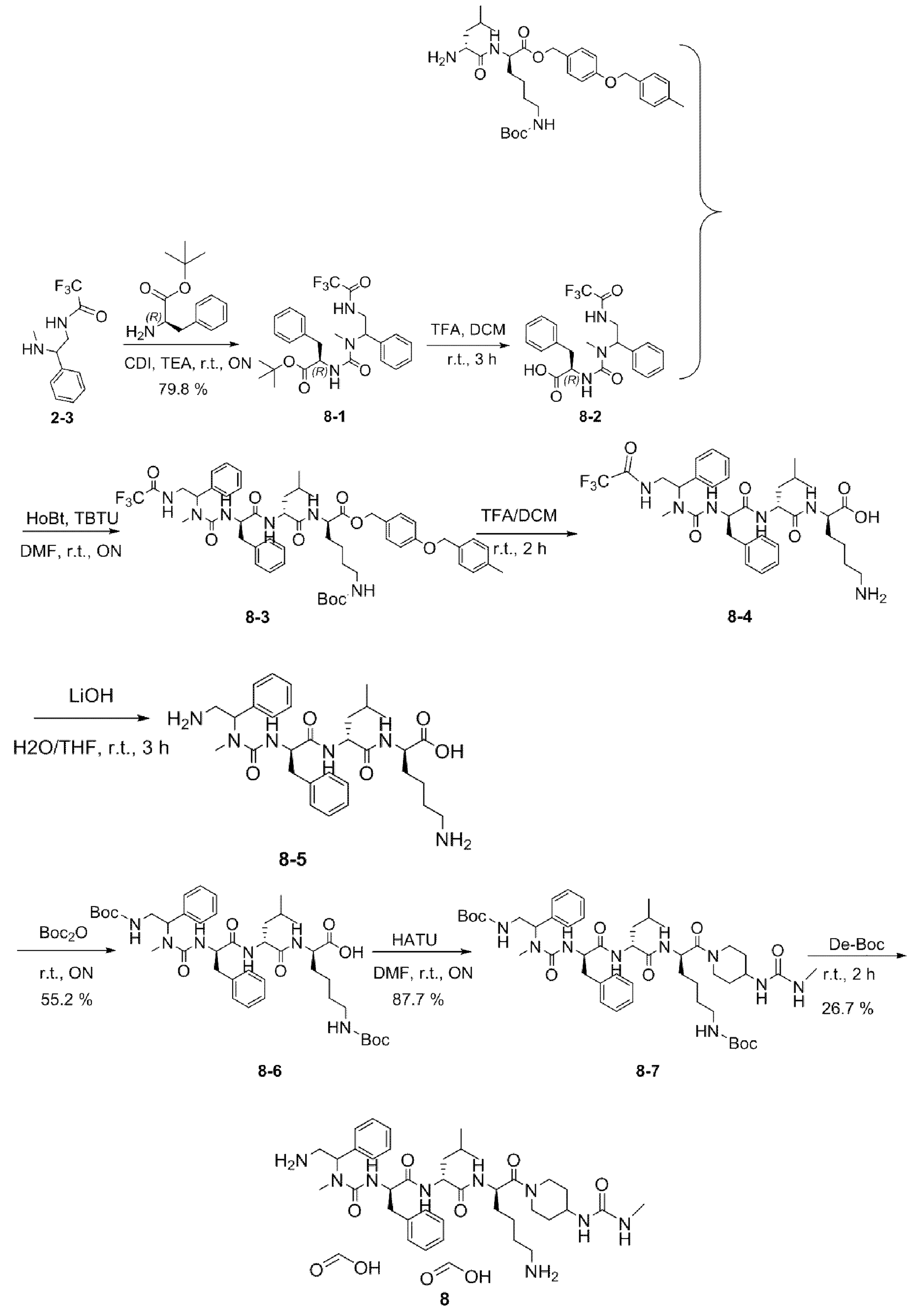


2-3

8-1

8-2

8-3

8-4

8-5

8-6

8-7

8

8.1 Synthesis of compound 8-1

*tert*-Butyl (2R)-2-amino-3-phenylpropionate (480 mg, 2.169 mmol, 1.00 equiv) and triethylamine (1.85 g, 18.282 mmol, 8.43 equiv) were dissolved in acetonitrile (19 mL, 361.469 mmol, 166.65 equiv). The resulting mixture was stirred at 0 °C for 30 min under nitrogen atmosphere. N,N'-Carbonyldiimidazole (1.2 g, 7.401 mmol, 3.41 equiv) and 2,2,2-trifluoro-N-[2-(methylamino)-2-phenylethyl]acetamide (12.80 g, 51.991 mmol, 23.97 equiv) were added to the mixture described above. The resulting mixture was stirred at room temperature overnight under nitrogen atmosphere and concentrated under reduced pressure. The residue was purified by reversed-phase flash chromatography, and the fractions were concentrated to dryness by rotary evaporation to give *tert*-butyl (2R)-2-({methyl[1-phenyl-2-(2,2,2-trifluoroacetamido) ethyl]carbamoyl}amino)-3-phenylpropionate (855 mg, 79.87%, purity: 100%).
LC-MS-8-1 (ES, m/z): [M+1] = 494

8.2 Synthesis of compound 8-2

*tert*-Butyl (2R)-2-({methyl[1-phenyl-2-(2,2,2-trifluoroacetamido)ethyl carbamoyl }amino)-3-phenylpropionate (67 mg, 0.136 mmol, 1 equiv) was dissolved in dichloromethane (7.70 mL), and trifluoroacetic acid (7.70 mL, 78.592 mmol, 45.09 equiv) was added dropwise. The resulting mixture was stirred in the air for 3 h. The resulting mixture was concentrated under reduced pressure to give (2R)-2-({methyl[1-phenyl-2-(2,2,2-trifluoroacetamido)ethyl]carbamoyl} amino)-3-phenylpropionic acid (600 mg, crude). The crude mixture was directly used in the next step without further purification.
LC-MS-8-2 (ES, m/z): [M+1] = 438

8.3 Synthesis of compound 8-3

{4-[(4-Methylphenyl)methoxy]phenyl}methyl (2R)-2-[(2R)-2-amino-4-methylpentanoylamino]-6-[(*tert*-butoxycarbonyl)amino]hexanoate (1445.79 mg, 2.538 mmol, 3 equiv), (2R)-2-({methyl[1-phenyl-2-(2,2,2-trifluoroacetamido) ethyl]carbamoyl}amino)-3-phenylpropionic acid (2600 mg, 5.944 mmol, 9.15 equiv) and HOBT (222 mg, 1.643 mmol, 2.53 equiv) were dissolved in DMF (50 mL, 64.609 mmol, 99.49 equiv) at room temperature, and TBTU (528 mg, 1.644 mmol) was added. The resulting mixture was stirred at room temperature overnight under nitrogen atmosphere to give {4-[(4-methylphenyl)methoxy]phenyl}methyl (2R)-6-[(*tert*-butoxycarbonyl)amino]-2-[(2R)-4-methyl-2-[(2R)-2-({methyl[1-phenyl-2-(2,2,2-trifluoroacetamido)ethyl]carbamoyl}amino)-3-phenylpropionylamino]pentanoylamino]hexanoate (2.6 g, crude).

8.4 Synthesis of compound 8-4

{4-[(4-Methylphenyl)methoxy]phenyl}methyl (2R)-6-[(*tert*-butoxycarbonyl)amino]-2-[(2R)-4-methyl-2-[(2R)-2-({methyl[1-phenyl-2-(2,2,2-trifluoroacetamido)ethyl]carbamoyl}amino)-3-phenylpropionylamino]pentanoylamino]hexanoate (2.8 g, 3.150 mmol, 1 equiv) was dissolved in DCM (20 mL) at room temperature, and trifluoroacetic acid (10 mL) was added. The resulting mixture was stirred at room temperature for 2 h under air atmosphere to give (2R)-6-amino-2-[(2R)-4-methyl-2-[(2R)-2-({methyl[1-phenyl-2-(2,2,2-trifluoroacetamido)ethyl]carbamoyl}amino)-3-phenylpropionylamino]pentanoylamino]hexanoic acid (470 mg, crude).
LC-MS-8-4 (ES, m/z): [M+1] = 679

8.5 Synthesis of compound 8-5

(2R)-6-Amino-2-[(2R)-4-methyl-2-[(2R)-2-({methyl[1-phenyl-2-(2,2,2-trifluoroacetamido)ethyl]carbamoyl} amino)-3-phenylpropionylamino]pentanoylamino]hexanoic acid (470 mg, 0.692 mmol, 1 equiv), $H_2O$ (10 mL) and lithium hydroxide (33.17 mg, 1.384 mmol, 2 equiv) were added to a 50 mL round-bottom flask at room temperature. The resulting mixture was stirred at room temperature for 3 h. The resulting mixture was concentrated under reduced pressure to give (2R)-6-amino-2-[(2R)-2-[(2R)-2-{[(2-amino-1-phenylethyl) (methyl)carbamoyl]amino}-3-phenylpropionylamino]-4-methylpentanoylamino]hexanoic acid (500 mg, crude).
LC-MS-8-5 (ES, m/z): [M+1] = 583

8.6 Synthesis of compound 8-6

(2R)-6-Amino-2-[(2R)-2-[(2R)-2-{[(2-amino-l-phenylethyl)(methyl)carbamoyl]amino}-3-phenylpropionylamino]-4-methylpentanoylamino]hexanoic acid (350 mg, 0.601 mmol, 1 equiv) and TEA (182.33 mg, 1.803 mmol, 3 equiv) were dissolved in THF (10 mL), and di-*tert*-butyl dicarbonate (262.1202 mg, 1 equiv) was added. The resulting mixture was

stirred at room temperature overnight. The resulting mixture was concentrated under reduced pressure and purified by reversed-phase flash chromatography to give (2R)-6-[(*tert*-butoxycarbonyl)amino]-2-[(2R)-2-[(2R)-2-[({2-[(*tert*-butoxycarbonyl)amino]]-1-phenylethyl}(methyl)carbamoyl) amino]-3-phenylpropionylamino]-4-methylpentanoylamino]hexanoic acid (260 mg, 55.29%, purity: 100%).

LC-MS-8-6 (ES, m/z): [M+1] = 783

8.7 Synthesis of compound 8-7

(2R)-6-[(*tert*-Butoxycarbonyl)amino]-2-[(2R)-2-[(2R)-2-[({2-[(*tert*-butoxycarbonyl)amino]-1-phenylethyl}(methyl)carbamoyl)amino]-3-phenylpropionylamino]-4-methylpentanoylamino]hexanoic acid (60 mg, 0.077 mmol, 1 equiv), 3-methyl-1-(piperidin-4-yl)urea (24.10 mg) and TEA (23.26 mg, 0.231 mmol, 3 equiv) were dissolved in DMF (5 mL, 64.609 mmol, 843.12 equiv) at room temperature under nitrogen atmosphere, and HATU (43.71 mg, 0.115 mmol, 3 equiv) was added. The resulting mixture was stirred at room temperature overnight under nitrogen atmosphere. The resulting mixture was purified by reversed-phase flash chromatography to give *tert*-butyl N-[(5R)-5-[(2R)-2-[(2R)-2-[({2-[(*tert*-butoxycarbonyl)amino]-1-phenylethyl)}(methyl)carbamoyl)amino]-3-phenylpropionylamino]-4-methylpentanoylamino]-6-{4-[(methylcarbamoyl)amino]piperidin-1-yl}-6-oxohexyl]carbamate (62 mg, 87.74%, purity: 100%).

LC-MS-8-7 (ES, m/z): [M+1] = 922

8.8 Synthesis of compound 8

*tert*-Butyl N-[(5R)-5-[(2R)-2-[(2R)-2-[({2-[(*tert*-butoxycarbonyl)amino]-1-phenylethyl}(methyl)carbamoyl) amino]-3-phenylpropionylamino]-4-methylpentanoylamino]-6-{4-[(methylcarbamoyl)amino]piperidin-1-yl}-6-oxohexyl]carbamate (62 mg, 0.067 mmol, 1 equiv) was dissolved in dioxane (10 mL), and a solution of HCl (gas) in 1,4-dioxane (5 mL) was added. The resulting mixture was stirred at room temperature for 2 h and The resulting mixture was concentrated under reduced pressure and purified by reversed-phase flash chromatography to give (2R)-2-[(2R)-2-{[(2-amino-1-phenylethyl)(methyl)carbamoyl)amino}-3-phenylpropionylamino]-N-[(2R)-6-amino-1-{4-[(methylcarbamoyl)amino]piperidin-1-yl}-1-oxohexyl-2-yl]-4-methylpentanamide (13.6 mg, 26.73%, purity: 95.0%).

LC-MS-8 (ES, m/z): [M+1] = 722

$^1$H NMR (400 MHz, Deuterium Oxide) δ 8.33 (s, 2H), 7.50 - 7.15 (m, 8H), 7.10 (d, *J* = 7.2 Hz, 2H), 5.49 (dd, *J* = 10.8, 5.2 Hz, 1H), 4.42 (dd, *J* = 9.1, 6.2 Hz, 1H), 4.37 - 4.25 (m, 1H), 4.24 - 4.00 (m, 1H), 3.95 - 3.40 (m, 7H), 3.26 - 3.02 (m, 2H), 2.99 - 2.69 (m, 4H), 2.70 - 2.42 (m, 6H), 2.02 - 1.75 (m, 6H), 1.73 - 1.43 (m, 4H), 1.42 - 1.13 (m, 7H), 0.80 (ddd, *J* = 15.3, 6.0, 2.9 Hz, 6H).

Example 9. Synthesis of Compound 9

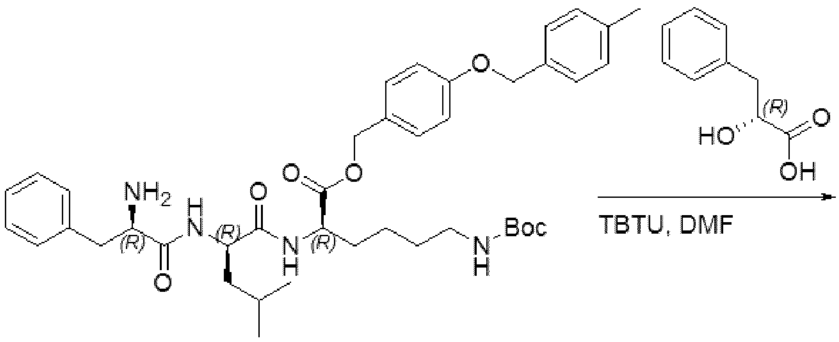


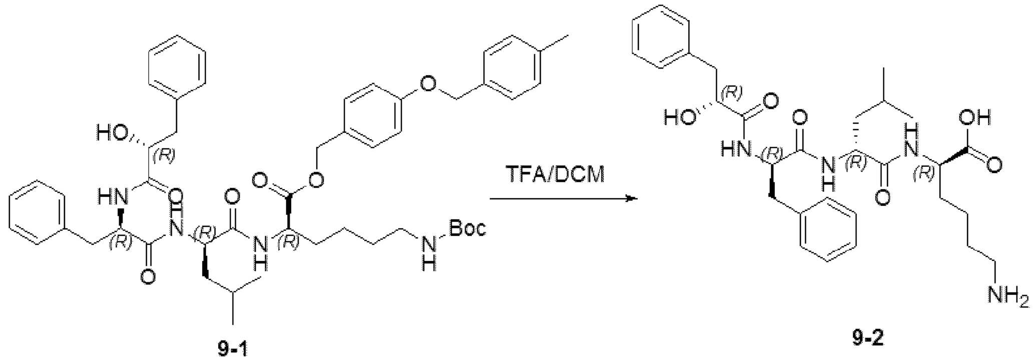


9-1

9-2

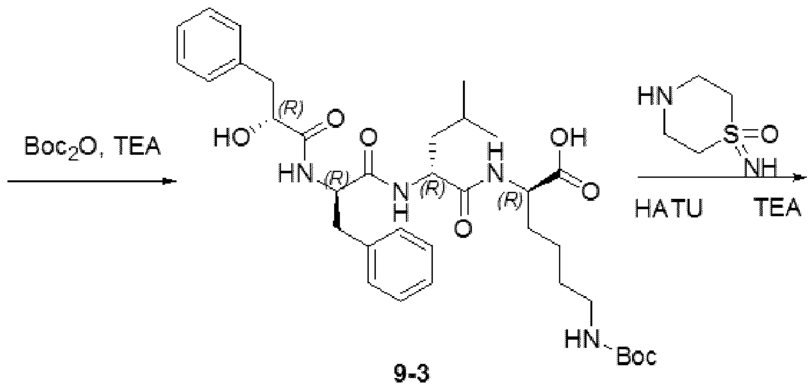


9-3

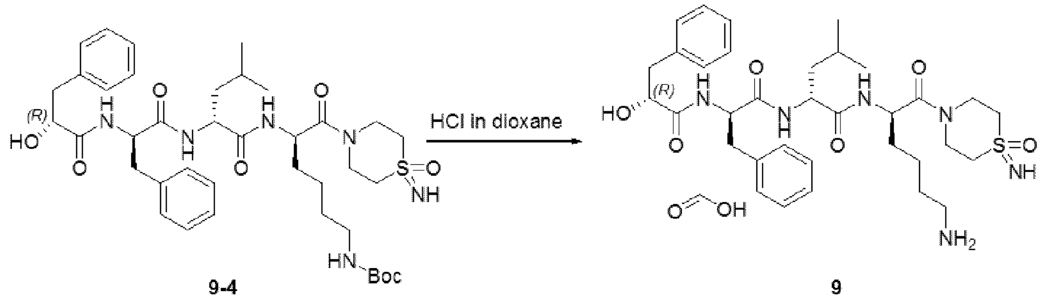


9-4

9

LC-MS-9 (ES, m/z): [M+1] = 671.20

$^1$H NMR (300 MHz, Deuterium Oxide) δ 8.37 (s, 1H), 7.43 - 6.81 (m, 10H), 4.49 (s, 2H), 4.46 - 3.55 (m, 6H), 3.60 - 2.94 (m, 5H), 3.03 - 2.52 (m, 6H), 1.83 - 1.10 (m, 9H), 0.78 (dd, *J* = 14.0, 5.0 Hz, 6H).

Example 10. Synthesis of Compound 10

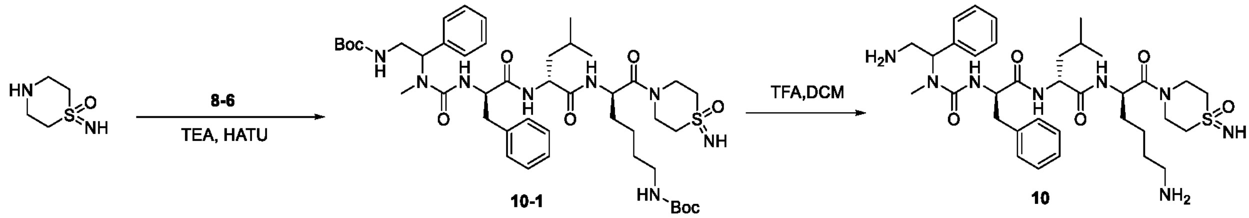


10-1

10

LC-MS-10 (ES, m/z): [M+1] = 699.20

$^1$H NMR (300 MHz, Deuterium Oxide) δ 7.42 - 6.89 (m, 10H), 5.51 (dd, $J$ = 10.6, 4.8 Hz, 1H), 4.43 (dd, $J$ = 9.0, 6.3 Hz, 1H), 4.34 - 4.12 (m, 2H), 4.10 - 3.71 (m, 2H), 3.65 - 3.44 (m, 3H), 3.32 (d, $J$ = 19.3 Hz, 2H), 3.18 (d, $J$ = 17.1 Hz, 2H), 3.09 (dd, $J$ = 13.8, 6.1 Hz, 1H), 3.00 - 2.93 (m, 1H), 2.85 (t, $J$ = 7.6 Hz, 2H), 2.57 (d, $J$ = 31.2 Hz, 4H), 1.82 - 1.43 (m, 7H), 1.42 - 1.16 (m, 2H), 0.82 (dd, $J$ = 12.3, 5.0 Hz, 6H).

Example 11. Synthesis of Compound 11

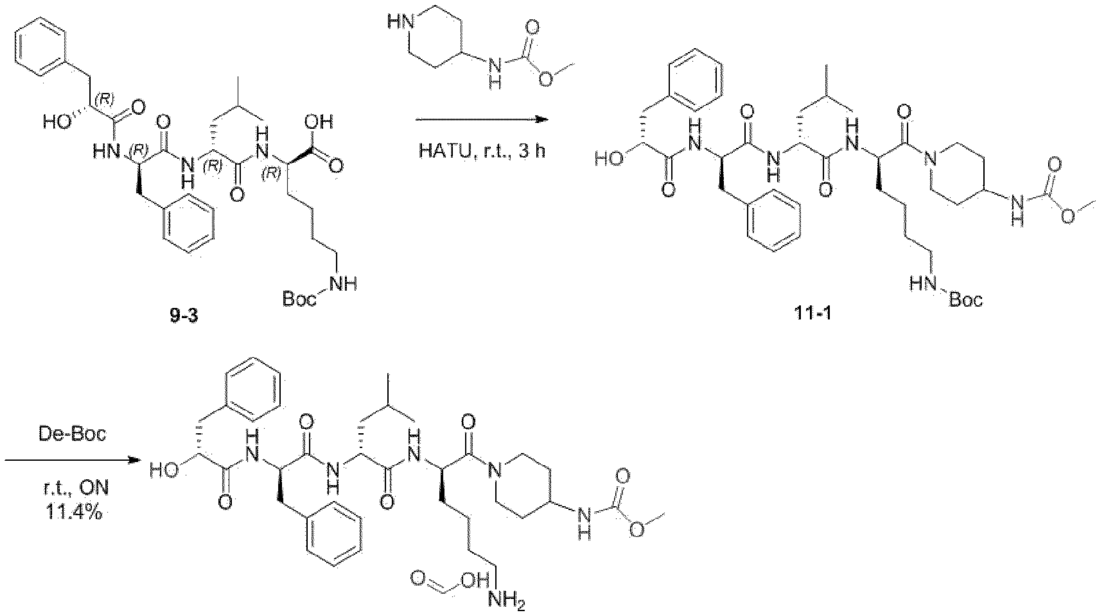


9-3

11-1

11

LC-MS-11 (ES, m/z): [M+1] = 695

$^1$H NMR (400 MHz, Deuterium Oxide) δ 8.39 (s, 1H), 7.40 - 7.19 (m, 6H), 7.08 (ddt, $J$ = 27.2, 5.4, 1.7 Hz, 4H), 4.59 - 4.47 (m, 1H), 4.30 (ddd, $J$ = 28.1, 8.0, 5.5 Hz, 2H), 4.14 (dd, $J$ = 47.0, 13.4 Hz, 1H), 3.88 (dd, $J$ = 36.1, 13.4 Hz, 1H), 3.70 - 3.50 (m, 4H), 3.24 (q, $J$ = 13.9, 12.7 Hz, 1H), 2.99 - 2.70 (m, 7H), 2.01 - 1.80 (m, 2H), 1.76 - 1.55 (m, 4H), 1.48 (q, $J$ = 9.6, 8.5 Hz, 3H), 1.41 - 1.17 (m, 4H), 0.83 (ddd, $J$ = 16.8, 5.1, 2.1 Hz, 6H).

Example 12. Synthesis of Compound 12

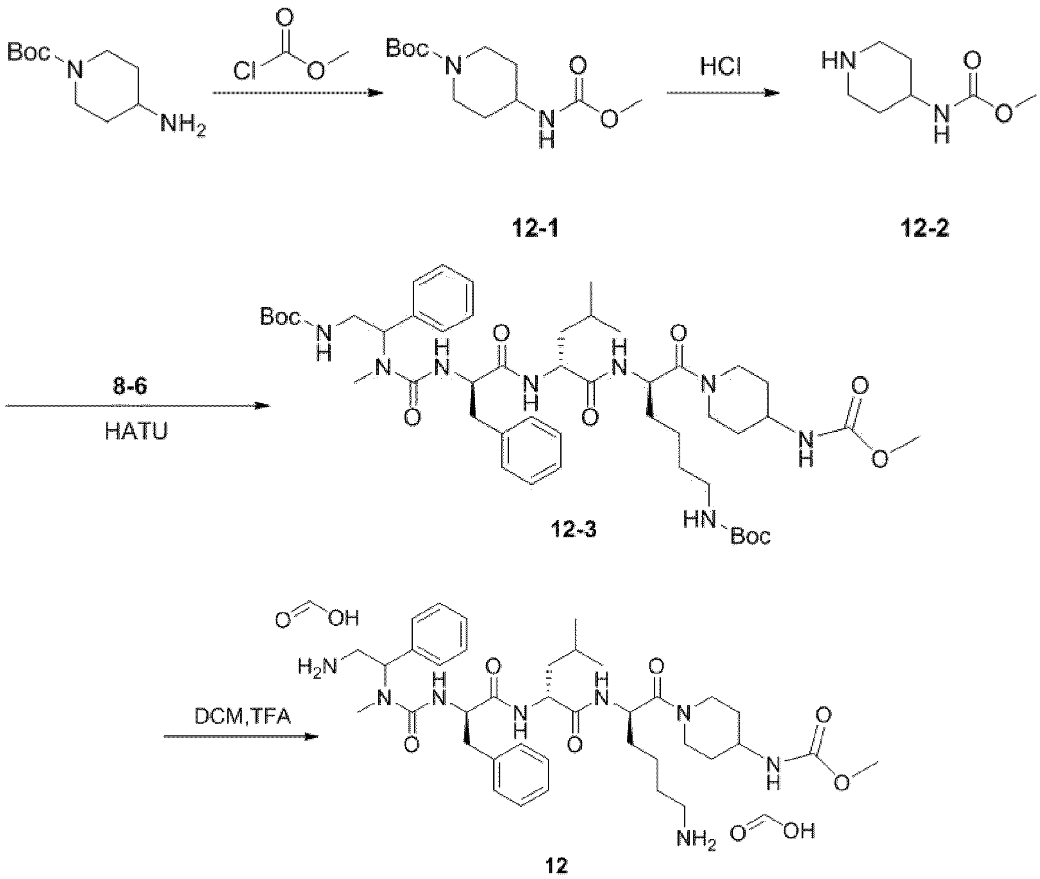


12-1 12-2 12-3 12

12.1 Synthesis of compound 12-1

*tert*-Butyl 4-aminopiperidine-1-carboxylate (500 mg, 2.496 mmol, 1 equiv) was dissolved in DCM, and TEA (505.25 mg, 4.992 mmol, 2 equiv) was added. Methyl chloroformate (353.84 mg, 3.744 mmol, 1.5 equiv) was added portionwise at 0 °C under nitrogen atmosphere. The resulting mixture was stirred at room temperature for 2 h and The reaction was quenched with water at room temperature. The resulting mixture was concentrated under reduced pressure, and the residue was purified by silica gel column chromatography and eluted with PE/EA (5:1). The fractions were concentrated to dryness by rotary evaporation to give *tert*-butyl 4-[(methoxycarbonyl)amino]piperidine-1-carboxylate (522 mg, 80.94%, purity: 100%).

LC-MS-12-1 (ES, m/z): [M+1] = 259.32

12.2 Synthesis of compound 12-2

*tert-Butyl* 4-[(methoxycarbonyl)amino]piperidine-1-carboxylate (552 mg, 2.137 mmol, 1 equiv) was added to a 4 M solution of HCl (gas) in 1,4-dioxane (5 mL) at room temperature. The resulting mixture was stirred at room temperature for 2 h and The reaction was quenched with water at room temperature. The resulting mixture was concentrated under reduced pressure to give methyl N-(piperidin-4-yl)carbamate (455 mg, crude). The crude product was directly used in the next step without further purification.

LC-MS-12-2 (ES, m/z): [M+1] = 159.20

12.3 Synthesis of compound 12-3

(2R)-6-[(*tert*-Butoxycarbonyl)amino]-2-[(2R)-2-[(2R)-2-[({2-[(*tert*-butoxycarbonyl)amino]-1-phenylethyl}(methyl)carbamoyl)amino]-3-phenylpropionylamino]-4-methylpentanoylamino]hexanoic acid (70 mg, 0.089 mmol, 1 equiv), TEA (27.14 mg, 0.267 mmol, 3 equiv) and methyl N-(piperidin-4-yl)carbamate (21.22 mg, 0.134 mmol, 1.5 equiv) were added to DMF (1 mL), and HATU (50.99 mg, 0.134 mmol, 1.5 equiv) was added portionwise at room temperature under nitrogen atmosphere. The resulting mixture was stirred at room temperature for another 2 h. The reaction was quenched with water at room temperature. The desired product could be detected by LCMS. The reaction solution was purified by reversed-phase flash chromatography to give *tert*-butyl N-[(5R)-5-[(2R)-2-[(2R)-2-[({2-[(*tert*-butoxycarbonyl)amino]-1-phenylethyl}(methyl)carbamoyl)amino]-3-phenylpropionylamino]-4-methylpentanoylamino]-6-{4-[(methoxycarbonyl)amino]piperidin-1-yl}-6-oxohexyl]carbamate (70 mg, 83.00%, purity: 100%).

LC-MS-12-3 (ES, m/z): [M+1] = 924.1

12.4 Synthesis of compound 12

*tert*-Butyl N-[(5R)-5-[(2R)-2-[(2R)-2-[({2-[(*tert*-butoxycarbonyl)amino]-1-phenylethyl}(methyl)carbamoyl)amino]-3-phenylpropionylamino]-4-methylpentanoylamino]-6-{4-[(methoxycarbonyl)amino]piperidin-1-yl}-6-oxohexyl]carbamate (70 mg, 0.076 mmol, 1 equiv) was added to DCM (4 mL) at room temperature under nitrogen atmosphere, and TFA (1 mL) was added dropwise. The resulting mixture was stirred at room temperature for another 3 h. The resulting mixture was concentrated under reduced pressure. The residue was purified by reversed-phase flash chromatography, and the fractions were concentrated to dryness by rotary evaporation to give methyl N-{1-[(2R)-6-amino-2-[(2R)-2-[(2R)-2-{[(2-amino-1-phenylethyl)(methyl)carbamoyl]amino}-3-phenylpropionylamino]-4-methylpentanoylamino]hexanoyl]piperidin-4-yl}carbamate (42.1 mg, 72.07%, purity: 93.9%).

LC-MS-12 (ES, m/z): [M+1] = 723.15

$^1$H NMR (400 MHz, Deuterium Oxide) δ 8.37 (s, 2H), 7.58 - 6.75 (m, 10H), 5.52 (s, 1H), 4.79 - 4.73 (m, 1H), 4.50 - 3.99 (m, 3H), 3.87 (d, *J* = 32.6 Hz, 1H), 3.57 (s, 6 H), 3.35 - 3.03 (m, 2H), 3.02 - 2.68 (m, 4H), 2.48 (dd, *J* = 39.8, 5.2 Hz, 3H), 1.90 (s, 2H), 1.74 - 1.07 (m, 11H), 1.01 - 0.61 (m, 6H).

Example 13. Synthesis of Compound 13

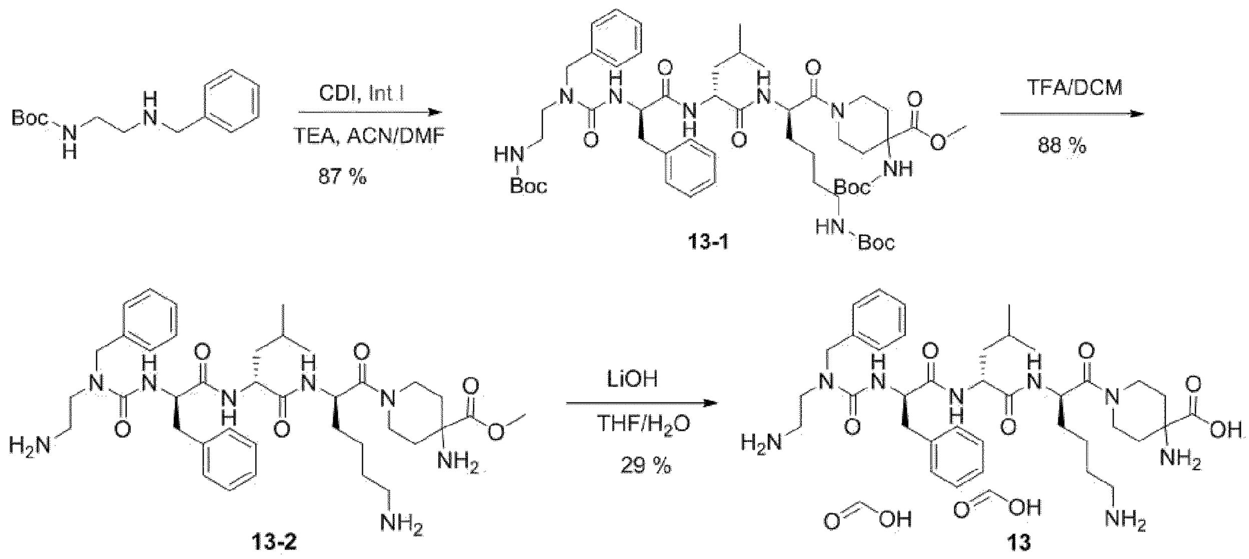


13.1 Synthesis of compound 13-1

Methyl 1-[(2R)-2-[(2R)-2-[(2R)-2-amino-3-phenylpropionylamino]-4-methylpentanoylamino]-6-[(tert-butoxycarbonyl)amino]hexanoyl]-4-[(tert-butoxycarbonyl)amino]piperidine-4-carboxylate (100 mg, 0.134 mmol, 1.00 equiv) was added to a solution of *tert-butyl* N-[2-(benzylamino)ethyl]carbamate (100 mg, 0.399 mmol, 2.98 equiv), TEA (40.64 mg, 0.402 mmol, 3 equiv) and N,N'-carbonyldiimidazole (23.88 mg, 0.147 mmol) in ACN (3 mL)/DMF (3 mL) at room temperature under nitrogen atmosphere. The resulting mixture was stirred at room temperature overnight under nitrogen atmosphere. The resulting mixture was purified by reversed-phase flash chromatography, and the fractions were concentrated to dryness by rotary evaporation to give methyl 1-[(2R)-2-[(2R)-2-[(2R)-2-{[benzyl({2-[(*tert*-butoxycarbonyl)amino]ethyl})carbamoyl]amino}-3-phenylpropionylamino]-4-methylpentanoylamino]-6-[(*tert*-butoxycarbonyl)amino]hexanoyl]-4-[(*tert*-butoxycarbonyl)amino]piperidine-4-carboxylate (120 mg, 87.59%, purity: 100%).

LC-MS-13-1 (ES, m/z): [M+1] = 1024.28

13.2 Synthesis of compound 13-2

Methyl 1-[(2R)-2-[(2R)-2-[(2R)-2-{[benzyl({2-[(*tert*-butoxycarbonyl)amino]ethyl})carbamoyl]amino}-3-phenylpropionylamino]-4-methylpentanoylamino]-6-[(*tert*-butoxycarbonyl)amino]hexanoyl]-4-[(tert-butoxycarbonyl)amino]piperidine-4-carboxylate (120 mg, 0.117 mmol, 1 equiv) was dissolved in DCM (1 mL, 5.612 mmol), and TFA (1 mL) was added. The solution was stirred at room temperature for 2 h under nitrogen atmosphere. The resulting mixture was concentrated under reduced pressure. The residue was purified by reversed-phase flash chromatography to give methyl 4-amino-1-[(2R)-6-amino-2-[(2R)-2-[(2R)-2-{[2-aminoethyl(benzyl)carbamoyl]amino}-3-phenylpropionylamino]-4-methylpentanoylamino]hexanoyl]piperidine-4-carboxylate (75 mg, 88.47%, purity: 100%).

LC-MS-13-2 (ES, m/z): [M+1] = 723.93

$^1$H NMR (400 MHz, Deuterium Oxide) δ 7.46 - 7.09 (m, 6H), 6.97 (dt, *J* = 14.3, 5.4 Hz, 4H), 4.52 - 4.36 (m, 2H), 4.31 (d, *J* = 17.5 Hz, 1H), 4.19 (p, *J* = 4.9, 4.1 Hz, 1H), 4.07 (d, *J* = 14.1 Hz, 1H), 3.92 (d, *J* = 14.4 Hz, 1H), 3.73 (dt, *J* = 12.4, 2.2 Hz, 3H), 3.64 (s, 1H), 3.60 - 3.31 (m, 3H), 3.30 - 3.16 (m, 1H), 3.10 - 2.89 (m,3H), 2.87 - 2.57 (m, 3H), 2.36 - 2.06 (m, 2H), 1.93-1.47 (m, 6H), 1.48 - 1.18 (m, 5H), 0.92 - 0.40 (m, 6H).

13.3 Synthesis of compound 13

Methyl 4-amino-1-[(2R)-6-amino-2-[(2R)-2-[(2R)-2-{[(2-aminoethyl)(benzyl)carbamoyl]amino}-3-phenylpropionylamino]-4-methylpentanoylamino]hexanoyl]piperidine-4-carboxylate (75 mg, 0.104 mmol, 1 equiv) was dissolved in THF (1 mL) and $H_2O$ (1 mL), and lithium hydroxide (5 mg, 0.209 mmol, 2.01 equiv) was added. The resulting mixture was stirred at room temperature for 2 h under nitrogen atmosphere. The resulting mixture was concentrated under reduced pressure to give a residue. The residue was purified by reversed-phase flash chromatography, and the fractions were concentrated to dryness by rotary evaporation to give 4-amino-1-[(2R)-6-amino-2-[(2R)-2-[(2R)-2-{[aminoethyl(benzyl) carbamoyl]]amino}-3-phenylpropionylamino]-4-methylpentanoylamino]hexanoyl]piperidine-4-carboxylic acid (25.5 mg, 29.28%, purity: 84.5%).

LC-MS-13 (ES, m/z): [M+1] = 709.25

$^1$H NMR (300 MHz, Deuterium Oxide) δ 8.33 (s, 2H), 7.33 - 7.15 (m, 6H), 7.00 (ddt, *J* = 12.9, 5.2, 2.6 Hz, 4H), 4.46 - 4.21 (m, 4H), 3.76 - 3.36 (m, 6H), 3.13 - 2.51 (m, 6H), 2.25 - 1.88 (m, 2H), 1.96 - 1.49 (m, 6H), 1.50 - 1.10 (m, 5H), 0.78 (dd, *J* = 14.4, 5.4 Hz, 6H).

Example 14. Synthesis of Compound 14

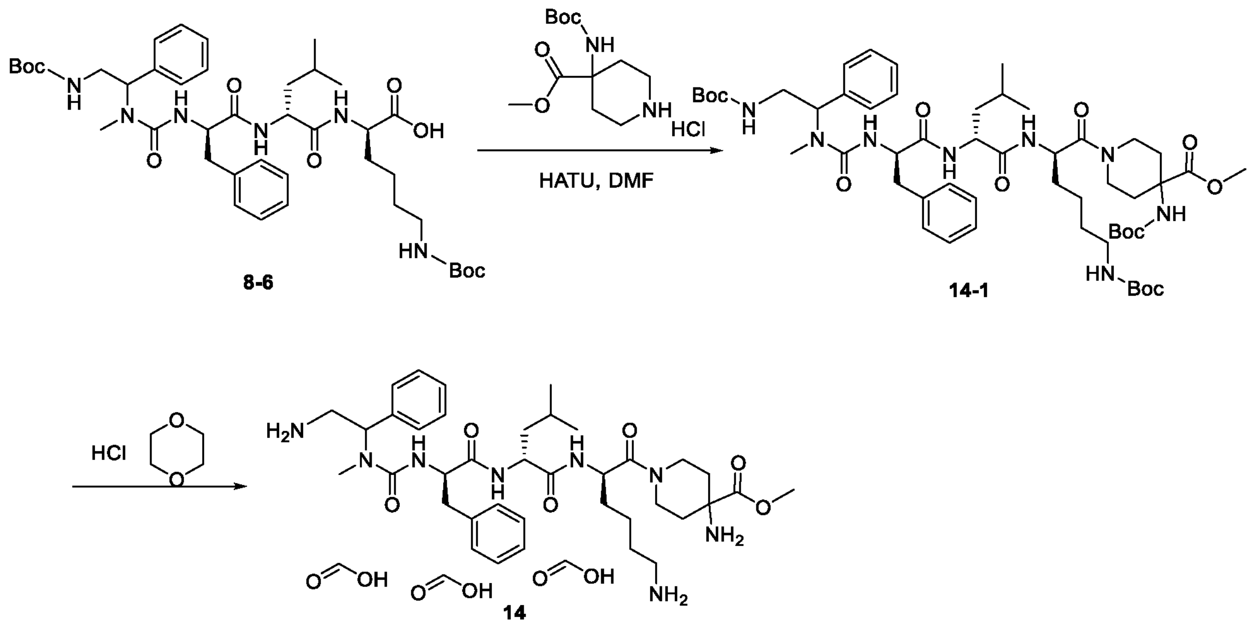


8-6

14-1

14

LC-MS-14 (ES, m/z): [M+1] = 723.25

$^1$H NMR (300 MHz, Deuterium Oxide) δ 8.33(s, 3H), 7.45 - 6.79 (m, 10H), 5.62 - 5.41 (m, 1H), 4.49 - 4.19 (m, 2H), 4.05 - 3.35 (m, 8H), 3.35 - 2.59 (m, 5H), 2.45 (dd, *J* = 30.9, 2.3 Hz, 3H), 2.20 (t, *J* = 16.0 Hz, 2H), 2.00 - 1.10 (m, 11H), 0.81 (dt, *J* = 11.9, 5.9 Hz, 6H).

Example 15. Synthesis of Compound 15

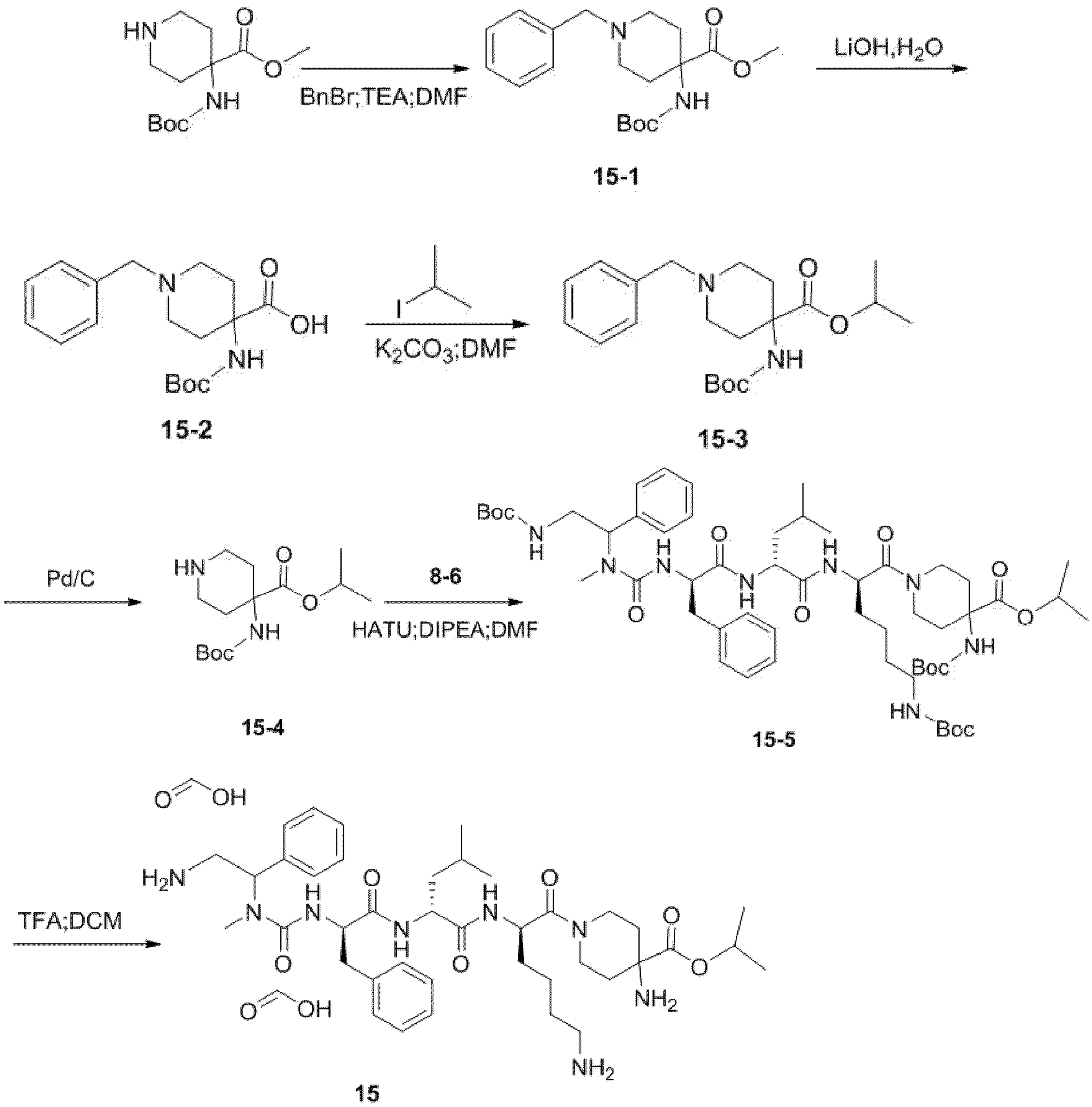


15-1

15-2

15-3

15-4

15-5

15

LC-MS-15 (ES, m/z): [M+1] = 751

$^1$H NMR (400 MHz, Deuterium Oxide) δ 8.32 (s, 2H), 7.52 - 6.72 (m, 10H), 5.70 - 5.31 (m, 2H), 4.48 - 4.09 (m, 3H), 4.11 - 3.34 (m, 7H), 3.29 - 3.14 (m, 1H), 3.14 - 2.68 (m, 5H), 2.49 (d, *J* = 5.1 Hz, 2H), 2.44 - 2.28 (m, 1H), 2.17 (d, *J* = 18.8 Hz, 2H), 1.93 - 1.34 (m, 10H), 1.20 (dd, *J* = 9.0, 6.3 Hz, 8H), 1.00 - 0.59 (m, 6H).

Example 16. Synthesis of Compound 16

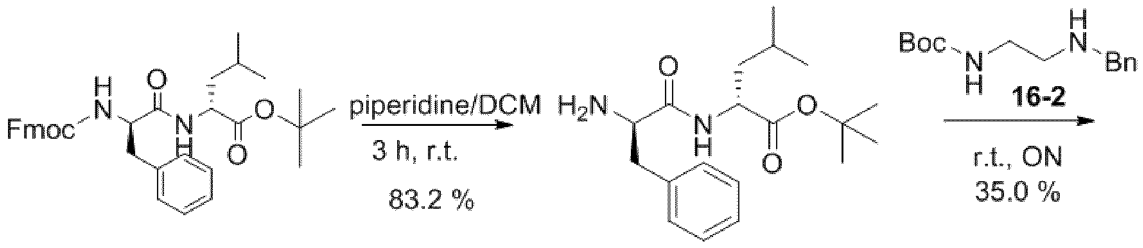


1-3 16-1

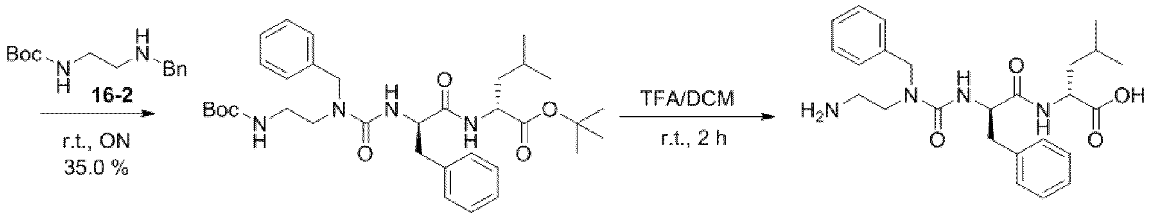


16-3 16-4

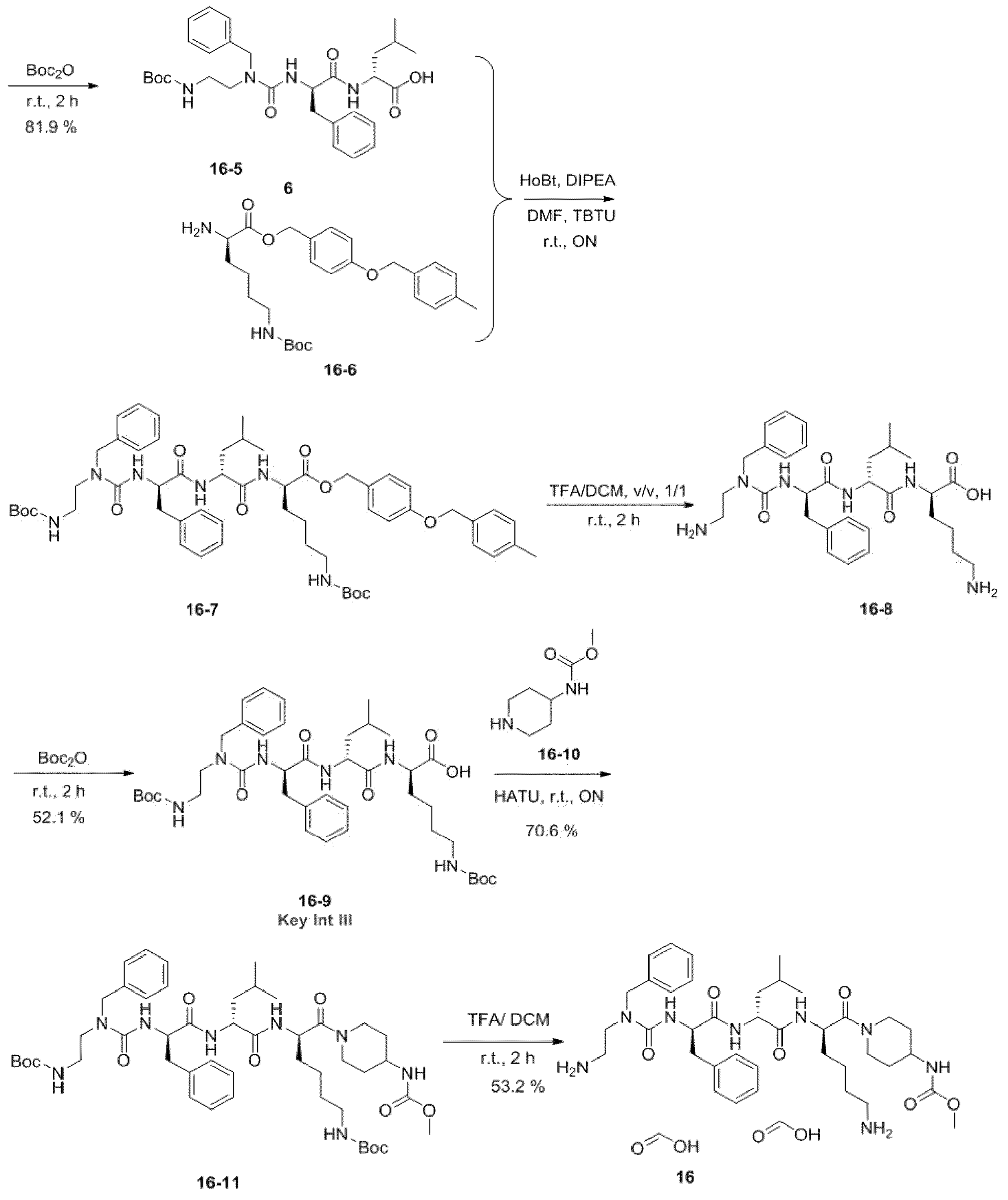

16-5
6
16-6
16-7
16-8
16-10
16-9
Key Int III
16-11
16

16.1 Synthesis of compound 16-1

Compound 1-3 (500 mg, 0.898 mmol, 1 equiv) was dissolved in DCM (5 mL), and then piperidine (0.5 mL) was added dropwise to the system described above. The resulting mixture was stirred at room temperature for 3 h. The reaction solution was then concentrated to dryness by rotary evaporation to give a yellow crude product. The resulting crude product was purified by reversed-phase column chromatography (conditions were as follows: C18 chromatographic column, mobile phase: solvent ACN and solvent $H_2O$ (FA, 0.1%), gradient 10% to 50% for 10 min, UV254 nanometer detector), and the fractions were concentrated to dryness by rotary evaporation to give compound 16-1 (250 mg, 83.22%, purity: 99%).

LC-MS-16-1 (ES, m/z): [M+1] = 335

16.2 Synthesis of compound 16-3

CDI (791.55 mg, 4.882 mmol, 1.1 equiv) was added portionwise to a solution of compound 16-1 (1482 mg, 4.438 mmol, 1 equiv) and compound 16-2 (711 mg, 4.438 mmol, 1.00 equiv) in ACN (2 mL)/DMF (0.4 mL) at 0 °C under nitrogen atmosphere. The resulting mixture was stirred at room temperature overnight. The reaction solution was then concentrated to dryness by rotary evaporation to give a crude product as a yellow oil. The resulting crude product was purified by reversed-phase column chromatography (conditions were as follows: C18 chromatographic column, mobile phase: solvent ACN and solvent $H_2O$ (FA, 0.1%), gradient 10% to 50% for 10 min, UV254 nanometer detector), and the fractions were concentrated to dryness by rotary evaporation to give compound 16-3 (950 mg, 35.05%, purity: 99%).
LC-MS-16-3 (ES, m/z): [M+1] = 611

16.3 Synthesis of compound 16-4

Compound 16-3 (760 mg, 1.244 mmol, 1 equiv) was dissolved in DCM (5 mL), and then TFA (5 mL) was added dropwise to the system described above. The resulting mixture was stirred at room temperature for 2 h. The reaction solution was then concentrated to dryness by rotary evaporation to give compound 16-4 (800 mg, crude).
LC-MS-16-4 (ES, m/z): [M+1] = 455

16.4 Synthesis of compound 16-5

Compound 16-4 (600 mg, 1.320 mmol, 1 equiv) was dissolved in THF (20 mL) at room temperature, and then TEA (267.13 mg, 2.640 mmol, 2 equiv) and $Boc_2O$ (432.11 mg, 1.980 mmol, 1.5 equiv) were added dropwise to the system described above. The resulting mixture was stirred at room temperature for 2 h. The reaction solution was then concentrated to dryness by rotary evaporation to give a crude compound. The resulting crude product was purified by silica gel column chromatography (PE/EA (1/1)), and the fractions were concentrated to dryness by rotary evaporation to give compound 16-5 (600 mg, 81.95%, purity: 99%).
LC-MS-16-5 (ES, m/z): [M+1] = 555

16.5 Synthesis of compound 16-7

Compound 16-5 (600 mg, 1.320 mmol, 1 equiv), compound 16-6 (1975.52 mg, 4.328 mmol, 4 equiv), HOBt (292.33 mg, 2.164 mmol, 2 equiv), DMF (50 mL), DIPEA (279.33 mg, 2.164 mmol, 2 equiv) and TBTU (694.64 mg, 2.164 mmol, 2 equiv) were added to a 100 mL single-necked flask at room temperature. The resulting mixture was stirred at room temperature overnight under nitrogen atmosphere. The reaction solution was filtered. The filter residue was washed with methanol and diethyl ether, and concentrated to dryness by rotary evaporation to give compound 16-7 (2.6 g, crude).

16.6 Synthesis of compound 16-8

Compound 16-7 (2.6 g, 2.618 mmol, 1 equiv), DCM (25 mL, 393.265 mmol, 150.24 equiv) and TFA (25 mL, 336.577 mmol, 128.58 equiv) were added to a 100 mL single-necked flask at room temperature. The resulting mixture was stirred at room temperature overnight under nitrogen atmosphere. The reaction solution was filtered. The filter residue was washed with TFA/DCM (v/v, 1/1), and the filtrate was concentrated to dryness by rotary evaporation to give compound 16-8 (1 g, crude).
LC-MS-16-8 (ES, m/z): [M+1] = 583

16.7 Synthesis of compound 16-9

Compound 16-8 (1 g, 1.716 mmol, 1 equiv) was dissolved in THF (20 mL) at room temperature, and then TEA (0.35 g, 3.432 mmol, 2 equiv) and $Boc_2O$ (0.75 g, 3.432 mmol, 2 equiv) were added dropwise to the system described above. The resulting mixture was stirred at room temperature for 2 h. The reaction solution was then concentrated to dryness by rotary evaporation to give a crude product of compound 10. The resulting crude product was purified by silica gel column chromatography and eluted with PE/EA (1/1), and the fractions were concentrated to dryness by rotary evaporation to give compound 16-9 (700 mg, 52.10%, purity: 99%).
LC-MS-16-9 (ES, m/z): [M+1] = 783

16.8 Synthesis of compound 16-11

Compound 16-9 (60 mg, 0.076 mmol, 1 equiv), compound 16-10 (18.18 mg, 0.115 mmol, 1.5 equiv), TEA (15.51 mg, 0.154 mmol, 2 equiv), DMF (2 mL) and HATU (43.71 mg, 0.115 mmol, 1.5 equiv) were added to a 50 mL single-necked flask at room temperature. The resulting mixture was stirred at room temperature overnight under nitrogen atmosphere. The reaction solution was purified by reversed-phase column chromatography (conditions were as follows: C18 chromatographic column, mobile phase: solvent ACN and solvent $H_2O$ (0.1% FA), gradient 10% to 50% for 10 min, UV254 nanometer detector), and the fractions were concentrated to dryness by rotary evaporation to give compound 16-11 (50 mg, 70.68%, purity: 99%).

LC-MS-16-11 (ES, m/z): [M+1] = 923

16.9 Synthesis of compound 16

Compound 16-11 (60 mg, 0.065 mmol, 1 equiv), DCM (4 mL) and TFA (4 mL) were added to a 50 mL single-necked flask at room temperature. The resulting mixture was stirred at room temperature for 2 h. The reaction solution was concentrated to dryness by rotary evaporation. The crude product was purified by reversed-phase column chromatography (conditions were as follows: C18 chromatographic column, mobile phase: solvent ACN and solvent $H_2O$ (0.1% FA), gradient 10% to 50% for 10 min, UV254 nanometer detector), and the fractions were concentrated to dryness by rotary evaporation to give compound 16 (25 mg, 53.21%, purity: 99.8%).

LC-MS-16 (ES, m/z): [M+1] = 723

$^1$H NMR (400 MHz, Deuterium Oxide) δ 8.37 (s, 2H), 7.49 - 7.17 (m, 6H), 7.06 (q, *J* = 14.0, 8.1 Hz, 4H), 4.58 - 4.33 (m, 3H), 4.33 - 3.99 (m, 2H), 3.87 (dd, *J* = 36.1, 13.2 Hz, 1H), 3.74 - 3.37 (m, 6H), 3.21 (t, *J* = 13.4 Hz, 1H), 3.13 - 2.68 (m, 7H), 2.02 - 1.77 (m, 2H), 1.80 - 1.16 (m, 11H), 0.82 (dd, *J*= 18.2, 5.7 Hz, 6H).

Example 17. Synthesis of Compound 17

According to the similar preparation procedures as in Example 16, the target compound was synthesized, with reactant compound

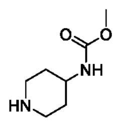

replaced by

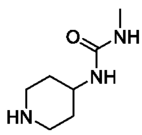

,

specifically, as shown below:

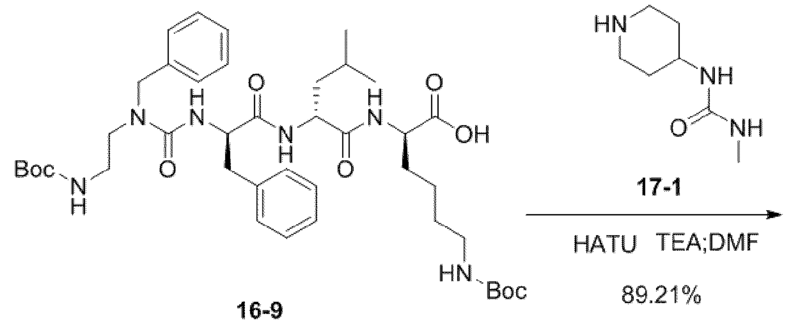


16-9
Key Int III

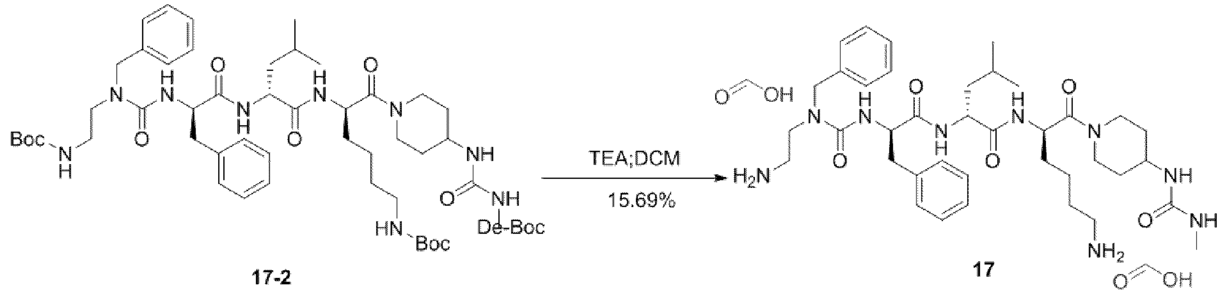


17-2

17

LC-MS-17 (ES, m/z): [M+1] = 721

$^1$H NMR (400 MHz, Deuterium Oxide) δ 8.37 (s, 2H), 7.32 (d, J = 7.3 Hz, 2H), 7.30 - 7.19 (m, 4H), 7.11 (d, J = 7.0 Hz, 1H), 7.05 (dd, J = 10.4, 6.5 Hz, 4H), 4.49 (d, J = 16.9 Hz, 2H), 4.45 - 4.35 (m, 1H), 4.27 (s, 1H), 3.83 (t, J = 13.7 Hz, 1H), 3.58 - 3.41 (m, 2H), 3.26 - 3.13 (m, 1H), 3.02 (did, J = 19.7, 11.6, 4.7 Hz, 3H), 2.96 - 2.87 (m, 3H), 2.87 - 2.75 (m, 2H), 2.62 - 2.56 (m, 2H), 2.54 (s, 1H), 1.87 (dd, J = 19.6, 8.9 Hz, 2H), 1.70 - 1.56 (m, 4H), 1.50 - 1.43 (m, 2H), 1.40 - 1.28 (m, 3H), 0.89 - 0.72 (m, 6H).

Example 18. Synthesis of Compound 18

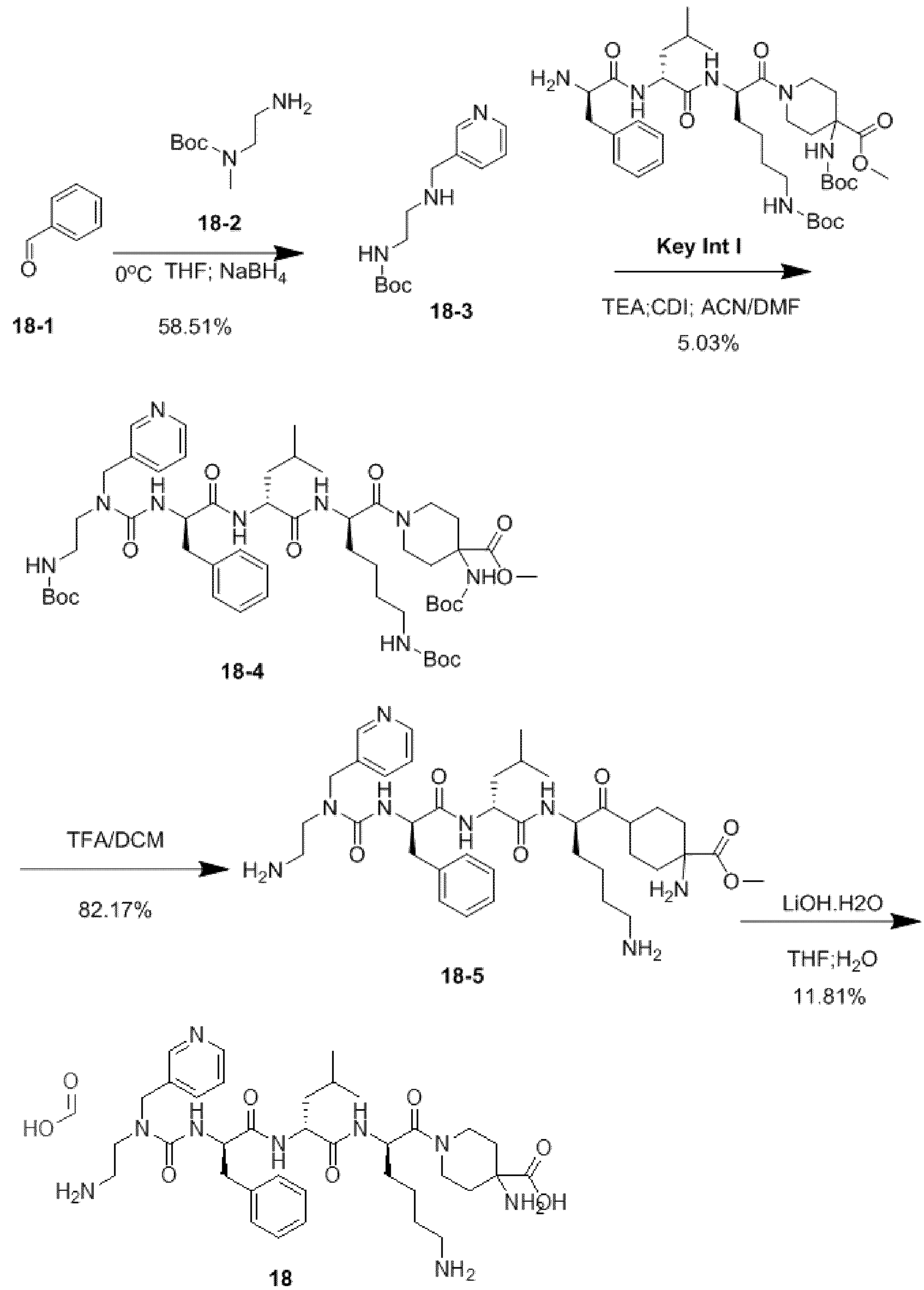


18-1 18-2 18-3 18-4 18-5 18

LC-MS-18 (ES, m/z): [M+1] = 709

$^1$H NMR (400 MHz, Deuterium Oxide) δ 8.32 (s, 1H), 8.29 (s, 1H), 8.08 (d, $J$ = 4.9 Hz, 1H), 7.46 - 7.35 (m, 1H), 7.29 (dd, $J$ = 8.1, 5.1 Hz, 1H), 7.10 (dd, $J$ = 4.4, 2.2 Hz, 3H), 6.96 (t, $J$ = 4.3 Hz, 2H), 4.48 (s, 1H), 4.46 - 4.32 (m, 2H), 4.22 (t, $J$ = 7.0 Hz, 1H), 3.85 - 3.22 (m, 7H), 3.05 - 2.87 (m, 3H), 2.83 (t, $J$ = 7.7 Hz, 2H), 2.73 (ddd, $J$ = 14.7, 9.6, 5.7 Hz, 1H), 2.27 - 1.94 (m, 2H), 1.88 - 1.09 (m, 11H), 0.86 - 0.64 (m, 6H).

Example 19. Synthesis of Compound 19

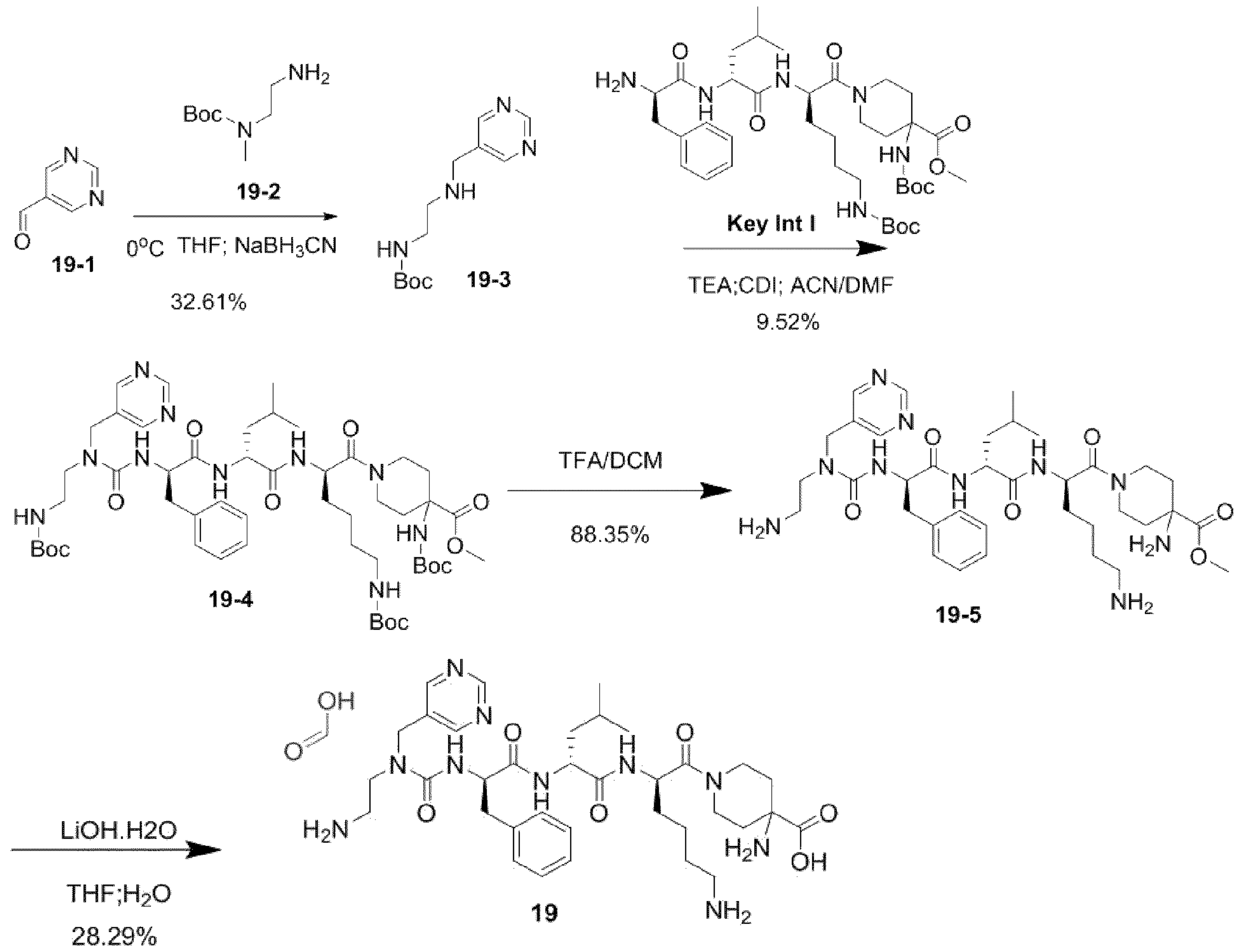


LC-MS-19 (ES, m/z): [M+1] = 710

$^1$H NMR (400 MHz, Deuterium Oxide) δ 8.95 (d, $J$ = 2.4 Hz, 1H), 8.37 (s, 2H), 8.35 (s, 1H), 7.09 (dq, $J$ = 45.5, 3.8, 3.3 Hz, 5H), 4.61 (d, $J$ = 18.1 Hz, 2H), 4.52 - 4.40 (m, 2H), 4.31 (t, $J$ = 6.7 Hz, 1H), 3.94 - 3.24 (m, 6H), 3.05 (dt, $J$ = 22.4, 6.0 Hz, 3H), 2.91 (t, $J$ = 7.7 Hz, 2H), 2.78 (ddd, $J$ = 14.1, 10.2, 7.5 Hz, 1H), 2.14 (ddd, $J$ = 35.9, 12.0, 5.5 Hz, 2H), 1.94 - 1.21 (m, 11H), 0.97 - 0.69 (m, 6H).

Example 20. Synthesis of Compound 20

According to the similar preparation procedures as in Example 16, the target compound was synthesized, with reactant compound

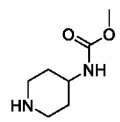

replaced by

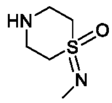
,

specifically, as shown below:

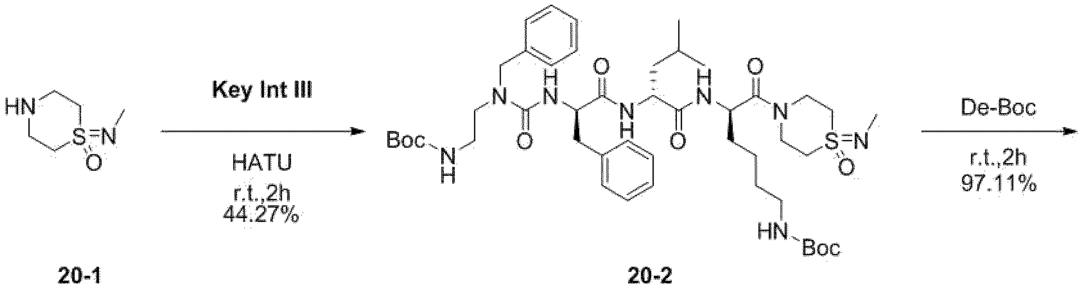


20-1

20-2

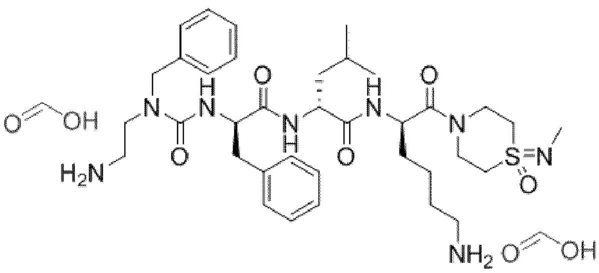

20

LC-MS-20 (ES, m/z): [M+1] = 713

$^1$H NMR (400 MHz, Deuterium Oxide) δ 8.36 (s, 2H), 7.33 (s, 1H), 7.33 - 7.23 (m, 2H), 7.27 - 7.21 (m, 2H), 7.10 - 7.00 (m, 5H), 4.74 (s, 1H), 4.54 - 4.44 (m, 2H), 4.39 (d, J = 17.5 Hz, 1H), 4.28 (s, 1H), 4.20 (d, J = 15.1 Hz, 1H), 4.05 (s, 1H), 3.98 - 3.76 (m, 1H), 3.72 (d, J = 14.2 Hz, 1H), 3.63 - 3.55 (m, 1H), 3.48 (dd, J = 13.9, 7.6 Hz, 2H), 3.36 (s, 2H), 3.32 - 3.21 (m, 1H), 3.02 (dq, J = 20.9, 5.7 Hz, 6H), 2.98 - 2.80 (m, 1H), 2.73 - 2.64 (m, 2H), 1.68 (d, J = 6.1 Hz, 1H), 1.62 (s, 5H), 1.47 (s, 4H), 1.35 (d, J = 12.0 Hz, 1H), 0.85 - 0.80 (d, J = 5.5 Hz, 6H).

Example 21. Synthesis of Compound 21

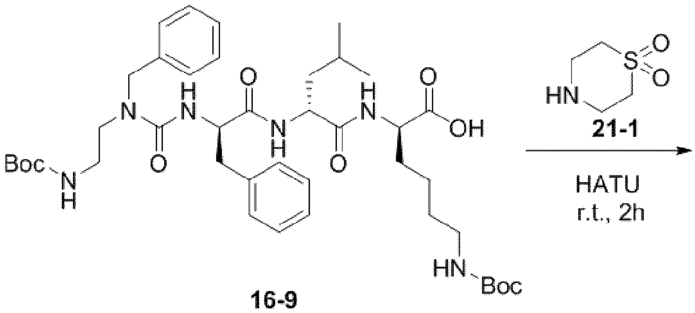


16-9
Key Int III

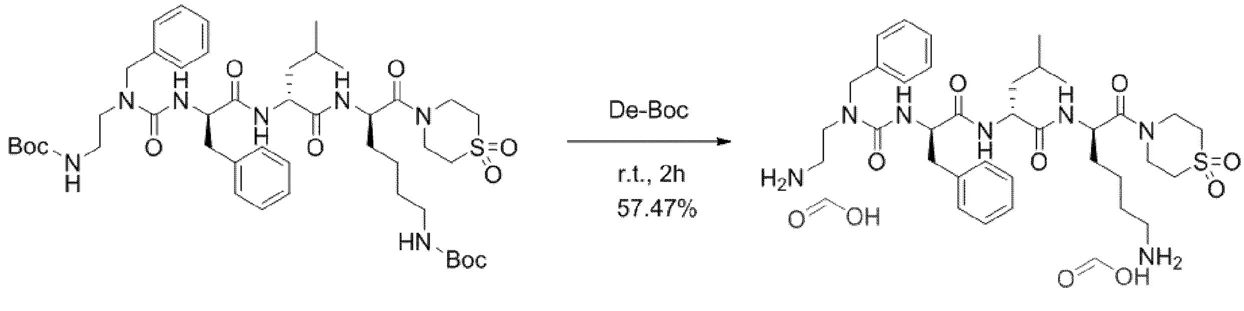


21-2

21

21.1 Synthesis of compound 21-2

Compound 21-1 (34.53 mg, 0.256 mmol, 4 equiv) was added to a solution of intermediate 3 (50 mg, 0.064 mmol, 1 equiv) in DMF (10 mL) at room temperature under nitrogen atmosphere. After the resulting mixture was stirred for 2 h, HATU (36.42 mg, 0.096 mmol, 1.5 equiv) and DIPEA (16.51 mg, 0.128 mmol, 2 equiv) were added dropwise at room temperature. The resulting mixture was extracted with EA (3 × 100 mL). The organic layer was washed with brine (3 × 10 mL) and dried over $Na_2SO_4$. The resulting filtrate was concentrated under reduced pressure. The resulting crude product was purified by reversed-phase column chromatography (conditions were as follows: C18 chromatographic column, mobile phase: solvent ACN and solvent $H_2O$ (0.1% FA), gradient 10% to 50% for 10 min, UV200 nanometer detector) to give compound 21-2 (58 mg, crude).
LC-MS-21-2 (ES, m/z): [M+1] = 900

21.2 Synthesis of compound 21

A mixed solution of compound 21-2 (40 mg, 0.044 mmol, 1 equiv) in TFA (1 mL)/DCM (4 mL) was stirred at room temperature for 2 h under nitrogen atmosphere. The resulting mixture filtrate was concentrated under reduced pressure. The resulting crude product was purified by reversed-phase column chromatography (conditions were as follows: C18 chromatographic column, mobile phase: solvent ACN and solvent $H_2O$ (0.1% FA), gradient 10% to 50% for 10 min, UV200 nanometer detector), and the fractions were concentrated to dryness by rotary evaporation to give compound 21 (18.6 mg, 57.47%, purity: 96.0%).
LC-MS-21 (ES, m/z): [M+1] = 700
$^1H$ NMR (400 MHz, Deuterium Oxide) δ 8.36 (s, 2H), 7.31 (d, *J* = 7.3 Hz, 3H), 7.24 (q, *J* = 3.6, 3.1 Hz, 3H), 7.05 (dd, *J* = 12.4, 5.8 Hz, 4H), 4.74 (s, 2H), 4.49 (dt, *J* = 13.3, 4.4 Hz, 2H), 4.39 (d, *J* = 17.5 Hz, 1H), 4.28 (s, 3H), 4.19 (d, *J* = 14.8 Hz, 1H), 3.88 (q, *J* = 15.4, 14.7 Hz, 1H), 3.70 (d, *J* = 15.5 Hz, 1H), 3.67 - 3.51 (m, 1H), 3.48 (s, 1H), 3.28 (d, *J* = 16.0 Hz, 1H), 3.19 (s, 2H), 3.09 - 2.96 (m, 3H), 2.87 (dt, *J* = 27.6, 8.3 Hz, 3H), 1.69 (t, *J* = 7.4 Hz, 2H), 1.62 (q, *J* = 7.5 Hz, 2H), 1.47 (s, 4H), 1.35 (dt, *J* = 16.9, 8.0 Hz, 2H), 0.83 (dt, *J* = 19.1, 3.9 Hz, 6H).

Example 22. Synthesis of Compound 22

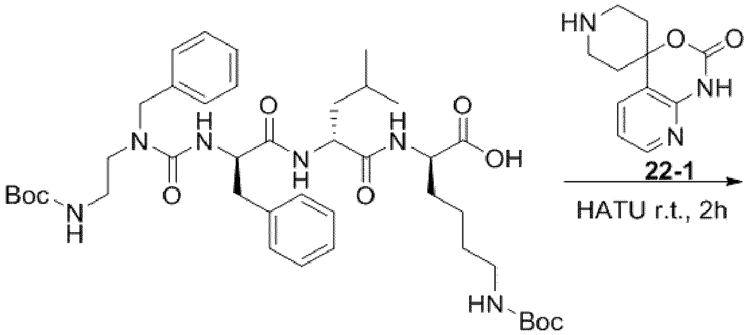


16-9
Key Int III

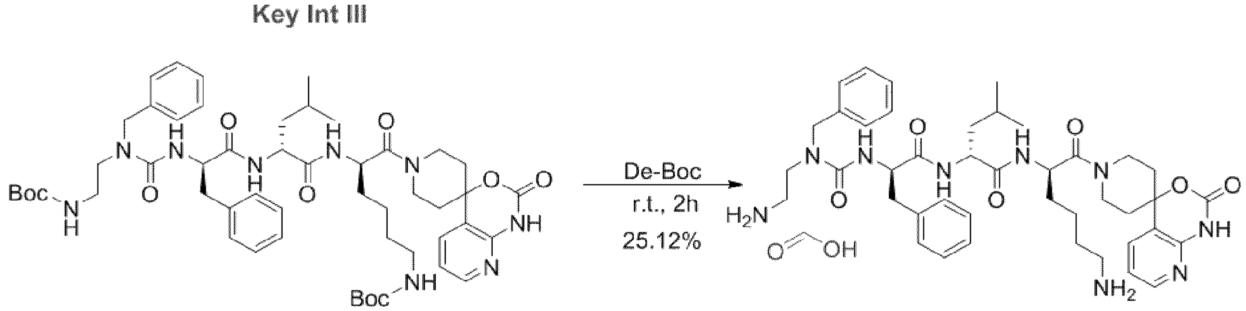


22-2

22

LC-MS-22 (ES, m/z): [M+1] = 784
$^1H$ NMR (400 MHz, Deuterium Oxide) δ 8.37 (s, 1H), 8.11 (s, 1H), 7.61 (t, *J* = 7.9 Hz, 1H), 7.40 (d, *J* = 3.4 Hz, 1H), 7.31 - 7.19 (m, 5H), 7.18 - 6.99 (m, 5H), 4.53 - 4.38 (m, 2H), 4.36 (s, 3H), 4.28 (t, *J*= 7.6 Hz, 1H), 4.00 (d, *J* = 13.8 Hz, 1H),

3.89 (s, 1H), 3.61 (d, *J* = 13.3 Hz, 1H), 3.55 (s, 1H), 3.47 (ddd, *J* = 21.8, 14.8, 6.7 Hz, 3H), 3.18 - 2.99 (m, 3H), 2.95 - 2.82 (m, 5H), 2.17 (s, 3H), 1.99 (d, *J* = 17.9 Hz, 3H), 1.92 (s, 2H), 1.62 (p, *J* = 6.8 Hz, 2H), 1.47 (d, *J* = 7.3 Hz, 1H), 1.37 (s, 4H), 1.20 (t, *J* = 7.3 Hz, 1H), 0.82 (dt, *J* = 16.5, 6.7 Hz, 6H), 0.67 (d, *J* = 6.2 Hz, 1H).

Example 23. Synthesis of Compound 23

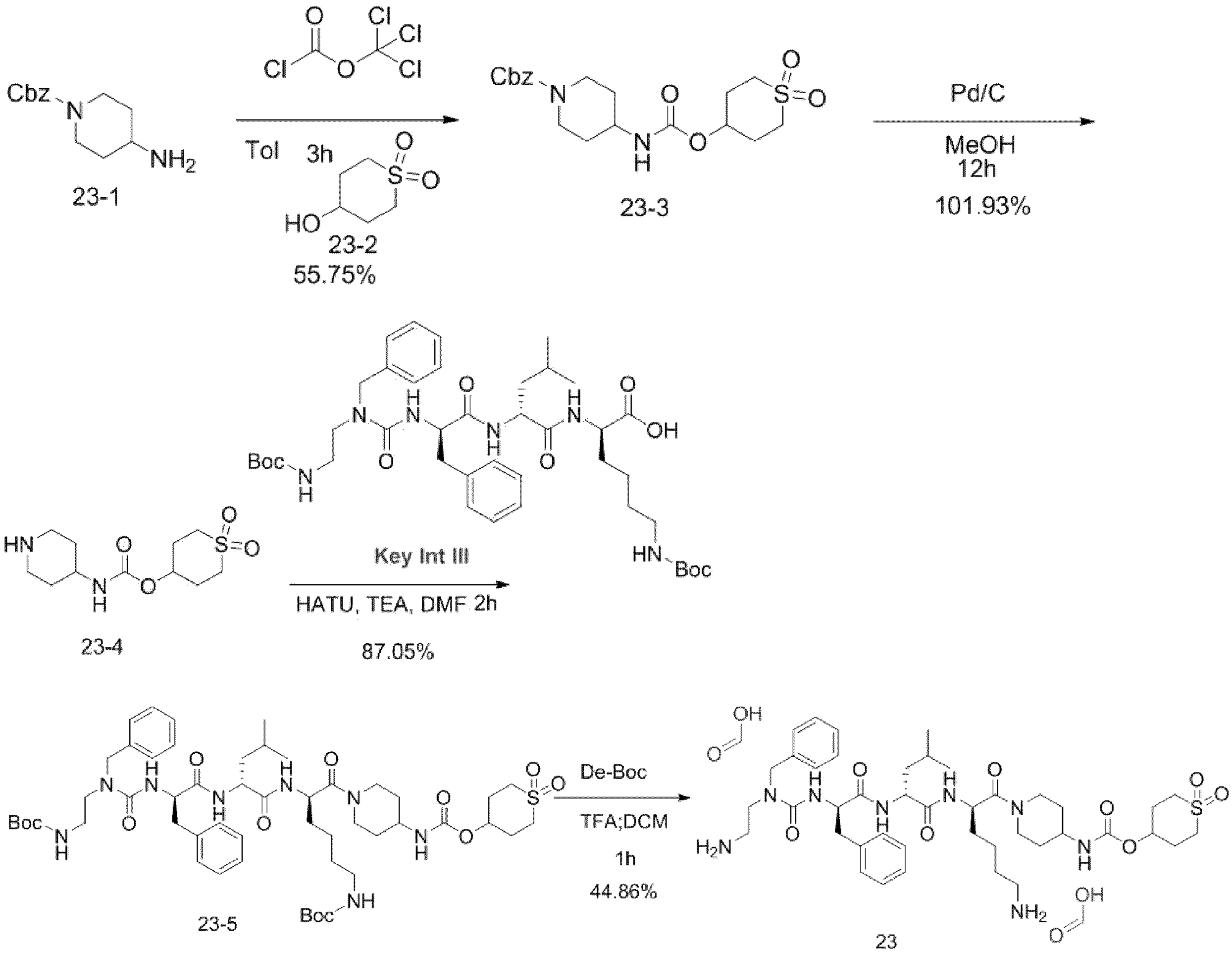

23-1 23-2 23-3 23-4 23-5 23

LC-MS-23 (ES, m/z): [M+1] = 840.46

$^1$H NMR (400 MHz, Deuterium Oxide) δ 8.29 (s, 2H), 7.31 - 7.08 (m, 6H), 7.00 (dd, *J* = 35.0, 7.4 Hz, 4H), 4.80 (s, 1H), 4.48 - 3.95 (m, 6H), 3.54 - 3.35 (m, 3H), 3.28 - 2.66 (m, 12H), 2.15 (s, 4H), 1.79 (s, 3H), 1.65 - 1.15 (m, 11H), 0.83 - 0.55 (m, 6H).

Example 24. Synthesis of Compound 24

According to the similar preparation procedures as in Example 16, the target compound was synthesized, with reactant compound

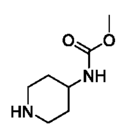

replaced by

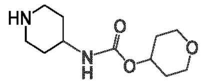

,

specifically, as shown below:

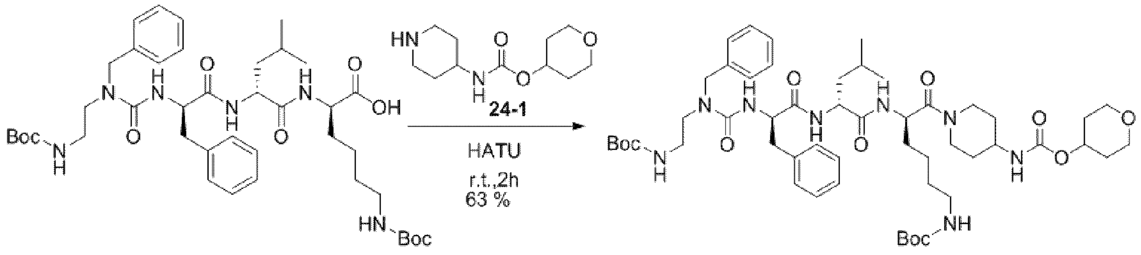


16-9
Key Int III

24-2

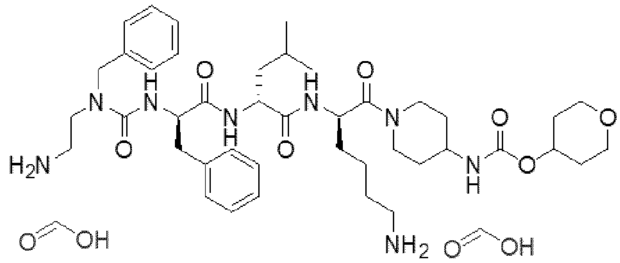

24

LC-MS-24 (ES, m/z): [M+1] = 793

$^1$H NMR (400 MHz, Deuterium Oxide) δ 8.37 (s, 2H), 7.31 (d, J = 7.4 Hz, 2H), 7.30 - 7.21 (m, 3H), 7.12 (d, J = 6.6 Hz, 1H), 7.04 (t, J = 9.5 Hz, 4H), 4.48 (d, J = 17.6 Hz, 3H), 4.39 (d, J = 17.8 Hz, 1H), 4.27 (s, 1H), 4.20 (d, J = 12.5 Hz, 1H), 4.07 (d, J = 13.1 Hz, 2H), 3.55 (s, 5H), 3.20 (d, J = 13.5 Hz, 1H), 2.96 (s, 6H), 2.89 (s, 1H), 1.86 (s, 4H), 1.60 (s, 6H), 1.46 (d, J = 6.9 Hz, 1H), 1.35 (s, 6H), 0.81-0.66 (dd, J = 17.8, 5.7 Hz, 6H).

Example 25. Synthesis of Compound 25

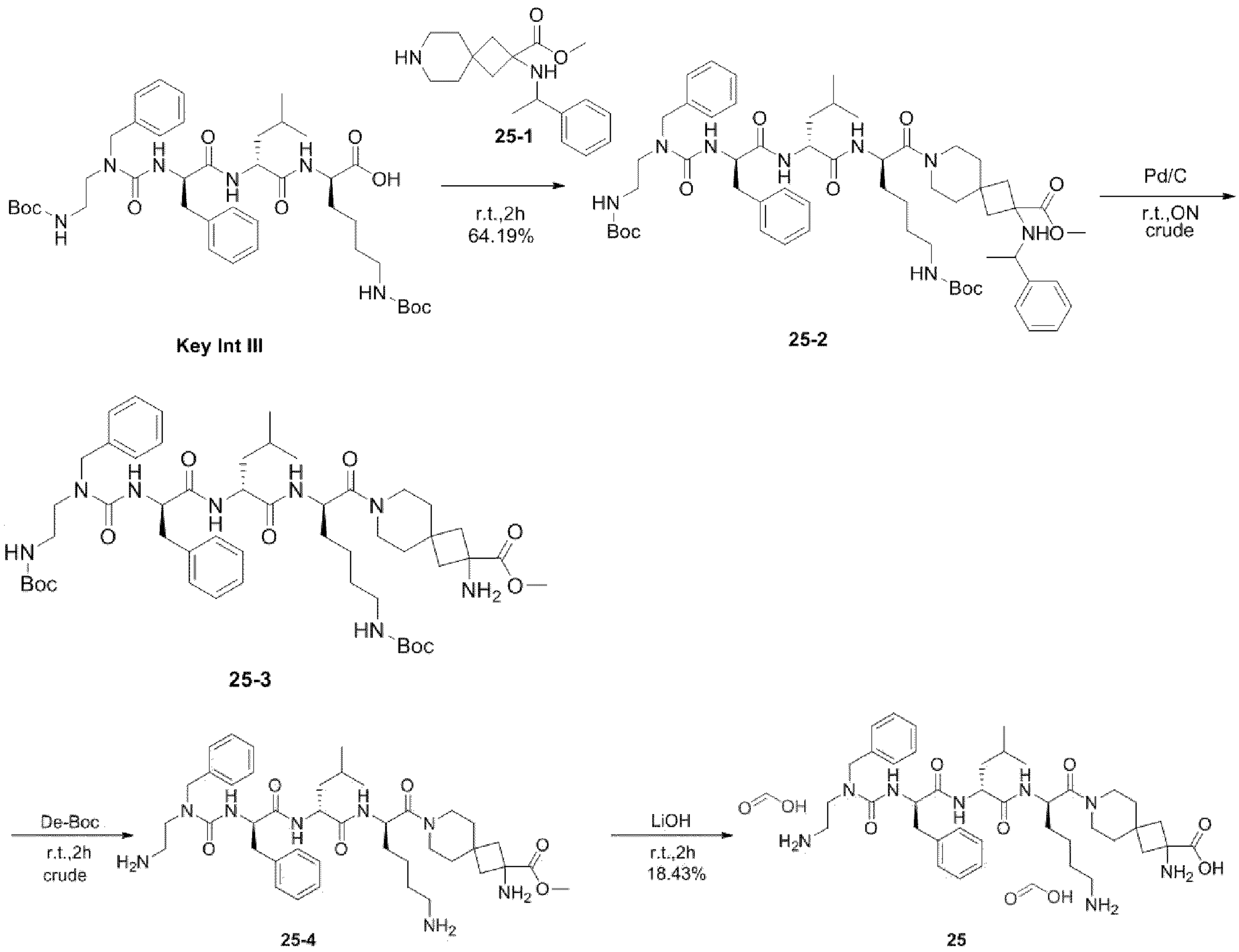


25-1

Key Int III

25-2

25-3

25-4

25

LC-MS-25 (ES, m/z): [M+1] = 853

$^1$H NMR (400 MHz, Deuterium Oxide) δ 8.38 (s, 2H), 7.32 (d, $J$ = 7.3 Hz, 3H), 7.32 - 7.20 (m, 3H), 7.05 (d, $J$ = 17.7 Hz, 1H), 7.05 (s, 3H), 4.27 (d, $J$ = 7.3 Hz, 1H), 3.53 (s, 4H), 3.41 (s, 1H), 3.31 (s, 1H), 3.03 (dd, $J$ = 15.0, 8.3 Hz, 3H), 2.89 (q, $J$ = 12.2, 9.9 Hz, 3H), 2.49 (t, $J$ = 12.5 Hz, 2H), 2.19 - 2.09 (m, 2H), 1.82 (s, 2H), 1.63 (s, 8H), 1.51 - 1.43 (m, 4H), 0.82 (dd, $J$ = 18.6, 5.9 Hz, 6H).

Example 26. Synthesis of Compound 26

According to the similar preparation procedures as in Example 16, the target compound was synthesized, with reactant compound

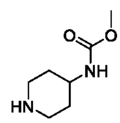

replaced by

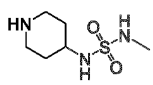,

specifically, as shown below:

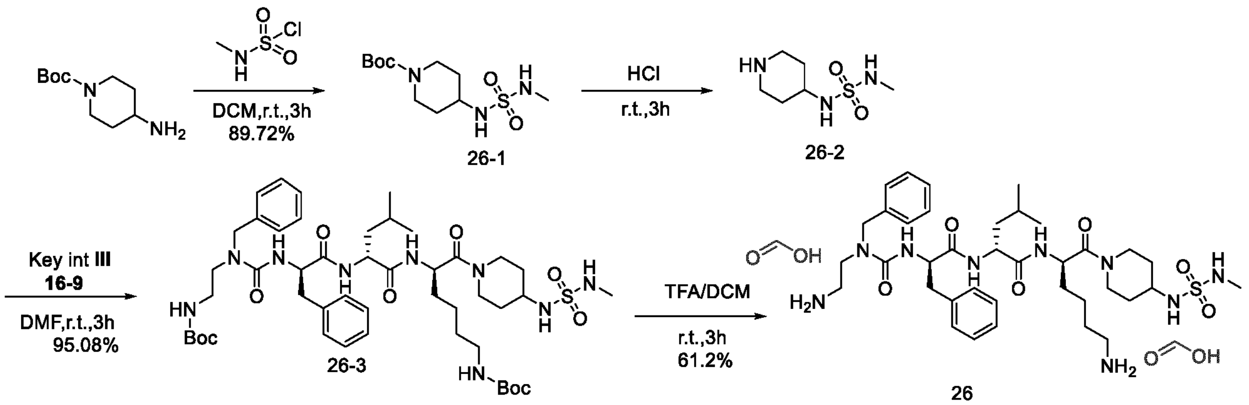


LC-MS-26 (ES, m/z): [M+1] = 758

$^1$H NMR (400 MHz, Deuterium Oxide) δ 8.39 (s, 2H), 7.40 - 7.18 (m, 6H), 7.16 - 7.00 (m, 4H), 4.56 - 4.47 (m, 2H), 4.47 - 4.35 (m, 2H), 4.30 (dq, *J* = 10.1, 5.2, 4.5 Hz, 1H), 4.20 (d, *J* = 12.0 Hz, 1H), 4.10 (td, *J* = 13.3, 11.5, 6.9 Hz, 1H), 3.58 (dd, *J* = 15.1, 6.5 Hz, 1H), 3.54 - 3.43 (m, 1H), 3.37 (dp, *J* = 11.2, 5.1 Hz, 1H), 3.28 - 3.14 (m, 1H), 3.10 - 2.94 (m, 3H), 2.92 (dd, *J* = 7.8, 4.0 Hz, 1H), 2.90 - 2.77 (m, 2H), 2.56 (dd, *J* = 11.6, 4.2 Hz, 3H), 2.00 (s, 1H), 1.94 (d, *J* = 8.6 Hz, 1H), 1.75 - 1.55 (m, 4H), 1.55 - 1.40 (m, 4H), 1.40 - 1.27 (m, 3H), 0.82 (ddd, *J* = 17.6, 6.3, 2.6 Hz, 4H), 0.74 (dd, *J* = 6.8, 3.3 Hz, 1H), 0.67 (t, *J* = 5.4 Hz, 1H).

Example 27. Synthesis of Compound 27

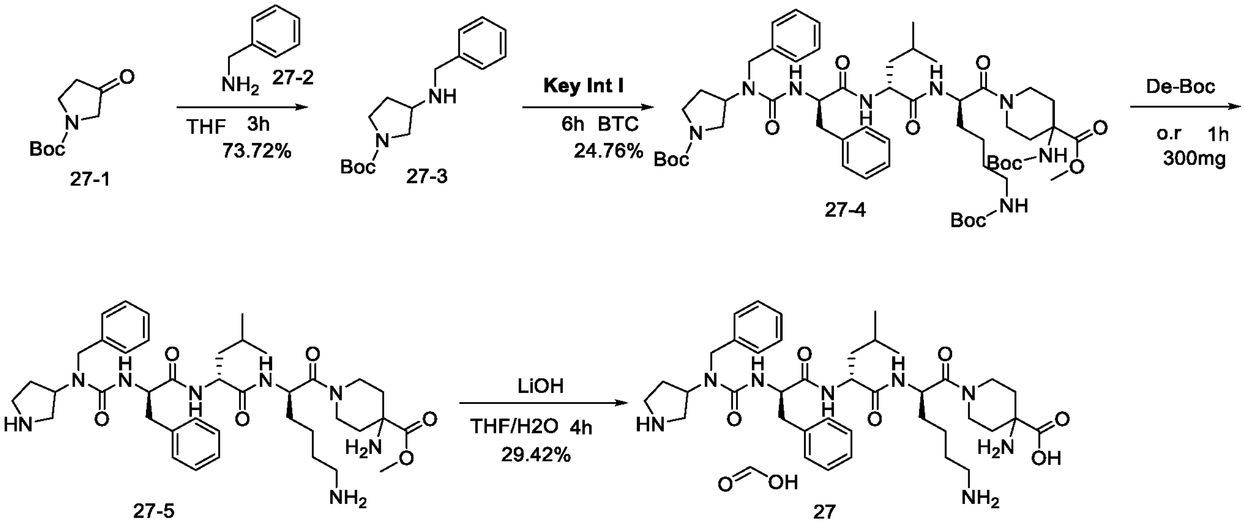


LC-MS-27 (ES, m/z): [M+1] = 736

$^1$H NMR (400 MHz, Deuterium Oxide) δ 8.33 (s, 1H), 7.52 - 7.10 (m, 6H), 6.98 (q, *J* = 4.8, 3.9 Hz, 4H), 4.57 - 4.26 (m, 4H), 4.21 (dd, *J* = 9.8, 4.8 Hz, 1H), 3.71 (tt, *J* = 13.0, 5.6 Hz, 3H), 3.63 - 3.31 (m, 3H), 3.25 (td, *J* = 12.2, 8.6 Hz, 1H), 3.17 - 2.90 (m, 3H), 2.91 - 2.58 (m, 3H), 2.39 - 1.98 (m, 3H), 1.94 - 1.15 (m, 12H), 0.96 - 0.44 (m, 6H).

Example 28. Synthesis of Compound 28

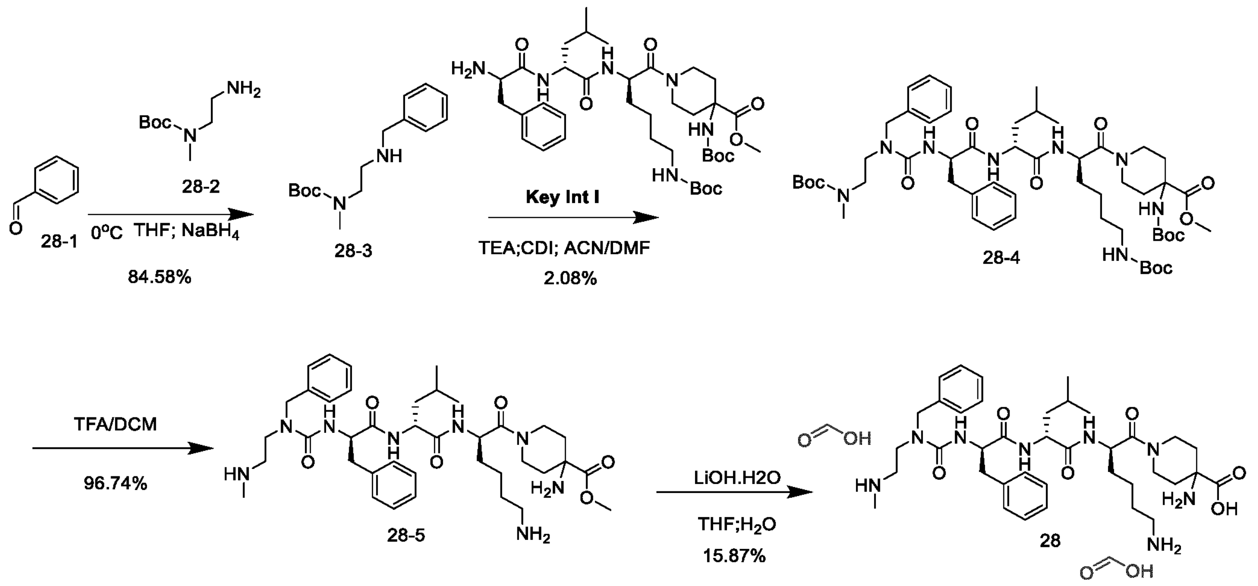


LC-MS-28 (ES, m/z): [M+1] = 722

$^1$H NMR (400 MHz, Deuterium Oxide) δ 8.38 (s, 2H), 7.41 - 7.18 (m, 6H), 7.14 - 6.91 (m, 4H), 4.58 - 4.22 (m, 5H), 3.95 - 3.35 (m, 7H), 3.16 - 2.78 (m, 7H), 2.61 (s, 3H), 2.33 - 2.01 (m, 3H), 1.94 - 1.25 (m, 12H), 0.84 (dd, J = 18.5, 5.4 Hz, 6H).

Example 29. Synthesis of Compound 29

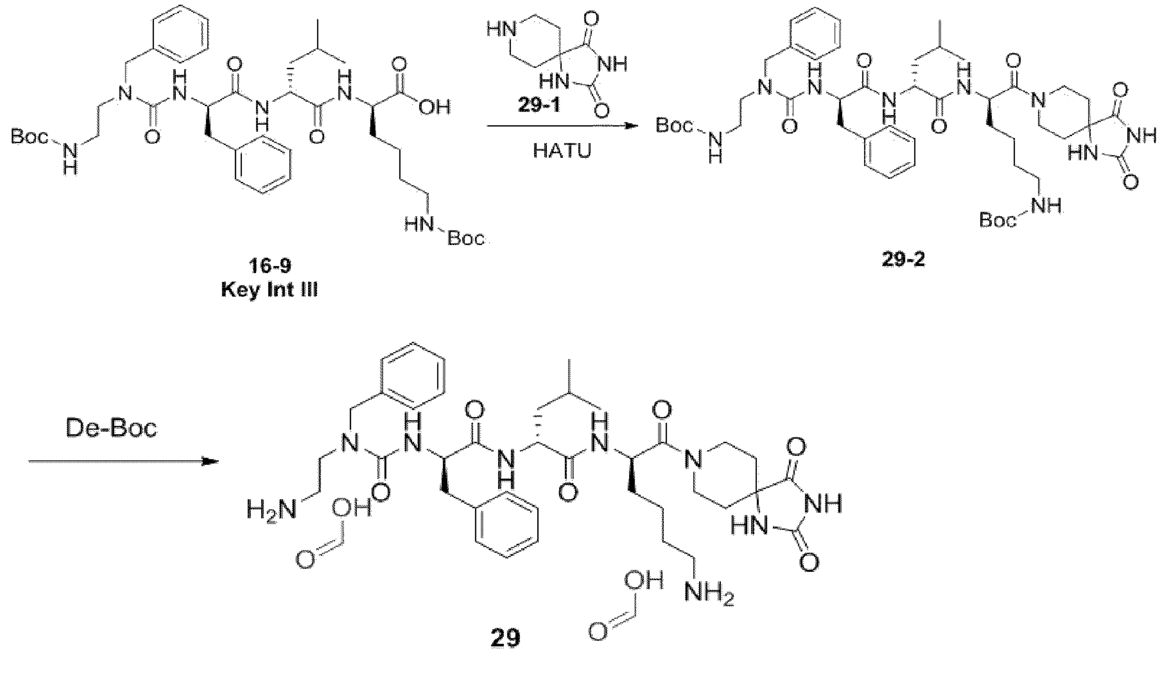


LC-MS-29 (ES, m/z): [M+1] = 734

$^1$H NMR (400 MHz, Deuterium Oxide) δ 8.36 (s, 2H), 7.32 (d, *J* = 7.5 Hz, 3H), 7.23 (dt, *J* = 7.7, 4.0 Hz, 3H), 7.04 (d, *J* = 7.1 Hz, 4H), 4.51 (s, 1H), 4.42 (dd, *J* = 22.2, 16.8 Hz, 1H), 4.31 - 4.20 (m, 3H), 4.09 - 3.97 (m, 2H), 3.94 (d, *J* = 10.0 Hz, 2H), 3.57 (s, 1H), 3.49 (dd, *J* = 9.9, 5.5 Hz, 1H), 3.33 (dd, *J* = 28.5, 14.0 Hz, 1H), 3.06 (d, *J* = 7.0 Hz, 1H), 3.01 (s, 3H), 2.95 - 2.80 (m, 3H), 1.88 (d, *J* = 9.0 Hz, 2H), 1.83 - 1.72 (m, 1H), 1.72 - 1.60 (m, 4H), 1.48 (s, 3H), 1.34 (s, 3H), 0.83 (dd, *J* = 19.5, 5.7 Hz, 6H).

Example 30. Synthesis of Compound 30

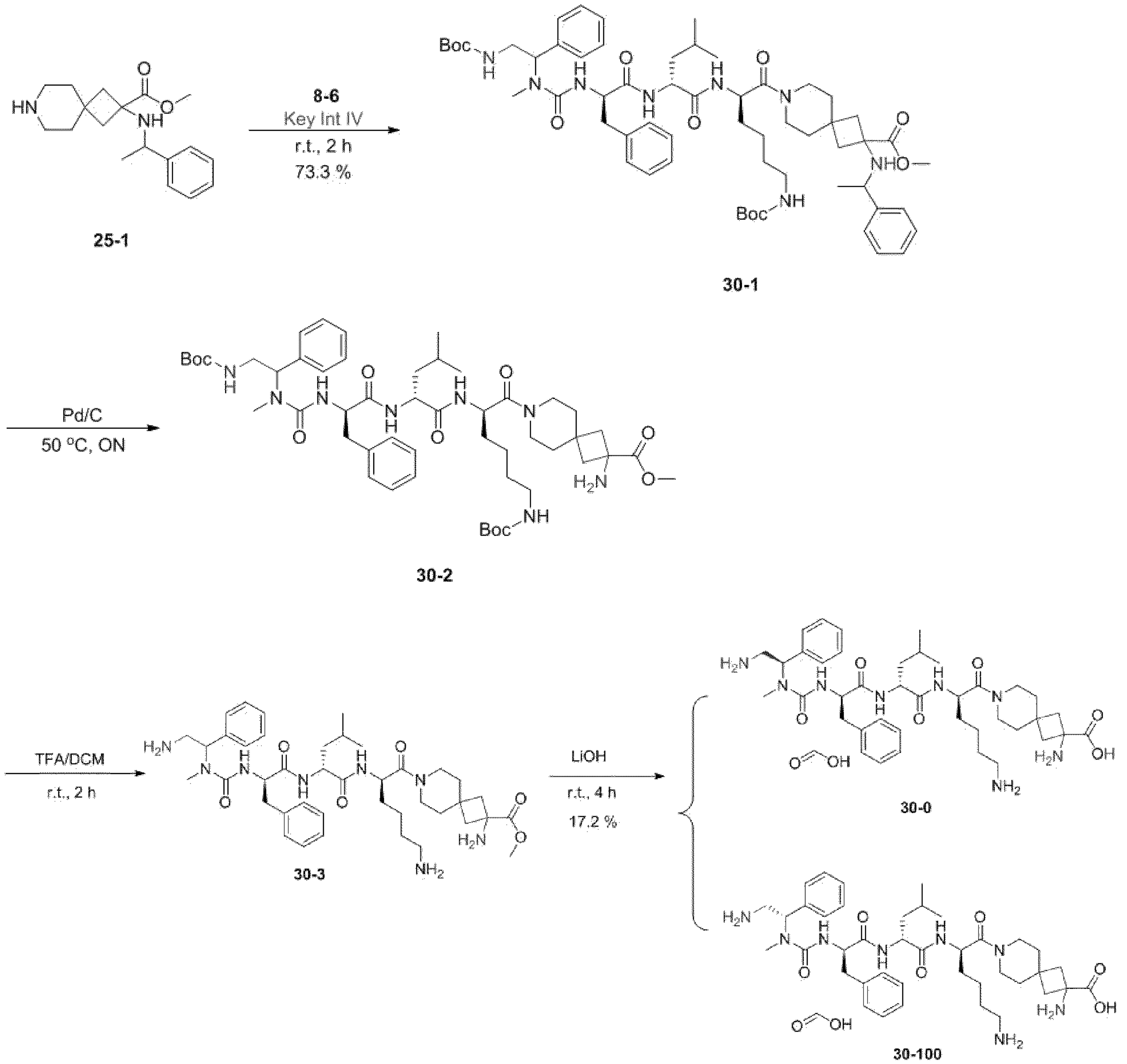


25-1

30-1

30-2

30-3

30-0

30-100

LC-MS-30-0 (ES, m/z): [M+1] = 749

$^1$H NMR (400 MHz, Deuterium Oxide) δ 8.37 (s, 1H), 7.68 - 6.76 (m, 10H), 5.47 (d, *J* = 11.3 Hz, 1H), 4.61 - 4.18 (m, 2H), 3.78 - 3.24 (m, 6H), 3.21 - 2.76 (m, 3H), 2.70 - 2.33 (m, 5H), 2.11 (dd, *J* = 14.2, 7.6 Hz, 2H), 1.95 - 1.15 (m, 14H), 1.15 - 0.39 (m, 6H).

LC-MS-30-100 (ES, m/z): [M+1] = 749

$^1$H NMR (400 MHz, Deuterium Oxide) δ 8.37 (s, 1H), 7.57 - 6.76 (m, 10H), 5.51 (d, *J* = 11.1 Hz, 1H), 4.35 (d, *J* = 6.8 Hz, 1H), 3.75 - 3.12 (m, 6H), 3.06 - 2.67 (m, 3H), 2.46 (d, *J* = 17.8 Hz, 5H), 2.12 (dd, *J* = 13.7, 7.4 Hz, 2H), 2.00 - 1.18 (m, 12H), 0.85 (dd, *J* = 17.1, 5.1 Hz, 6H).

Example 31. Synthesis of Compound 31

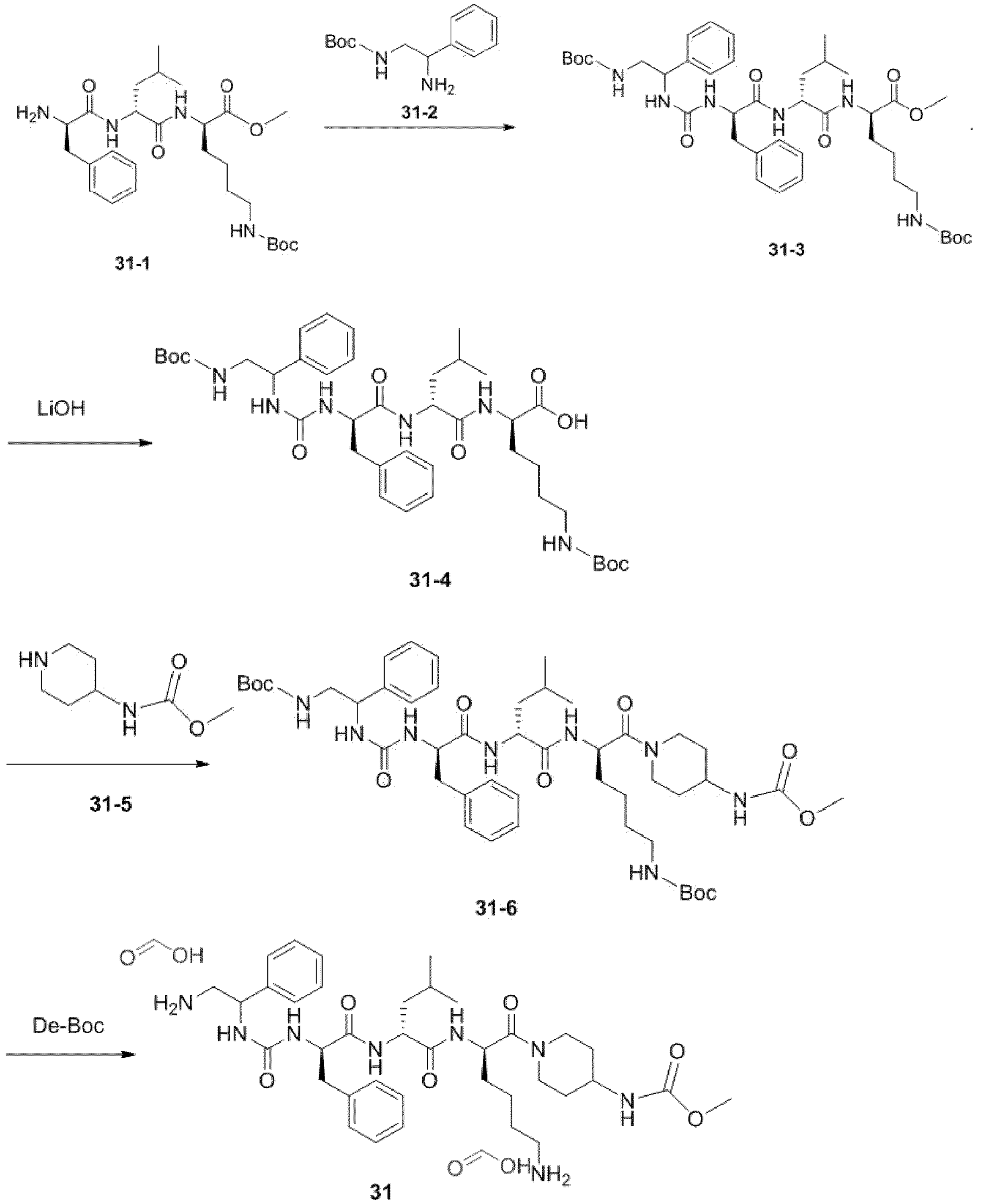


LC-MS-31 (ES, m/z): [M+1] = 709

$^1$H NMR (400 MHz, Deuterium Oxide) δ 8.34 (s, 2H), 7.76 - 6.68 (m, 10H), 5.15 - 4.80 (m, 2H), 4.56 - 3.67 (m, 6H), 3.61 (d, *J* = 16.4 Hz, 4H), 3.30 - 2.97 (m, 4H), 3.11 - 2.70 (m, 4H), 2.19 - 1.79 (m, 2H), 1.76 - 1.06 (m, 11H), 0.95 - 0.39 (m, 6H).

Example 32. Synthesis of Compound 32

According to the similar preparation procedures as in Example 8, the target compound was synthesized, with reactant compound

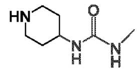

replaced by

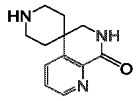

,

specifically, as shown below:

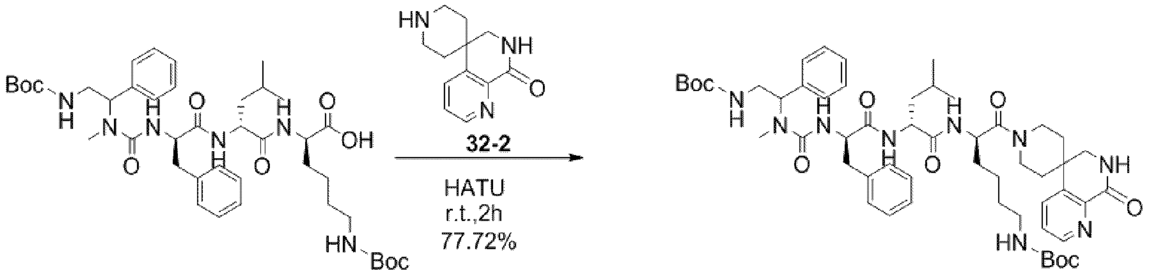


32-1

32-3

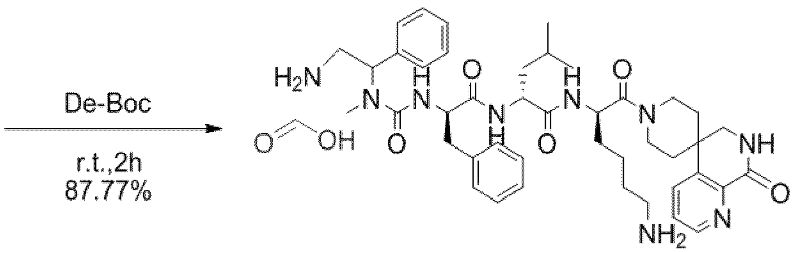


32

LC-MS-32 (ES, m/z): [M+1] = 782

$^{1}$H NMR (400 MHz, Deuterium Oxide) δ 8.51 (s, 1H), 8.36 (s, 1H), 7.93 (s, 1H), 7.57 (s, 1H), 7.35 (d, J = 7.2 Hz, 2H), 7.31 - 7.26 (m, 4H), 7.22 (d, J = 7.7 Hz, 2H), 7.13 (d, J = 7.4 Hz, 1H), 6.89 (d, J = 6.8 Hz, 1H), 5.57 - 5.49 (m, 1H), 4.49 - 4.41 (m, 1H), 4.33 (s, 2H), 4.24 - 4.16 (m, 1H), 3.94 (s, 1H), 3.69 - 3.50 (m, 1H), 3.49 (s, 3H), 3.24 (dd, J = 13.7, 5.2 Hz, 1H), 3.07 (s, 1H), 3.00 - 2.78 (m, 2H), 2.53 (d, J = 8.6 Hz, 3H), 2.43 (d, J = 2.8 Hz, 3H), 1.87 (s, 2H), 1.68 - 1.58 (m, 2H), 1.57 (s, 6H), 1.52 (s, 1H), 1.35 (s, 3H), 0.90 - 0.78 (m, 2H), 0.83 (s, 5H).

Example 33. Synthesis of Compound 33

According to the similar preparation procedures as in Example 8, the target compound was synthesized, with reactant compound

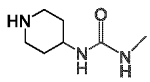

replaced by

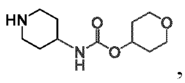

,

specifically, as shown below:

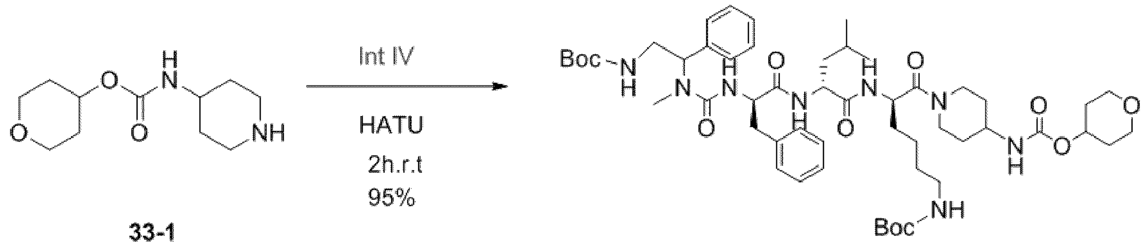


33-1

33-2

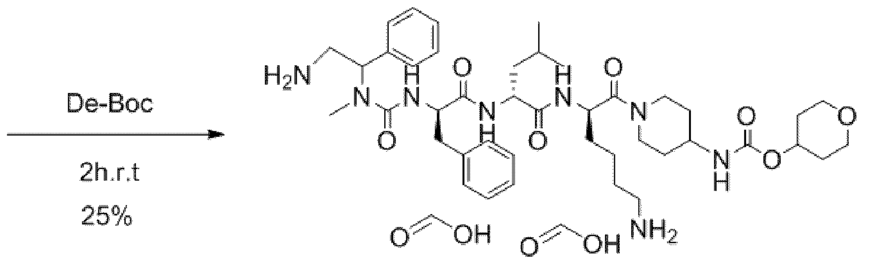


33

LC-MS-33 (ES, m/z): [M+1] = 793

$^1$H NMR (400 MHz, Deuterium Oxide) δ 8.36 (s, 2H), 7.59 - 7.10 (m, 9H), 6.89 (d, *J* = 6.7 Hz, 1H), 5.54 (dd, *J* = 11.4, 4.5 Hz, 1H), 4.56 - 4.25 (m, 2H), 4.25 - 4.03 (m, 1H), 3.96 - 3.78 (m, 3H), 3.72 - 3.42 (m, 5H), 3.34 - 3.05 (m, 2H), 3.05 - 2.73 (m, 4H), 2.49 (d, *J* = 39.1 Hz, 3H), 1.87 (s, 4H), 1.57 (d, *J* = 31.1 Hz, 9H), 1.34 (dd, *J* = 24.6, 11.5 Hz, 4H), 0.85 (dt, *J* = 13.0, 7.0 Hz, 6H).

Example 34. Synthesis of Compound 34

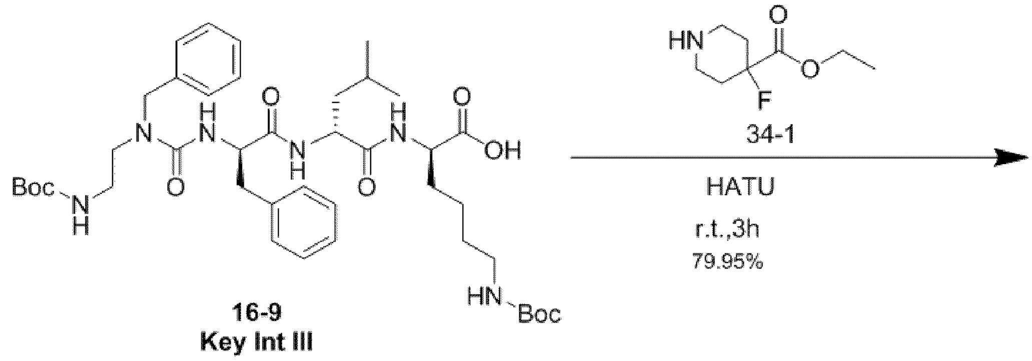


16-9
Key Int III

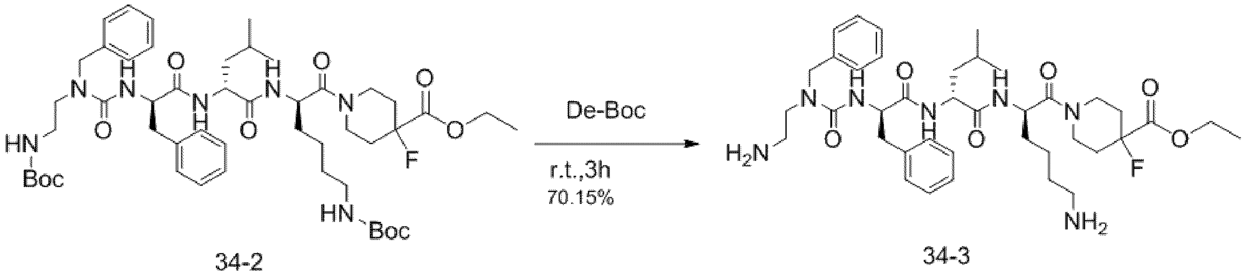


34-2

34-3

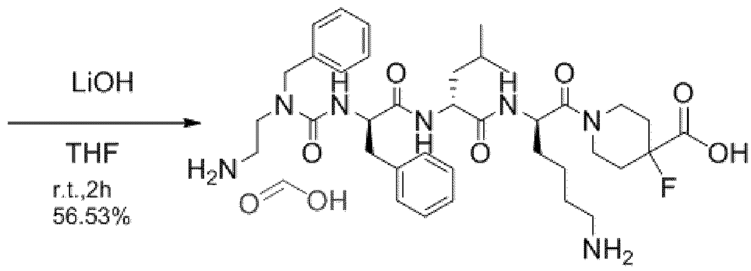


34

LC-MS-34 (ES, m/z): [M+1] = 712

$^1$H NMR (400 MHz, Deuterium Oxide) δ 8.26 (s, 1H), 7.28 - 7.18 (m, 3H), 7.15 (td, J = 4.4, 1.9 Hz, 3H), 7.01 - 6.90 (m, 4H), 4.67 (s, 1H), 4.47 - 4.31 (m, 3H), 4.31 - 4.18 (m, 2H), 3.81 (s, 1H), 3.51 (dt, J = 15.1, 5.8 Hz, 1H), 3.35 (ddd, J = 28.0, 13.3, 7.3 Hz, 2H), 3.02 - 2.87 (m, 4H), 2.80 (dddd, J = 23.1, 13.9, 9.1, 4.7 Hz, 4H), 2.03 - 1.77 (m, 4H), 1.56 (ddt, J = 20.7, 8.8, 4.7 Hz, 4H), 1.44 - 1.20 (m, 5H), 0.74 (ddd, J = 15.8, 5.2, 2.4 Hz, 6H).

Example 35. Synthesis of Compound 35

According to the similar preparation procedures as in Example 16, the target compound was synthesized, with reactant compound

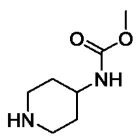

replaced by

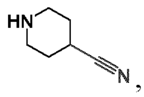,

specifically, as shown below:

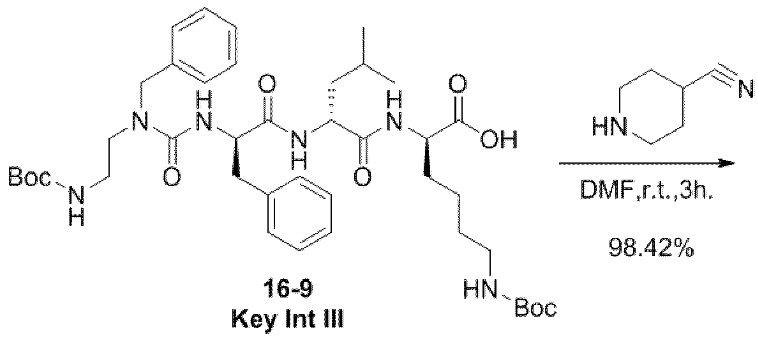


16-9
**Key Int III**

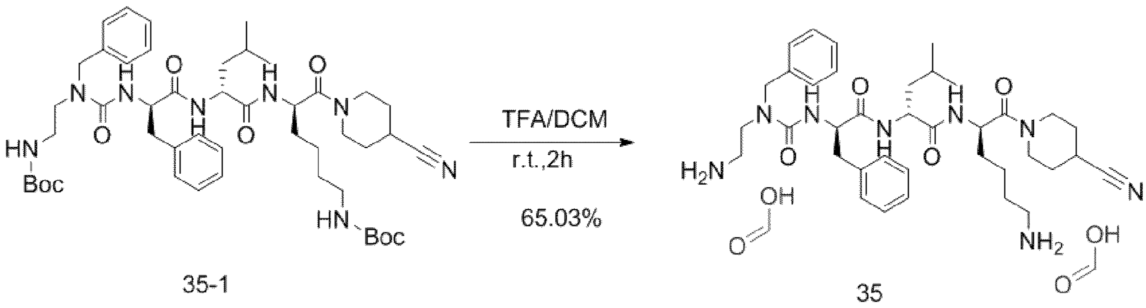


35-1

35

LC-MS-35 (ES, m/z): [M+1] = 675

$^1$H NMR (400 MHz, Deuterium Oxide) δ 8.29 (s, 2H), 7.18 (dtd, *J* = 29.2, 8.0, 7.1, 2.7 Hz, 6H), 7.08 - 6.88 (m, 4H), 4.49 - 4.11 (m, 4H), 3.62 - 3.23 (m, 5H), 3.16 - 2.71 (m, 8H), 1.88 - 1.14 (m, 14H), 0.78 - 0.53 (m, 6H).

Example 36. Synthesis of Compound 36

According to the similar preparation procedures as in Example 8, the target compound was synthesized, with reactant compound

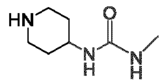

replaced by

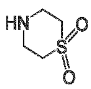 ,

specifically, as shown below:

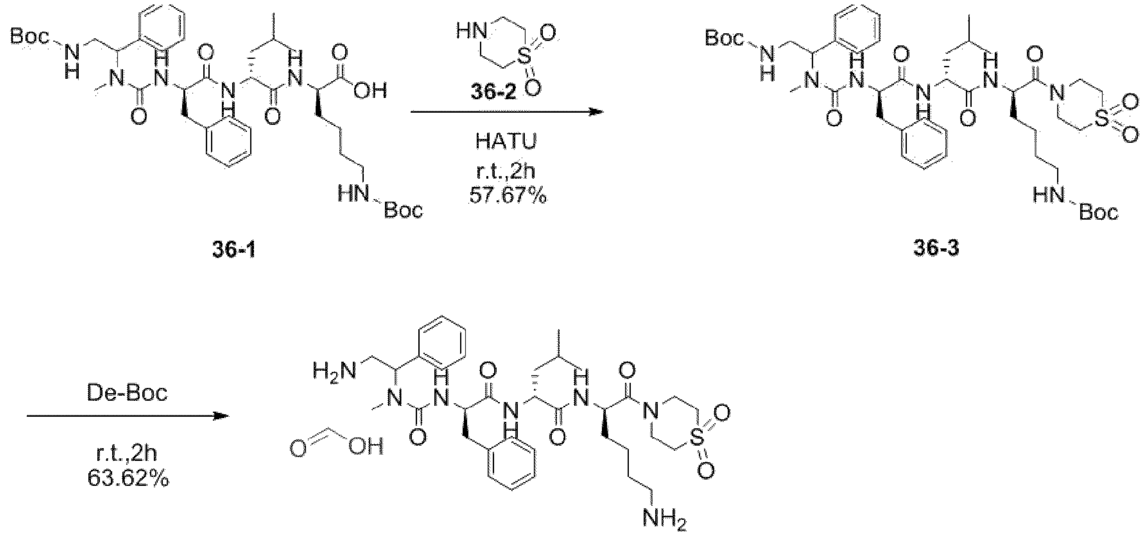


36-1

36-3

36

LC-MS-36 (ES, m/z): [M+1] = 700

$^1$H NMR (400 MHz, Deuterium Oxide) δ 8.37 (s, 1H), 7.43 - 7.17 (m, 8H), 7.13 (d, J = 7.4 Hz, 1H), 6.91 - 6.85 (m, 1H), 5.56 - 5.49 (m, 1H), 4.45 (dd, J = 9.0, 6.2 Hz, 1H), 4.29 (d, J = 8.4 Hz, 2H), 4.17 (d, J = 15.9 Hz, 1H), 3.94 - 3.84 (m, 1H), 3.67 (dd, J = 25.3, 12.6 Hz, 1H), 3.61 - 3.45 (m, 2H), 3.35 (t, J = 11.9 Hz, 1H), 3.28 (s, 1H), 3.20 (s, 5H), 3.18 - 3.06 (m, 1H), 3.01 - 2.72 (m, 3H), 2.54 (s, 2H), 2.43 (s, 1H), 1.63 (s, 1H), 1.61 - 1.49 (m, 4H), 1.34 (t, J = 7.9 Hz, 1H), 0.85 (ddd, J = 15.5, 9.9, 5.4 Hz, 6H).

Example 37. Synthesis of Compound 37

According to the similar preparation procedures as in Example 8, the target compound was synthesized, with reactant compound

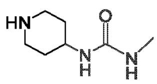

replaced by

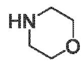,

specifically, as shown below:

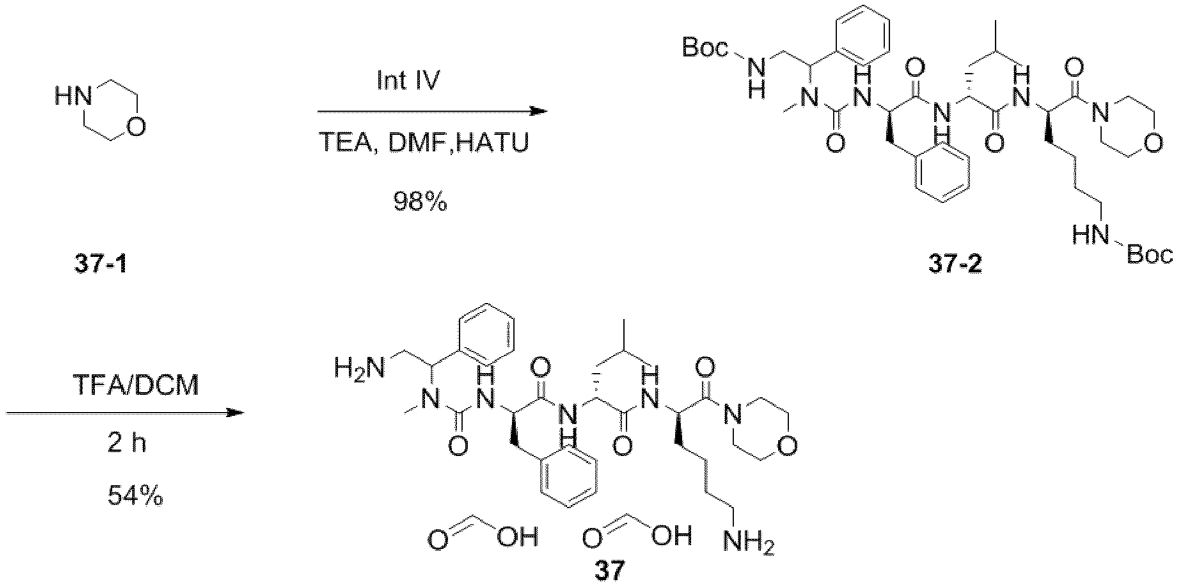


37-1

37-2

37

LC-MS-37 (ES, m/z): [M+1] = 652

$^1$H NMR (400 MHz, Deuterium Oxide) δ 8.36 (s, 2H), 7.54 - 6.66 (m, 10H), 5.52 (dd, *J* = 11.4, 5.7 Hz, 1H), 4.64 - 4.13 (m, 2H), 3.91 - 3.30 (m, 10H), 3.35 - 2.73 (m, 4H), 2.48 (d, *J* = 40.5 Hz, 3H), 1.95 - 1.22 (m, 9H), 0.84 (dt, *J* = 15.4, 7.6 Hz, 6H).

Example 38. Synthesis of Compound 38

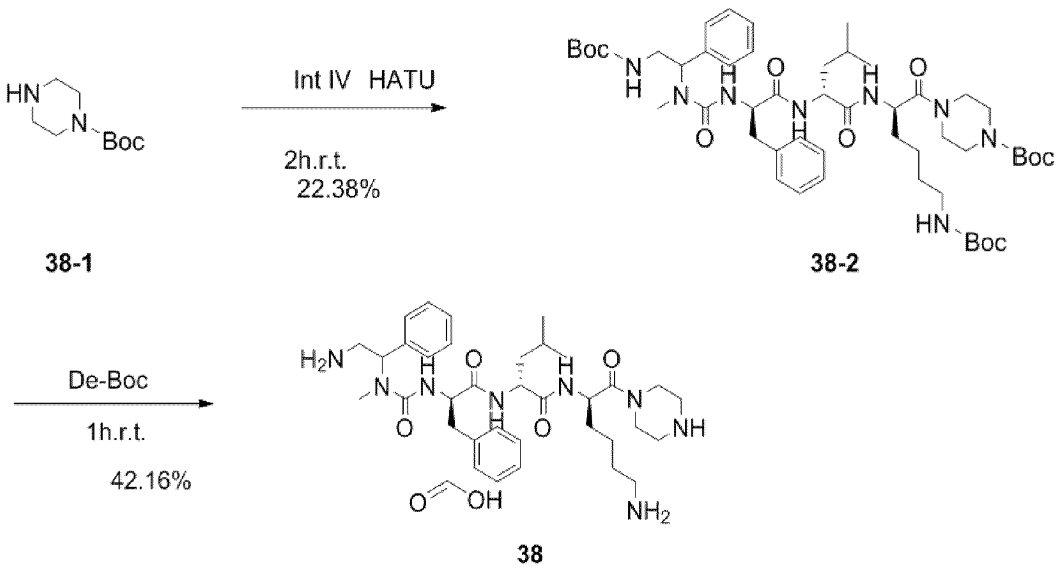


38-1

38-2

38

LC-MS-38 (ES, m/z): [M+1] = 651

$^1$H NMR (400 MHz, Deuterium Oxide) δ 8.38 (s, 1H), 7.57 - 6.68 (m, 10H), 5.52 (dd, *J* = 11.0, 4.5 Hz, 1H), 4.70 - 4.10 (m, 2H), 4.13 - 3.32 (m, 6H), 3.27 - 2.73 (m, 8H), 2.73 - 2.30 (m, 3H), 1.90 - 1.41 (m, 9H), 1.44 - 1.01 (m, 3H), 1.01 - 0.42 (m, 6H).

Example 39. Synthesis of Compound 39

According to the similar preparation procedures as in Example 16, the target compound was synthesized, with reactant compound

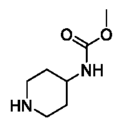

replaced by

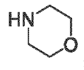,

specifically, as shown below:

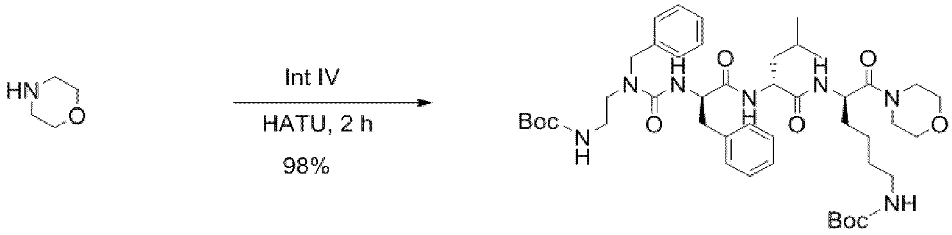


39-1

39-2

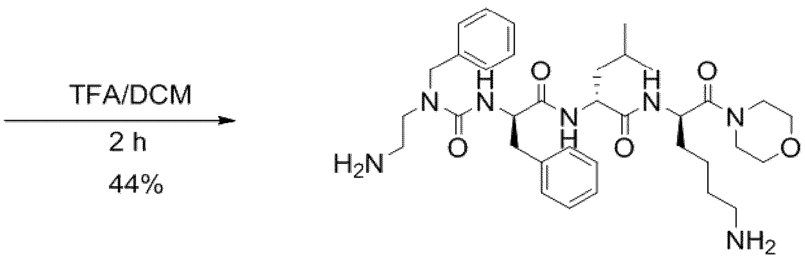


39

LC-MS-39 (ES, m/z): [M+1] = 652

$^{1}$H NMR (400 MHz, Deuterium Oxide) δ 7.68 - 6.57 (m, 10H), 4.50 - 3.83 (m, 4H), 3.77 - 3.21 (m, 10H), 3.16 - 2.57 (m, 6H), 1.76 - 1.03 (m, 9H), 0.98 - 0.23 (m, 6H).

Example 40. Synthesis of Compound 40

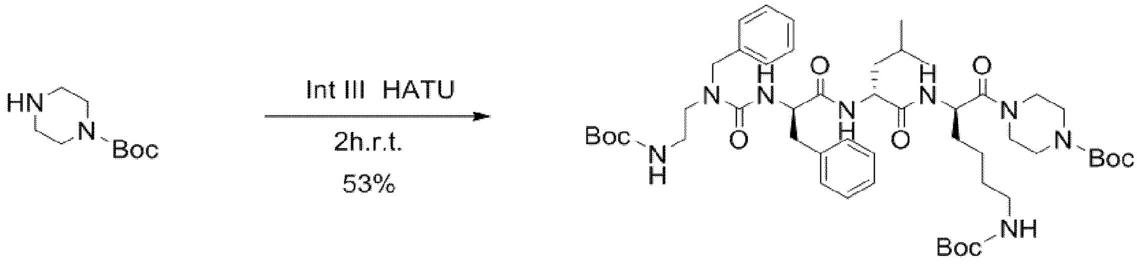


40-1 40-2

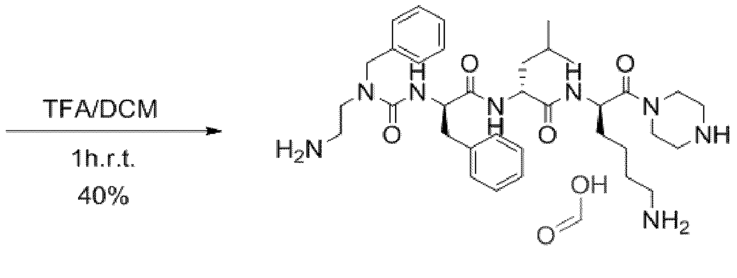


40

LC-MS-40 (ES, m/z): [M+1] = 651

$^1$H NMR (400 MHz, Deuterium Oxide) δ 8.36 (s, 1H), 7.53 - 7.15 (m, 6H), 7.08 - 6.79 (m, 4H), 4.58 - 4.15 (m, 5H), 3.96 - 3.34 (m, 6H), 3.34 - 2.77 (m, 10H), 1.77-21.22 (m, 9H), 0.80 (dd, *J* = 19.9, 5.3 Hz, 6H).

Example 41. Synthesis of Compound 41

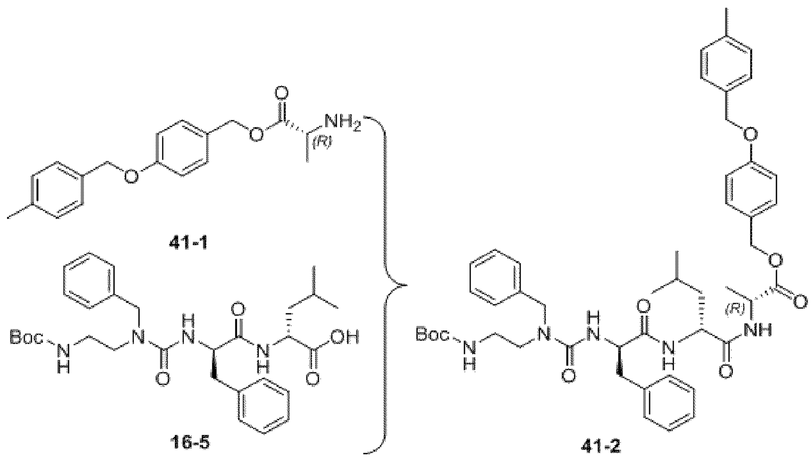

41-1

16-5

41-2

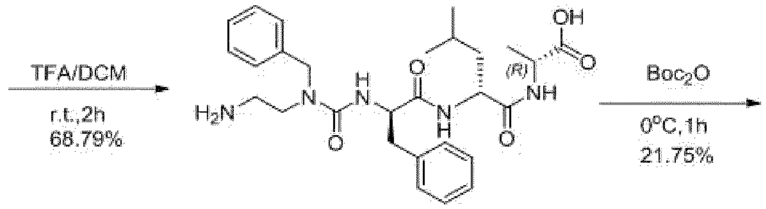


41-3

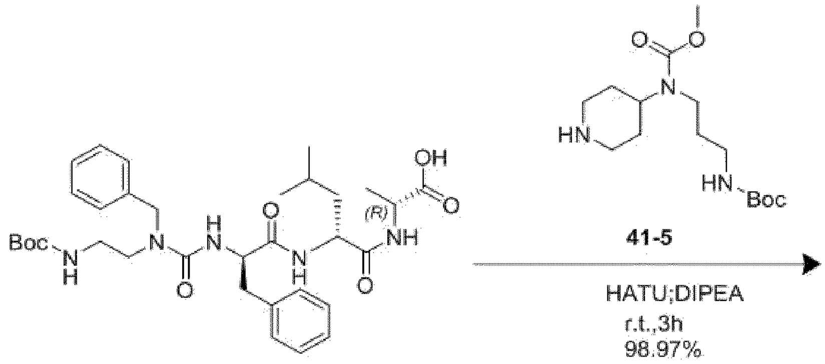


41-5

41-4

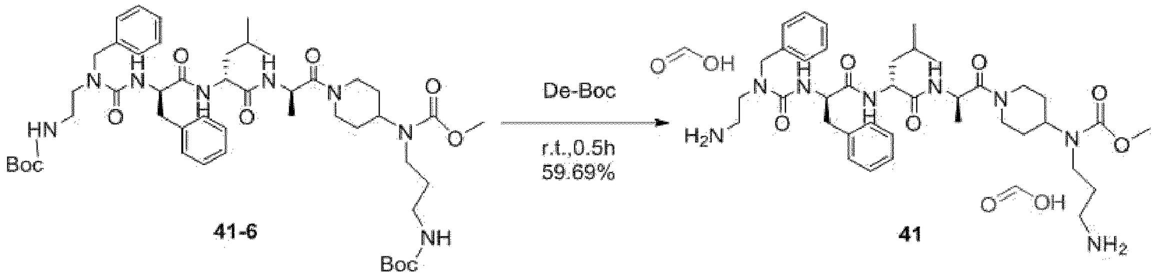


41-6

41

LC-MS-41 (ES, m/z): [M+1] = 723

$^1$H NMR (400 MHz, Deuterium Oxide) δ 8.37 (s, 2H), 7.32 (q, J = 7.1 Hz, 3H), 7.26 - 7.20 (m, 3H), 7.06 (t, J = 4.7 Hz, 4H), 4.54 - 4.46 (m, 2H), 4.40 (d, J = 17.4 Hz, 2H), 4.28 (d, J = 6.8 Hz, 1H), 4.01 (s, 2H), 3.67 - 3.54 (m, 4H), 3.48 (dt, J = 15.2, 6.8 Hz, 1H), 3.28 - 3.11 (m, 3H), 2.95 (dqd, J = 44.1, 15.2, 14.4, 6.9 Hz, 6H), 2.67 (t, J = 13.1 Hz, 1H), 1.87 - 1.59 (m, 6H), 1.48 (t, J = 7.5 Hz, 3H), 1.26 (dt, J = 13.0, 6.8 Hz, 3H), 0.83 (dq, J = 18.4, 3.4 Hz, 6H).

Example 42. Synthesis of Compound 42

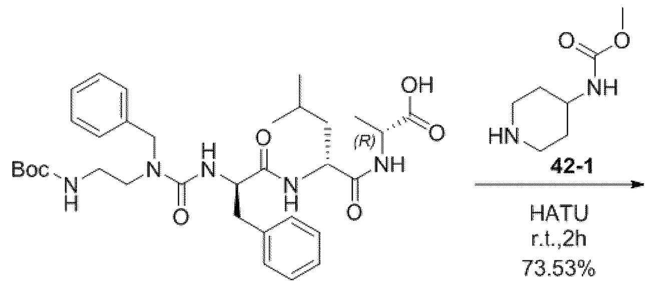


41-4

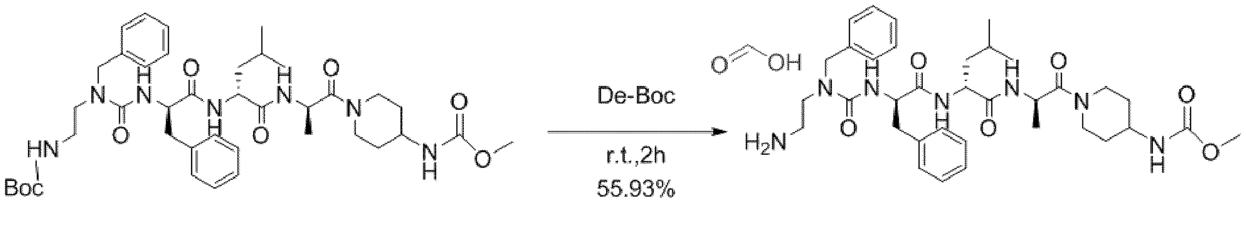


42-2

42

LC-MS-42 (ES, m/z): [M+1] = 666

$^1H$ NMR (400 MHz, Deuterium Oxide) δ 8.38 (s, 1H), 7.32 (q, J = 7.0 Hz, 3H), 7.23 (q, J = 3.8 Hz, 3H), 7.05 (t, J = 5.2 Hz, 4H), 4.53 - 4.45 (m, 2H), 4.40 (d, J = 17.5 Hz, 1H), 4.28 (t, J = 6.9 Hz, 1H), 4.21 - 4.09 (m, 1H), 3.84 (d, J = 16.9 Hz, 1H), 3.57 (t, J = 8.5 Hz, 5H), 3.45 (dq, J = 15.4, 5.4, 5.0 Hz, 1H), 3.23 (d, J = 13.5 Hz, 1H), 3.03 (dd, J = 13.0, 6.4 Hz, 1H), 2.97 (d, J = 6.6 Hz, 2H), 2.91 - 2.79 (m, 1H), 2.86 (s, 1H), 1.93 (d, J = 12.6 Hz, 1H), 1.86 (d, J = 15.1 Hz, 1H), 1.48 (s, 2H), 1.49 - 1.44 (m, 2H), 1.24 (q, J = 7.0 Hz, 4H), 0.88 - 0.77 (m, 6H).

Example 43. Synthesis of Compound 43

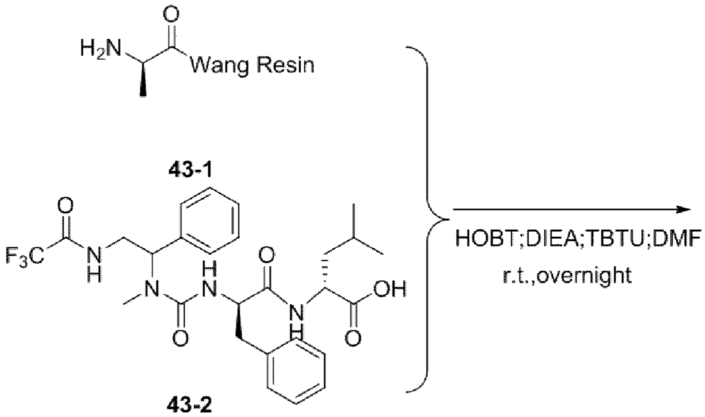


43-1

43-2

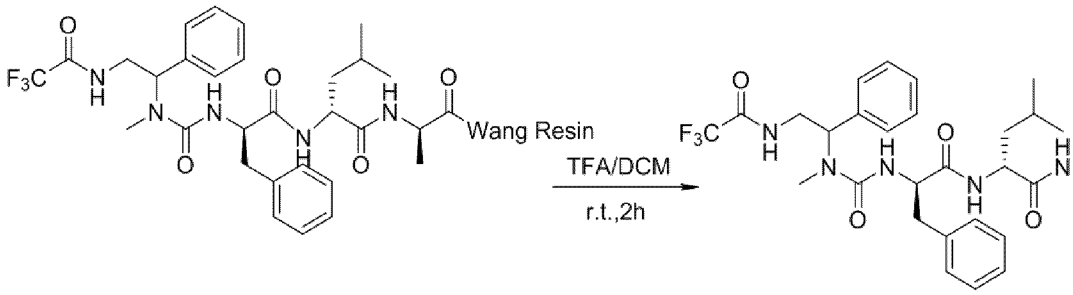


43-3

43-4

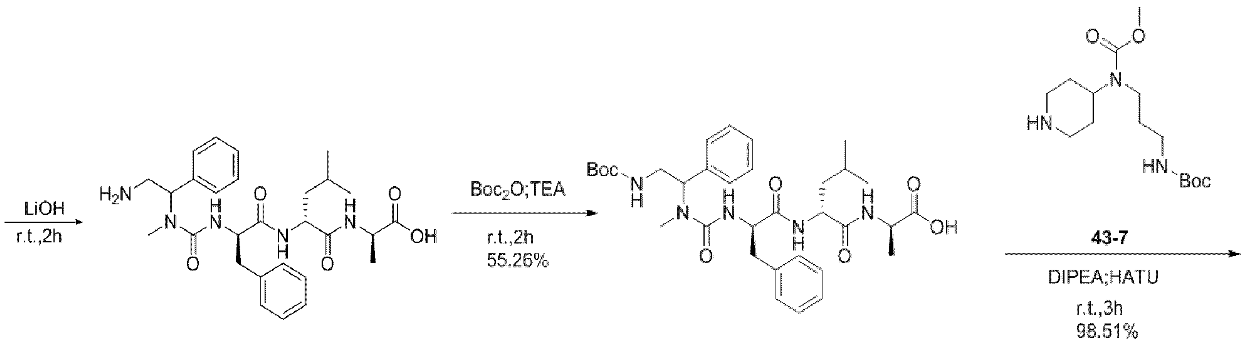


43-7

43-5

43-6

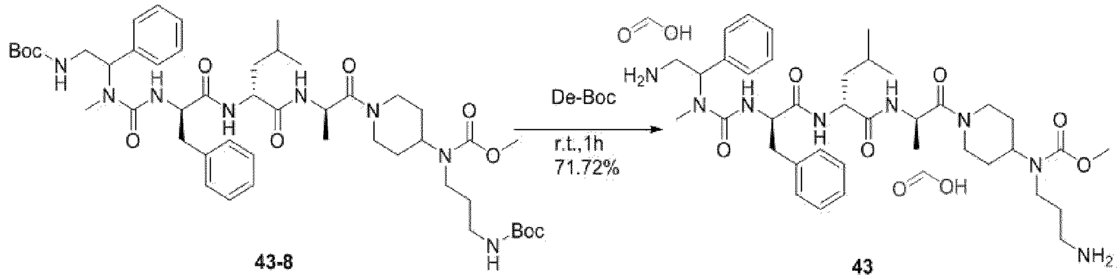


43-8

43

LC-MS-43 (ES, m/z): [M+1] = 723

$^1$H NMR (400 MHz, Deuterium Oxide) δ 8.35 (s, 2H), 7.35 (dd, J = 12.2, 5.2 Hz, 3H), 7.30 - 6.81 (m, 7H), 5.70 - 5.42 (m, 1H), 4.46 - 4.28 (m, 2H), 4.12 - 3.83 (m, 2H), 3.61 (d, J = 11.6 Hz, 3H), 3.50 (q, J = 11.8, 9.2 Hz, 2H), 3.30 - 3.02 (m, 4H), 2.91 (h, J = 10.1, 9.2 Hz, 3H), 2.70 - 2.50 (m, 3H), 2.43 (d, J = 4.9 Hz, 1H), 2.00 - 1.63 (m, 5H), 1.60 - 1.44 (m, 3H), 1.37 - 0.98 (m, 3H), 0.95 - 0.50 (m, 6H).

Example 44. Synthesis of Compound 44

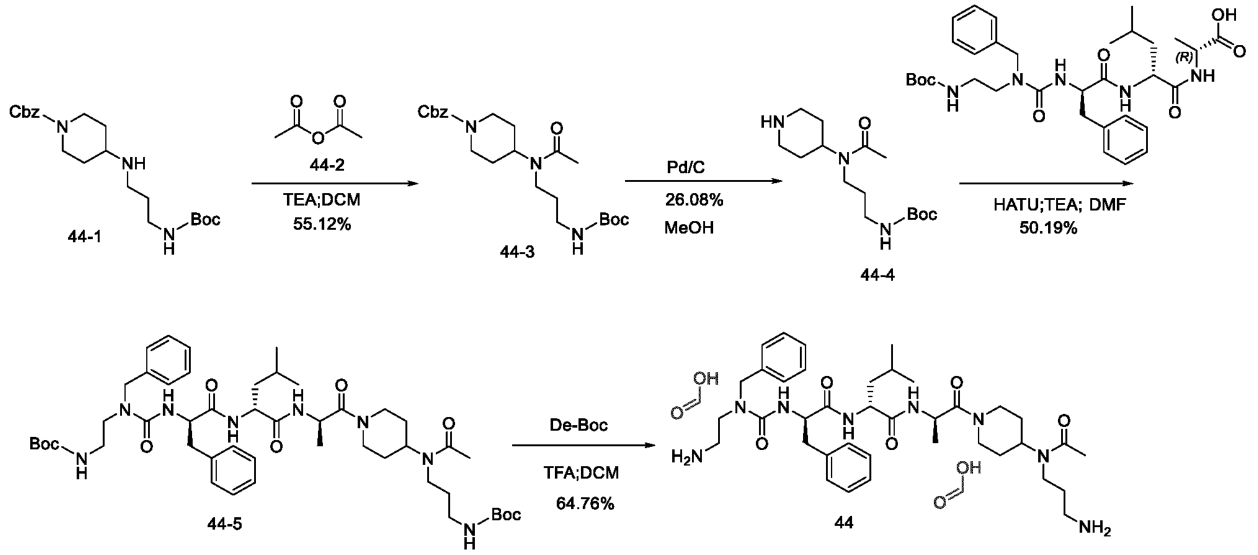


LC-MS-44 (ES, m/z): [M+1] = 706

$^1$H NMR (400 MHz, Deuterium Oxide) δ 8.28 (s, 2H), 7.28 - 7.09 (m, 6H), 7.07 - 6.90 (m, 4H), 4.49 - 4.10 (m, 5H), 4.04 - 3.79 (m, 2H), 3.59 - 3.28 (m, 2H), 3.13 (ddt, *J* = 36.4, 14.4, 7.9 Hz, 3H), 2.99 - 2.67 (m, 6H), 2.68 - 2.52 (m, 2H), 2.13 - 1.94 (m, 3H), 1.86 - 1.02 (m, 12H), 0.84 - 0.51 (m, 6H).

Example 45. Synthesis of Compound 45

According to the similar preparation procedures as in Example 16, the target compound was synthesized, with reactant compound

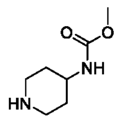

replaced by

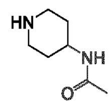,

specifically, as shown below:

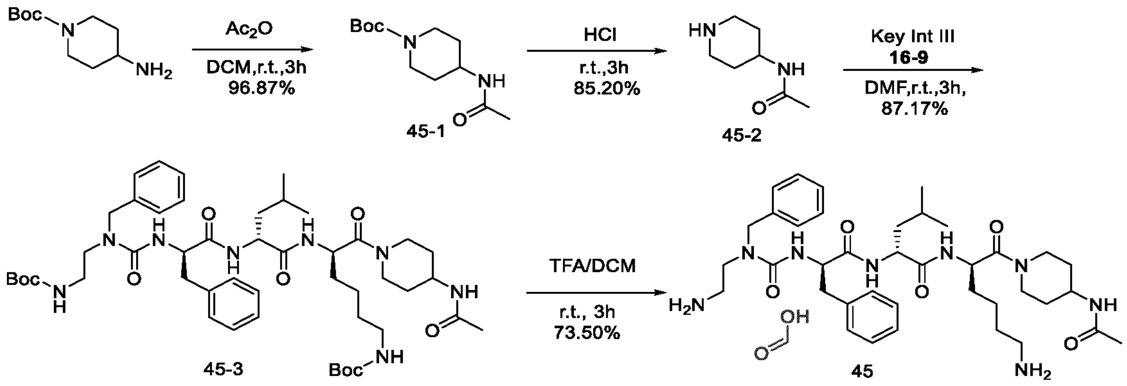

45-1
45-2
45-3
45

LC-MS-45 (ES, m/z): [M+1] = 708

$^1$H NMR (400 MHz, Deuterium Oxide) δ 8.29 (s, 1H), 7.29 - 7.07 (m, 6H), 7.07 - 6.91 (m, 4H), 4.46 - 4.25 (m, 3H), 4.25 - 3.93 (m, 2H), 3.90 - 3.58 (m, 2H), 3.58 - 3.29 (m, 2H), 3.13 (t, *J* = 13.6 Hz, 1H), 3.02 - 2.64 (m, 7H), 1.79 (dd, *J* = 19.2, 5.9 Hz, 5H), 1.64 - 1.08 (m, 11H), 0.79 - 0.54 (m, 6H).

Example 46. Synthesis of Compound 46

According to similar preparation procedures as in Example 16, the target compound was synthesized, with reactant compound

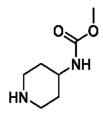

replaced by

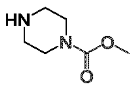
,

specifically, as shown below:

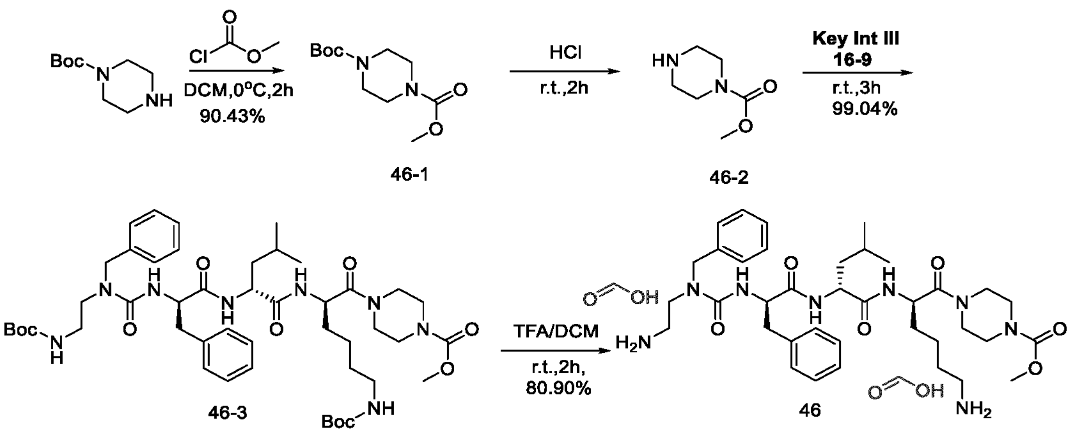

46-1
46-2
46-3
46

LC-MS-46 (ES, m/z): [M+1] = 709

$^1$H NMR (400 MHz, Deuterium Oxide) δ 8.34 (d, *J* = 2.0 Hz, 2H), 7.33 - 7.17 (m, 6H), 7.01 (dq, *J* = 13.1, 5.3, 4.5 Hz, 4H), 4.52 - 4.41 (m, 2H), 4.35 (dd, *J* = 17.6, 4.9 Hz, 1H), 4.25 (dd, *J* = 8.8, 5.9 Hz, 1H), 3.61 (d, *J* = 3.0 Hz, 6H), 3.50 (t, *J* = 6.6 Hz, 3H), 3.38 (s, 1H), 3.01 (dt, *J* = 14.3, 4.5 Hz, 1H), 2.96 - 2.76 (m, 5H), 1.68 - 1.52 (m, 4H), 1.43 (q, *J* = 10.1, 8.0 Hz, 3H), 1.35 - 1.25 (m, 2H), 0.84 - 0.74 (m, 6H).

Example 47. Synthesis of Compound 47

According to similar preparation procedures as in Example 16, the target compound was synthesized, with reactant compound

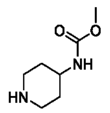

replaced by

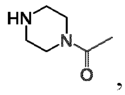

,

specifically, as shown below:

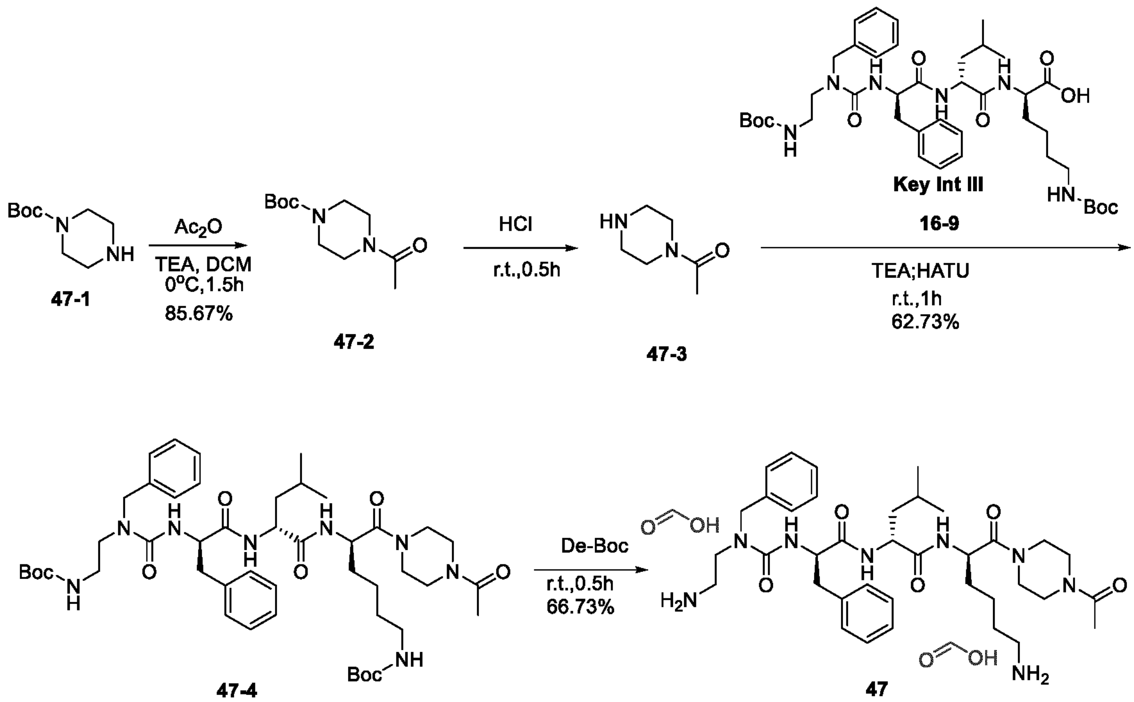


47-1 47-2 47-3 16-9

47-4 47

LC-MS-47 (ES, m/z): [M+1] = 693

$^1$H NMR (400 MHz, Deuterium Oxide) δ 8.33 (s, 2H), 7.31 - 7.14 (m, 6H), 7.00 (dd, J = 11.1, 5.8 Hz, 4H), 4.47 (dd, J = 13.2, 7.5 Hz, 2H), 4.38 - 4.22 (m, 2H), 3.68 - 3.51 (m, 5H), 3.38 (tdt, J = 24.0, 18.3, 9.2 Hz, 5H), 3.05 - 2.79 (m, 6H), 2.03 (t, J = 6.9 Hz, 3H), 1.70 - 1.53 (m, 4H), 1.43 (hept, J = 6.6, 5.9 Hz, 3H), 1.36 - 1.22 (m, 2H), 0.79 (dd, J = 18.2, 5.9 Hz, 6H).

Example 48. Synthesis of Compound 48

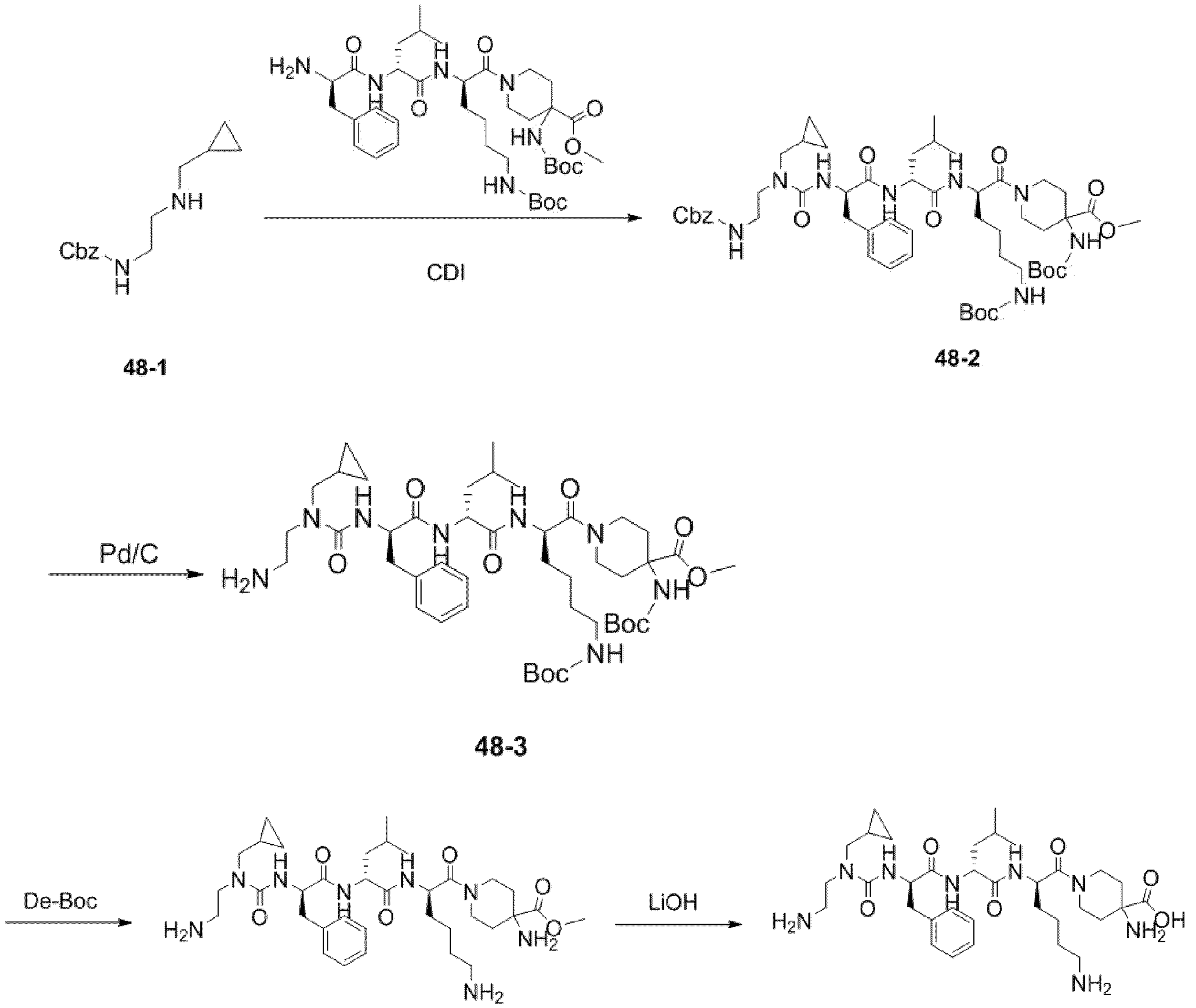


48-1

48-2

48-3

48-4

48

LC-MS-48 (ES, m/z): [M+1] = 673.43

$^1$H NMR (400 MHz, Deuterium Oxide) δ 7.28 - 7.14 (m, 5H), 4.70 (s, 1H), 4.65 (s, 2H), 4.43 (dd, *J* = 9.5, 5.7 Hz, 1H), 4.28 - 4.21 (m, 1H), 3.56 (s, 2H), 3.35 (dt, *J* = 14.1, 7.2 Hz, 2H), 3.13 - 2.89 (m, 2H), 2.87 (s, 2H), 2.86 (d, *J* = 7.6 Hz, 2H), 2.85 - 2.75 (m, 2H), 1.98 (dd, *J* = 13.5, 6.8 Hz, 1H), 1.87 (d, *J* = 9.5 Hz, 1H), 1.55 (dq, *J* = 15.0, 7.6 Hz, 6H), 1.45 (s, 5H), 0.77 (dd, *J* = 17.0, 5.1 Hz, 7H), 0.66 - 0.59 (m, 1H), 0.33 (d, *J* = 7.8 Hz, 2H).

Example 49. Synthesis of Compound 49

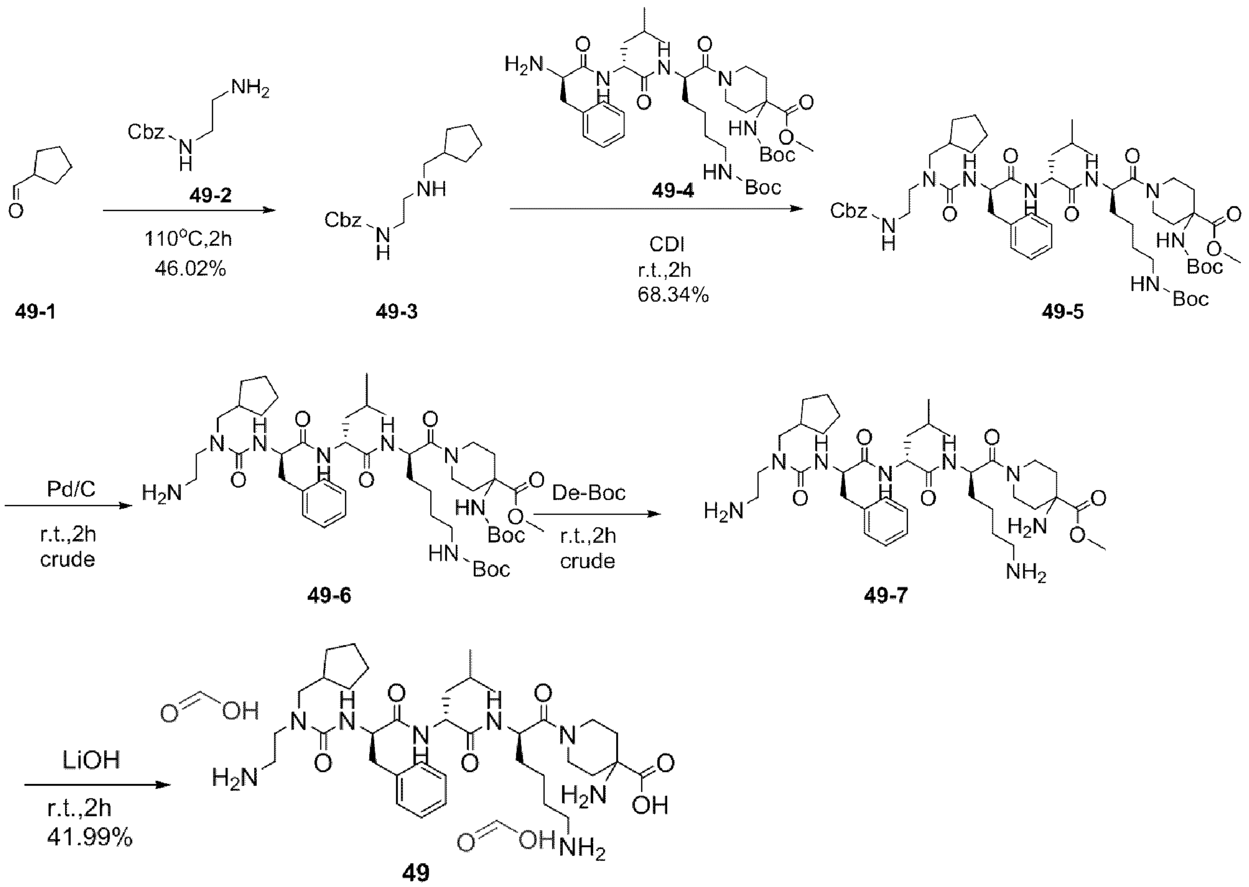


LC-MS-49 (ES, m/z): [M+1] = 700

$^1$H NMR (400 MHz, Deuterium Oxide) δ 8.37 (s, 2H), 7.31 (t, J = 7.3 Hz, 2H), 7.23 (dd, J = 17.4, 7.3 Hz, 3H), 4.76 (d, J = 6.2 Hz, 1H), 4.69 (s, 1H), 4.49 (dd, J = 9.7, 5.4 Hz, 1H), 4.30 (d, J = 8.6 Hz, 1H), 3.74 (s, 2H), 3.56 (ddd, J = 29.1, 13.5, 7.1 Hz,2H), 3.34 (ddt, J = 16.1, 10.9, 5.8 Hz, 1H), 3.14 (dd, J = 14.4, 6.6 Hz, 2H), 2.93 (dq, J = 14.1, 6.5 Hz, 6H), 2.22 - 2.11 (m, 1H), 2.09 (s, 1H), 1.72 (s, 5H), 1.62 (t, J = 7.7 Hz, 2H), 1.47 (s, 10H), 1.37 (s, 1H), 0.94 (s, 1H), 0.84 (dd, J = 17.7, 5.3 Hz, 7H).

Example 50. Synthesis of Compound 50

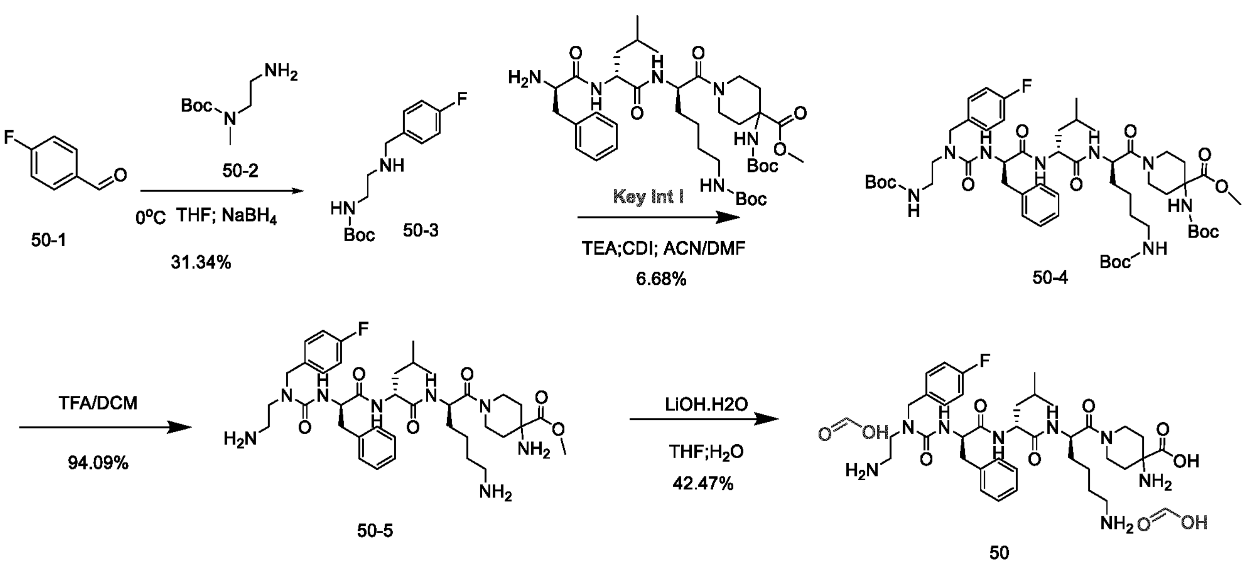

LC-MS-50 (ES, m/z): [M+1] = 726

$^1$H NMR (400 MHz, Deuterium Oxide) δ 8.29 (s, 2H), 7.18 - 7.12 (m, 3H), 6.98 (d, J = 5.5 Hz, 2H), 6.91 (td, J = 8.0, 6.9, 3.2 Hz, 4H), 4.45 - 4.34 (m, 2H), 4.31 - 4.17 (m, 2H), 3.75 - 3.57 (m, 3H), 3.51 (dq, J = 13.0, 6.3, 5.7 Hz, 2H), 3.35 (dtt, J = 21.5, 10.9, 6.0 Hz, 1H), 2.97 (dt, J = 14.0, 4.6 Hz, 1H), 2.90 (q, J = 5.4 Hz, 2H), 2.83 (t, J = 7.7 Hz, 2H), 2.75 (ddd, J = 13.5, 9.2, 3.4 Hz, 1H), 2.15 - 2.06 (m, 1H), 2.01 (dd, J = 13.5, 7.7 Hz, 1H), 1.75 (dt, J = 13.3, 5.6 Hz, 1H), 1.68 - 1.59 (m, 2H), 1.56 (s, 1H), 1.53 (d, J = 7.5 Hz, 1H), 1.40 (q, J = 9.4, 8.3 Hz, 3H), 1.35 - 1.22 (m, 2H), 0.74 (dd, J = 18.9, 5.1 Hz, 6H).

Example 51. Synthesis of Compound 51

According to similar preparation procedures as in Example 16, the target compound was synthesized, with reactant compound

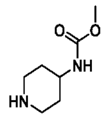

replaced by

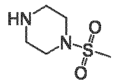

,

specifically, as shown below:

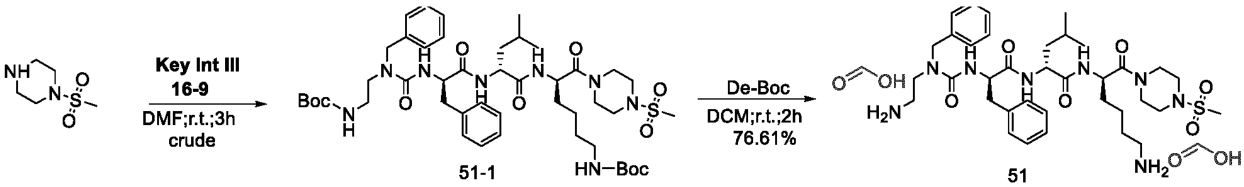


LC-MS-51 (ES, m/z): [M+1] = 829

$^1$H NMR (400 MHz, Deuterium Oxide) δ 8.34 (s, 2H), 7.34 - 7.22 (m, 3H), 7.21 (dt, *J* = 6.3, 2.6 Hz, 3H), 7.07 - 6.97 (m, 4H), 4.52 - 4.42 (m, 2H), 4.36 (d, *J* = 17.5 Hz, 1H), 4.25 (dd, *J* = 8.8, 5.7 Hz, 1H), 3.74 (d, *J* = 13.5 Hz, 2H), 3.63 - 3.37 (m, 4H), 3.28 (ddd, *J* = 17.5, 11.7, 4.4 Hz, 2H), 3.16 (td, *J* = 8.7, 8.2, 4.1 Hz, 1H), 3.04 (ddt, *J* = 20.9, 12.1, 5.0 Hz, 2H), 2.99 - 2.77 (m, 7H), 1.71 - 1.63 (m, 1H), 1.60 (ddd, *J* = 14.5, 9.7, 5.6 Hz, 3H), 1.44 (s, 3H), 1.35 (ddd, *J* = 30.9, 14.8, 7.2 Hz, 1H), 0.79 (dd, *J* = 18.9, 5.7 Hz, 6H).

Example 1A. Synthesis of Compound 1A

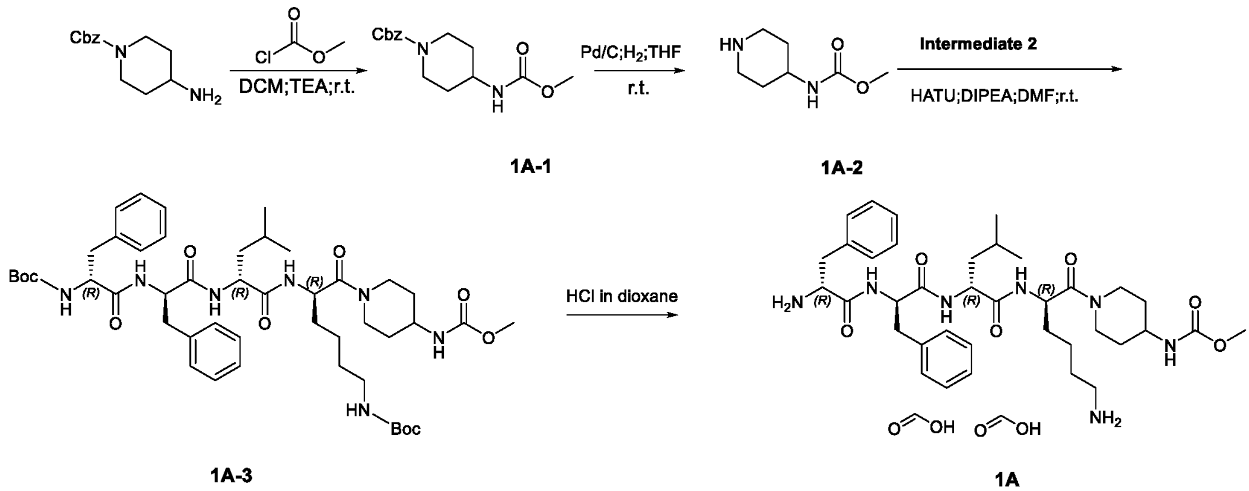


1A-1 1A-2

1A-3 1A

1.1 Synthesis of compound 1A-1

Benzyl 4-aminopiperidine-1-carboxylate (100 mg, 0.427 mmol, 1.00 equiv) and TEA (0.2 mL, 2.693 mmol, 6.31 equiv) were dissolved in DCM (15 mL), and methyl chloroformate (60.49 mg, 0.640 mmol, 1.5 equiv) was added portionwise. The mixture was stirred at room temperature for 2 h. The reaction was then quenched with a saturated ammonium chloride solution and extracted with DCM (15 mL × 3). The organic phases were combined, dried, and concentrated to dryness by rotary evaporation to give a crude product. The crude product was purified by silica gel column chromatography and eluted with PE/EA (1:1). The fractions were concentrated to dryness by rotary evaporation to give benzyl 4-[(methoxycarbonyl)amino]piperidine-1-carboxylate.

LC-MS-1A-1 (ES, m/z): [M+1] = 293

1.2 Synthesis of compound 1A-2

Pd/C (18 mg, w/t, 10%) was added to a stirred solution of benzyl 4-[(methoxycarbonyl)amino]piperidine-1-carboxylate (180 mg, 0.616 mmol, 1.00 equiv) in THF (12 mL). The mixture was stirred for 13 h under hydrogen atmosphere. The resulting mixture was washed with MeOH (7 × 50 mL). The filtrate was concentrated under reduced pressure to give methyl N-(piperidin-4-yl)carbamate (100 mg, crude).

LC-MS-1A-2 (ES, m/z): [M+1] = 159

1.3 Synthesis of compound 1A-3

DIPEA (1.48 mg, 0.123 mmol, 3 equiv), intermediate 2 (31 mg, 0.041 mmol, 1.00 equiv) and HATU (23.45 mg, 0.061 mmol, 1.5 equiv) were added to a stirred solution of methyl N-(piperidin-4-yl)carbamate (19.51 mg, 0.123 mmol, 3.00 equiv) in DMF (10 mL). The reaction was carried out at room temperature for 3 h under nitrogen atmosphere. Water (12 mL) was added to quench the reaction, and the resulting mixture was extracted with ethyl acetate (3 × 10 mL). The organic phases were combined, dried, and concentrated to dryness by rotary evaporation to give a crude product. The crude product was purified by reversed-phase chromatography and eluted with $CH_3CN/H_2O$ (0.1% FA) (6:1), and the fractions were concentrated to dryness by rotary evaporation to give *tert*-butyl N-[(5R)-5-[(2R))-2-[(2R)-2-[(2R)-2-[(*tert*-butoxycarbonyl)amino]-3-phenylpropionylamino]-3-phenylpropionylamino]-4-methylpentanoylamino]-6-{4-[(methoxycarbonyl))amino]piperidin-1-yl}-6-oxohexyl]carbamate.

LC-MS-1A-3 (ES, m/z): [M+1] = 894

1.4 Synthesis of compound 1A

*tert*-Butyl N-[(5R)-5-[(2R)-2-[(2R)-2-[(2R)-2-[(*tert*-butoxycarbonyl)amino]-3-phenylpropionylamino]-3-phenylpropionylamino]-4-methylpentanoylamino]-6-{4-[(methoxycarbonyl)amino]piperidin-1-yl}-6-oxohexyl]carbamate (350 mg, 0.391 mmol, 1 equiv) was dissolved in 4 M HCl (gas)/1,4-dioxane (5 mL). The resulting mixture was reacted at room temperature for 4 h. The resulting mixture was concentrated under reduced pressure. The residue was purified by preparative HPLC, and the fractions were concentrated to dryness by rotary evaporation to give methyl N-{1-[(2R)-6-amino-2-[(2R)-2-[(2R)-2-[(2R)-2-amino-3]-phenylpropionylamino]-3-phenylpropionylamino]-4-methylpentanoylamino] hexanoyl]piperidin-4-yl}carbamate (169.1 mg, 62.01%, purity: 99.2%).

LC-MS-1A (ES, m/z): [M+1] = 693

$^1H$ NMR (400 MHz, Deuterium Oxide) δ 8.36 (s, 2H), 7.45 - 6.86 (m, 10H), 4.63 - 4.47 (m, 2H), 4.27 - 4.06 (m, 3H), 3.85 (dd, *J* = 37.5, 14.1 Hz, 1H), 3.55 (d, *J* = 3.6 Hz, 4H), 3.32 - 2.69 (m, 8H), 2.14 - 1.71 (m, 2H), 1.73 - 1.13 (m, 11H), 0.82 (dq, *J* = 20.0, 2.6 Hz, 6H).

Example 2A. Synthesis of Compound 2A

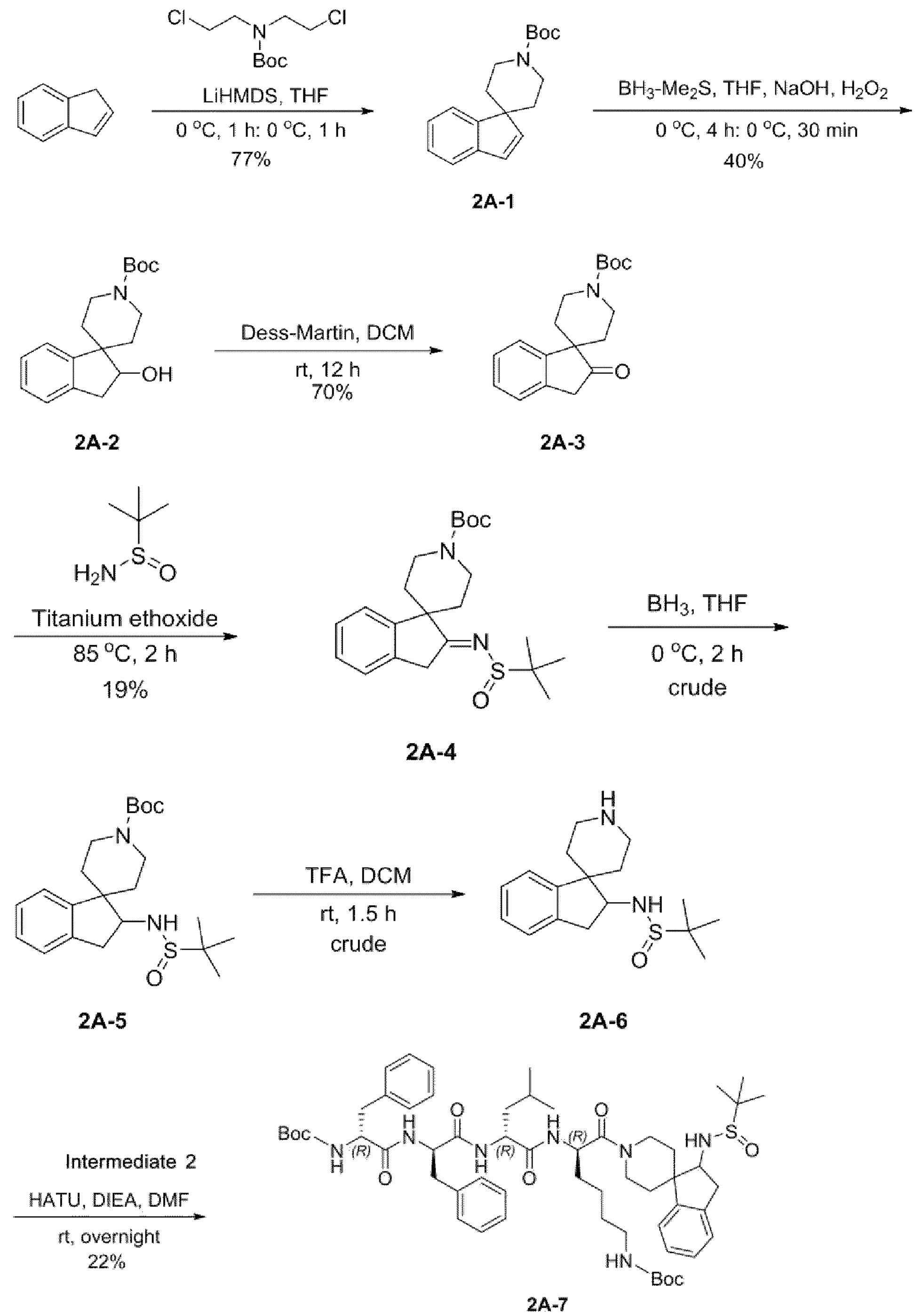


2A-1

2A-2

2A-3

2A-4

2A-5

2A-6

2A-7

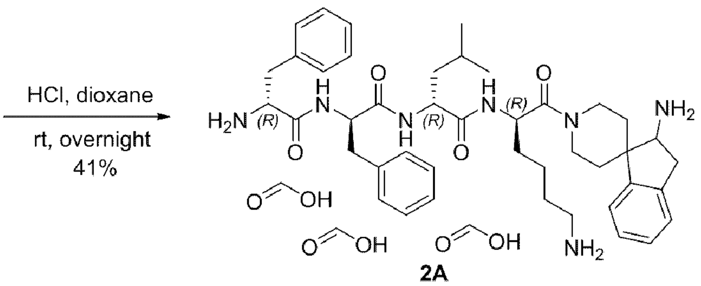

2A

2.1 Synthesis of **compound 2A-1**

Lithium bis(trimethylsilyl)amide (19.97 mL, 119.348 mmol, 11.95 equiv) was added dropwise to a stirred solution of indene (1.16 g, 9.986 mmol, 1.00 equiv) in THF (12.00 mL, 148.116 mmol, 14.83 equiv) at 0 °C under nitrogen atmosphere. The resulting mixture was stirred at 0 °C for 1 h under nitrogen atmosphere. *tert*-Butyl N,N-bis(2-chloroethyl) carbamate (2.42 g, 9.994 mmol, 1.00 equiv) was added dropwise to the mixture described above at 0 °C. The resulting mixture was stirred at 0 °C for another 1 h and concentrated under reduced pressure to give a crude product. The crude product was purified by silica gel column chromatography and eluted with petroleum ether/ethyl acetate (9:1), and the fractions were concentrated to dryness by rotary evaporation to give *tert*-butyl spiro[indene-1,4'-piperidine]-1'-carboxylate (2.22 g, 77.90%, purity: 99%).

LC-MS-2A-1 (ES, m/z): [M+1] = 286

2.2 Synthesis of compound 2A-2

Borane dimethyl sulfide complex 2M (THF) (19.45 mL, 38.895 mmol, 5 equiv) was added to a stirred solution of *tert*-butyl spiro[indene-1,4'-piperidine]-1'-carboxylate (2.22 g, 7.779 mmol, 1.00 equiv) in THF (22 mL) at 0 °C under nitrogen atmosphere. The resulting mixture was stirred at 0 °C for 4 h under nitrogen atmosphere. Sodium hydroxide (2 M) (23.34 mL, 46.674 mmol, 6 equiv) and hydrogen peroxide (6.66 mL, 285.878 mmol, 36.75 equiv) were added dropwise to the mixture described above at 0 °C. The resulting mixture was stirred at 0 °C for another 30 min and then extracted with ethyl acetate (1 × 150 mL). The combined organic phase was dried over anhydrous sodium sulfate and filtered. The filtrate was concentrated under reduced pressure. The residue was purified by silica gel column chromatography and eluted with petroleum ether/ethyl acetate (1:1), and the fractions were concentrated to dryness by rotary evaporation to give *tert*-butyl 2-hydroxy-2,3-dihydrospiro[indene-1,4'-piperidine]-1'-carboxylate (959 mg, 40.63%, purity: 99%).

LC-MS-2A-2 (ES, m/z): [M+1] = 304

2.3 Synthesis of compound 2A-3

Dess-Martin reagent (2180.81 mg, 5.142 mmol, 2 equiv) was added portionwise to a solution of *tert*-butyl 2-hydroxy-2,3-dihydrospiro[indene-1,4'-piperidine]-1'-carboxylate (780 mg, 2.571 mmol, 1.00 equiv) in dichloromethane (16 mL) at room temperature. The resulting mixture was stirred at room temperature for 12 h. The resulting mixture was filtered, and the filter cake was washed with dichloromethane (1 × 20 mL). The combined organic layer was washed with aqueous sodium bicarbonate solution (1 × 15 mL) and brine (1 × 15 mL) and extracted with ethyl acetate, and the organic phases were combined, dried over anhydrous sodium sulfate, and filtered. The filtrate was concentrated under reduced pressure. The residue was purified by silica gel column chromatography and eluted with petroleum ether/ethyl acetate (4:1), and the fractions were concentrated to dryness by rotary evaporation to give *tert*-butyl 2-oxo-3H-spiro[indene-1,4'-piperidine]-1'-carboxylate (550 mg, 70.98%, purity: 99%).

LC-MS-2A-3 (ES, m/z): [M+1] = 302

2.4 Synthesis of compound 2A-4

*tert*-Butanesulfinamide (2.08 g, 17.121 mmol, 3 equiv) was added portionwise to a stirred mixture of *tert*-butyl 2-oxo-3H-spiro[indene-1,4'-piperidine]-1'-carboxylate (1.72 g, 5.707 mmol, 1.00 equiv) and ethyl titanate (4 mL) at room temperature. The resulting mixture was stirred at 85 °C for 2 h. The reaction was quenched with water at room temperature. The resulting mixture was filtered, and the filter cake was washed with ethyl acetate (3 × 10 mL). The filtrate was concentrated under reduced pressure. The resulting mixture was extracted with ethyl acetate (2 × 50 mL). The combined organic phase (1 × 40 mL) was dried over anhydrous sodium sulfate and filtered. The filtrate was concentrated under reduced pressure. The residue was purified by silica gel column chromatography and eluted with petroleum ether/ethyl acetate (2:1), and the fractions were concentrated to dryness by rotary evaporation to give *tert*-butyl (2E)-2-[(2-methylpropane-2-sulfinyl)imino]-3H-spiro[indene-1,4'-piperidine]-1'-carboxylate (450 mg, 19.49%, purity: 99%).

LC-MS-2A-4 (ES, m/z): [M+1] = 405

2.5 Synthesis of compound 2A-5

$BH_3$.THF (1.73 mL, 1.730 mmol, 2 equiv) was added portionwise to a stirred solution of *tert*-butyl (2E)-2-[(2-methylpropane-2-sulfinyl)imino]-3H-spiro[indene-1,4'-piperidine]-1'-carboxylate (350 mg, 0.865 mmol) in tetrahydrofuran (5 mL) at 0 °C under nitrogen atmosphere. The resulting mixture was stirred at 0 °C for 2 h under nitrogen atmosphere. The reaction was quenched with methanol at room temperature. The resulting mixture was concentrated under reduced pressure to give *tert*-butyl 2-[(2-methylpropane-2-sulfinyl)amino]-2,3-dihydrospiro[indene-1,4'-piperidine]-1'-carboxylate (380 mg, crude).

LC-MS-2A-5 (ES, m/z): [M+1] = 407

2.6 Synthesis of compound 2A-6

TFA (0.3 mL) was added to a stirred solution of *tert*-butyl 2-[(2-methylpropane-2-sulfinyl)amino]-2,3-dihydrospiro [indene-1,4'-piperidine]-1'-carboxylate (100 mg, 0.246 mmol) in dichloromethane (1.7 mL) at room temperature. The resulting mixture was stirred at room temperature for 1.5 h. The resulting mixture was concentrated *in vacuo* to give N-{2,3-dihydrospiro[indene-1,4'-piperidin]-2-yl}-2-methylpropane-2-sulfinamide (100 mg, crude).

LC-MS-2A-6 (ES, m/z): [M+1] = 307

2.7 Synthesis of compound 2A-7

Diisopropylethylamine (115.01 mg, 0.891 mmol, 3 equiv), intermediate 2 (223.64 mg, 0.297 mmol, 1.00 equiv) and HATU (135.34 mg, 0.3156 mmol, 1.2 equiv) were added portionwise to a solution of N-{2,3-dihydrospiro[indene-1,4'-piperidin]-2-yl}-2-methylpropane-2-sulfinamide (100.00 mg, 0.327 mmol, 1.1 equiv) in dimethylformamide (2 mL) at room temperature. The resulting mixture was stirred at room temperature overnight. The resulting solution was purified by reversed-phase flash chromatography, and the fractions were concentrated to dryness by rotary evaporation to give *tert*-butyl N-[(5R)-5-[(2R)-2-[(2R)-2-[(2R)-2-[(*tert*-butoxycarbonyl)amino]-3-phenylpropionylamino]-3-phenylpropionylamino]-4-methylpentanoylamino]-6-{2-[(2-methylpropane-2-sulfinyl)amino]-2,3-dihydrospiro[indene-1,4'-piperidin]-1'-y1} -6-oxohexyl carbamate (70 mg, 22.64%, purity: 99%).

LC-MS-2A-7 (ES, m/z): [M+1] = 1042

2.8 Synthesis of compound 2A

4 M HCl (gas)/1,4-dioxane (2 mL) was added portionwise to a stirred solution of *tert*-butyl N-[(5R)-5-[(2R)-2-[(2R)-2-[(2R)-2-[(*tert*-butoxycarbonyl)amino]-3-phenylpropionylamino]-3-phenylpropionylamino]-4-methylpentanoylamino]-6-{2-[(2-methylpropane-2-sulfinyl)amino]-2,3-dihydrospiro [indene-1,4'-piperidin]-1'-yl}-6-oxohexyl]carbamate (70 mg, 0.067 mmol, 1.00 equiv) in dioxane (2 mL) at room temperature. The resulting mixture was stirred at room temperature overnight. The resulting mixture was concentrated under reduced pressure. The crude product (50 mg) was purified by preparative HPLC to give (2R)-N-[(2R)-6-amino-1-{2-amino-2,3-dihydrospiro[indene-1,4'-piperidin]-1'-yl}-1-oxohex-2-yl]-2-[(2R)-2-[(2R)-2-amino-3-phenylpropionylamino]-3-phenylpropionylamino]-4-methylpentanamide (24.7 mg, 41.65%, purity: 83.3%).

LC-MS-2A (ES, m/z): [M+1] = 738

$^1$H NMR-2 (400 MHz, Deuterium Oxide) δ 8.26 (s, 3H), 7.48 - 6.89 (m, 14H), 4.52 (ddt, *J* = 25.9, 13.0, 7.1 Hz, 2H), 4.14 (dq, *J* = 17.7, 5.4, 4.2 Hz, 2H), 3.99 (q, *J* = 8.3, 7.1 Hz, 2H), 3.86 - 3.23 (m, 3H), 3.16 - 2.65 (m, 7H), 2.25 - 1.18 (m, 13H), 0.97 - 0.56 (m, 6H).

Example 3A. Synthesis of Compound 3A

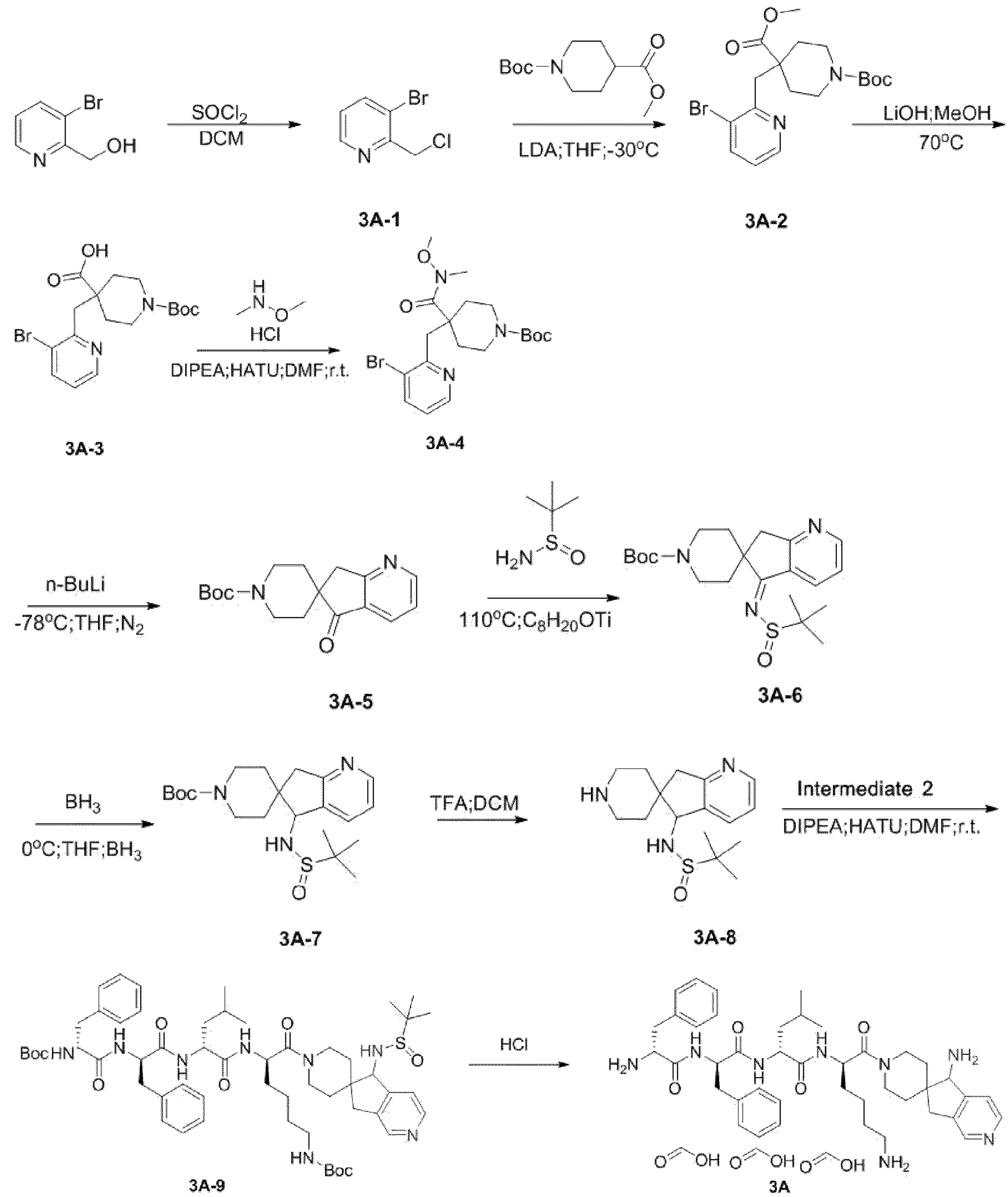


3A-1 3A-2 3A-3 3A-4 3A-5 3A-6 3A-7 3A-8 3A-9 3A

3.1 Synthesis of compound 3A-1

Thionyl chloride (1.27 g, 10.636 mmol, 2.0 equiv) was added portionwise to a stirred solution of (3-bromopyridin-2-yl)methanol (1 g, 5.318 mmol, 1.00 equiv) in DCM at 0 °C. The resulting mixture was stirred at 0 °C for 4 h. The mixture was neutralized with saturated $NaHCO_3$ (aq.) to pH 7. The crude product was purified by silica gel column chromatography and eluted with PE/EA (5:1), and the fractions were concentrated to dryness by rotary evaporation to give 3-bromo-2-(chloromethyl)pyridine (700 mg, 64.4%, purity: 99%).

LC-MS-3A-1 (ES, m/z): [M+1] = 205

3.2 Synthesis of compound 3A-2

LDA (10 mL, 73.747 mmol, 10.15 equiv) was added dropwise to a solution of 1-*tert*-butyl 4-methylpiperidine-1,4-dicarboxylate (2.65 g, 10.897 mmol, 1.5 equiv) in THF (50 mL) at -30 °C under nitrogen atmosphere. After 30 min, a

solution of 3-bromo-2-(chloromethyl)pyridine (1.5 g, 7.265 mmol, 1 equiv) in THF was added dropwise to the solution described above. The resulting mixture was stirred at room temperature overnight. The reaction was quenched with saturated $NH_4Cl$ (aq.) at room temperature. The resulting mixture was extracted with ethyl acetate. The organic phases were combined and concentrated to dryness by rotary evaporation. The crude product was purified by silica gel column chromatography and eluted with PE/EA (1:1), and the fractions were concentrated to dryness by rotary evaporation to give 1-*tert*-butyl 4-methyl 4-[(3-bromopyridin-2-yl)methyl]piperidine-1,4-dicarboxylate (3.7 g, 82.4%, purity: 99%).

LC-MS-3A-2 (ES, m/z): [M+1] = 413

3.3 Synthesis of compound 3A-3

LiOH (0.37 g, 8.709 mmol, 3 equiv) was added to a solution of 1-*tert*-butyl 4-methyl 4-[(3-bromopyridin-2-yl) methyl]piperidine-1,4-dicarboxylate (1.2 g, 2.903 mmol, 1.00 equiv) in methanol at 70 °C. The mixture was stirred at 70 °C for 6 h. The reaction solution was cooled, and then the pH of the mixture was adjusted to 6 with HCl (aq.). The resulting mixture was extracted with ethyl acetate. The organic phases were combined, dried, and concentrated to dryness by rotary evaporation. The crude product was purified by silica gel column chromatography and eluted with PE/EA (1:1), and the fractions were concentrated to dryness by rotary evaporation to give 4-[(3-bromopyridin -2-yl)methyl]-1-(tert-butoxycarbonyl)piperidine-4-carboxylic acid (630 mg, 54.34%, purity: 99%).

LC-MS-3A-3 (ES, m/z): [M+1] = 399

3.4 Synthesis of compound 3A-4

DIPEA (786.87 mg, 6.087 mmol, 3 equiv) and HATU (1543.30 mg, 4.058 mmol, 2 equiv) were added to a stirred solution of N,O-dimethylhydroxylamine hydrochloride (200 mg, 2.029 mmol, 1 equiv) and 4-[(3-bromopyridin-2-yl) methyl]-1-(*tert*-butoxycarbonyl)piperidine-4-carboxylic acid (1604.22 mg, 4.058 mmol, 2 equiv) in DMF at room temperature under nitrogen atmosphere. The mixture was stirred at 0 °C for 3 h under nitrogen atmosphere. The reaction solution was quenched with water and extracted with ethyl acetate. The organic phases were combined, dried, and concentrated to dryness by rotary evaporation. The crude product was purified by silica gel column chromatography and eluted with PE/EA (1:1), and the fractions were concentrated to dryness by rotary evaporation to give *tert*-butyl 4-[(3-bromopyridin-2-yl)methyl]-4-(methoxy(methyl)carbamoyl)piperidine-1-carboxylate (120 mg, 13.52%, purity: 99%).

LC-MS-3A-4 (ES, m/z): [M+1] = 442

3.5 Synthesis of compound 3A-5

Butyl lithium (1.74 mL, 27.130 mmol, 10 equiv) was added to a mixture of *tert*-butyl 4-[(3-bromopyridin-2-yl) methyl]-4-(methoxy(methyl)carbamoyl)piperidine-1-carboxylate (1.2 g, 2.713 mmol, 1 equiv) in THF at -78 °C under nitrogen atmosphere. The mixture was stirred at -78 °C for 3 h under nitrogen atmosphere. The reaction was quenched with a saturated $NH_4Cl$ (aq.) (2 mL) solution at room temperature. The resulting mixture was extracted with ethyl acetate. The organic phases were combined, dried, and concentrated to dryness by rotary evaporation. The crude product was purified by silica gel column chromatography and eluted with PE/EA (1:1), and the fractions were concentrated to dryness by rotary evaporation to give *tert*-butyl 5-oxo-7H-spiro[cyclopenta[b]pyridine-6,4'-piperidine]-1'-carboxylate (590 mg, 71.93%, purity: 99%).

LC-MS-3A-5 (ES, m/z): [M+1] = 303 3.6 Synthesis of compound 3A-6

*tert*-Butanesulfinamide (30.06 mg, 0.249 mmol, 3 equiv) was added dropwise to a stirred solution of *tert*-butyl 5-oxo-7H-spiro[cyclopenta[b]pyridine-6,4'-piperidine]-1'-carboxylate (25 mg, 0.083 mmol, 1 equiv) under air atmosphere. The mixture was stirred at 110 °C for 4 h under nitrogen atmosphere. The reaction solution was cooled to room temperature, and then the reaction was quenched with water. The resulting mixture was extracted with ethyl acetate. The organic phases were combined, dried, and concentrated to dryness by rotary evaporation. The crude product was purified by silica gel column chromatography and eluted with PE/EA (1:1), and the fractions were concentrated to dryness by rotary evaporation to give *tert*-butyl (5Z)-5-[(2-methylpropane-2-sulfinyl)imino]-7H-spiro[cyclopenta[b]pyridine-6,4'-piperidine]-1'-carboxylate (10 mg, 29.82%, purity: 99%).

LC-MS-3A-6 (ES, m/z): [M+1] = 413

3.7 Synthesis of compound 3A-7

A solution of *tert*-butyl (5Z)-5-[(2-methylpropane-2-sulfinyl)imino]-7H-spiro[cyclopenta[b]pyridine-6,4'-piperidine]-1'-carboxylate (20 mg, 0.049 mmol, 1 equiv) and $BH_3$-THF (0.2 mL, 2.327 mmol, 47.19 equiv) in THF was stirred at 0 °C for 3 h under nitrogen atmosphere. The reaction was quenched with MeOH (2 mL) at room temperature. The reaction solution was purified by reversed-phase flash chromatography under the following conditions: column: silica gel

column; mobile phase: MeCN/aqueous solution, gradient 10% to 100% within 10 min; detector: UV 220 nm. The fractions were concentrated to dryness by rotary evaporation to give *tert*-butyl 5-[(2-methylpropane-2-sulfinyl)amino]-5,7-dihydrospiro[cyclopenta|b]pyridine-6.4'-piperidine]-1'-carboxylate (10 mg, 49.75%, purity: 99%).

LC-MS-3A-7 (ES, m/z): [M+1] = 408

3.8 Synthesis of compound 3A-8

*tert*-Butyl 5-[(2-methylpropane-2-sulfinyl)amino]-5,7-dihydrospiro[cyclopenta[b]pyridine-6,4'-piperidine]-1'-carboxylate (180 mg, 0.4421 mmol, 1 equiv) and TFA (1 mL, 13.463 mmol, 30.48 equiv) were dissolved in DCM at room temperature under air atmosphere and stirred for 3 h. The resulting mixture was concentrated under reduced pressure to give N-{5,7-dihydrospiro[cyclopenta[b]pyridine-6,4'-piperidin]-5-yl}-2-methylpropane-2-sulfinamide (66 mg, crude), which was directly used in the next step without further purification.

LC-MS-3A-8 (ES, m/z): [M+1] = 308

3.9 Synthesis of compound 3A-9

A solution of N-{5,7-dihydrospiro[cyclopenta[b]pyridine-6,4'-piperidin]-5-yl}-2-methylpropane-2-sulfinamide (66 mg, 0.107 mmol, 1 equiv) and intermediate 2 (126.70 mg, 0.086 mmol, 0.8 equiv) in DMF was stirred at room temperature for 1 min under air atmosphere. DIPEA (41.62 mg, 0.323 mmol, 1.5 equiv) and HATU (163.24 mg, 0.430 mmol, 2 equiv) were added to the mixture described above at room temperature. The mixture was stirred at room temperature for 3 h under nitrogen atmosphere. The reaction solution was purified by reversed-phase flash chromatography under the following conditions: column: silica gel; mobile phase: MeCN/aqueous solution, gradient 10% to 50% within 10 min; detector: UV 220 nm. The fractions were concentrated to dryness by rotary evaporation to give *tert*-butyl N-[(5R)-5-[(2R)-2-[(2R)-2-[(2R)-2-[(*tert*-butoxycarbonyl)amino]-3-phenylpropionylamino]-3-phenylpropionylamino]-4-methylpentanoylamino]-6-{5-[(2-methylpropane-2-sulfinyl)amino]-5,7-dihydrospiro [cyclopenta[c]pyridine-6,4'-piperidin]-1'-yl }-6-oxohexyl]carbamate (30 mg, 13.39%, purity: 99%).

LC-MS-3A-9 (ES, m/z): [M+1] = 1043

3.10 Synthesis of compound 3A

4 M HCl (gas)/1,4-dioxane (5 mL) was added dropwise to *tert*-butyl N-[(5R)-5-[(2R)-2-[(2R)-2-[(2R)-2-[(*tert*-butoxycarbonyl)amino]-3-phenylpropionylamino]-3-phenylpropionylamino]-4-methylpentanoylamino]-6-{5-[(2-methylpropane-2-sulfinyl)amino]-5,7-dihydrospiro[cyclopenta[c]pyridine-6,4'-piperidin]-1'-yl}-6-oxohexyl]carbamate (110 mg, 0.105 mmol, 1 equiv). The resulting mixture was stirred at room temperature for 3 h under nitrogen atmosphere and concentrated under reduced pressure to give a crude product. The crude product (100 mg) was purified by preparative HPLC under the following conditions (column: C18 silica gel; mobile phase: acetonitrile/aqueous solution, gradient 10% to 50% within 10 min; detector: UV 220 nm), and the fractions were concentrated to dryness by rotary evaporation to give (2R)-N-[(2R)-6-amino-1-{5-amino-5,7-dihydrospiro[cyclopenta[c]pyridine-6,4'-piperidin]-1'-yl}-1-oxohex-2-yl]-2-[(2R)-2-amino-3-phenylpropionylamino]-3-phenylpropionylamino]-4-methylpentanamide (20.1 mg, 18.2%, purity: 70.3%).

LC-MS-3A (ES, m/z): [M+1] = 739

$^1$H NMR (400 MHz, Deuterium Oxide) δ 8.40 (s, 1H), 8.27 (s, 3H), 7.81 (dd, *J* = 8.2, 4.4 Hz, 1H), 7.41 - 6.90 (m, 11H), 4.58 - 4.38 (m, 2H), 4.36 - 4.16 (m, 2H), 4.10 (s, 2H), 3.98 - 3.71 (m, 1H), 3.39 (d, *J* = 12.4 Hz, 1H), 3.23 - 2.72 (m, 9H), 1.81 - 1.20 (m, 13H), 0.79 (dt, *J* = 20.4, 5.1 Hz, 6H).

Example 4A. Synthesis of Compound 4A

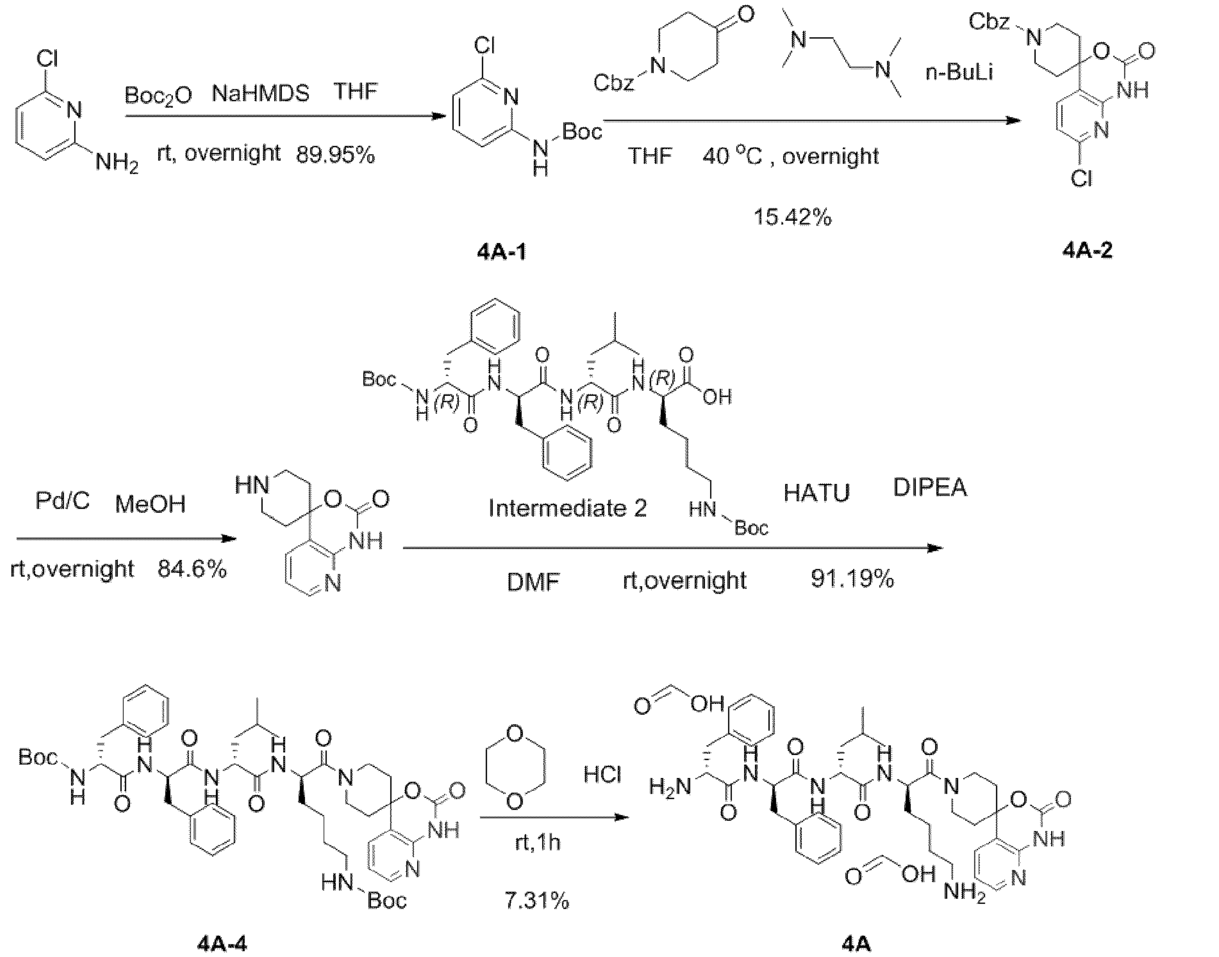


4A-1 4A-2 4A-4 4A

4.1 Synthesis of compound 4A-1

Sodium bis(trimethylsilyl)amide (1426.37 mg, 7.778 mmol) was to a stirred solution of 6-chloropyridin-2-amine (500 mg, 3.889 mmol, 1.00 equiv) in tetrahydrofuran (10 mL) at 0 °C under nitrogen atmosphere. Di-*tert*-butyl dicarbonate (933.69 mg, 4.278 mmol, 1.1 equiv) was added dropwise to the mixture described above. The resulting mixture was stirred at room temperature overnight. The reaction was quenched with water/ice at 0 °C. The resulting mixture was extracted with ethyl acetate (3 × 10 mL). The combined organic layer was washed with brine (1 × 10 mL), dried over anhydrous sodium sulfate, and filtered. The filtrate was concentrated under reduced pressure, purified by silica gel column chromatography, and eluted with petroleum ether/ethyl acetate (10:1), and the fractions were concentrated to dryness by rotary evaporation to give *tert*-butyl N-(6-chloropyridin-2-yl)carbamate (800 mg, 89.95%, purity: 99%).

LC-MS-4A-1 (ES, m/z): [M+1] = 229

4.2 Synthesis of compound 4A-2

In a two-necked round-bottom flask, tetramethylethylenediamine (3353.91 mg, 28.862 mmol, 2.2 equiv) was dissolved in tetrahydrofuran (78.43 mL). *n*-Butyllithium (1848.71 mg, 28.862 mmol, 2.2 equiv) was added dropwise to the mixture described above at -78 °C. The mixture was stirred at -20 °C for another 30 min. Benzyl 4-oxopiperidine-1-carboxylate (4590.26 mg, 19.678 mmol, 1.5 equiv) was added dropwise to the mixture described above at -78 °C. The mixture was stirred at -78 °C for another 1 h. *tert*-Butyl N-(6-chloropyridin-2-yl)carbamate (3000 mg, 13.119 mmol, 1.00 equiv) was added dropwise to the mixture described above at -50 °C. The resulting mixture was stirred at 40 °C overnight. The resulting mixture was concentrated under reduced pressure to give a residue. The residue was purified by silica gel column chromatography and eluted with petroleum ether/ethyl acetate (2:1) to give benzyl 7'-chloro-2'-oxo-1'H-spiro [piperidine-4,4'-pyrido[2,3-d][1,3]oxazine]-1-carboxylate (3000 mg, 58.97%, purity: 99%).

LC-MS-4A-2 (ES, m/z): [M+1] = 388

4.3 Synthesis of compound 4A-3

Benzyl 7'-chloro-2'-oxo-1'H-spiro[piperidine-4,4'-pyrido[2,3-d][1,3]oxazine]-1-carboxylate (230 mg, 0.612 mmol, 1.00 equiv) was dissolved in methanol (20 mL), and Pd/C (12.62 mg, w/t, 10%) was added. The system was purged 3 times with hydrogen. The resulting mixture was stirred at room temperature overnight under hydrogen atmosphere. The resulting mixture was filtered, and the filter cake was washed with methanol (3 × 10 mL). The filtrate was concentrated under reduced pressure to give spiro[piperidine-4,4'-pyrido[2,3-d][1,3]oxazin]-2'(1'H)-one (110 mg, crude).

LC-MS-4A-3 (ES, m/z): [M+1] = 220

4.4 Synthesis of compound 4A-4

Spiro[piperidine-4,4'-pyrido[2,3-d][1,3]oxazin]-2'(1'H)-one (44 mg, 0.138 mmol,1.00 equiv) and intermediate 2 (100 mg, 0.067 mmol, 0.5 equiv) were dissolved in DMF (2 mL), and diisopropylethylamine (34 mg, 0.4 mmol, 3.00 equiv) and HATU (76 mg, 0.207 mmol, 1.50 equiv) were added portionwise. The mixture was stirred at room temperature overnight under nitrogen atmosphere. Water (10 mL) was added to quench the reaction, and the resulting mixture was extracted with ethyl acetate (3 × 10 mL). The organic phases were combined, dried, and concentrated *in vacuo* to give *tert*-butyl ((10R,13R,16R,19R)-16-benzyl-13-isobutyl-2,2-dimethyl-4,12,15,18-tetraoxo-10-(2'-oxo-1',2'-dihydrospiro[piperidine-4,4'-pyrido[2,3-d][1,3]oxazine]-1-carbonyl)-20-phenyl-3-oxa-5,11,14,17-tetraazaeicosan-19-yl)carbamate (120 mg, crude).

LC-MS-4A-4 (ES, m/z): [M+1] = 955

4.5 Synthesis of compound 4A

*tert*-Butyl ((10R,13R,16R,19R)-16-benzyl-13-isobutyl-2,2-dimethyl-4,12,15,18-tetraoxo-10-(2'-oxo-1',2'-dihydrospiro[piperidine-4,4'-pyrido[2,3-d][1,3]oxazine]-1-carbonyl)-20-phenyl-3-oxa-5,11,14,17-tetraazaeicosan-19-yl)carbamate (120 mg, 0.126 mmol, 1.00 equiv) and a 4 M solution of HCl (gas) in 1,4-dioxane (4 mL) were added to a 50 mL round-bottom flask. The resulting mixture was stirred at room temperature for 1 h. The resulting mixture was concentrated under reduced pressure and purified by reverse flash chromatography, and the fractions were concentrated to dryness by rotary evaporation to give (2R)-N-[(2R)-6-amino-1-oxo-1-{2'-oxo-1'H-spiro[piperidine-4,4'-pyrido[2,3-d][1,3]oxazin]-1-yl}hex-2-yl]-2-[(2R)-2-[(2R)-2-amino-3-phenylpropionylamino]-3-phenylpropionylamino]-4-methylpentanamide (7 mg, 7.31%, purity: 99.2%).

LC-MS-4A (ES, m/z): [M+1] = 755

$^1$H NMR (400 MHz, Deuterium Oxide) δ 8.36 (s, 2H), 8.10 (dd, *J* = 10.1, 5.0 Hz, 1H), 7.58 (dd, *J* = 15.2, 7.7 Hz, 1H), 7.24 (h, *J* = 7.1 Hz, 6H), 7.12 (q, *J* = 7.7 Hz, 5H), 4.67 (s, 1H), 4.55 (q, *J* = 7.5 Hz, 1H), 4.33 (d, *J* = 13.6 Hz, 1H), 4.25 - 4.16 (m, 1H), 4.07 - 3.82 (m, 2H), 3.66 - 3.43 (m, 1H), 3.09 (q, *J* = 13.2 Hz, 1H), 3.04 - 2.82 (m, 6H), 2.15 (d, *J* = 27.4 Hz, 2H), 2.05 - 1.82 (m, 2H), 1.78 - 1.55 (m, 4H), 1.57 - 1.23 (m, 5H), 0.84 (dt, *J* = 20.5, 5.2 Hz, 6H).

Example 5A. Synthesis of Compound 5A

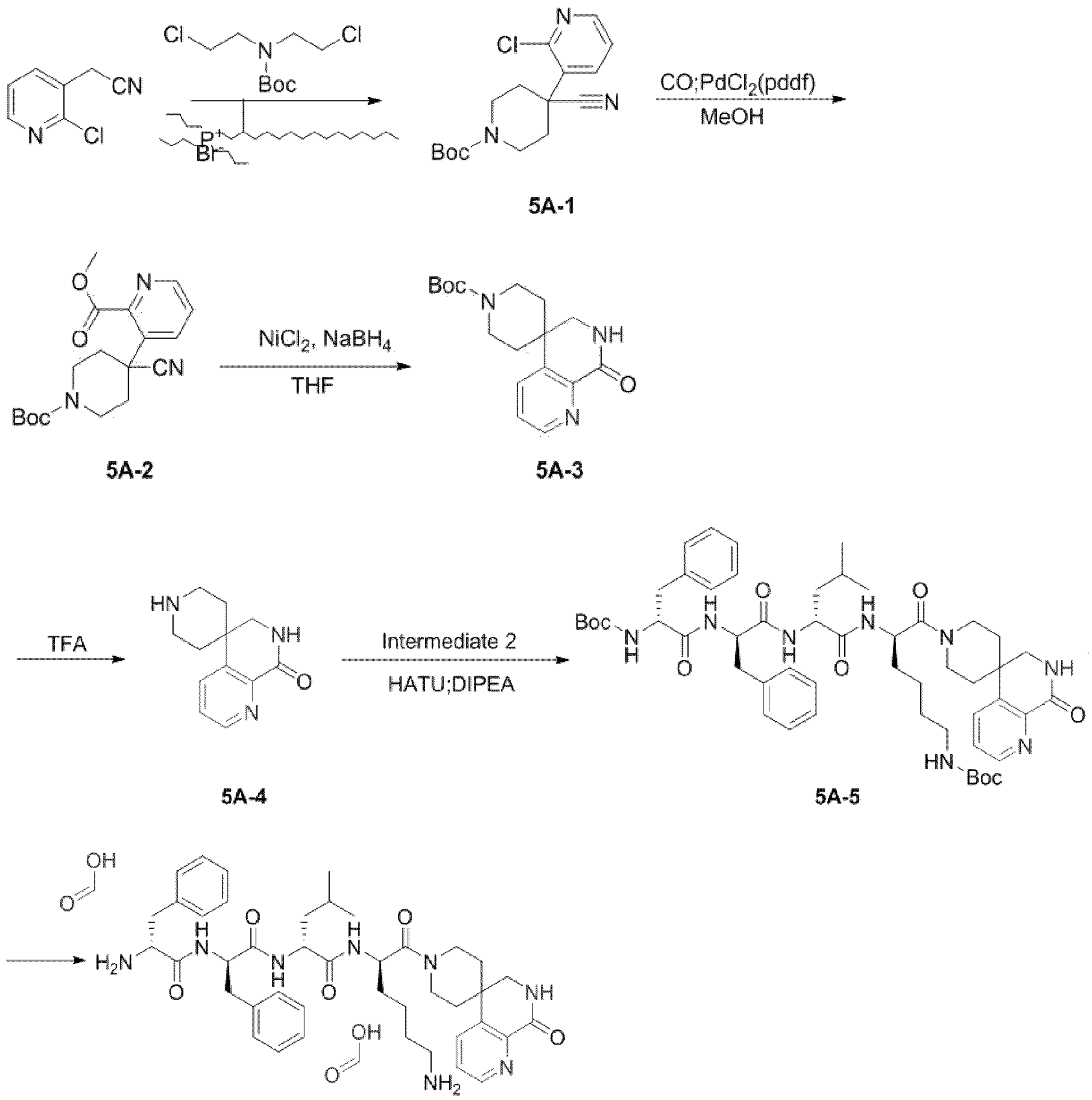


5A-1

5A-2

5A-3

5A-4

5A-5

5A

LC-MS-5A (ES, m/z): [M+1] = 753

$^1$H NMR (300 MHz, Deuterium Oxide) δ 8.55 - 8.39 (m, 1H), 8.35 (s, 2H), 7.97 - 7.74 (m, 1H), 7.56 (d, *J* = 5.1 Hz, 1H), 7.37 - 7.00 (m, 10H), 4.85-4.70(m, 2H), 4.58 - 4.46 (m, 1H), 4.33 - 4.13 (m, 2H), 4.05 - 3.79 (m, 2H), 3.71 - 3.54 (m, 2H), 3.50 - 3.26 (m, 1H), 3.02 - 2.82 (m, 6H), 1.95 - 1.17 (m, 13H), 0.83 (dd, *J* = 14.9, 6.7 Hz, 6H).

Example 6A. Synthesis of Compound 6A

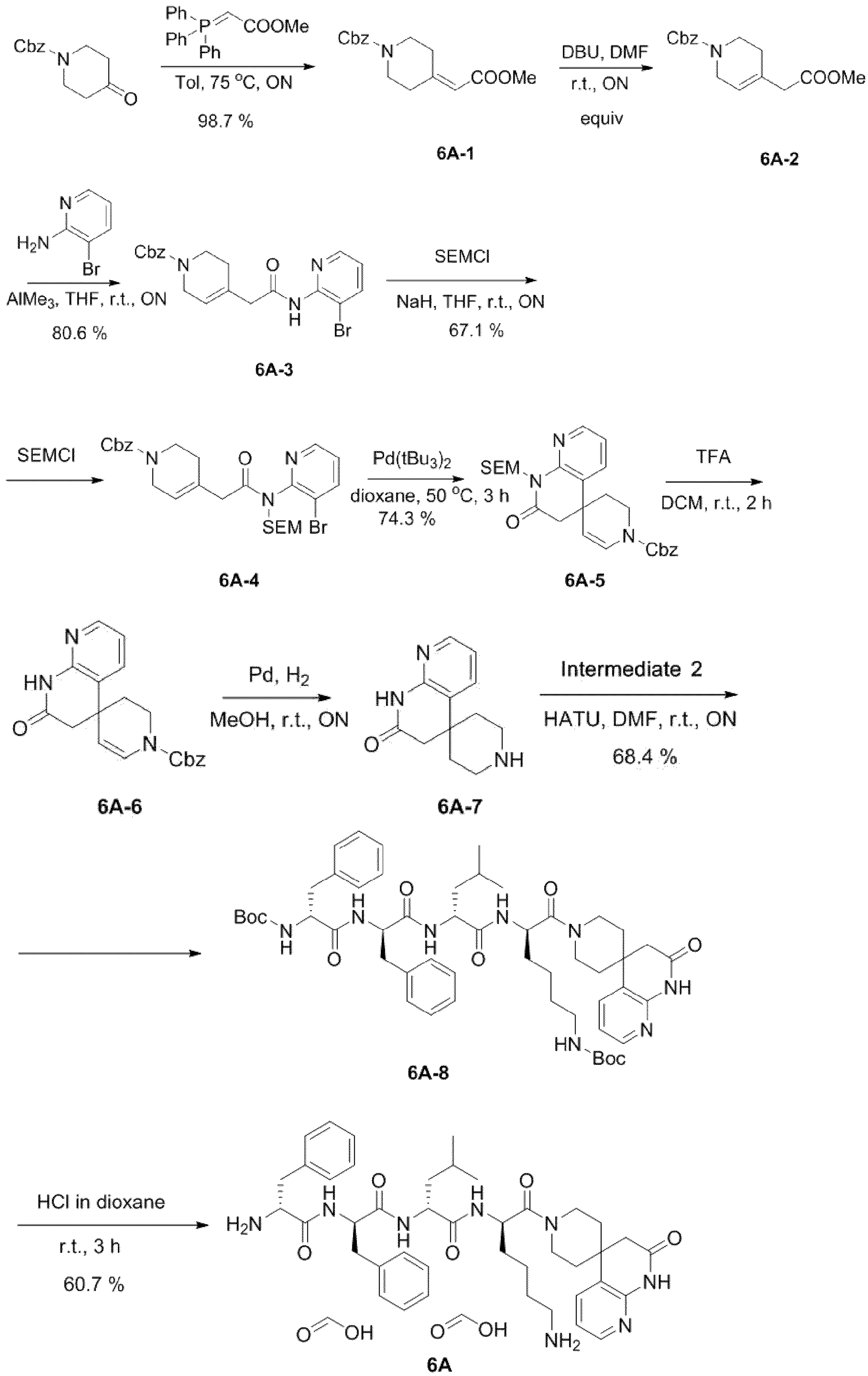


6A-1

6A-2

6A-3

6A-4

6A-5

6A-6

6A-7

6A-8

6A

LC-MS-6A (ES, m/z): [M+1] = 753

$^1H$ NMR (400 MHz, Deuterium Oxide) δ 8.37 (s, 2H), 8.23 - 7.98 (m, 1H), 7.77 (dd, *J* = 27.3, 7.8 Hz, 1H), 7.47 - 6.96 (m, 11H), 4.85 - 4.77 (m, 1H), 4.58 (t, *J* = 7.5 Hz, 1H), 4.34 - 4.01 (m, 3H), 3.86 (dd, *J* = 53.3, 14.0 Hz, 1H), 3.46 (q, *J* = 12.7, 12.2 Hz, 1H), 3.25 - 2.68 (m, 9H), 2.10 - 1.58 (m,8H), 1.47 (d, *J* = 6.9 Hz, 5H), 1.13 - 0.44 (m, 6H).

Example 7A. Synthesis of Compound 7A

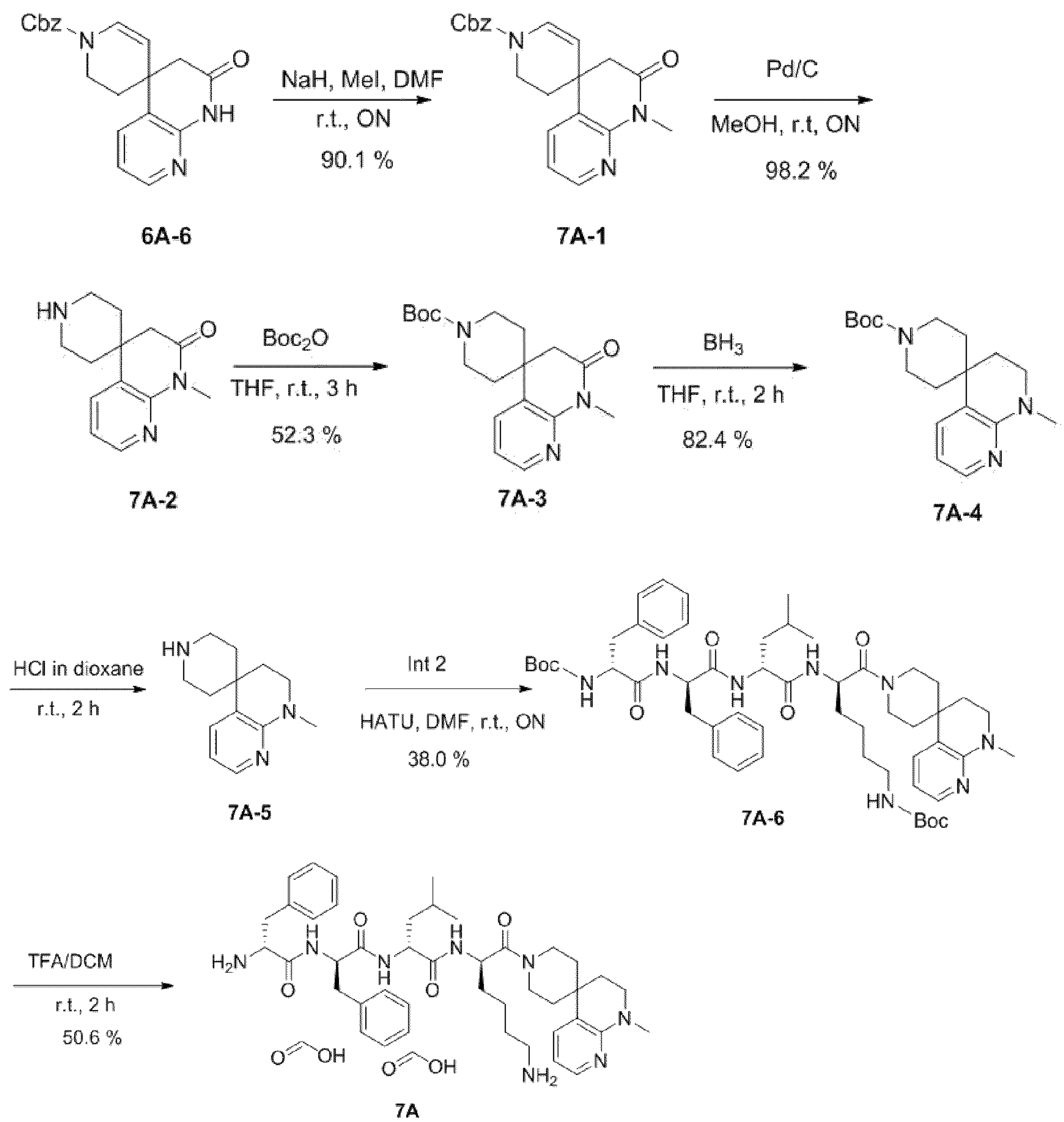


6A-6 7A-1 7A-2 7A-3 7A-4 7A-5 7A-6 7A

LC-MS-7A (ES, m/z): [M+1] = 753

$^1H$ NMR (400 MHz, Deuterium Oxide) δ 8.38 (s, 2H), 7.88 - 7.37 (m, 2H), 7.42 - 7.00 (m, 10H), 6.90 - 6.47 (m, 1H), 4.57 (t, *J* = 7.4 Hz, 2H), 4.37 - 3.97 (m, 3H), 3.86 (dd, *J* = 44.1, 13.8 Hz, 1H), 3.63 - 3.34 (m, 3H), 3.20 - 2.66 (m, 9H), 2.28 - 1.11 (m, 15H), 0.84 (dd, *J* = 20.1, 5.4 Hz, 6H).

Example 8A. Synthesis of Compound 8A

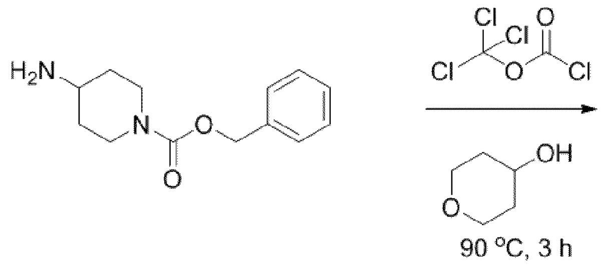


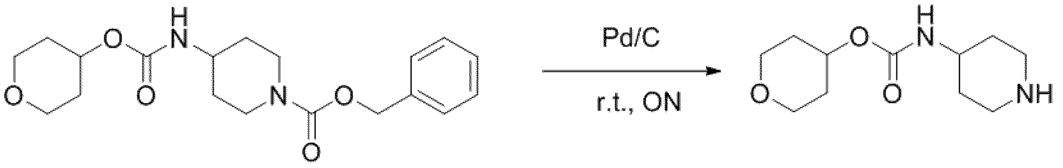


8A-1 8A-2

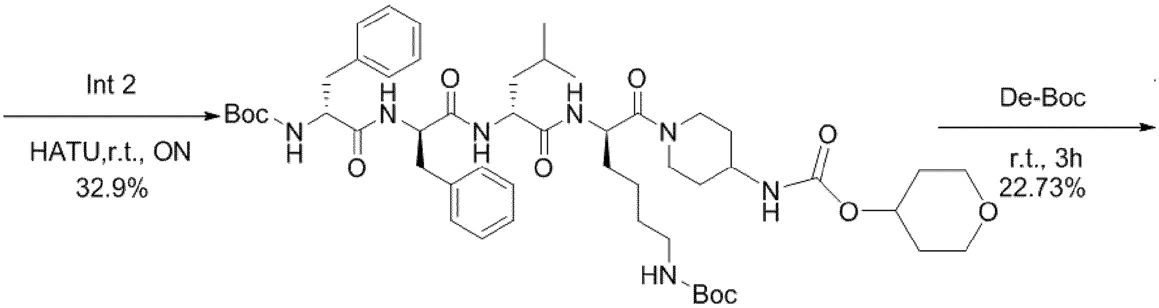


8A-3

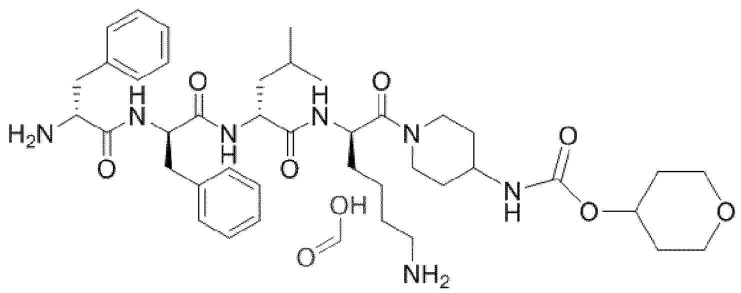

8A

8.1 Synthesis of compound 8A-1

Benzyl 4-aminopiperidine-1-carboxylate (500 mg, 2.134 mmol, 1 equiv) was dissolved in toluene (5 mL), and diphosgene (260 µL, 1.314 mmol, 0.62 equiv) was added. The resulting mixture was stirred at 90 °C for 3 h, and then tetrahydropyran-4-ol (205 µL, 2.007 mmol, 0.94 equiv) was added dropwise. The resulting mixture was stirred at 90 °C overnight under nitrogen atmosphere. The reaction solution was cooled to room temperature and then poured into ice water, and the resulting mixture was extracted with EtOAc (3 × 10 mL). The combined organic layer was washed with ethyl acetate (3 × 10 mL), dried over anhydrous $Na_2SO_4$, and filtered. The filtrate was concentrated under reduced pressure. The crude product was directly used in the next step without further purification.

8.2 Synthesis of compound 8A-2

Compound 8A-1 (433 mg, 1.195 mmol, 1 equiv) and Pd/C (100 mg, w/t, 10%) were added to methanol (10 mL, 246.988 mmol, 206.73 equiv), and the mixture was stirred at room temperature overnight under hydrogen atmosphere. The resulting mixture was filtered, and the filter cake was washed with MeOH (3 × 10 mL). The filtrate was concentrated

under reduced pressure, and the filtrate was concentrated to dryness by rotary evaporation to give oxacyclohexan-4-yl N-(piperidin-4-yl)carbamate (448 mg, crude).

8.3 Synthesis of compound 8A-3

Oxacyclohexan-4-yl N-(piperidin-4-yl)carbamate (113 mg, 0.495 mmol, 1.5 equiv) was dissolved in DMF (10 mL) at room temperature under nitrogen atmosphere, and intermediate 2 (248.79 mg, 0.330 mmol, 1 equiv) was added. The resulting mixture was stirred, and then DIPEA (85.30 mg, 0.660 mmol, 2 equiv) and HATU (188.21 mg, 0.495 mmol, 1.5 equiv) were added. After the addition was completed, the resulting mixture was stirred overnight. Water was added to quench the reaction, and the resulting mixture was extracted with EtOAc (3 × 20 mL). The combined organic layer was washed with diethyl ether (3 × 20 mL), dried over anhydrous $Na_2SO_4$, and filtered. The filtrate was concentrated under reduced pressure. The residue was purified by reversed-phase flash chromatography under the following conditions: column: silica gel; mobile phase: MeCN aqueous solution, gradient 10% to 50% within 10 min; detector: UV 254 nm. The fractions were concentrated to dryness by rotary evaporation to give compound 8A-3 (104 mg, 32.69%, purity: 99%).

8.4 Synthesis of compound 8A

Compound 8A-3 (104 mg, 0.108 mmol, 1 equiv) and TFA (4 mL) were added to DCM (16 mL) at room temperature under nitrogen atmosphere and stirred for 3 h. The resulting mixture was concentrated under reduced pressure to give a residue. The residue was purified by reversed-phase flash chromatography under the following conditions: column: silica gel; mobile phase: MeCN aqueous solution, gradient 10% to 50% within 10 min; detector: UV 254 nm. The fractions were concentrated to dryness by rotary evaporation to give compound 8A (19.7 mg, 22.73%, purity: 95.0%).

LC-MS-8A (ES, m/z): [M+1] = 764

$^1$H NMR (400 MHz, Deuterium Oxide) δ 8.36 (s, 1H), 7.26 (h, *J* = 6.6 Hz, 6H), 7.13 (t, *J* = 7.1 Hz, 4H), 4.55 (dt, *J* = 8.0, 4.0 Hz, 2H), 4.30 - 3.99 (m, 2H), 3.98 - 3.72 (m, 4H), 3.56 (d, *J*= 10.5 Hz, 3H), 3.22 (q, *J* = 13.9 Hz, 1H), 2.96 (t, *J* = 6.7 Hz, 3H), 2.90 (td, *J* = 7.6, 2.7 Hz, 3H), 1.85 (s, 4H), 1.74 - 1.52 (m, 6H), 1.52 - 1.17 (m, 7H), 0.93 - 0.75 (m, 6H).

Example 9A. Synthesis of Compound 9[a]

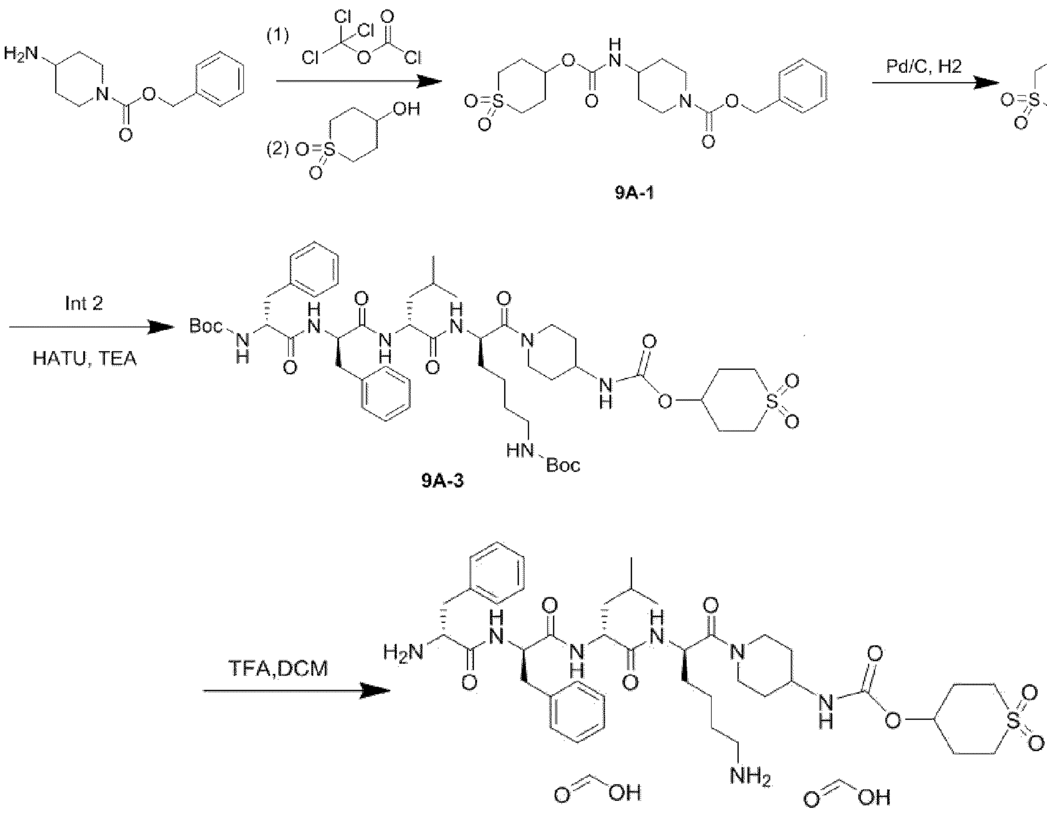


9A-1

9A-2

9A-3

9A

LC-MS-9A (ES, m/z): [M+1] = 812.25

$^1$H NMR (300 MHz, Deuterium Oxide) δ 8.41 (s, 2H), 7.38 - 7.16 (m, 10H), 4.92 (s, 1H), 4.60 (s, 2H), 4.26 (s, 2H), 4.02 (d, $J$ = 38.6 Hz, 2H), 3.67 (s, 1H), 3.41 - 2.90 (m, 12H), 2.28 (s, 4H), 1.93 (d, $J$ = 21.3 Hz, 2H), 1.71 - 1.28 (m, 11H), 0.95 - 0.83 (m, 6H).

Example 10A. Synthesis of Compound 10A

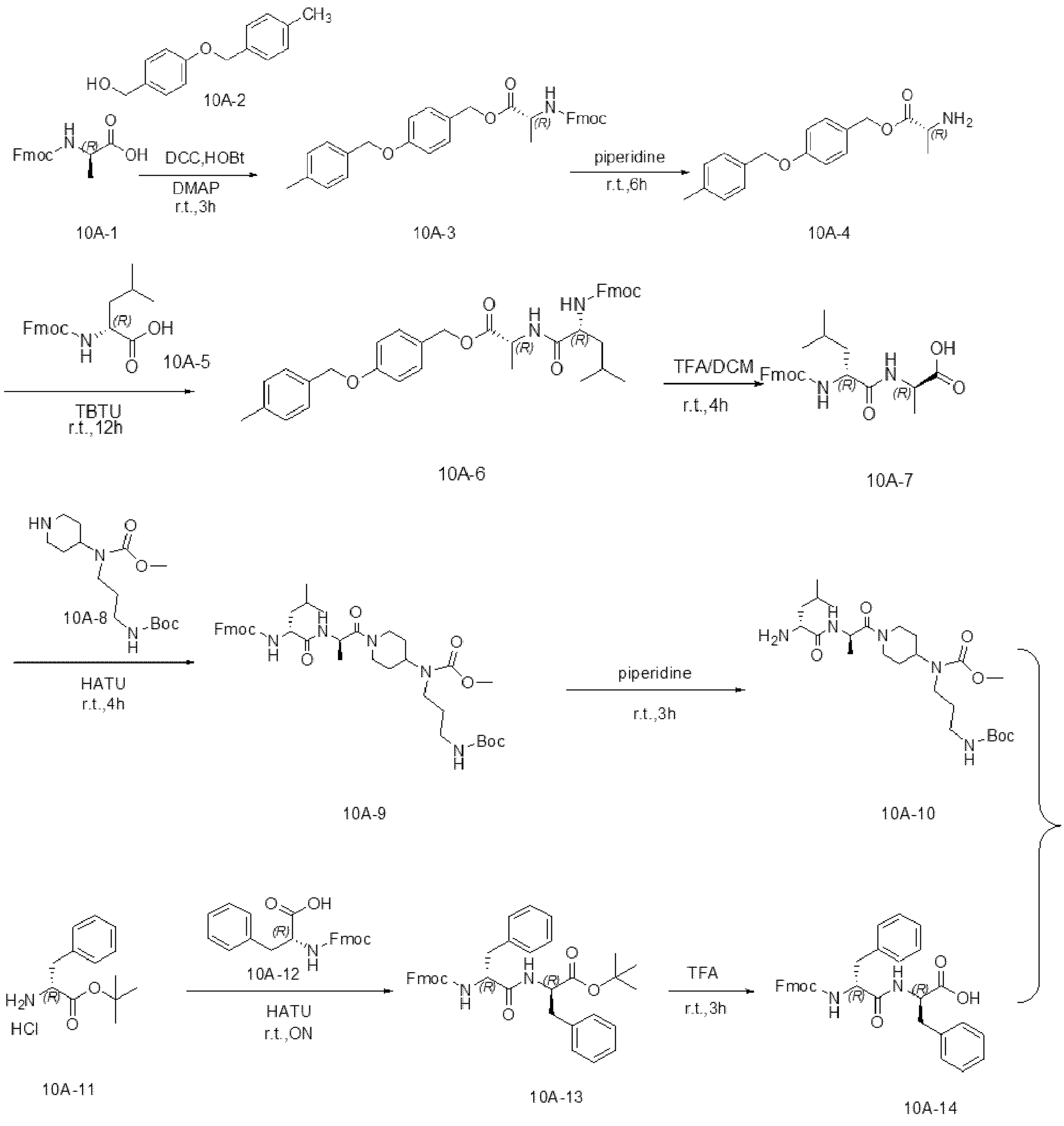

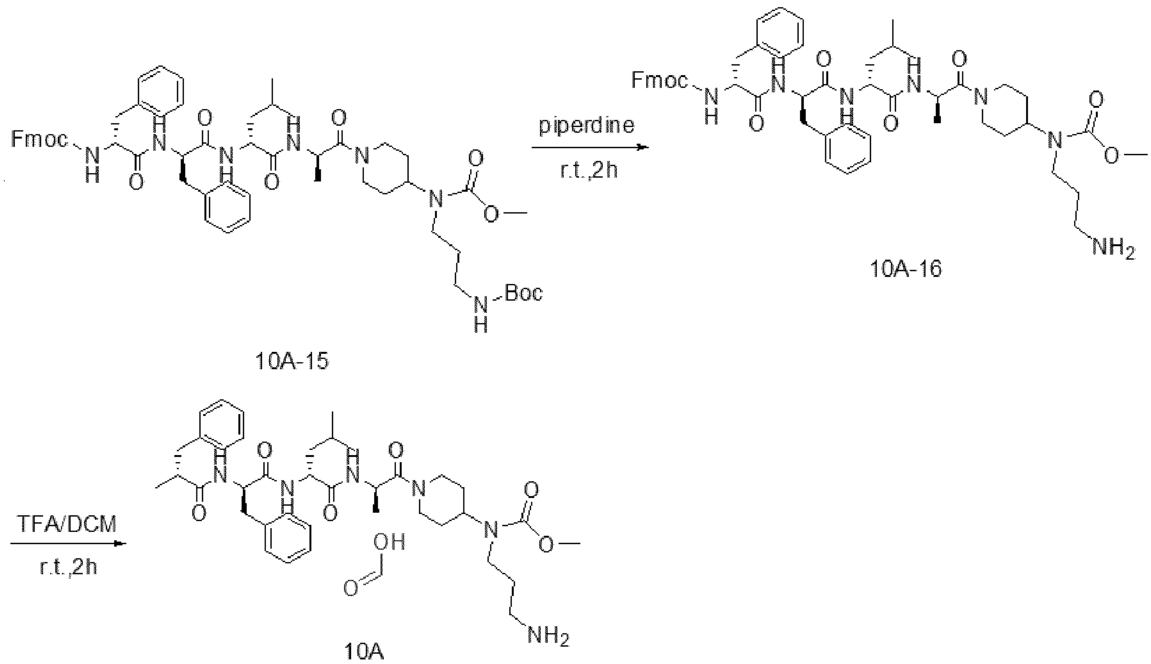

10A-15

10A-16

10A

10.1 Synthesis of compound 10A-3

Compound 10A-2 (1 g) was added to a 100 mL round-bottom flask, and stirred and swollen in DCM (40 mL) for 30 min at room temperature. The resulting mixture was filtered, and the solid was washed with DCM and dried. The solid was dissolved in DMF (40 mL), followed by addition of compound 10A-1 (2 g, 8.761 mmol, 1.00 equiv), DCC (5.42 g, 26.283 mmol, 3 equiv) and HOBT (2.37 g, 17.522 mmol, 2 equiv). The resulting mixture was stirred at room temperature for 3 h, and finally, DMAP (0.54 g, 4.380 mmol, 0.5 equiv) was added and stirred overnight at room temperature. The resulting mixture was filtered and washed with MeOH, and the solid was collected to give compound 10A-3 (3 g, crude) as a white solid. The resulting mixture was directly used in the next step without further purification.

10.2 Synthesis of compound 10A-4

A solution of compound 10A-3 (2.9 g, 5.560 mmol, 1 equiv) in DMF (40 mL) and piperidine (10 mL) was stirred at room temperature for 6 h under nitrogen atmosphere. The resulting mixture was filtered and washed with MeOH, and the solid was collected and concentrated to dryness by rotary evaporation. The resulting white crude product (2 g) was directly used in the next step.

10.3 Synthesis of compound 10A-6

Under nitrogen atmosphere, compound 10A-5 (3.54 g, 10.021 mmol, 1.5 equiv) was added to a solution of compound 10A-4 (2 g, 6.681 mmol, 1 equiv) in DMF (10 mL) at room temperature, and DIEA (1.30 g, 10.021 mmol, 1.5 equiv), HOBT (1.35 g, 10.021 mmol, 1.5 equiv) and TBTU (3.22 g, 10.021 mmol, 1.5 equiv) were added at room temperature. After stirring at room temperature for 2 h, the resulting mixture was filtered and washed with MeOH, and the solid was collected and concentrated under reduced pressure to give compound 10A-6 (2.9 g, crude).

10.4 Synthesis of compound 10A-7

A mixed solution of compound 10A-6 (2 g, 3.151 mmol, 1 equiv) in TFA (10 mL, 134.631 mmol, 42.73 equiv) and DCM (40 mL, 629.224 mmol, 199.71 equiv) was stirred at room temperature for 4 h under nitrogen atmosphere. The resulting mixture filtrate was concentrated under reduced pressure and concentrated to dryness by rotary evaporation to give compound 10A-7 (1.6 g, crude).

LC-MS-10A-7 (ES, m/z): [M+1] = 424

10.5 Synthesis of compound 10A-9

Compound 10A-8 (229.60 mg, 0.728 mmol, 1.5 equiv) was added to a solution of compound 10A-7 (206 mg, 0.485 mmol, 1 equiv) in DMF (10 mL) at room temperature under nitrogen atmosphere, and HATU (276.78 mg, 0.728 mmol, 1.5 equiv) and DIPEA (125.44 mg, 0.970 mmol, 2 equiv) were added at room temperature. After the resulting mixture was stirred for 4 h, water was added to quench the reaction. The resulting mixture was extracted with EA (3 × 100 mL). The organic layer was washed with EA (3 × 10 mL), dried over $Na_2SO_4$, and filtered. The resulting solution was concentrated under reduced pressure. The resulting crude product was purified by reversed-phase column chromatography (conditions were as follows: C18 chromatographic column, mobile phase: solvent ACN and solvent $H_2O$ (0.1% FA), gradient 10% to 50% for 10 min, UV254 nanometer detector), and the fractions were concentrated to dryness by rotary evaporation to give compound 10A-9 (165 mg, 47.10%).

LC-MS-10A-9 (ES, m/z): [M+1] = 721

10.6 Synthesis of compound 10A-10

A solution of compound 10A-9 (165 mg, 0.229 mmol, 1 equiv) in piperidine (1 mL) and DCM (10 mL) was stirred at room temperature for 3 h under nitrogen atmosphere. The resulting solution was concentrated under reduced pressure. The resulting crude product was purified by reversed-phase column chromatography under the following conditions: C18 chromatographic column, mobile phase: solvent ACN and solvent $H_2O$ (0.1% FA), gradient 10% to 50% for 10 min, UV254 nanometer detector. The fractions were concentrated to dryness by rotary evaporation to give compound 10A-10 (100 mg, 87.5%).

LC-MS-10A-10 (ES, m/z): [M+1] = 499

10.7 Synthesis of compound 10A-13

Compound 10A-12 (1.93 g, 4.981 mmol, 1.28 equiv) was added to a solution of compound 10A-11 (1 g, 3.880 mmol, 1 equiv) in DMF (10 mL) at room temperature under nitrogen atmosphere, and HATU (2.1 g, 8.712 mmol, 2.25 equiv) and DIPEA (1.8 g, 13.927 mmol, 3.59 equiv) were added dropwise at room temperature and stirred overnight. The resulting mixture was quenched with water and extracted with EA (3 × 100 mL). The organic phases were combined, dried, concentrated to dryness by rotary evaporation, and filtered. The resulting solution was concentrated under reduced pressure. The resulting crude product was purified by reversed-phase column chromatography (conditions were as follows: C18 chromatographic column, mobile phase: solvent ACN and solvent $H_2O$ (0.1% FA), gradient 10% to 50% for 10 min, UV254 nanometer detector), and the fractions were concentrated to dryness by rotary evaporation to give compound 10A-13 (1.86 g, 81.16%, purity: 99%).

LC-MS-10A-13 (ES, m/z): [M+1] = 590

10.8 Synthesis of compound 10A-14

A mixed solution of compound 10A-13 (1.86 g, 3.149 mmol, 1 equiv) in TFA (6 mL, 61.209 mmol, 19.44 equiv)/DCM (24 mL, 377.534 mmol, 119.90 equiv) was stirred at room temperature for 3 h under nitrogen atmosphere. The resulting mixture was concentrated under reduced pressure to give compound 10A-14 (2.7 g, crude).

LC-MS-10A-14 (ES, m/z): [M+1] = 535

10.9 Synthesis of compound 10A-15

Compound 10A-14 (74.90 mg, 0.140 mmol, 1 equiv) was added to a solution of compound 10A-10 (70 mg, 0.140 mmol, 1 equiv) in DMF (10 mL) at room temperature under nitrogen atmosphere, and HATU (53.27 mg, 0.140 mmol, 1.5 equiv) and DIPEA (24.14 mg, 0.187 mmol, 1.25 equiv) were added at room temperature and stirred for 4 h. The reaction solution was quenched with water and extracted with EA (3 × 100 mL). The organic phases were combined, dried, and concentrated to dryness by rotary evaporation. The resulting crude product was purified by reversed-phase column chromatography (conditions were as follows: C18 chromatographic column, mobile phase: solvent ACN and solvent $H_2O$ (0.1% FA), gradient 10% to 50% for 10 min, UV254 nanometer detector), and the fractions were concentrated to dryness by rotary evaporation to give compound 10A-15 (118 mg, 83.1%, purity: 99%).

LC-MS-10A-15 (ES, m/z): [M+1] = 1017

10.10 Synthesis of compound 10A-16

A solution of compound 10A-15 (118 mg, 0.116 mmol, 1 equiv) in piperidine (1 mL) and DCM (10 mL) was stirred

at room temperature for 2 h under nitrogen atmosphere. The resulting solution was concentrated under reduced pressure to give compound 10A-16 (94 mg, crude), which was directly used in the next step.

LC-MS-10A-16 (ES, m/z): [M+1] = 916

10.11 Synthesis of compound 10A

A mixed solution of compound 10A-16 (94 mg, 0.118 mmol, 1 equiv) in TFA (1 mL)/DCM (5 mL) was stirred at room temperature for 2 h under nitrogen atmosphere. The resulting mixture filtrate was concentrated under reduced pressure. The resulting crude product was purified by reversed-phase column chromatography (conditions were as follows: C18 chromatographic column, mobile phase: solvent ACN and solvent $H_2O$ (0.1% FA), gradient 10% to 50% for 10 min, UV254 nanometer detector), and the fractions were concentrated to dryness by rotary evaporation to give compound 10A (0.0233 g, 28.22%, purity: 99.0%).

LC-MS-10A (ES, m/z): [M+1] = 694

$^1H$ NMR (400 MHz, Deuterium Oxide) δ 8.37 (s, 1H), 7.30 (s, 3H), 7.30 - 7.19 (m, 4H), 7.14 (dd, *J* = 6.8, 4.0 Hz, 3H), 4.61 (t, *J* = 6.7 Hz, 1H), 4.53 (t, *J* = 7.5 Hz, 1H), 4.37 (d, *J* = 13.1 Hz, 1H), 4.22 (t, *J* = 7.9 Hz, 1H), 4.00 (t, *J* = 13.1 Hz, 2H), 3.91 (d, *J* = 5.7 Hz, 1H), 3.64 (s, 3H), 3.21 (s, 2H), 3.13 (d, *J* = 13.5 Hz, 1H), 2.94 (ddd, *J* = 15.8, 9.8, 4.5 Hz, 6H), 2.66 (s, 1H), 1.81 (q, *J* = 7.3 Hz, 3H), 1.74 (d, *J* = 14.4 Hz, 2H), 1.46 (d, *J* = 8.1 Hz, 4H), 1.26 (dd, *J* = 14.7, 7.1 Hz, 3H), 0.85 (dt, *J* = 20.3, 5.0 Hz, 6H).

Example 11A. Synthesis of Compound 11A

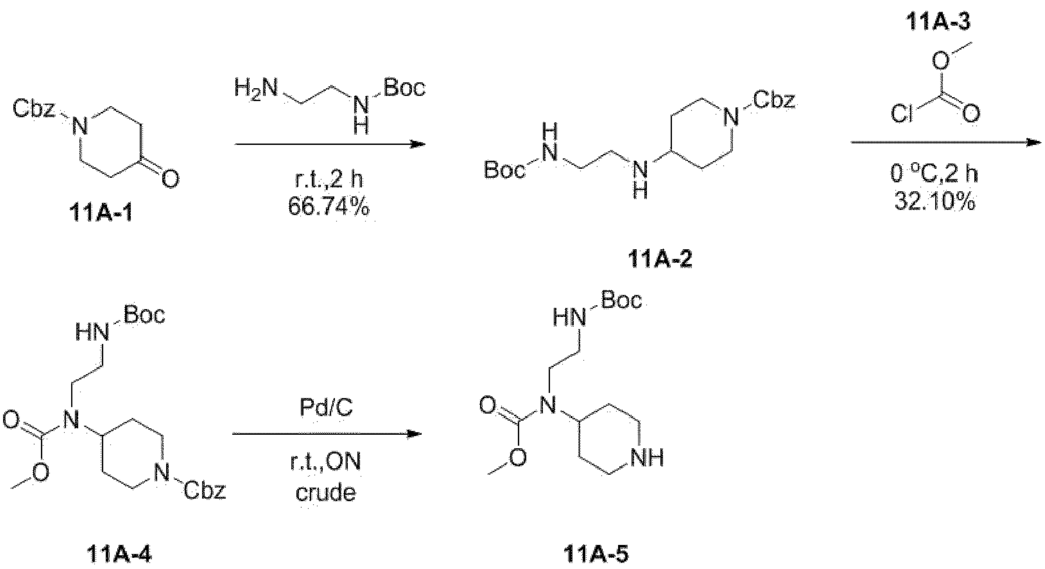


11A-1 11A-2 11A-3 11A-4 11A-5

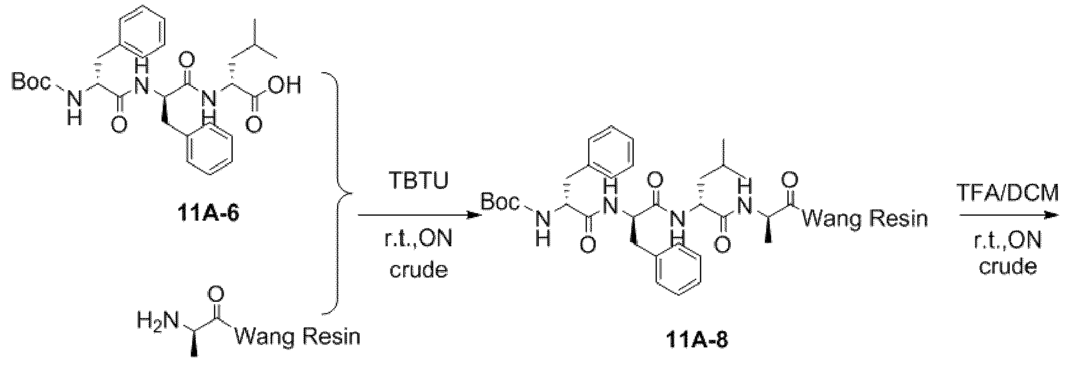


11A-6 10A-4 11A-8

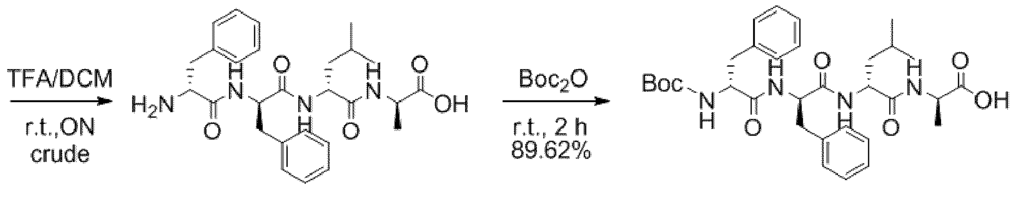


11A-9 11A-10

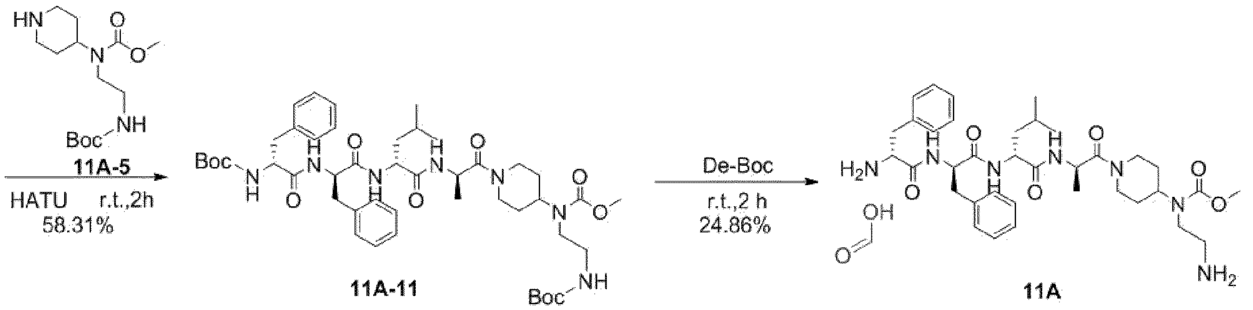


11A-11 11A

LC-MS-11A (ES, m/z): [M+1] = 680

$^{1}$H NMR (400 MHz, Deuterium Oxide) δ 8.35 (s, 1H), 7.24 (ddd, J = 11.2, 7.7, 5.5 Hz, 6H), 7.10 (t, J = 9.0 Hz, 4H), 4.69 (s, 2H), 4.58 (t, J = 7.1 Hz, 1H), 4.48 (t, J = 7.4 Hz, 1H), 4.36 (s, 1H), 4.20 (t, J = 8.0 Hz, 1H), 4.08 (s, 1H), 4.00 (d, J = 13.6 Hz, 1H), 3.68 (d, J = 6.7 Hz, 1H), 3.64 (s, 1H), 3.47 - 3.39 (m, 2H), 3.14 (t, J = 13.3 Hz, 1H), 3.06 - 2.98 (m, 2H), 2.90 (s, 2H), 2.85 (dd, J = 17.8, 6.6 Hz, 2H), 2.65 (s, 1H), 1.75 (s, 2H), 1.51 (s, 3H), 1.43 (s, 3H), 1.24 (dd, J = 12.7, 7.0 Hz, 3H), 0.82 (dt, J = 20.9, 5.0 Hz, 6H).

Example 12A. Synthesis of Compound 12A

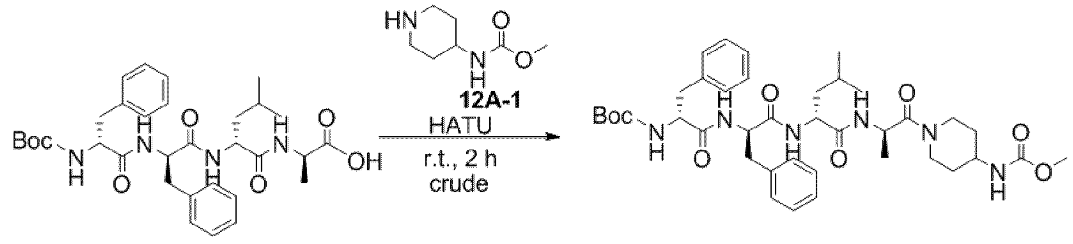


11A-10 12A-2

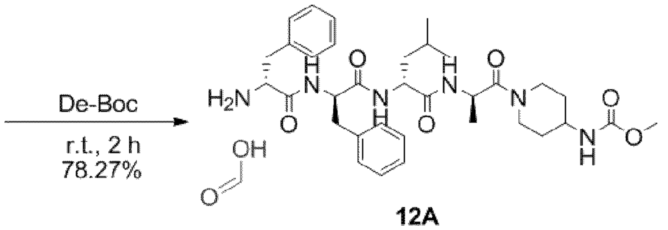


**12A**

LC-MS-12A (ES, m/z): [M+1] = 637

$^1$H NMR (400 MHz, Deuterium Oxide) δ 8.37 (s, 1H), 7.32 - 7.17 (m, 6H), 7.12 (dd, J = 7.2, 3.8 Hz, 4H), 4.68 (s, 1H), 4.61 - 4.46 (m, 1H), 3.88 (t, J = 6.9 Hz, 2H), 3.61 (s, 2H), 3.56 (s, 4H), 3.21 (q, J = 13.1 Hz, 3H), 2.98 - 2.88 (m, 4H), 1.93 (s, 1H), 1.84 (d, J = 13.1 Hz, 1H), 1.43 (s, 2H), 1.30 (s, 2H), 1.23 (t, J = 7.0 Hz, 4H), 0.85 - 0.80 (d, J = 4.9 Hz, 6H).

Example 1B. Synthesis of Compound 1B

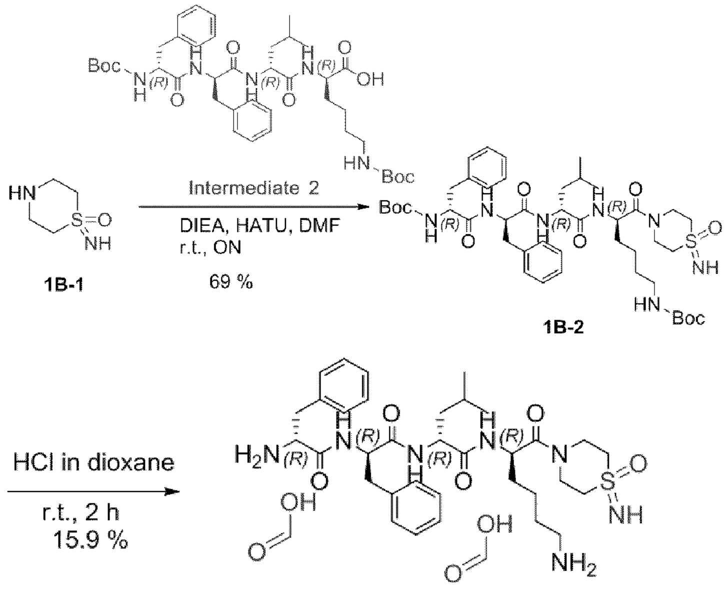


**1B**

1.1 Synthesis of compound 1B-2

Intermediate 2 (750 mg, 0.995 mmol, 1.00 equiv) and DIEA (192.85 mg, 1.492 mmol, 1.5 equiv) were dissolved in DMF (30 mL) at room temperature under nitrogen atmosphere, and HATU (567.37 mg, 1.492 mmol, 1.5 equiv) and compound 1B-1 (200 mg, 1.492 mmol, 1.5 equiv) were added portionwise. The resulting mixture was stirred at room temperature overnight under nitrogen atmosphere. The resulting crude product was purified by a reversed-phase column under the following conditions: C18 column, mobile phase: solvent ACN and solvent $H_2O$ (0.1% formic acid), gradient 10% to 50% for 10 min, UV200 nanometer detector. The resulting fractions were concentrated to dryness by rotary evaporation to give *tert*-butyl N-[(5R)-5-[(2R)-2-[(2R)-2-[(2R)-2-[(*tert*-butoxycarbonyl)amino]-3]-phenylpropionylamino]-3-phenylpropionylamino]-4-methylpentanoylamino]-6-(1-imino-1-oxo-1λ6-thiomorpholin-4-yl)-6-oxohexyl|carbamate (600 mg, 69.32%, purity: 99%) as a pale yellow solid.

LC-MS-1B-2 (ES, m/z): [M+1] = 870

1.2 Synthesis of compound 1B

*tert*-Butyl N-[(5R)-5-[(2R)-2-[(2R)-2-[(2R)-2-[(*tert*-butoxycarbonyl)amino]-3]-phenylpropionylamino]-3-phenylpropionylamino]-4-methylpentanoylamino]-6-(1-imino-1-oxo-1λ6-thiomorpholin-4-yl)-6-oxohexyl]carbamate (80 mg, 0.092 mmol, 1.00 equiv) and a 4 M solution of HCl in 1,4-dioxane (8 mL) were added to a 25 mL round-bottom flask. The resulting mixture was stirred at room temperature for 2 h. The resulting mixture was concentrated under reduced

pressure. The resulting crude product was purified by a reversed-phase column under the following conditions: C18 column, mobile phase: solvent ACN and solvent $H_2O$ (0.1% formic acid), gradient 10% to 50% for 10 min, detector: UV200 nm to give (R)-N-((R)-6-amino-1-(1-imino-1-oxo-1λ6-thiomorpholine)-1-oxohex-2-yl)-2-((R)-2-((R)-2-amino-3-phenylpropionylamino)-3-phenylpropionylamino)-4-methylpentanamide (10.2 mg, 15.90%, purity: 95.9%).

LC-MS-1B (ES, m/z): [M+1] = 670

$^1$H NMR (400 MHz, Deuterium Oxide) δ 8.37 (s, 2H)(HCOOH), 7.63 - 7.01 (m, 10H), 4.56 (t, J = 7.5 Hz, 1H), 4.37 - 3.87 (m, 5H), 3.85 - 3.67 (m, 1H), 3.52 (t, J = 12.4 Hz, 1H), 3.40 - 3.07 (m, 4H), 3.11 - 2.81 (m, 6H), 1.64 (tt, J = 15.1, 7.8 Hz, 4H), 1.52 - 1.17 (m, 5H), 0.85 (dd, J = 21.2, 5.0 Hz, 6H).

Example 2B. Synthesis of Compound 2B

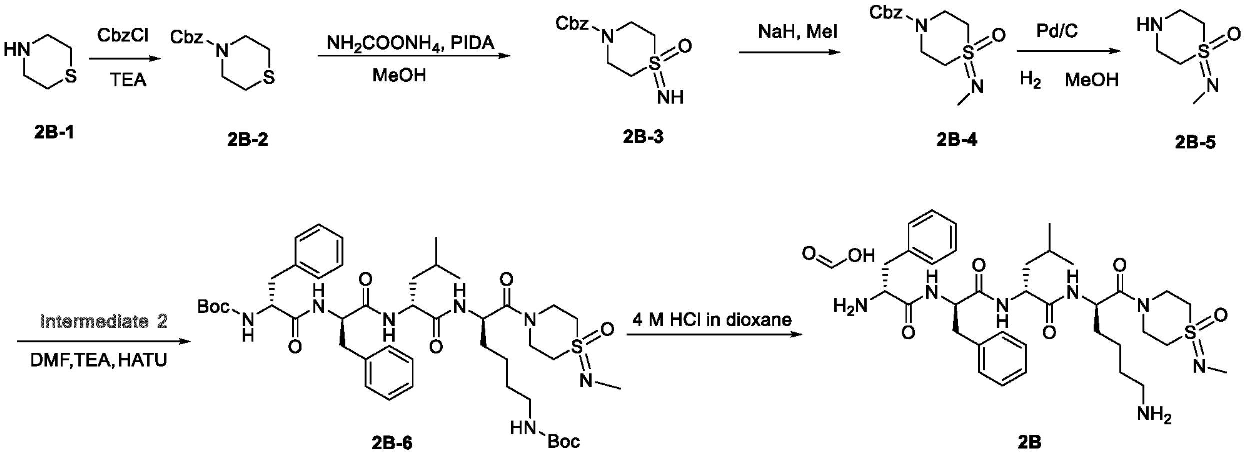


According to similar preparation procedures as in Example 1B, the target compound was synthesized, with the difference being that reactant compound

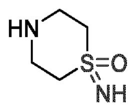

was replaced with

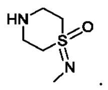.

LC-MS-2B (ES, m/z): [M+1] = 684.20

$^1$H NMR (400 MHz, Deuterium Oxide) δ 8.30 (d, *J* = 6.9 Hz, 1H), 7.51 - 7.05 (m, 10H), 4.62 (dt, *J* = 15.2, 7.6 Hz, 2H), 4.33 - 4.12 (m, 3H), 4.12 - 3.90 (m, 1H), 3.87 - 3.66 (m, 1H), 3.62 - 3.36 (m, 4H), 3.36 - 3.04 (m, 3H), 2.98 (dq, *J* = 24.6, 7.8 Hz, 4H), 2.73 (s, 3H), 1.92 - 1.60 (m, 4H), 1.57 - 1.21 (m, 5H), 0.87 (dd, *J* = 20.8, 50 Hz, 6H).

Example 3B. Synthesis of Compound 3B

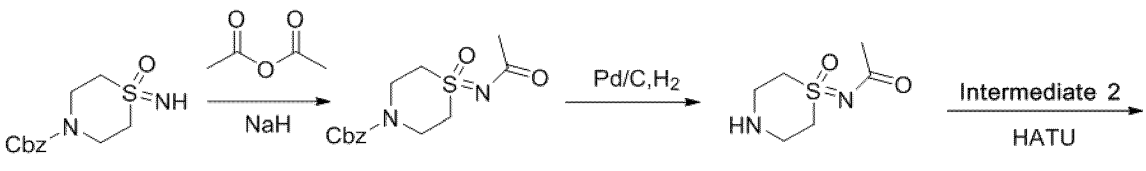


3B-1 3B-2 3B-3

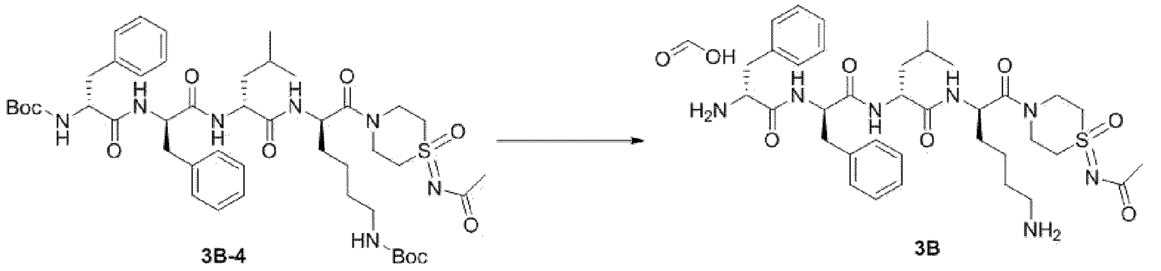

3B-4 3B

According to similar preparation procedures as in Example 1B, the target compound was synthesized, with the difference being that reactant compound

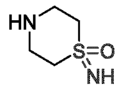

was replaced with

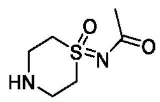

.

LC-MS-3B (ES, m/z): [M+1] = 712.20

$^{1}$H NMR (400 MHz, Deuterium Oxide) δ 8.37 (s, 1H), 7.43 - 7.01 (m, 10H), 4.63 - 4.50 (m, 1H), 4.43 (s, 1H), 4.33 - 3.98 (m, 4H), 3.98 - 3.26 (m, 6H), 3.12 - 2.70 (m, 6H), 2.18 - 1.91 (m, 3H), 1.74 - 1.27 (m, 9H), 0.98 - 0.72 (m, 6H).

Example 4B. Synthesis of Compound 4B

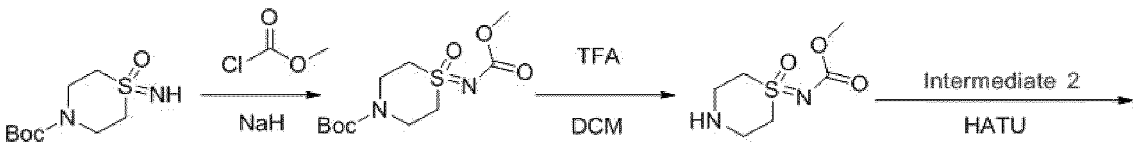


4B-1 4B-2 4B-3

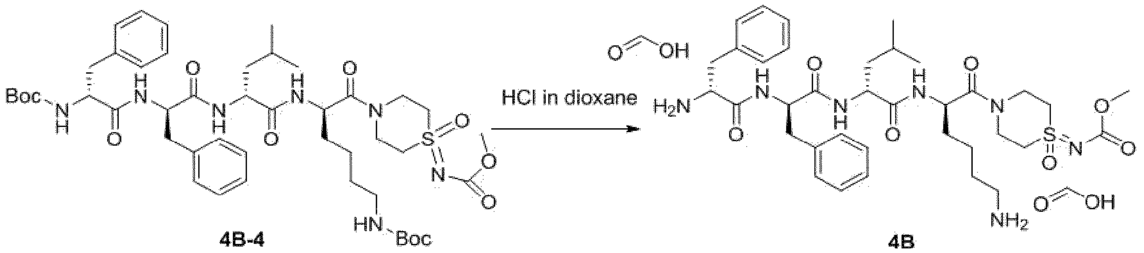


4B-4 4B

According to similar preparation procedures as in Example 1B, the target compound was synthesized, with the difference being that reactant compound

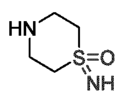

was replaced with

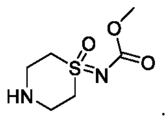

LC-MS-4B (ES, m/z): [M+1] = 728.10

$^{1}$H NMR (300 MHz, Deuterium Oxide) δ 8.34 (s, 2H), 7.38 - 7.07 (m, 10H), 4.63 - 4.04 (m, 5H), 3.96 (q, *J* = 6.5 Hz, 1H), 3.86 - 3.30 (m, 8H), 3.22 (s, 1H), 3.12 - 2.59 (m, 6H), 1.90 - 1.09 (m, 9H), 0.89 (dd, *J* = 13.9, 5.0 Hz, 6H).

The present specification illustrates the preparation of the compounds described above, and it can be understood by those skilled in the art that other compounds not illustrated in the present invention can also be prepared by reference to the general methods and specific examples described above.

Comparative Example 1B

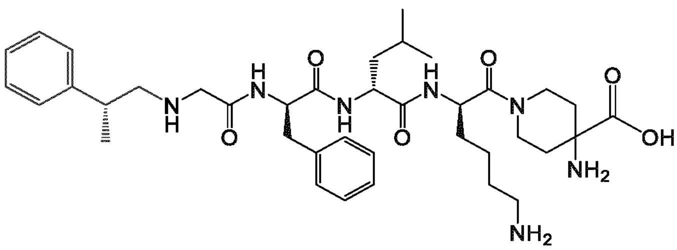

This compound was prepared by reference to the method in Example 5 of CN108290926A.

Biological Test Evaluation

The present invention is further described below using test examples, but these examples are not intended to limit the scope of the present invention.

The following CR845 in the present disclosure was prepared by reference to the method in Example 2 of CN 101627049 A.

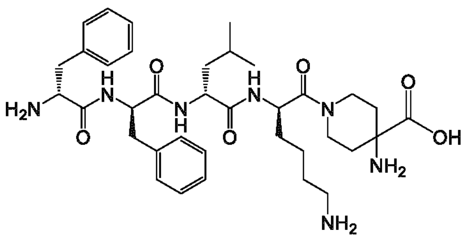

CR845

Test Example 1. *In Vitro* Receptor Binding Assay

**1.1 Objective**

The affinities of the compounds for μ, Kappa, and delta receptors were detected by using an *in vitro* isotope labeling method.

**1.2 Experimental materials**

Cell lines: CHO-K1-μ, CHO-K1-Kappa, and CHO-K1-delta stably transfected cell strains (Nanjing GenScript Biotech Co., Ltd.)

Table 1-Related information about labeled ligands and preparation

| Name of labeled ligands | | Source | Cat. No. | Batch No. | Suitable receptor | Storage condition |
|---|---|---|---|---|---|---|
| No. | Name | | | | | |
| 1 | $^3$H-DAMGO | PE | NET902250 | 2139100 | Mu OR | -20 °C |
| 2 | $^3$H-U69593 | PE | NET952250 | 2126416 | Kappa | -20 °C |
| 3 | $^3$H-DADLE | PE | NET648250 | 2060549 | Delta | -20 °C |
| Note: PE: PerkinElmer Inc.; | | | | | | |

Table 2-Related information about non-labeled compounds

| Name of non-labeled compounds | Molecular weight | Source | Cat. No. | Batch No. | Suitable receptor | Storage condition |
|---|---|---|---|---|---|---|
| DAMGO | 513.59 | Abcam | Ab120674 | Apn113800-1-1 | Mu OR | 2-8 °C |
| U69593 | 356.50 | Sigma | U103-25MG | 0000102364 | Kappa | 2-8 °C |
| DADLE | 569.66 | Abcam | Ab120673 | APN11299-1-3 | Delta | 2-8 °C |
| Note: 5-HT: 5-hydroxytryptamine. | | | | | | |

Table 3-Information about reagents and consumables

| **Name** | **Cat. No./batch No.** | **Manufacturer** |
|---|---|---|
| Absolute ethanol | 20150422 | Yonghua Technology Co., Ltd. |
| Tris | T1378-1KG/BCBQ2051V | SIGMA |
| EDTA | T20110308 | Sinopharm (Hushi) |
| Magnesium chloride | E010094/GF290086 | Sun Chemical Technology (Shanghai) Co., Ltd. |
| MICROSCINT PS scintillation solution | 6013631/94-19221 | Perkin Elmer |
| PEI | 306185-100G/07518TE | SIGMA |
| UniFilter-96 GF/C plate | 6005174 | Perkin Elmer |
| Note: Tris: tris(hydroxymethyl)aminomethane; EDTA: ethylenediaminetetraacetic acid; PEI: polyetherimide. | | |

Table 4-Instrument information

| **Name** | **Model** | **Manufacturer** |
|---|---|---|
| PH meter | PHS-3C | Shanghai REX Sensor Technology Co., Ltd. |
| Precision electronic balance | MS105DU | METTLER TOLEDO |
| Liquid scintillation instrument | Microbeta 2450 | Perkin Elmer Inc. |
| High-speed centrifuge | J26xpi | Beckman Coulter |
| High-speed dispersion machine | IKA-T10 | IKA |

### 1.3 Experimental methods

#### 1.3.1 Buffer preparation

A: (for the preparation of μ, Kappa, and delta receptor membranes): 11.7 mg of EDTA and 380.84 mg of $MgCl_2$ were weighed out, and 50 mM Tris-HCl buffer was added to reach a total volume of 400 mL, and the solution was adjusted to pH 7.4 to make final concentrations of 0.1 mM for EDTA and 10 mM for $MgCl_2$.

B: preparation of compound: the theoretical sample weighing amount was calculated according to the designed concentration and the required volume. $5.0 \times 10^{-3}$ M was used as an initial preparation dose, and the solution was dissolved in DMSO; the mixture was sequentially diluted with DMSO to $5.0 \times 10^{-4}$ M-$5.0 \times 10^{-9}$ M; the diluted DMSO solution was diluted with Buffer to a working concentration, $5.0 \times 10^{-5}$ M-$5.0 \times 10^{-11}$ M; and the final concentration of DMSO in the working solution was 1% (the final concentration of DMSO in the reaction system was 0.2%). The test sample was prepared and then stored at 4 °C. After the test was completed, the test sample was discarded.

**1.3.2 Preparation of receptor membranes**

CHO-μ, Kappa, delta, etc. cells were taken out of a refrigerator at -80 °C, thawed spontaneously, and centrifuged at 1000 g for 10 min at 4 °C. The precipitate was collected, and the supernatant was discarded. Buffer was added to the precipitate, and the mixture was homogenized for 20-30 s and then centrifuged at 50000 g for 15 min at 4 °C. The supernatant was carefully discarded, and Buffer was added into the precipitate again. The cells were mixed well, and the mixture was centrifuged at 50000 g for 15 min at 4 °C. The procedures were repeated three times. The sample was stored at -80 °C.

**1.3.3 Competitive receptor binding assay**

3.3.1 μ receptor affinity assay

Step 1: 50 μL of a vehicle (1% DMSO) was added into a total binding tube (TB), 50 μL of DAMGO (with a final concentration of $1.0 \times 10^{-5}$ M) was added into a non-specific binding tube (NB), and 50 μL of a test compound was added into each compound binding tube (CB).
Step 2: 100 μL of a buffer (homogenate A) was added into each reaction tube.
Step 3: the prepared membrane was suspended in the homogenate A to obtain a 10 mg/mL membrane suspension for later use.
Step 4: 50 μL of radioligand [$^3$H] DAMGO was added into each reaction tube to reach a final concentration of 2 nM.
Step 5: 50 μL of the membrane solution was added into each reaction tube.
Step 6: each reaction tube was incubated at 25 °C for 90 min. After the reaction was completed, the binding ligands were rapidly filtered under reduced pressure. The UniFilter GF/C plate was saturated with 0.5% PEI solution 1 h in advance, fully washed with cold Tris buffer, filtered under vacuum, then placed into a thermostatic dryer, and dried for 30 min. The filter plate was taken out, and MICROSCINT PS scintillation solution was added at 40 μL/well.
Step 7: the scintillation vial was put into a liquid scintillation counter for counting.

3.3.2 Competitive Kappa receptor binding assay

Step 1: 50 μL of a vehicle (1% DMSO) was added into a total binding tube (TB), 50 μL of U69593 (with a final concentration of $1.0 \times 10^{-5}$ M) was added into a non-specific binding tube (NB), and 50 μL of a test compound was added into each compound binding tube (CB).
Step 2: 100 μL of a buffer (homogenate A) was added into each reaction tube.
Step 3: the prepared membrane was suspended in the homogenate A to obtain a 15 mg/mL membrane suspension for later use.
Step 4: 50 μL of radioligand $^3$H-U69593 was added into each reaction tube to reach a final concentration of 2 nM.
Step 5: 50 μL of the membrane solution was added into each reaction tube.
Step 6: each reaction tube was incubated at 25 °C for 90 min. After the reaction was completed, the binding ligands were rapidly filtered under reduced pressure. The UniFilter GF/C plate was saturated with 0.5% PEI solution 1 h in advance, fully washed with cold Tris buffer, filtered under vacuum, then placed into a thermostatic dryer, and dried for 30 min. The filter plate was taken out, and MICROSCINT PS scintillation solution was added at 40 μL/well.
Step 7: the filter plate was put into a liquid scintillation counter for counting.

3.3.3 Competitive Delta receptor binding assay

Step 1: 50 μL of a vehicle (1% DMSO) was added into a total binding tube (TB), 50 μL of DADLE (with a final concentration of $1.0 \times 10^{-5}$ M) was added into a non-specific binding tube (NB), and 50 μL of a test compound was added into each compound binding tube (CB).
Step 2: 100 μL of a buffer (homogenate A) was added into each reaction tube.
Step 3: the prepared membrane was suspended in the homogenate A to obtain a 10 mg/mL membrane

suspension for later use.

Step 4: 50 μL of radioligand $^3$H-DADLE was added into each reaction tube to reach a final concentration of 4 nM.

Step 5: 50 μL of the membrane solution was added into each reaction tube.

Step 6: each reaction tube was incubated at 25 °C for 90 min. After the reaction was completed, the binding ligands were rapidly filtered under reduced pressure. The UniFilter GF/C plate was saturated with 0.5% PEI solution 1 h in advance, fully washed with cold Tris buffer, filtered under vacuum, then placed into a thermostatic dryer, and dried for 30 min. The filter plate was taken out, and MICROSCINT PS scintillation solution was added at 40 μL/well.

Step 7: the filter plate was put into a liquid scintillation counter for counting.

### 1.4 Data analysis

Based on the effect values of different concentration test points of the compound samples, the GraphPad Prism software was used to fit the action curve of the compound samples to the receptors, and the Ki values were calculated. The results are shown in Table 5.

Table 5-Receptor binding Ki values

| Compound | κ receptor Ki (nM) | μ receptor Ki (nM) | δ receptor Ki (nM) |
|---|---|---|---|
| 2-0 | 1.15 | >1000 | >1000 |
| 2-100 | 32.51 | >1000 | >1000 |
| 3 | 3.31 | >1000 | >1000 |
| 4-0 | 2.80 | >1000 | >1000 |
| 4-100 | 59.93 | >1000 | >1000 |
| 8 | 0.09 | >1000 | >1000 |
| 9 | 11.15 | >1000 | >1000 |
| 10 | 0.86 | >1000 | >1000 |
| 11 | 0.94 | >1000 | >1000 |
| 12 | 0.53 | >1000 | >1000 |
| 13 | 0.13 | >1000 | >1000 |
| 13-2 | 0.13 | >1000 | >1000 |
| 14 | 1.10 | >1000 | >1000 |
| 15 | 0.83 | >1000 | >1000 |
| 16 | 0.26 | >1000 | >1000 |
| 17 | 0.44 | >1000 | >1000 |
| 18 | 0.21 | >1000 | >1000 |
| 19 | 0.90 | >1000 | >1000 |
| 20 | 0.58 | >1000 | >1000 |
| 21 | 0.33 | >1000 | >1000 |
| 22 | 0.35 | >1000 | >1000 |
| 23 | 0.36 | >1000 | >1000 |
| 24 | 0.36 | >1000 | >1000 |
| 25 | 0.45 | >1000 | >1000 |
| 26 | 0.31 | >1000 | >1000 |
| 27 | 0.88 | >1000 | >1000 |
| 28 | 3.31 | >1000 | >1000 |
| 29 | 0.63 | >1000 | >1000 |
| 30 | 1.61 | >1000 | >1000 |

(continued)

| Compound | κ receptor Ki (nM) | μ receptor Ki (nM) | δ receptor Ki (nM) |
|---|---|---|---|
| 31 | 3.49 | >1000 | >1000 |
| 32 | 0.58 | >1000 | >1000 |
| 33 | 0.56 | >1000 | >1000 |
| 34 | 0.60 | >1000 | >1000 |
| 35 | 0.18 | >1000 | >1000 |
| 36 | 0.98 | >1000 | >1000 |
| 37 | 0.41 | >1000 | >1000 |
| 38 | 0.17 | >1000 | >1000 |
| 39 | 0.27 | >1000 | >1000 |
| 40 | 0.58 | >1000 | >1000 |
| 41 | 0.57 | >1000 | >1000 |
| 42 | 0.47 | >1000 | >1000 |
| 43 | 2.00 | >1000 | >1000 |
| 44 | 0.55 | >1000 | >1000 |
| 45 | 0.52 | >1000 | >1000 |
| 46 | 0.29 | >1000 | >1000 |
| 47 | 0.49 | >1000 | >1000 |
| 48 | 3.79 | >1000 | >1000 |
| 49 | 0.93 | >1000 | >1000 |
| 50 | 0.27 | >1000 | >1000 |
| 51 | 0.35 | >1000 | >1000 |
| 55 | 10.63 | >1000 | >1000 |
| 69 | 14.37 | >1000 | >1000 |
| 1A | 0.40 | >1000 | >1000 |
| 2A | 0.34 | >1000 | >1000 |
| 3A | 0.26 | >1000 | >1000 |
| 4A | 0.30 | >1000 | >1000 |
| 5A | 0.34 | >1000 | >1000 |
| 6A | 0.43 | >1000 | >1000 |
| 7A | 0.60 | >1000 | >1000 |
| 8A | 0.31 | >1000 | >1000 |
| 9A | 0.37 | >1000 | >1000 |
| 10A | 0.48 | >1000 | >1000 |
| 11A | 0.48 | >1000 | >1000 |
| 12A | 0.34 | >1000 | >1000 |
| 1B | 0.42 | >1000 | >1000 |
| 2B | 0.91 | >1000 | >1000 |
| 3B | 1.05 | >1000 | >1000 |
| 4B | 1.88 | >1000 | >1000 |

It can be seen that the compounds of the present invention have excellent selectivity for κ-opioid receptors and high affinity for the κ-opioid receptors.

Test Example 2. *In Vitro* Functional Assay

## 2.1 Objective

Using the Cisbio HTRF cAMP-Gi kit, changes in the cAMP concentration of the κ-opioid receptor (Kappa) signaling pathway were detected with a microplate reader, and $EC_{50}$ values of the compounds were calculated to evaluate agonistic effects of the compounds on the κ-opioid receptors.

## 2.2 Experimental materials

Cell line: CHO-K1-OPRK1 stably transfected cell strain (Nanjing GenScript Biotech Co., Ltd.)
Cell culture conditions: F12, 10% FBS, 200 μg/mL Zeocin, 100 μg/mL Hygromycin B
Reagents and consumables:

F12 (meilunbio, MA0229)
FBS (BOVOGEN, SFBS)
Zeocin (invitrogen, R25001)
Hygromycin B (invitrogen, 10687010)
PBS (meilunbio, MA0015)
Pancreatin (Gibco, 25200-072)
96 cell plate (cisbio, 66PL96025)
cAMP-Gi kit (cisbio, 62AM9PEB)
$CO_2$ incubator (Thermo, 311)
Centrifuge (Shanghai Anting Scientific Instrument Factory, TGL-16C)
Cell counter (Countstar, IC1000)
Microplate reader (PerkinElmer, EnVision)

## 2.3 Experimental methods

(1) A reaction buffer (1× Stimulation buffer) required for the experiment was prepared as follows: 5× Stimulation buffer and $ddH_2O$ in the Cisbio cAMP-Gi kit were diluted according to a ratio of 1:4 for later use.
(2) Preparation of compound: the compound was diluted with DMSO to obtain a 5 mM stock solution, followed by a 3.16-fold dilution to obtain 10 gradients, and then the prepared compound was diluted with the Stimulation buffer to the corresponding concentrations (2.5×) for later use.
(3) Cell preparation: CHO-K1-OPRK1 cells on the culture dish were digested with the pancreatin, and the cells were eluted with the medium and collected into a 5 mL centrifuge tube. The cells were centrifuged at 1000 rpm for 5 min, and the supernatant was discarded. 3 mL of PBS was added, and the resulting mixture was gently pipetted and mixed well using a pipette. The cells were centrifuged again at 1000 rpm for 5 min, and the supernatant was discarded. The cells were resuspended in 1× Stimulation buffer and counted using a Countstar cell counter, and the cell density was adjusted to $6 \times 10^5$ cells/mL for later use.
(4) Cell addition: the cell suspension was added to the assay plate at 5 μL/well (i.e., about 3000 cells/well).
(5) Compound addition: the compound diluted with the Stimulation buffer was added to the assay plate described above at 4 μL/well.
(6) Reaction and incubation: after gentle shaking, the assay plate was incubated at 37 °C for 20 min.
(7) Agonist Forskolin addition: a 10× adenylate cyclase agonist (with a final concentration of 1 μM Forskolin) solution was added at 1 μL/well.
(8) Reaction and incubation: after gentle shaking, the assay plate was incubated at 37 °C for 45 min.
(9) Detection reagent addition: cAMP-cryptate and Anti-cAMP-d2 were separately diluted according to a ratio of 1:20 with Lysis & detection buffer in the Cisbio cAMP-Gi detection kit, and the diluted cAMP-cryptate and Anti-cAMP-d2 were separately added to the assay plate at 5 μL/well. After shaking, the assay plate was left to stand at room temperature for 60 min.

(10) Experimental readings: the plate was read on Envision, readings from 665 nm and 615 nm channels were measured, and the ratio of 665 nm/615 nm readings was calculated.

**2.4. Data analysis**

Based on the agonistic effect values of different concentration test points of the compound samples, the GraphPad Prism software was used to fit the agonistic effect curve of the compound samples to the κ-opioid receptors, and the $EC_{50}$ values were calculated. The experimental results are shown in Table 6.

Table 6-*In vitro* agonistic effects on κ-opioid receptors

| Compound | $EC_{50}$ (nM) |
|---|---|
| 4-0 | 3.62 |
| 8 | 0.90 |
| 10 | 1.1 |
| 11 | 5.1 |
| 12 | 0.17 |
| 13 | 0.52 |
| 13-2 | 0.13 |
| 14 | 8.20 |
| 15 | 0.63 |
| 16 | 0.079 |
| 17 | 0.30 |
| 18 | 0.18 |
| 19 | 0.87 |
| 20 | 0.52 |
| 21 | 0.68 |
| 22 | 0.36 |
| 23 | 0.34 |
| 24 | 0.27 |
| 25 | 0.46 |
| 26 | 0.25 |
| 27 | 0.87 |
| 29 | 0.058 |
| 31 | 1.23 |
| 32 | 0.65 |
| 33 | 0.81 |
| 34 | 0.71 |
| 35 | 0.17 |
| 36 | 0.35 |
| 37 | 0.39 |
| 38 | 0.54 |
| 39 | 0.35 |
| 40 | 0.13 |
| 41 | 0.57 |

(continued)

| Compound | $EC_{50}$ (nM) |
|---|---|
| 42 | 0.46 |
| 43 | 6.13 |
| 44 | 0.09 |
| 45 | 0.16 |
| 46 | 0.22 |
| 47 | 0.24 |
| 48 | 12.01 |
| 49 | 3.06 |
| 50 | 0.43 |
| 1A | 0.05 |
| 2A | 0.36 |
| 3A | 0.40 |
| 4A | 0.20 |
| 5A | 0.30 |
| 6A | 0.95 |
| 7A | <0.1 |
| 8A | 0.075 |
| 9A | <0.1 |
| 10A | 0.59 |
| 11A | 0.21 |
| 12A | 0.07 |
| 1B | 0.52 |
| 2B | 0.30 |
| 3B | 1.00 |
| 4B | 0.65 |

It can be seen that the compounds of the present invention have relatively strong agonistic effects on the κ-opioid receptors.

Test Example 3. Sedation Safety Window Assay

### 3.1 Acetic acid writhing test in mice

#### 3.1.1 Objective

The analgesic effects of the compounds after single administration were investigated by an acetic acid writhing test in mice, and the $ED_{50}$ values were calculated.

#### 3.1.2 Test system

##### 3.1.2.1 Experimental animal information

Species and strain: ICR mice
Grade: normal

Number and sex: male, 100 mice (as the case may be)
Age range: 6-8 weeks old
Weight range: 23-32 g
Supplier: SPF (Beijing) Biotechnology Co., Ltd.
Animal use license: SYXK (Jiangsu) 2019-053
Treatment of the remaining animals: the remaining animals were handed over to the department of the experimental system for treatment.

**3.1.2.2 Instruments and reagents**

Table 7-Main instruments and reagents

| Name | Model | Manufacturer |
|---|---|---|
| Precision electronic balance | BS224s | Sartorius Scientific Instruments (Beijing) Co., Ltd. |
| Electronic balance | YP6001N | Shanghai Precision Instrument Co., Ltd. |
| Hot/cold plate | 35100 | UGO Basile, Italy |
| Acetic acid | 20160408 | Shanghai Lingfeng Chemical Reagents Co., Ltd. |

**3.1.3 Test method:**

The acetic acid writhing test in mice is a classic visceral pain test model. The peritoneum is stimulated to cause a lasting pain response by intraperitoneal injection of a 0.6% aqueous acetic acid solution in the mice, further causing a writhing reaction of the mice. The analgesic drugs can inhibit the writhing reaction of the mice. The mice were adapted for 15 min after intravenous injection of the drug at the tail, then a 0.6% acetic acid solution (0.1 mL/10 g) was intraperitoneally injected. A vehicle control group (0.9% sodium chloride injection) was set. The times of writhing of the mice within 15 min after the injection of acetic acid were observed and recorded, and the writhing inhibition rate of the test compound was calculated to preliminarily evaluate the analgesic effect of single administration.

Writhing inhibition rate% = (times of vehicle group - times of administration group) × 100%/times of vehicle group.

**3.1.4 Data processing**

The experimental data were expressed as mean±standard deviation (Mean±SD). The one-way ANOVA was carried out by using the GraphPad Prism 8 statistical software, pairwise comparisons were carried out by using the Dunnett t test, and $ED_{50}$ values were calculated using a non-linear fitting method. The differences were considered statistically significant when $P < 0.05$.

**3.2 Autonomous activity test in mice**

**3.2.1 Objective**

The effects of the compounds on the autonomic activity of the mice after single administration were determined, and $ED_{50}$ values were calculated.

**3.2.2 Test system**

3.2.2.1 Experimental animal information

Species and strain: ICR mice
Grade: normal
Number and sex: male, 200 mice (as the case may be)
Age range: 6-8 weeks old

Weight range: 23-35 g
Supplier: SPF (Beijing) Biotechnology Co., Ltd.
Animal use license: SYXK (Jiangsu) 2019-053
Treatment of the remaining animals: the remaining animals were handed over to the department of the experimental system for treatment.

### 3.2.2.2 Instruments and reagents

Table 8-Main instruments and reagents

| Name | Model | Manufacturer |
|---|---|---|
| Precision electronic balance | BS224s | Sartorius Scientific Instruments (Beijing) Co., Ltd. |
| Electronic balance | YP6001N | Shanghai Precision Instrument Co., Ltd. |
| Dahua L series hard disk drive video recorder | DH/DVR1604LE-L | Zhejiang Dahua Technology Co., Ltd. |
| TopScan Version 3.00 (animal video analysis system) | Version 3.00 | Clever Sys, Inc. |

### 3.2.3. Test method

The autonomous activity test in mice is a classical test model for evaluating the inhibition of compounds on the central nervous system. The mice were intravenously injected with a test sample solution with a corresponding concentration (freshly prepared before use) at the tail, and the mice in the blank group were given a vehicle (0.9% sodium chloride injection). After the administration, the mice were immediately placed into an autonomous activity test box (a black polyethylene box with the specification of 29 cm × 29 cm × 30 cm) to be recorded for 30 min. Video analysis was carried out after the recording was completed, and the autonomous activity of the mice after the administration was evaluated.

### 3.2.4 Data processing

The experimental data were expressed as mean±standard deviation (Mean±SD). The one-way ANOVA was carried out by using the GraphPad Prism 8 statistical software, pairwise comparisons were carried out by using the Dunnett t test, and $ED_{50}$ values were calculated using a non-linear fitting method. The differences were considered statistically significant when $P < 0.05$.

### 3.3 Safety window

Sedation is the most significant side effect of peripheral Kappa receptor agonists, and the sedation safety window can be obtained according to the sedation side effect $ED_{50}$ (autonomic activity test)/efficacy $ED_{50}$ (acetic acid writhing test), with a larger safety window indicating higher safety and a lower probability of the clinical sedation side effect at the same dose. The experimental results are shown in Table 9.

Table 9-Safety window

| Compound | Acetic acid writhing in mice $ED_{50}$ (mg/kg) | Autonomous activity in mice $ED_{50}$ (mg/kg) | Safety window |
|---|---|---|---|
| 4-0 | 1.14 | 7.34 | 6.44 |
| 11 | 1.82 | 9.60 | 5.27 |
| 13 | 0.23 | 2.83 | 12.30 |
| 13-2 | 0.17 | 2.75 | 16.18 |
| 16 | 0.27 | 1.37 | 5.13 |
| 21 | 0.50 | 1.42 | 2.82 |

(continued)

| Compound | Acetic acid writhing in mice $ED_{50}$ (mg/kg) | Autonomous activity in mice $ED_{50}$ (mg/kg) | Safety window |
|---|---|---|---|
| 27 | 0.88 | 4.59 | 5.22 |
| 29 | 0.41 | 1.35 | 3.28 |
| 1A | 0.38 | 1.08 | 2.84 |
| 3A | 0.45 | 1.41 | 3.15 |
| 4A | 0.41 | 1.25 | 3.05 |
| 8A | 0.16 | 5.93 | 37.06 |
| 10A | 1.61 | 5.52 | 3.43 |
| 1B | 0.20 | 0.50 | 2.50 |
| 2B | 0.31 | 3.33 | 10.74 |
| 3B | 0.42 | 3.25 | 7.74 |
| Comparative Example 1B | 0.25 | 0.59 | 2.36 |

It can be seen that the compounds of the present invention have a very large safety window, i.e., have a greater safe dose range of administration, or are less likely to have side effects at the same dose.

**Test Example 4. PK and Blood-Brain Barrier Permeability in Mice**

**4.1 Objective**

The compounds of the present disclosure were investigated for pharmacokinetic characteristics and blood-brain barrier permeability in mice.

**4.2 Test devices**

Table 10-Device information

| **Name** | **Model** | **Manufacturer** |
|---|---|---|
| AB triple quadrupole liquid chromatography-mass spectrometry instrument | 5500+ | Sciex |
| Mettler-Toledo analytical balance | XSE105 | Mettler-Toledo Group |
| Benchtop high-speed refrigerated centrifuge | TL-18M | Shanghai Centrifuge Institute Co., Ltd. |
| Eppendorf micropipette | 20/200/1000 μL | Eppendorf, Germany |
| Digital display constant-temperature oscillator | SHA-B(A) | Jinyi Instrument Technology Company Limited |
| Benchtop ultrasonic cleaner | CPX8800H | Emerson, USA |
| Vortex oscillator | Vortex genie 2 | Scientific Industries, USA |
| Acetonitrile | Chromatography grade | Tedia, USA |
| Methanol | Chromatography grade | Tedia, USA |
| Watsons purified water | 600 mL | Watsons Group |
| Formic acid | 095324 | MREDA, USA |

**4.3 Test method and data processing**

Healthy male ICR mice were randomly grouped, 6 animals per group. For each test compound, 3 groups of animals were used. The first group of animals was used for collecting blood and brain 5 min after the administration, and the second group of animals and the third group of animals were used for cross blood collection at subsequent time points; each test compound was intravenously administered (1 mg/kg) to corresponding groups of mice, and blood sampling via submaxillary veins of the mice was carried out at different time points. Drug contents in plasma and brain tissues at different time points were measured by an LC-MS/MS method, the brain-to-blood ratio B/P was calculated by using EXCEL software, and pharmacokinetic parameters were calculated by using DAS3.0 software. The experimental results are shown in Table 11.

Table 11-Pharmacokinetic parameters

| Compound | Cmax (ng/mL) | AUC(0-∞) (μg/L*h) | Clearance (L/h/kg) | Vd (L/kg) | t1/2 (h) | B/P |
|---|---|---|---|---|---|---|
| 10 | 3224.52 | 4010.04 | 0.25 | 0.29 | 0.82 | 0.007 |
| 12 | 2264.57 | 3681.68 | 0.28 | 0.54 | 1.42 | 0.008 |
| 13 | 2538.43 | 1351.65 | 0.74 | 0.38 | 0.35 | 0.015 |
| 13-2 | 2929.28 | 1685.68 | 0.60 | 0.63 | 0.72 | BQL** |
| 16 | 4729.52 | 2503.55 | 0.40 | 0.51 | 0.87 | 0.010 |
| 21 | 3461.88 | 2667.60 | 0.38 | 0.63 | 1.15 | 0.010 |
| 27 | 2316.41 | 1163.46 | 0.88 | 0.63 | 0.50 | 0.010 |
| 29 | 4707.93 | 3343.19 | 0.31 | 0.32 | 0.74 | 0.010 |
| CR845 | 1621.13 | 652.67 | 1.62 | 0.60 | 0.26 | 0.015 |
| **: below the lower limit of quantitation, indicating that the test substance was undetectable in the brain tissue. | | | | | | |

It can be seen that the compounds of the present invention have excellent pharmacokinetic properties. Particularly, compared to CR845, the compounds of the present invention have comparable or lower brain-to-blood ratios B/P, which can selectively act on the peripheral Kappa receptors, and the compounds of the present invention have significantly improved pharmacokinetic characteristics and significantly longer half-lives.

Test Example 5. Toxicity Test of Single Administration in Mice

**5.1 Objective**

Safety data were supplemented by researching the toxic responses of the compounds of the present invention after a single intravenous administration in mice.

**5.2 Test system**

Species and strain: ICR mice
Grade: SPF grade
Sex: male
Weight range: 24-28 g
Supplier: SPF (Beijing) Biotechnology Co., Ltd.
Animal production license: SCXK (Beijing) 2019-0010
Animal use license: SYXK (Jiangsu) 2019-0053
Treatment of the remaining animals: the remaining animals were handed over to the department of the test system for treatment.

**5.3 Instruments and reagents**

Table 12-Main instruments and reagents

| Name | Model | Manufacturer |
|---|---|---|
| Precision electronic balance | BS224s | Sartorius Scientific Instruments (Beijing) Co., Ltd. |
| Electronic balance | YP6001N | Shanghai Precision Instrument Co., Ltd. |
| Sodium chloride injection | 2204141334 | Shandong Cisen Pharmaceutical Co., Ltd. |

### 5.4 Test method

The ICR mice were randomly grouped, 10 mice per group. The mice were intravenously administrated via the tail at 10 mL/kg. The animals were observed for state and mortality and recorded. The administration was carried out sequentially according to the recommended dose of AOT425 software until the $LD_{50}$ values were determined.

### 5.5 Data processing

The $LD_{50}$ values were calculated using the AOT425 software. The results are shown in Table 13.

Table 13-Acute toxicity

| Example No. | $LD_{50}$ (mg/kg) |
|---|---|
| 8A | 175 |
| 2B | 151.6 |
| 3B | 150 |

It can be seen that the compounds of the present invention have relatively low single administration toxicity, and thus have improved safety.

## Test Example 6. Research on Effect of Compounds on hERG Potassium Ion Channel

### 6.1 Objective

In this experiment, the inhibitory effects of the compounds on the hERG channel were investigated by using the manual patch-clamp technique.

### 6.2 Test system

Non-cardiac drugs may cause prolongation of myocardial action potential duration by inhibiting hERG (IKr) channels, thereby increasing the likelihood of life-threatening torsades de pointes (TdP) ventricular arrhythmias. In this experiment, an HEK293 cell line without endogenous IKr current was used as a host cell. The cell line is widely applied to the detection of hERG.

HEK293 cells stably expressing the hERG channel were cultured in a 35 mm culture dish and placed in a 37 °C C/5% $CO_2$ incubator for at least 24 h for later use. The hERG cell line was routinely cultured and passaged in DMEM containing 10% fetal bovine serum and 250 μg/mL of G418.

### 6.3 Liquid preparation

The extracellular fluid used in the whole-cell patch-clamp experiment was composed of (mM): NaCl 145; $MgCl_2$ 1; KCl 4; Glucose 10; HEPES 10; $CaCl_2$ 2; the pH value was adjusted to 7.4 with NaOH, and osmotic pressure value was adjusted to 300 mOsm with sucrose.

The intracellular fluid was composed of (mM): KCl 140; $MgCl_2$ 1; EGTA 5; HEPES 10 and $Na_2ATP$ 4; the pH value was adjusted to 7.2 with KOH, and osmotic pressure value was adjusted to 290 mOsm with sucrose.

Table 14-Main reagent concentrations

| Reagent | Extracellular fluid (mM) | Intracellular fluid (mM) |
|---|---|---|
| $CaCl_2$ | 2 | - |
| $MgCl_2$ | 1 | 1 |
| KCl | 4 | 140 |
| NaCl | 145 | - |
| Glucose | 10 | - |
| HEPES | 10 | 10 |
| EGTA | - | 5 |
| Na2ATP | - | 4 |
| pH | 7.4 | 7.2 |

**6.4 Test method**

Electrophysiological recording: one culture dish was taken out for each experiment, washed twice with the extracellular fluid and placed on the stage of an inverted microscope. The whole-cell patch-clamp experiment was carried out at room temperature, and the tip resistance of the borosilicate glass microelectrode was 3-5 MΩ.

Voltage stimulation protocol and current recording: after the whole-cell recording mode, the membrane potential was clamped at -80 mV, the cells were given a depolarization voltage stimulation of +50 mV every 30 s for 2 s, and repolarization was carried out to -50 mV for 3 s, causing an hERG tail current. Before the depolarization voltage stimulation, the cells were given a repolarization voltage of -50 mV for 50 ms, and the current recorded at this voltage served as the baseline for calculation of the hERG tail current. Only cells that met the recording criteria could be used for detection of the test compounds. Before the addition of the compounds, the hERG tail current in the extracellular fluid was stably recorded for at least 3 min. After perfusion administration, when the hERG tail current amplitude change was less than 5%, it was considered that the drug action reached a steady state. If the current did not reach steady state within 6 min, the compound detection at this concentration was terminated.

**6.5 Data processing**

The raw data were recorded by using Clampex 10.2, and data acquisition and analysis were carried out by using the pCLAMP 10.1 software program. 4-5 sweeps with the current in a steady state before the addition of the compounds were selected, and the average value of peak values was calculated to serve as a control current amplitude. 4-5 sweeps with the current in a steady state after the addition of the compounds were selected, and the average value of peak values was calculated to serve as the residual amplitude after the current was inhibited. The inhibition rate of the test compound on the hERG current was calculated according to the following equation:

$$\%\ \text{inhibition rate} = \{1 - (\text{current residual amplitude})/(\text{control current amplitude})\} \times 100$$

The inhibition rate of the test compounds on the hERG current at a concentration of 10 μM was obtained according to the calculation method described above. The results are shown in Table 15.

Table 15-Cardiotoxicity

| Example No. | % inhibition rate |
|---|---|
| 4 | 0.54% ± 1.72% |
| 12 | 2.13% ± 2.96% |
| 13 | 0.80% ± 0.77% |
| 1A | -0.94% ± 0.76% |
| 2A | 1.00% ± 1.86% |
| 4A | 0.90% ± 3.50% |

(continued)

| Example No. | % inhibition rate |
|---|---|
| 5A | 4.15% ± 0.07% |
| 6A | 1.04% ± 0.53% |
| 1B | 1.20% ± 0.39% |
| 3B | 1.13% ± 0.00% |

It can be seen that the compounds of the present invention have relatively low cardiotoxicity, and thus have improved safety.

The above examples merely illustrate the activity data of certain representative compounds of the present invention, and other compounds provided by the present invention also have similar effects when tested by the same methods.

## Claims

**1.** A compound of formula IA, a stereoisomer thereof, or a pharmaceutically acceptable salt thereof:

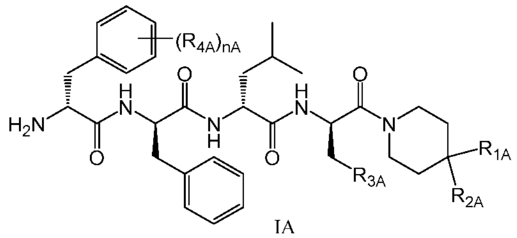

IA

wherein,

$R_{1A}$ is H or -COOH, preferably H;

$R_{2A}$ is $-NR_{aA}C(=O)OR_{bA}$ or $-NR_{aA}S(=O)_2OR_{bA}$, preferably $-NR_{aA}C(=O)OR_{bA}$, wherein $R_{aA}$ is H or $C_{1-6}$ alkyl substituted with one or more groups selected from amino, $C_{1-6}$ alkylamino, $C_{1-6}$ alkoxy, halogen, hydroxyl, nitro, cyano, $NH_2C(=O)$-, and $C_{1-6}$ alkoxy, preferably H or $C_{1-6}$ alkyl substituted with amino, $C_{1-6}$ alkylamino, or $C_{1-6}$ alkoxy, and more preferably H or $C_{1-6}$ alkyl substituted with amino or $C_{1-6}$ alkylamino; $R_{bA}$ is selected from $C_{1-6}$ alkyl, $C_{6-14}$ aryl, 5- to 14-membered heteroaryl, $C_{3-8}$ cycloalkyl, and 3- to 8-membered heterocyclyl, preferably from $C_{1-6}$ alkyl and 3- to 8-membered heterocyclyl, more preferably from $C_{1-6}$ alkyl and

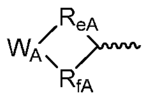

,

and even more preferably from $C_{1-6}$ alkyl and

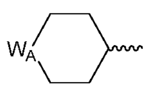

,

the group $R_{bA}$ described above is optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, wherein $R_{eA}$ and $R_{fA}$ are each independently $-(CH_2)_{n1A}-$ and $-(CH_2)_{n1A'}-$, wherein n1A and n1A' are each independently selected from 0, 1, 2, and 3, preferably 2, and n1A and n1A' are not both 0; $W_A$ is selected from -NH-C(=O)-, $-NH-S(=O)_2-$, $-NR_{5A}-$, -O-, -S-, and $-S(=O)_2-$, preferably from -O- and - $S(=O)_2$-, wherein $R_{5A}$ is selected from H, $C_{1-6}$ alkyl, amidino, and $HOOC-(CH_2)_{n3A}-$, wherein n3A is selected from 1, 2, and 3;

or $R_{1A}$ and $R_{2A}$, together with the carbon atom to which they are both attached form an optionally substituted 9- to 10-membered bicyclic moiety; preferably, the bicyclic moiety, together with the piperidine ring attached thereto,

forms a structure selected from:

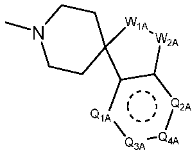

and

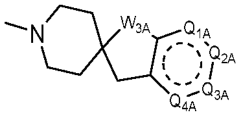 ,

when any one of $Q_{1A}$-$Q_{4A}$ is N, the rest are C, or $Q_{1A}$-$Q_{4A}$ are all C;
$W_{1A}$ and $W_{2A}$ are each independently -C(=O)-NH-, -NH-C(=O)-, $-S(=O)_2-NH-$, $-NH-S(=O)_2-$, -S-, -O-, $-NR_{6A}-$, $-NR_{6A}-CH_2-$, $-(CH_2)_{n2A}-$ optionally substituted with one or more groups selected from $-NH_2$, -OH, halogen, nitro, cyano, $C_{1-6}$ haloalkyl, $NH_2C(=O)-$, and $C_{1-6}$ alkoxy, or absent, wherein $R_{6A}$ is selected from H, $C_{1-6}$ alkyl, amidino, and $HOOC-(CH_2)_{n3A}-$; preferably, $W_{1A}$ and $W_{2A}$ cannot be simultaneously absent;
$W_{3A}$ is $-(CH_2)_{n2A}-$ optionally substituted with one or more groups selected from $NH_2$, -OH, halogen, nitro, cyano, $C_{1-6}$ haloalkyl, $NH_2C(=O)-$, and $C_{1-6}$ alkoxy;
n2A is selected from 0, 1, 2, and 3;
more preferably, the bicyclic moiety, together with the piperidine ring attached thereto, forms a structure selected from:

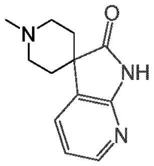 , 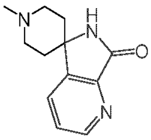 , 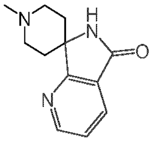 , 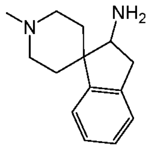 , 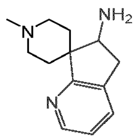 , 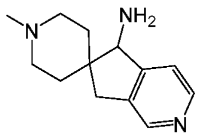 ,

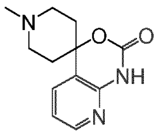 , 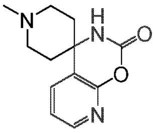 , 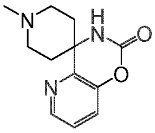 , 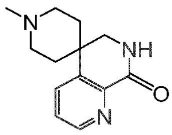 , 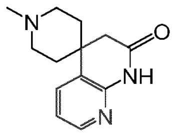 , 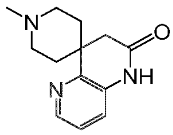 ,

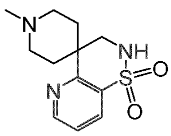 , 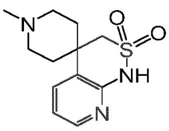 , 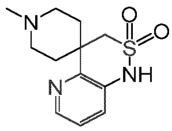 , 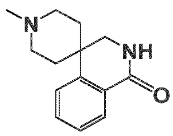 , 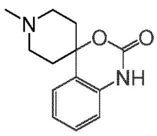 , 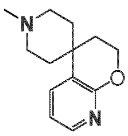 ,

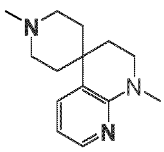 , 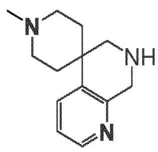 , and 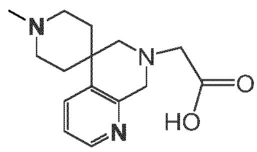 ,

more preferably from

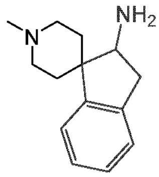
,
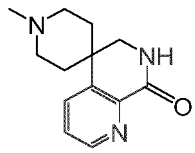
,

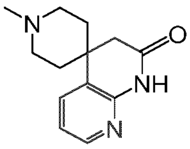
,
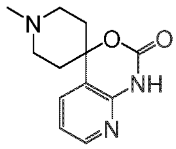
,
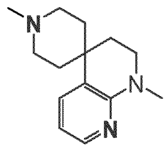
,

and

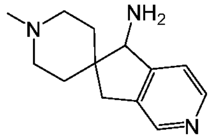
,

and even more preferably from

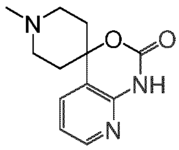

and

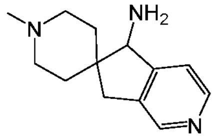
;

$R_{3A}$ is selected from H or $-(CH_2)_{mA}NR_{cA}R_{dA}$;
$R_{cA}$ and $R_{dA}$ are each independently selected from H, $C_{1-6}$ alkyl, amidino, and $C_{1-6}$ alkoxycarbonyl;
$R_{4A}$ is selected from halogen, $NO_2$, $C_{1-6}$ alkyl, $C_{1-6}$ haloalkyl, cyano, $NH_2C(=O)-$, and $C_{1-6}$ alkoxy;
mA and nA are each independently 0, 1, 2, 3, 4, or 5.

**2.** The compound, the stereoisomer thereof, or the pharmaceutically acceptable salt thereof according to claim 1, being a compound of formula IIA, a stereoisomer thereof, or a pharmaceutically acceptable salt thereof:

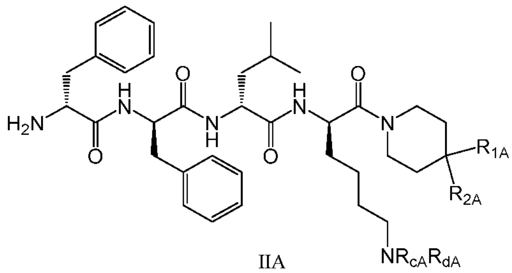
IIA

wherein $R_{1A}$, $R_{2A}$, $R_{cA}$, and $R_{dA}$ are as defined in claim 1;
preferably a compound of formula IIIA, a stereoisomer thereof, or a pharmaceutically acceptable salt thereof:

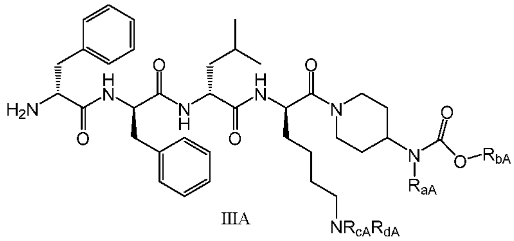

IIIA

wherein $R_{bA}$, $R_{aA}$, $R_{cA}$, and $R_{dA}$ are as defined in claim 1;
more preferably a compound of formula IVA, a stereoisomer thereof, or a pharmaceutically acceptable salt thereof:

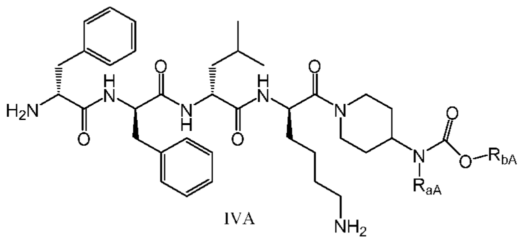

IVA

wherein $R_{bA}$ and $R_{aA}$ are as defined in claim 1;
and even more preferably a compound of formula VA, a stereoisomer thereof, or a pharmaceutically acceptable salt thereof:

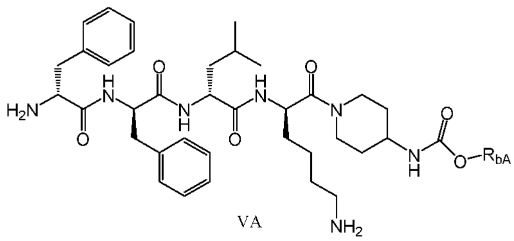

VA

wherein $R_{bA}$ is as defined in claim 1.

**3.** The compound, the stereoisomer thereof, or the pharmaceutically acceptable salt thereof according to claim 1 or 2, wherein $R_{bA}$ is selected from $C_{1-6}$ alkyl or 3- to 8-membered heterocyclyl; preferably from $C_{1-6}$ alkyl or

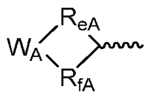
;

more preferably $C_{1-6}$ alkyl or

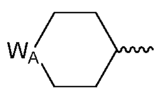
;

and further preferably

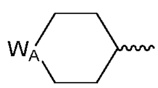
,

$W_a$ is selected from -O- or -S(=O)$_2$-, wherein $R_{eA}$ and $R_{fA}$ are as defined in claim 1; and $R_{aA}$, $R_{cA}$, and $R_{dA}$ are as defined in claim 1.

4. The compound, the stereoisomer thereof, or the pharmaceutically acceptable salt thereof according to claim 1, being a compound of formula VIA, a stereoisomer thereof, or a pharmaceutically acceptable salt thereof:

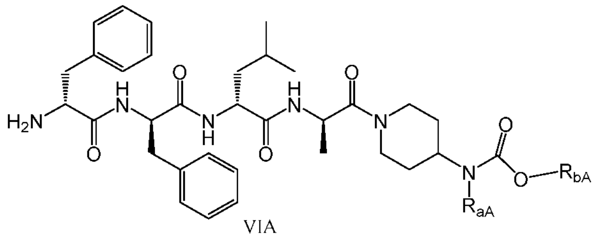
VIA

,

wherein $R_{aA}$ is H or $C_{1-6}$ alkyl substituted with amino, $C_{1-6}$ alkylamino, or $C_{1-6}$ alkoxy, more preferably H or $C_{1-6}$ aminoalkyl; $R_{bA}$ is as defined in claim 1.

5. The compound, the stereoisomer thereof, or the pharmaceutically acceptable salt thereof according to claim 1, being selected from the following compounds, stereoisomers thereof, or pharmaceutically acceptable salts thereof:

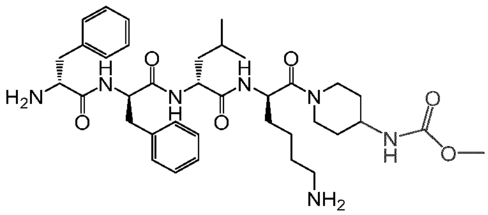

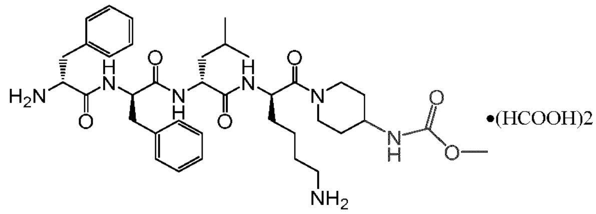
•(HCOOH)2

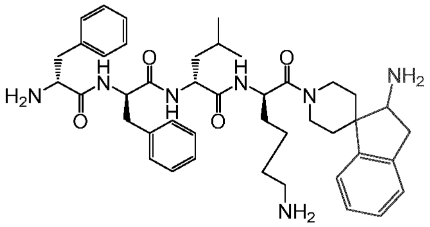

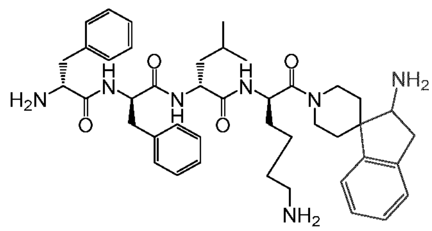
•(HCOOH)3

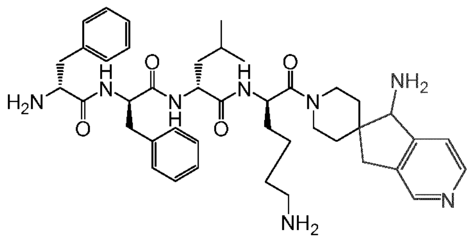

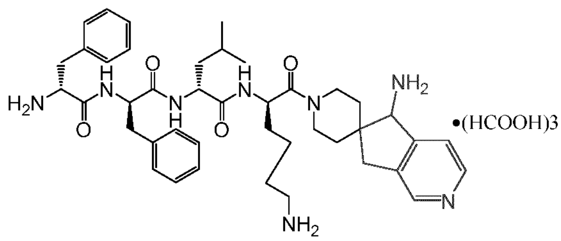
•(HCOOH)3

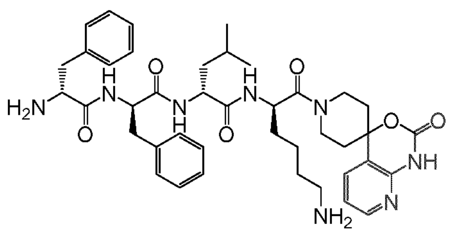

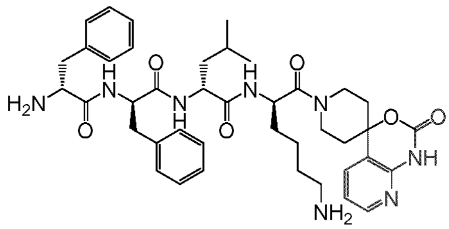
•(HCOOH)2

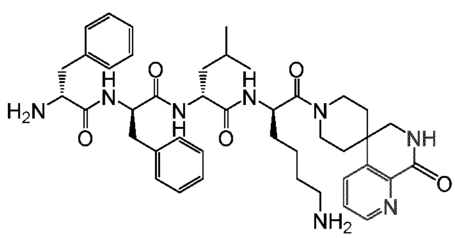

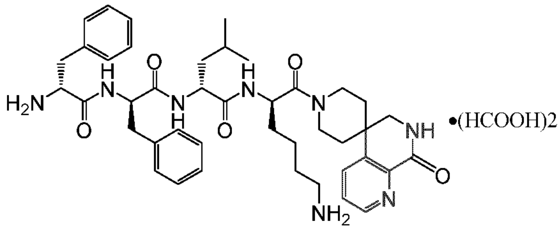

•(HCOOH)2

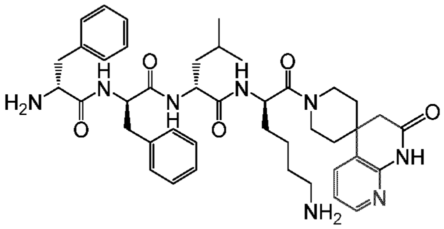

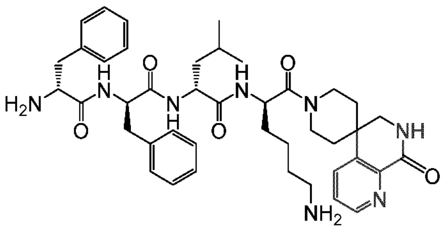

•(HCOOH)2

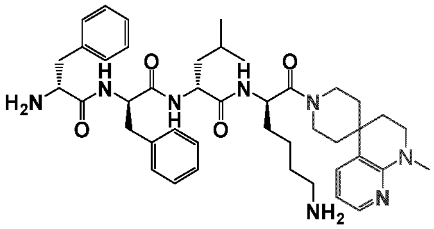

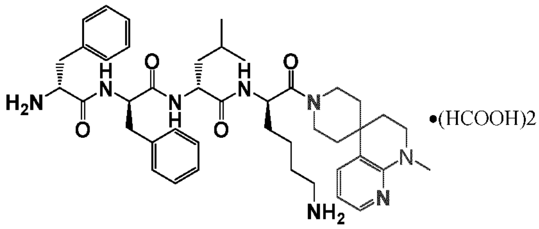

•(HCOOH)2

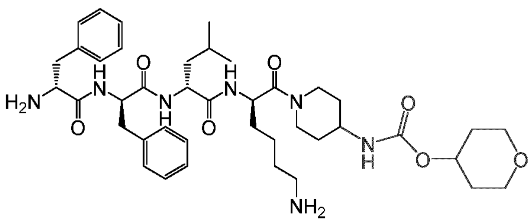

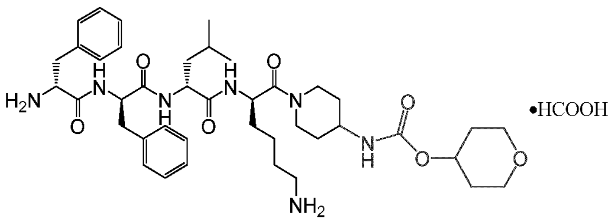

•HCOOH

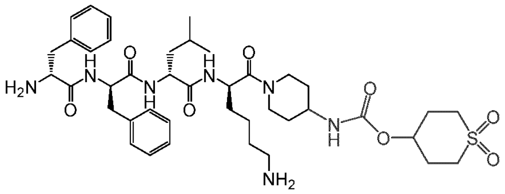

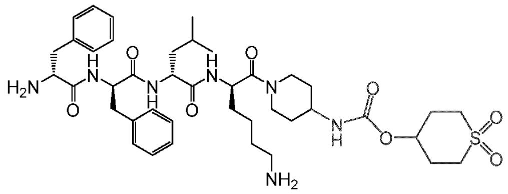

•(HCOOH)2

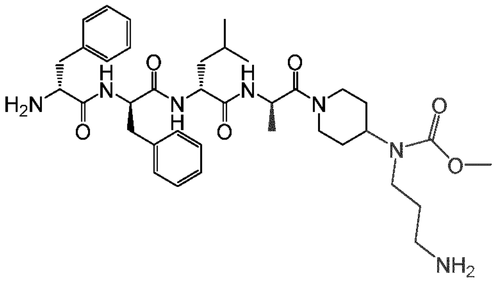

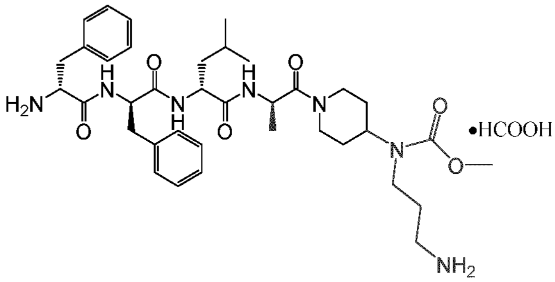

•HCOOH

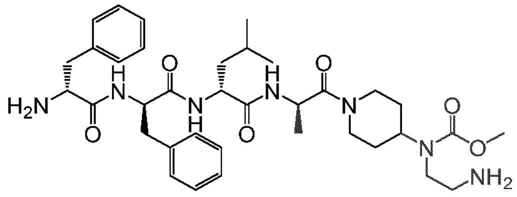

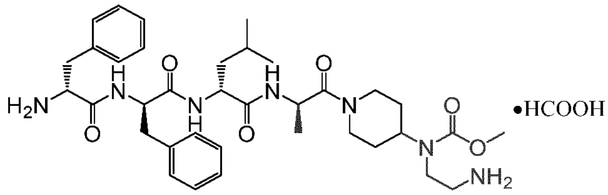 •HCOOH

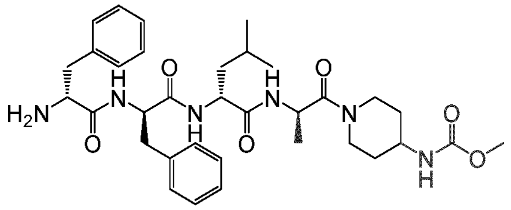

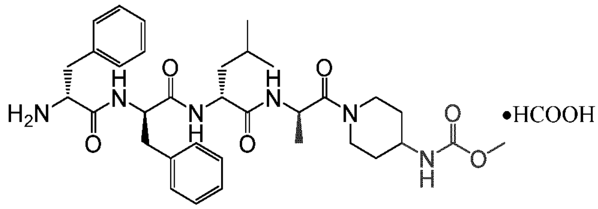 •HCOOH

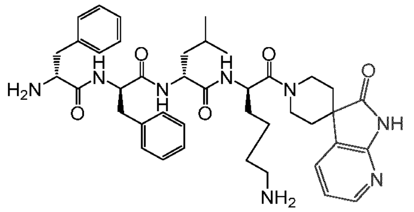

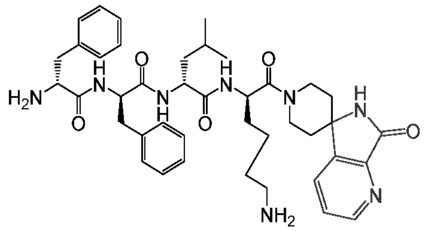

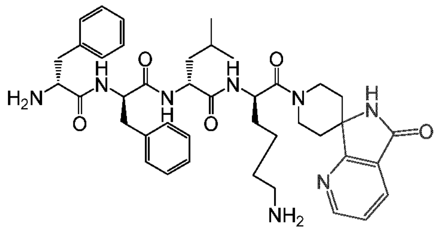

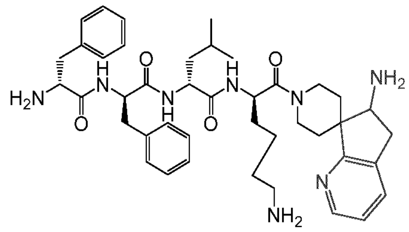

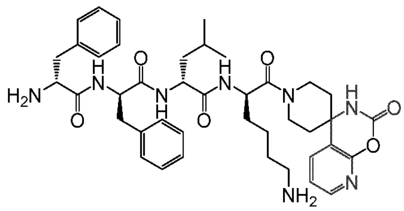

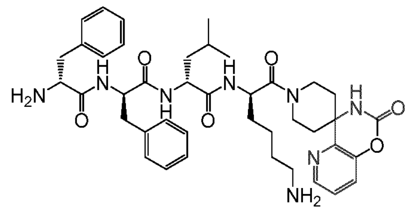

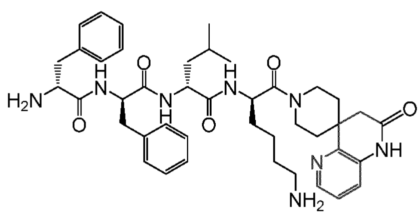

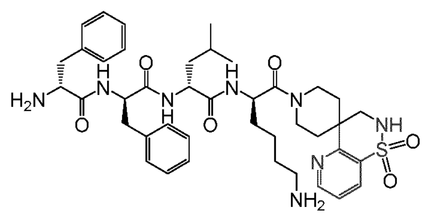

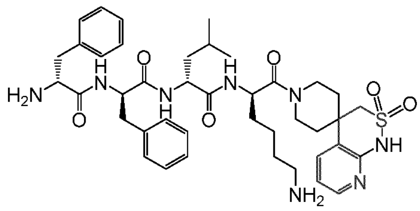

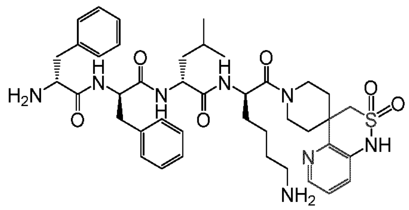

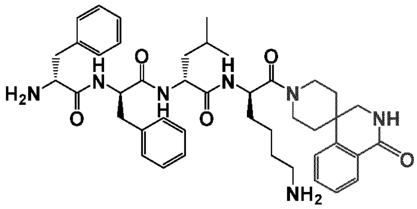

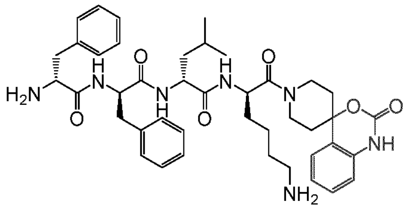

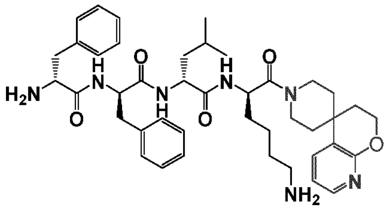

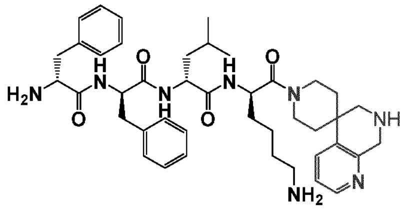

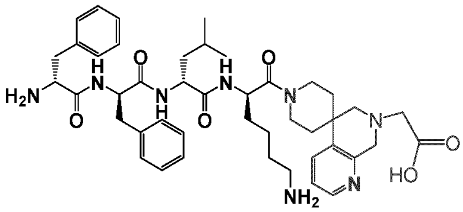

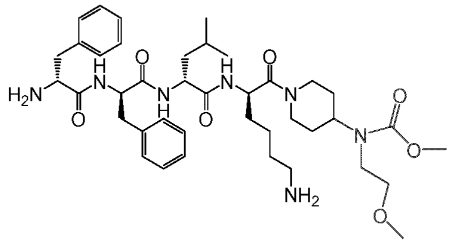

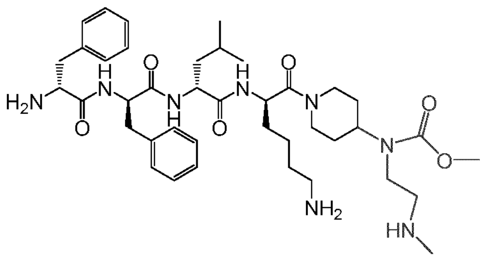

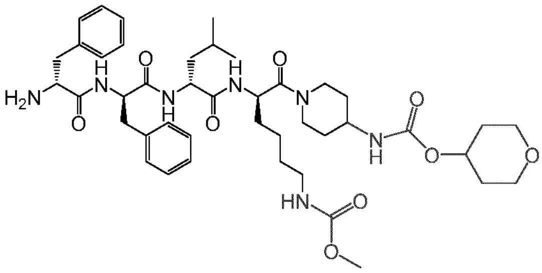

.

6. A method for preparing the compound, the stereoisomer thereof, or the pharmaceutically acceptable salt thereof according to any one of claims 1-5, being selected from the following methods:

method I:
method I comprising the following steps:

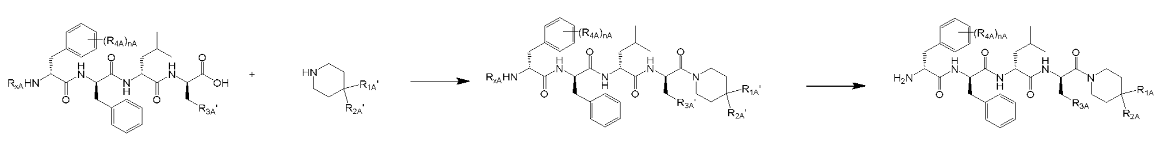

iA-2 iA-3 iA-1 iA

method II:
method II comprising the following steps:

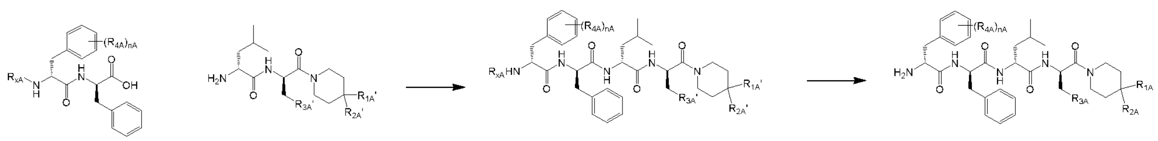

iiA-2 iiA-3 iA-1 iA

wherein $R_{1A}$' is $R_{1A}$ or $R_{1A}$ in which $NH_2$ is protected with an amino-protecting group, $R_{2A}$' is $R_{2A}$ or $R_{2A}$ in which

$NH_2$ is protected with an amino-protecting group, $R_{3A}$' is $R_{3A}$ or $R_{3A}$ in which $NH_2$ is protected with an amino-protecting group, $R_{1A}$, $R_{2A}$, $R_{3A}$, $R_{4A}$, and nA are as defined in any one of claims 1-5, and $R_{xA}$ is an amino-protecting group;

the amino-protecting groups are preferably each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, *tert*-butylsulfinyl, or ethoxycarbonyl.

**7.** A compound of the following general formula, a stereoisomer thereof, or a salt thereof,

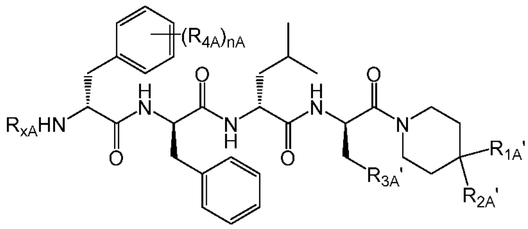

iA-1

wherein $R_{1A}$' is $R_{1A}$ or $R_{1A}$ in which $NH_2$ is protected with an amino-protecting group, $R_{2A}$' is $R_{2A}$ or $R_{2A}$ in which $NH_2$ is protected with an amino-protecting group, $R_{3A}$' is $R_{3A}$ or $R_{3A}$ in which $NH_2$ is protected with an amino-protecting group, $R_{1A}$, $R_{2A}$, $R_{3A}$, $R_{4A}$, and nA are as defined in any one of claims 1-5, and $R_{xA}$ is an amino-protecting group;

the amino-protecting groups are preferably each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, *tert*-butylsulfinyl, or ethoxycarbonyl.

**8.** A compound of formula IB, a stereoisomer thereof, or a pharmaceutically acceptable salt thereof:

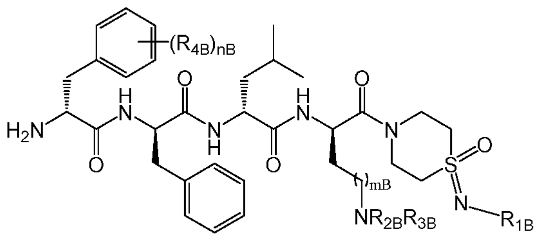

IB

wherein,

$R_{1B}$ is selected from H, $C_{1-6}$ alkyl, $C_{1-6}$ alkylcarbonyl, $C_{1-6}$ alkoxycarbonyl, $C_{6-14}$ aryl, $C_{6-14}$ arylcarbonyl, $C_{6-14}$ aryloxycarbonyl, $C_{3-8}$ cycloalkyl, $C_{3-8}$ cycloalkylcarbonyl, $C_{3-8}$ cycloalkoxycarbonyl, 5- to 14-membered heteroaryl, 5- to 14-membered heteroarylcarbonyl, 5- to 14-membered heteroaryloxycarbonyl, 3- to 8-membered heterocyclyl, 3- to 8-membered heterocyclylcarbonyl, and 3- to 8-membered heterocyclyloxycarbonyl, the above substituents are each optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;

$R_{2B}$ and $R_{3B}$ are each independently selected from H, $C_{1-6}$ alkyl, amidino, and $C_{1-6}$ alkoxycarbonyl;

$R_{4B}$ is selected from halogen, $NO_2$, $C_{1-6}$ alkyl, $C_{1-6}$ haloalkyl, cyano, $NH_2C(=O)$-, and $C_{1-6}$ alkoxy;

mB and nB are each independently 0, 1, 2, 3, 4, or 5.

**9.** The compound, the stereoisomer thereof, or the pharmaceutically acceptable salt thereof according to claim 8, being a compound of formula IIB, a stereoisomer thereof, or a pharmaceutically acceptable salt thereof:

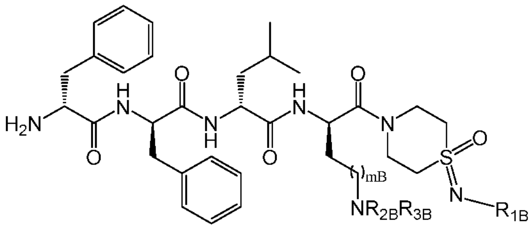

IIB

,

preferably a compound of formula IIIB, a stereoisomer thereof, or a pharmaceutically acceptable salt thereof:

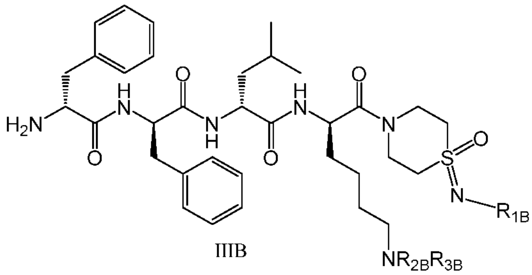

IIIB

,

and preferably a compound of formula IVB, a stereoisomer thereof, or a pharmaceutically acceptable salt thereof:

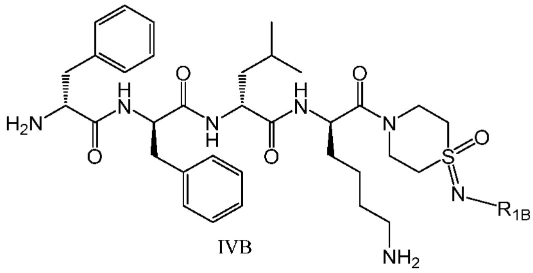

IVB

.

**10.** The compound, the stereoisomer thereof, or the pharmaceutically acceptable salt thereof according to any one of claims 8-9, wherein $R_{1B}$ is selected from $C_{1-6}$ alkyl and $C_{1-6}$ alkylcarbonyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, preferably $C_{1-6}$ alkyl or $C_{1-6}$ alkylcarbonyl, and more preferably methyl or methylcarbonyl.

**11.** The compound, the stereoisomer thereof, or the pharmaceutically acceptable salt thereof according to claim 8, being selected from the following compounds, stereoisomers thereof, or pharmaceutically acceptable salts thereof:

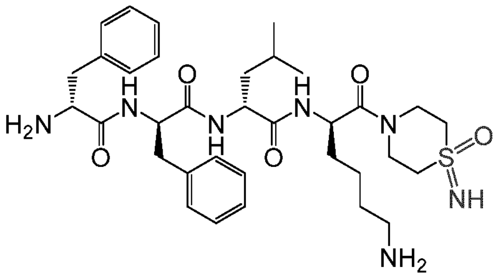

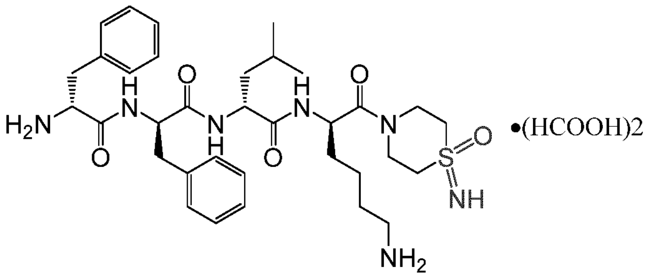

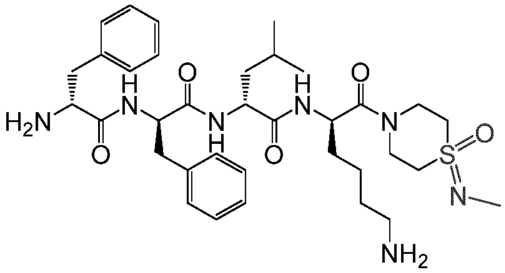

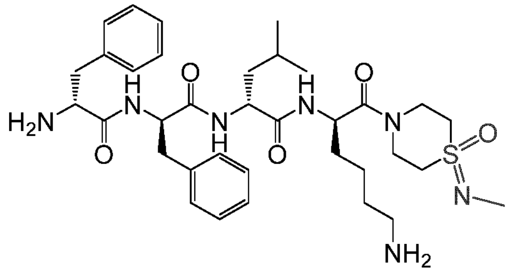

•HCOOH

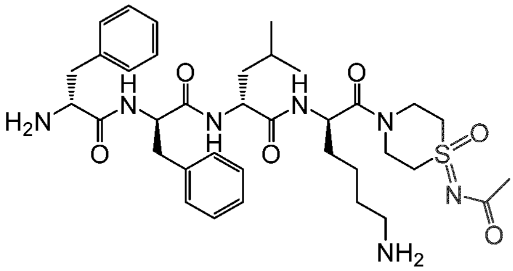

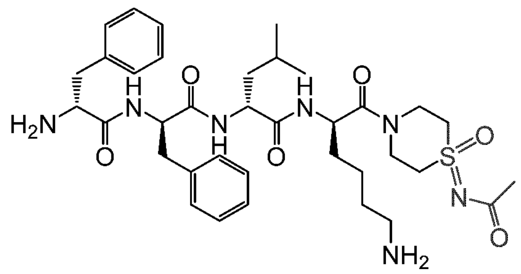

•HCOOH

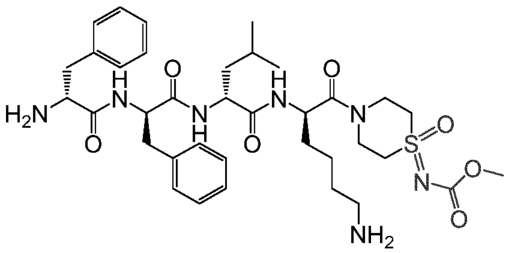

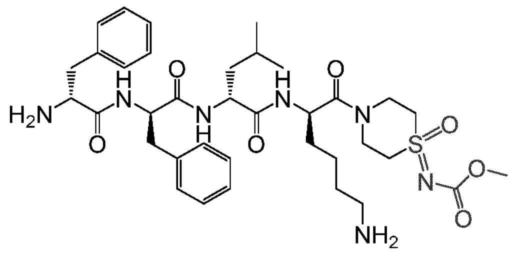

•(HCOOH)2

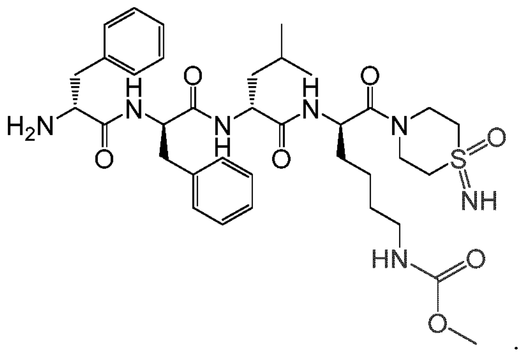

.

**12.** A method for preparing the compound, the stereoisomer thereof, or the pharmaceutically acceptable salt thereof according to any one of claims 8-11, comprising the following steps:

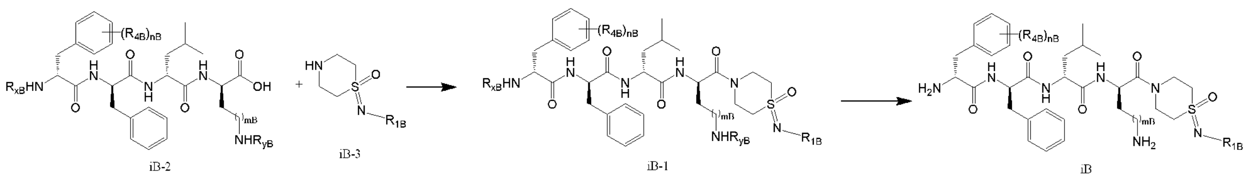

iB-2 iB-3 iB-1 iB

wherein $R_{1B}$, $R_{4B}$, mB, and nB are as defined in any one of claims 8-11, and $R_{xB}$ and $R_{yB}$ are amino-protecting groups, which are preferably each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, or ethoxycarbonyl.

**13.** A compound of formula iB-1, a stereoisomer thereof, or a salt thereof,

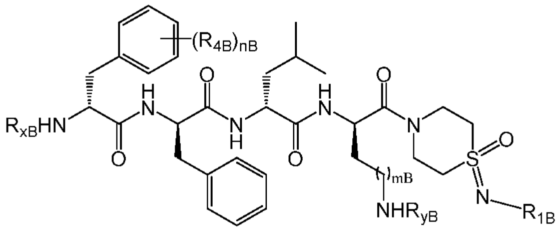

iB-1

wherein $R_{1B}$, $R_{4B}$, mB, and nB are as defined in any one of claims 8-11, and $R_{xB}$ and $R_{yB}$ are amino-protecting groups, which are preferably each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, or ethoxycarbonyl.

**14.** A compound of formula IC, a stereoisomer thereof, or a pharmaceutically acceptable salt thereof:

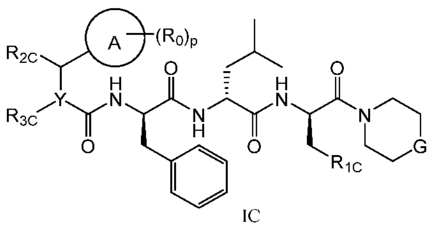

IC

wherein,

ring A is $C_{3-8}$ cycloalkyl, $C_{6-14}$ aryl, or 5- to 14-membered heteroaryl, preferably phenyl; Y is selected from CH or N; G is selected from -S-, -O-, $-CR_{4C}R_{5C}-$, $-NR_{6C}-$, $-S(=O)_2-$, $-S(=O)(=NR_{6C}')-$, and

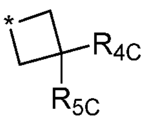

,

preferably -O-, - $CR_{4C}R_{5C}-$, $-NR_{6C}-$, $-S(=O)_2-$, $-S(=O)(=NR_{6C}')-$, and

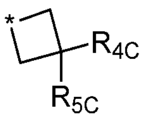

;

$R_{1C}$ is selected from H or $-(CH_2)_tNR_{aC}R_{bC}$;
$R_{2C}$ is selected from H, amino, hydroxyl, $C_{1-6}$ alkyl, $C_{1-6}$ alkylamino, and $C_{1-6}$ aminoalkyl, wherein the alkyl is optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy;
$R_{3C}$ is selected from H, hydroxyl, $C_{1-6}$ alkyl, 3- to 8-membered heterocyclyl, and $C_{1-6}$ alkoxy when Y is CH, and is selected from H, $C_{1-6}$ alkyl, $C_{3-8}$ cycloalkyl, $C_{3-8}$ cycloalkyl-$(CH_2)_m$-, 3- to 8-membered heterocyclyl, 3- to 8-membered heterocyclyl-$(CH_2)_{mC}$-, and $-(CH_2)_{mC}NR_{10}R_{11}$ when Y is N, wherein the alkyl, cycloalkyl, heterocyclyl, and alkoxy are optionally substituted with one or more groups selected from halogen, hydroxyl, amino, nitro, cyano, $C_{1-6}$ alkyl, $C_{1-6}$ haloalkyl, $NH_2C(=O)-$, $C_{1-6}$ alkoxy, and $C_{1-6}$ alkylamino;
$R_{4C}$ and $R_{5C}$ are each independently selected from H, $C_{1-6}$ alkyl, $C_{1-6}$ alkyl-O-, hydroxyl, $-C(O)OR_7$, $-NR_8R_9$, $-NR_{cC}C(O)NR_8R_9$, $C_{1-6}$ alkylamino, 3- to 8-membered heterocyclyl-$(CH_2)_{mC}$-, halogen, cyano, $-NR_{cC}S(=O)_2NR_8R_9$, $-NR_{cC}C(O)OR_{dC}$, $-NR_{cC}S(=O)_2OR_{dC}$, $-NR_{cC}C(O)R_7'$, and $-NH(CH_2)_{mC}NR_8R_9$, wherein the alkyl and heterocyclyl are optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy;
or $CR_{4C}R_{5C}$ forms a 3- to 8-membered heterocyclic or 9- to 10-membered bicyclic moiety, wherein the 3- to 8-membered heterocyclic or 9- to 10-membered bicyclic moiety, together with the piperidine ring attached thereto, forms a structure selected from:

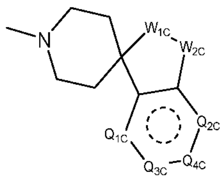 , 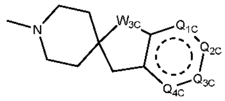 ,

and

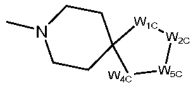 ,

when any one of $Q_{1C}$-$Q_{4C}$ is N, the rest are C, or $Q_{1C}$-$Q_{4C}$ are all C;

$W_{1C}$ and $W_{2C}$ are each independently -C(=O)-NH-, -NH-C(=O)-, $-S(=O)_2$-NH-, $-NH-S(=O)_2$-, -S-, -O-, $-NR_{12}$-, $-NR_{12}-CH_2$-, $-(CH_2)_{n2C}$- optionally substituted with one or more groups selected from $-NH_2$, -OH, halogen, nitro, cyano, $C_{1-6}$ haloalkyl, $NH_2C(=O)$-, and $C_{1-6}$ alkoxy, or absent, wherein $R_{12}$ is selected from H, $C_{1-6}$ alkyl, amidino, and HOOC-$(CH_2)_{n3C}$-; preferably, $W_{1C}$ and $W_{2C}$ cannot be simultaneously absent;

$W_{3C}$ is $-(CH_2)_{n2C}$- optionally substituted with one or more groups selected from $NH_2$, -OH, halogen, nitro, cyano, $C_{1-6}$ haloalkyl, $NH_2C(=O)$-, and $C_{1-6}$ alkoxy, or absent; preferably $-(CH_2)_{n2C}$- optionally substituted with one or more groups selected from $NH_2$, -OH, halogen, nitro, cyano, $C_{1-6}$ haloalkyl, $NH_2C(=O)$-, and $C_{1-6}$ alkoxy;

$W_{4C}$ and $W_{5C}$ are $-(CH_2)_{n2C}$- optionally substituted with one or more groups selected from $NH_2$, -OH, halogen, nitro, cyano, $C_{1-6}$ haloalkyl, $NH_2C(=O)$-, and $C_{1-6}$ alkoxy, or absent;

n2C is selected from 0, 1, 2, and 3;

more preferably, the 3- to 8-membered heterocyclic or 9- to 10-membered bicyclic moiety, together with the piperidine ring attached thereto, forms a structure selected from:

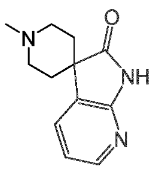 , 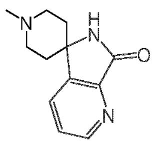 , 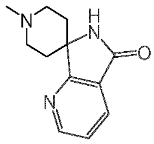 , 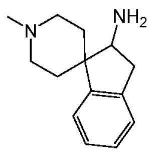 , 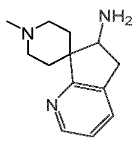 , 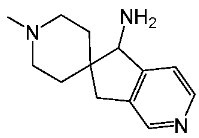 ,

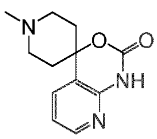 , 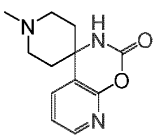 , 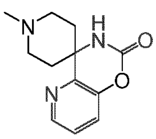 , 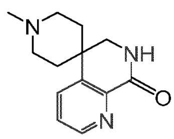 , 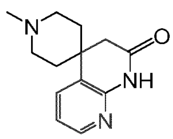 , 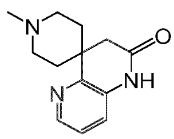 ,

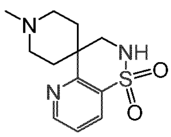 , 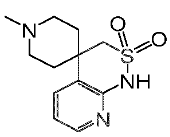 , 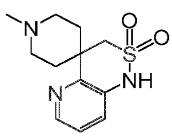 , 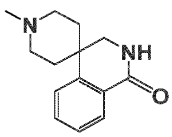 , 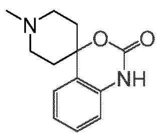 , 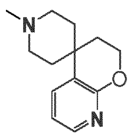 ,

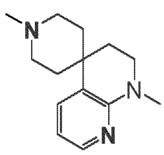 , 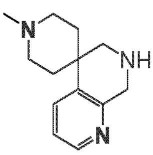 , 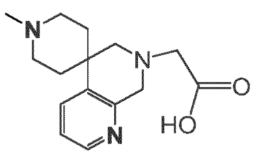 , 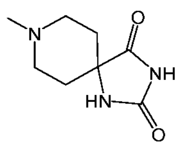 , and 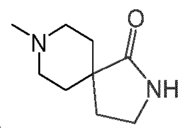 ,

more preferably from

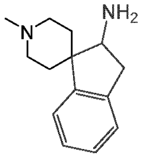, 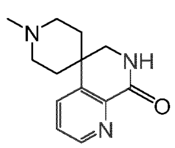, 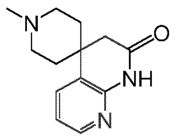, 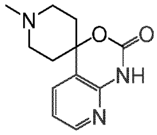, 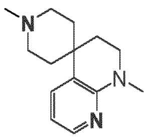, 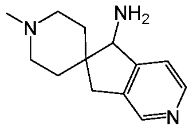,

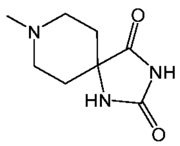,

and

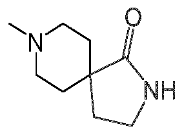;

$R_{aC}$ and $R_{bC}$ are each independently selected from H, $C_{1-6}$ alkyl, amidino, and $C_{1-6}$ alkoxycarbonyl;
$R_{cC}$ is H or $C_{1-6}$ alkyl substituted with one or more groups selected from amino, $C_{1-6}$ alkylamino, $C_{1-6}$ alkoxy, halogen, hydroxyl, nitro, cyano, $NH_2C(=O)$-, and $C_{1-6}$ alkoxy, preferably H or $C_{1-6}$ alkyl substituted with amino, $C_{1-6}$ alkylamino, or $C_{1-6}$ alkoxy, and more preferably H or $C_{1-6}$ alkyl substituted with amino or $C_{1-6}$ alkylamino;
$R_{dC}$ is selected from $C_{1-6}$ alkyl, $C_{6-14}$ aryl, 5- to 14-membered heteroaryl, $C_{3-8}$ cycloalkyl, and 3- to 8-membered heterocyclyl, preferably from $C_{1-6}$ alkyl and 3- to 8-membered heterocyclyl, more preferably from $C_{1-6}$ alkyl and

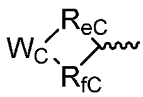,

and even more preferably from $C_{1-6}$ alkyl and

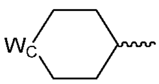,

the group $R_{dC}$ described above is optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy, wherein $R_{eC}$ and $R_{fC}$ are each independently $-(CH_2)_{n1C}$- and $-(CH_2)_{n1C'}$-, wherein n1C and n1C' are each independently selected from 0, 1, 2, and 3, preferably 2, and n1C and n1C' are not both 0; Wc is selected from -NH-C(=O)-, -NH-S$(=O)_2$-, $-NR_{12}$-, -O-, -S-, and -S$(=O)_2$-, preferably from -O- and - S$(=O)_2$-, wherein $R_{12}$ is selected from H, $C_{1-6}$ alkyl, amidino, and HOOC-$(CH_2)_{n3C}$-, wherein $_{n3C}$ is selected from 1, 2, and 3;
$R_{6C}$ and $R_{6C}$' are independently selected from H, $C_{1-6}$ alkyl, $C_{1-6}$ alkylcarbonyl, $C_{1-6}$ alkyl-S$(=O)_2$-, $C_{1-6}$ alkoxycarbonyl, $C_{6-14}$ aryl, $C_{6-14}$ arylcarbonyl, $C_{6-14}$ aryloxycarbonyl, $C_{3-8}$ cycloalkyl, $C_{3-8}$ cycloalkylcarbonyl, $C_{3-8}$ cycloalkoxycarbonyl, 5- to 14-membered heteroaryl, 5- to 14-membered heteroarylcarbonyl, 5- to 14-membered heteroaryloxycarbonyl, 3- to 8-membered heterocyclyl, 3- to 8-membered heterocyclylcarbonyl, and 3- to 8-membered heterocyclyloxycarbonyl, the above substituents are each optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;
$R_7$ and $R_7$' are each independently selected from H, $C_{1-6}$ alkyl, $C_{3-8}$ cycloalkyl, 3- to 8-membered heterocyclyl, $C_{6-14}$ aryl, and 5- to 14-membered heteroaryl, wherein the alkyl, cycloalkyl, heterocyclyl, aryl, and heteroaryl are optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;
$R_8$ and $R_9$ are each independently H or $C_{1-6}$ alkyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;
$R_{10}$ and $R_{11}$ are each independently H or $C_{1-6}$ alkyl, or $R_{10}$ and $R_{11}$, together with the nitrogen atom to which they are both attached form 3- to 8-membered heterocyclyl, wherein the alkyl and heterocyclyl are optionally

substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy;
p and t are each independently selected from 0, 1, 2, 3, 4, or 5;
mC is independently selected from 1, 2, 3, and 4 at each occurrence;
$R_0$ is selected from H, halogen, $NO_2$, cyano, $NH_2C(=O)$-, $C_{1-6}$ alkoxy, and $C_{1-6}$ alkyl optionally substituted with one or more groups selected from halogen, hydroxyl, amino, nitro, cyano, $C_{1-6}$ alkyl, $C_{1-6}$ haloalkyl, $NH_2C(=O)$-, and $C_{1-6}$ alkoxy.

**15.** The compound, the stereoisomer thereof, or the pharmaceutically acceptable salt thereof according to claim 14, being a compound of formula IIC-1 or IIC-2, a stereoisomer thereof, or a pharmaceutically acceptable salt thereof:

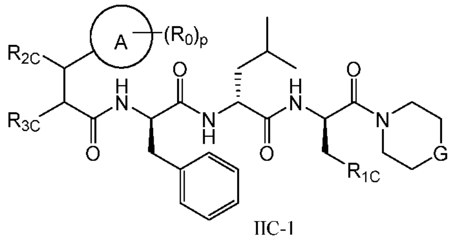

IIC-1

,

wherein preferably, in IIC-1, $R_{2C}$ is $C_{1-6}$ aminoalkyl or amino, and $R_{3C}$ is H or $C_{1-6}$ alkyl; more preferably, $R_{2C}$ is amino, and $R_{3C}$ is H;

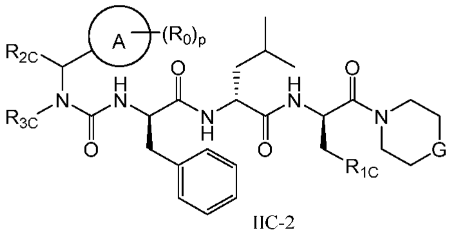

IIC-2

.

preferably, in formula IIC-2, $R_{2C}$ is $C_{1-6}$ aminoalkyl or amino, and $R_{3C}$ is H or $C_{1-6}$ alkyl, or $R_{2C}$ is H or $C_{1-6}$ alkyl, and $R_{3C}$ is $C_{1-6}$ aminoalkyl or 3- to 8-membered heterocyclyl containing nitrogen;
more preferably, formula IIC-2 is formula IIIC or formula IVC:

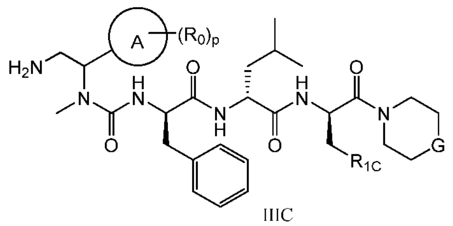

IIIC

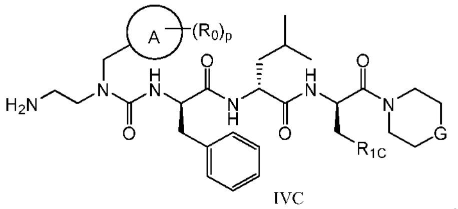

IVC

.

**16.** The compound, the stereoisomer thereof, or the pharmaceutically acceptable salt thereof according to claim 14 or 15, wherein one or more of the following criteria are satisfied:

(1) $R_{1C}$ is $-(CH_2)_tNR_{aC}R_{bC}$, preferably $-(CH_2)_3NH_2$;
(2) ring A is phenyl; and

(3) p is 0.

**17.** The compound, the stereoisomer thereof, or the pharmaceutically acceptable salt thereof according to any one of claims 14-16, wherein G is selected from one of the following:

(1) G is $-S(=O)(=NR_{6C}')-$;
(2) G is $-CR_{4C}R_{5C}-$, wherein $R_{4C}$ is H, and $R_{5C}$ is $-NR_{cC}C(O)OR_{dC}$; and
(3) G is $-CR_{4C}R_{5C}-$, wherein $R_{4C}$ is $-NR_8R_9$, and $R_{5C}$ is $-C(O)OR_7$, wherein $R_7$ is selected from H, $C_{1-6}$ alkyl, $C_{3-8}$ cycloalkyl, 3- to 8-membered heterocyclyl, $C_{6-14}$ aryl, and 5- to 14-membered heteroaryl, wherein the alkyl, cycloalkyl, heterocyclyl, aryl, and heteroaryl are optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy; and $R_7$ is preferably H or $C_{1-6}$ alkyl optionally substituted with one or more groups selected from halogen, hydroxyl, amino, nitro, cyano, $C_{1-6}$ alkyl, $C_{1-6}$ haloalkyl, $NH_2C(=O)-$, and $C_{1-6}$ alkoxy.

**18.** The compound, the stereoisomer thereof, or the pharmaceutically acceptable salt thereof according to any one of claims 14-17, being a compound of the following general formula, a stereoisomer thereof, or a pharmaceutically acceptable salt thereof:

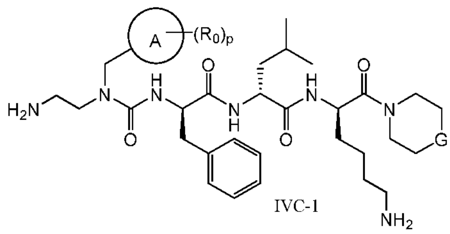

IVC-1 ,

more preferably

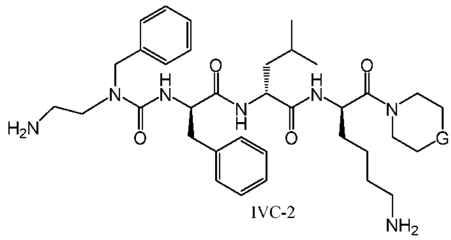

IVC-2 ,

and even more preferably

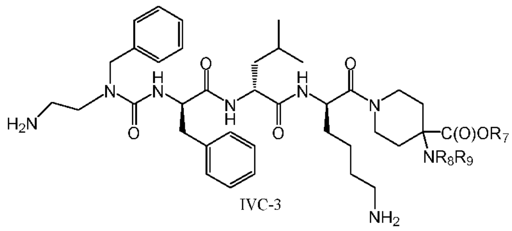

IVC-3 .

**19.** The compound, the stereoisomer thereof, or the pharmaceutically acceptable salt thereof according to claim 14, being selected from the following compounds, stereoisomers thereof, or pharmaceutically acceptable salts thereof:

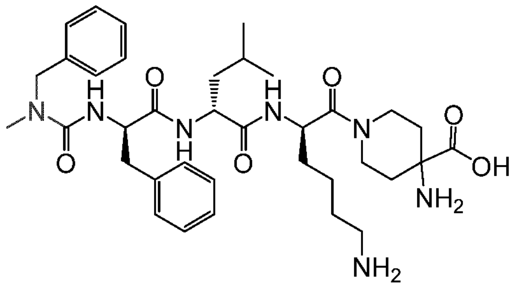

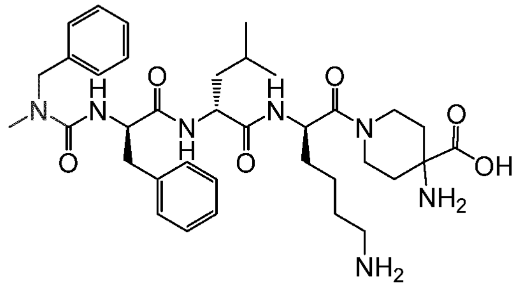

•HCOOH

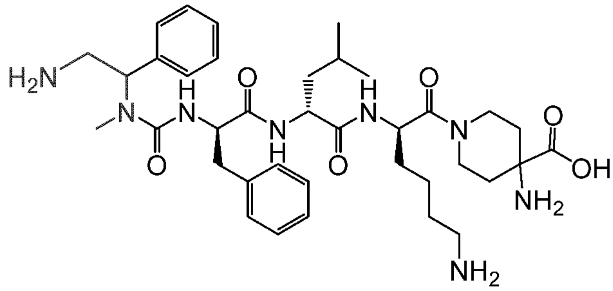

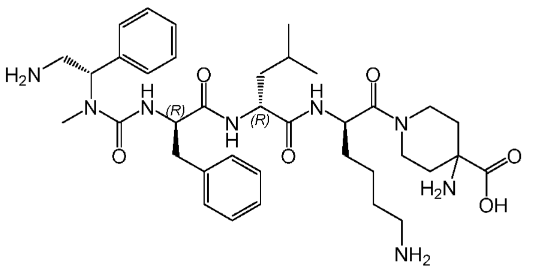

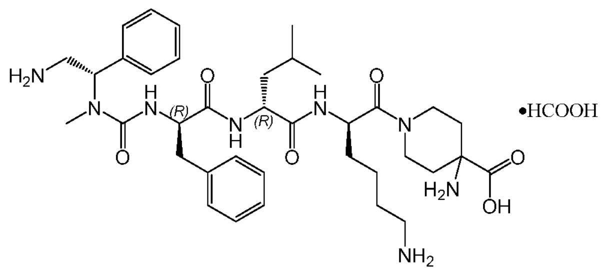

•HCOOH

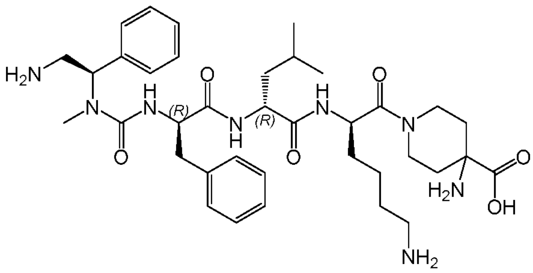

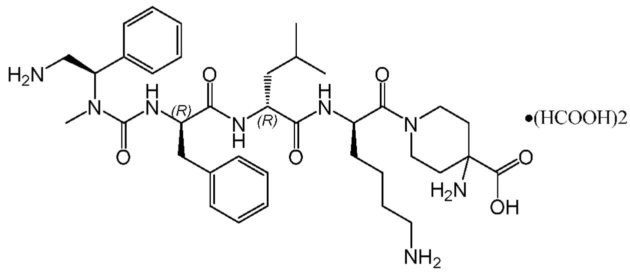

•(HCOOH)2

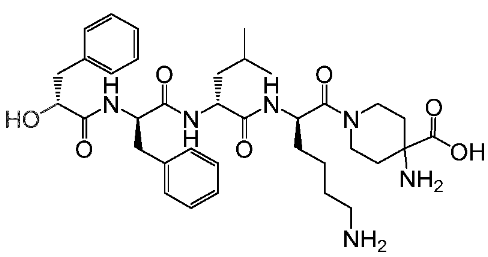

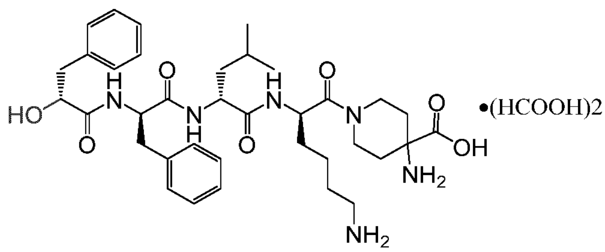

•(HCOOH)2

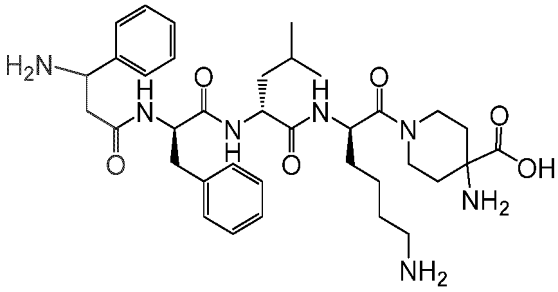

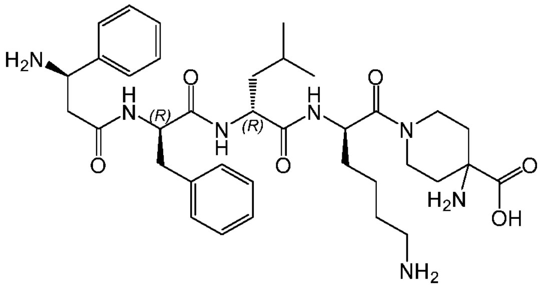

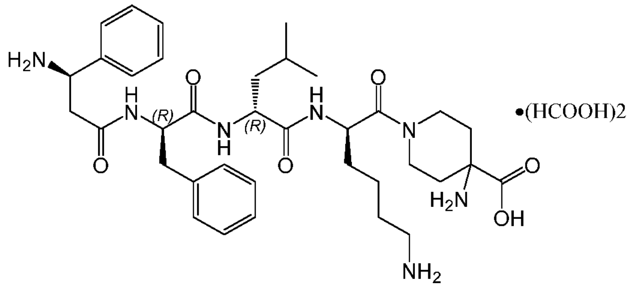

•(HCOOH)2

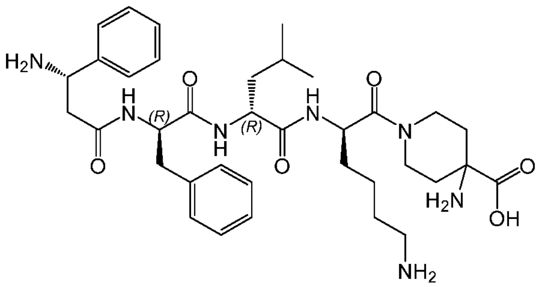

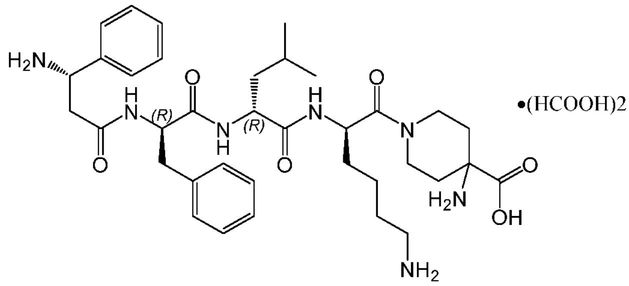

•(HCOOH)2

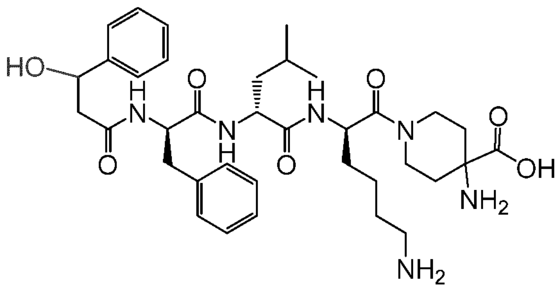

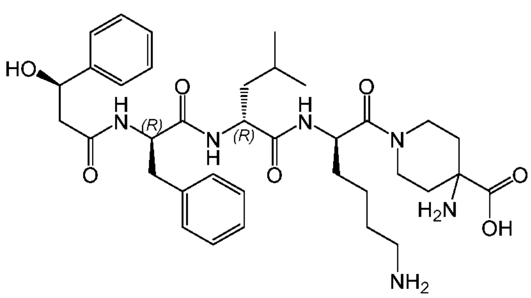

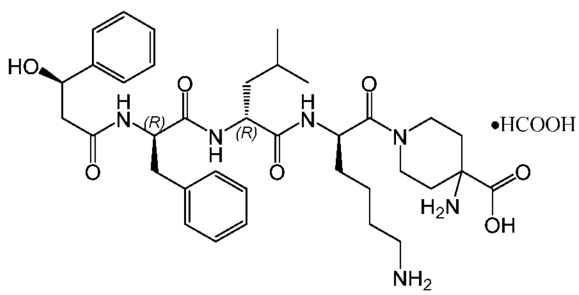

•HCOOH

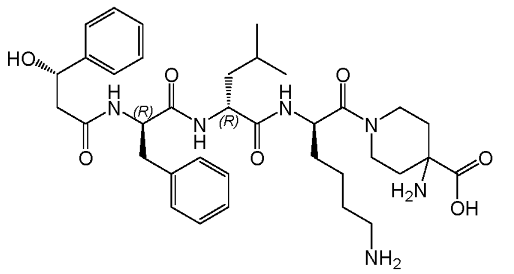

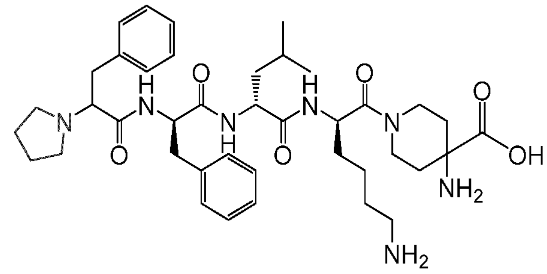

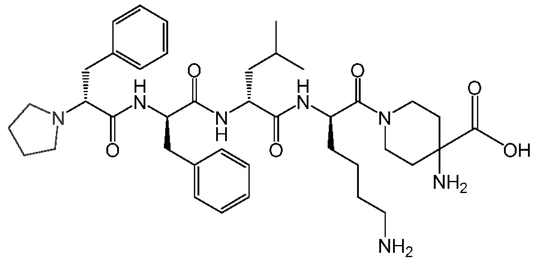

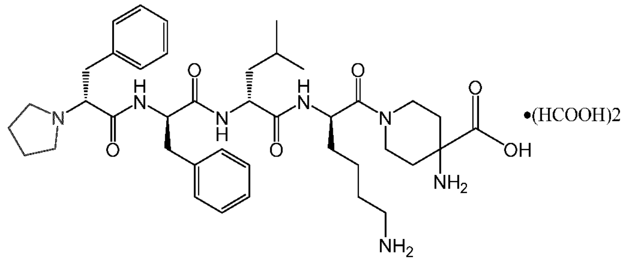

•(HCOOH)2

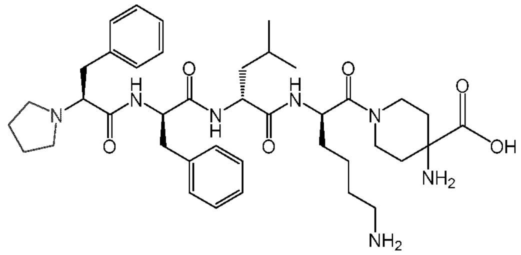

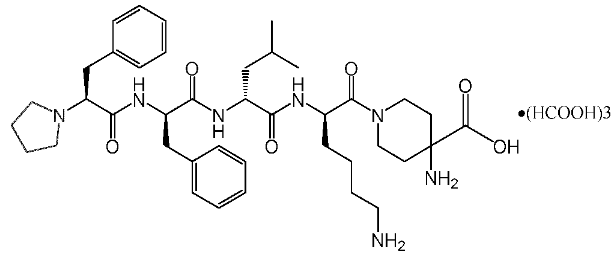

•(HCOOH)3

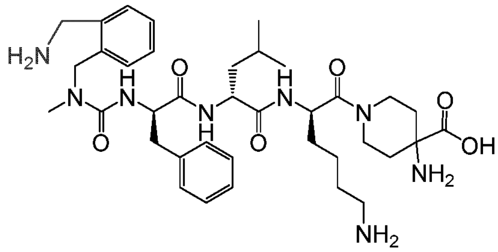

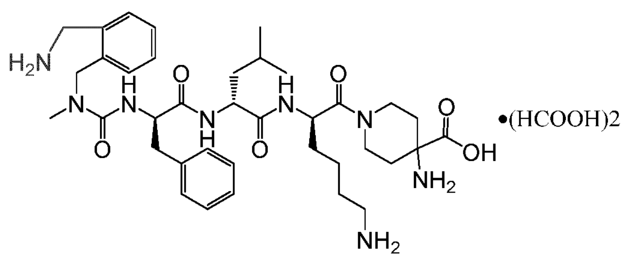

•(HCOOH)2

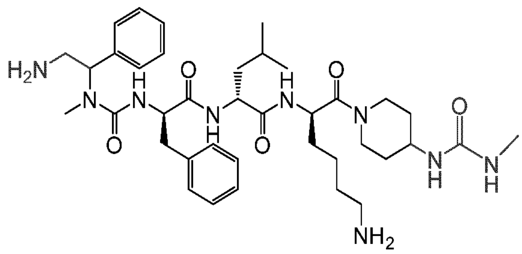

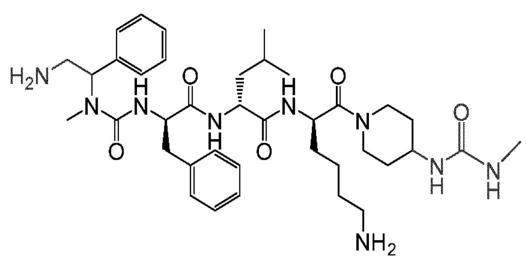

•(HCOOH)2

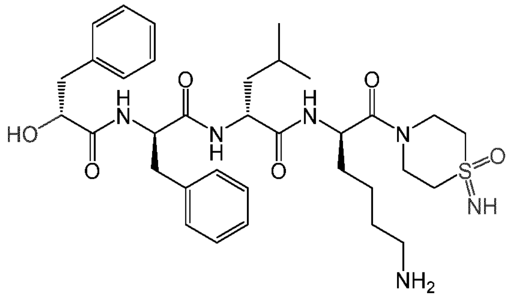

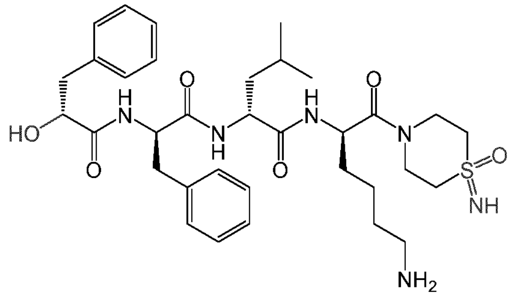

•HCOOH

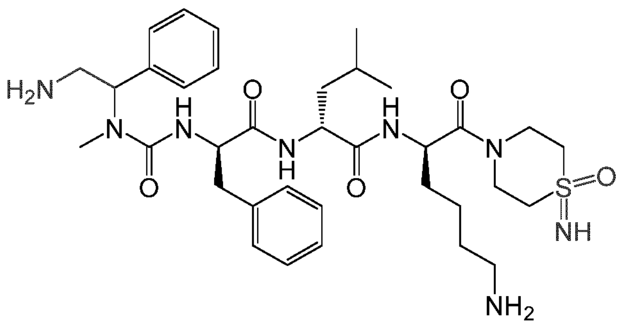

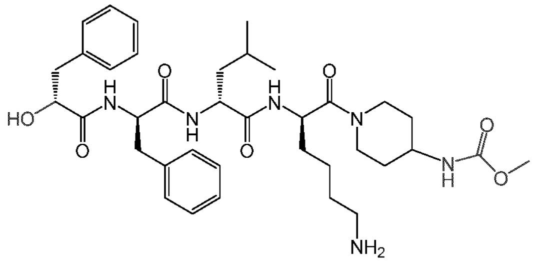

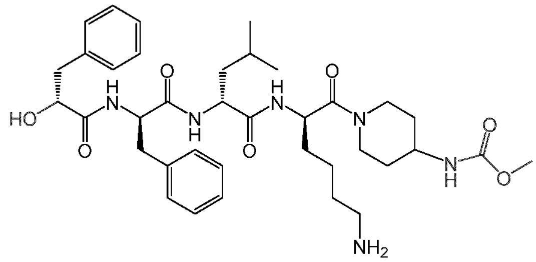

•HCOOH

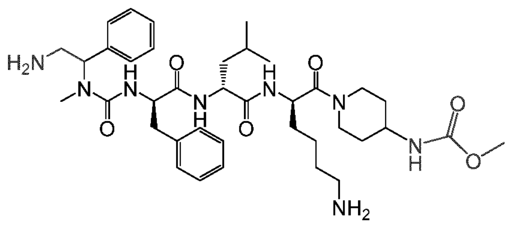

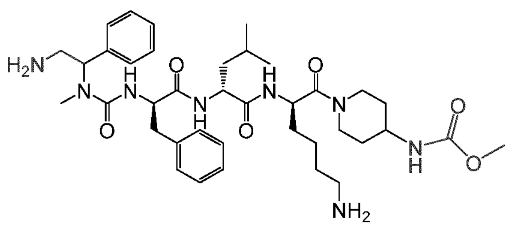

•(HCOOH)2

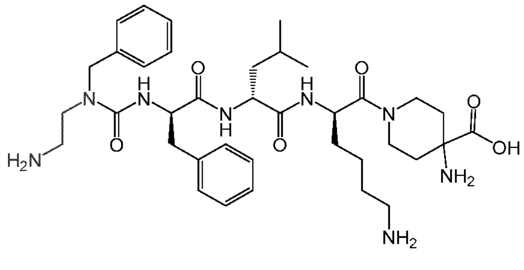

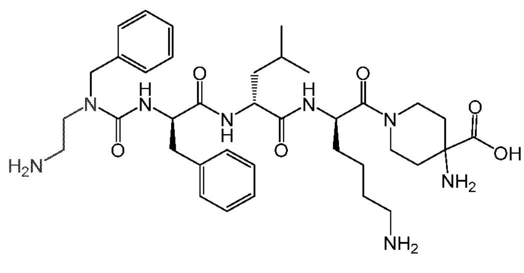

•(HCOOH)2

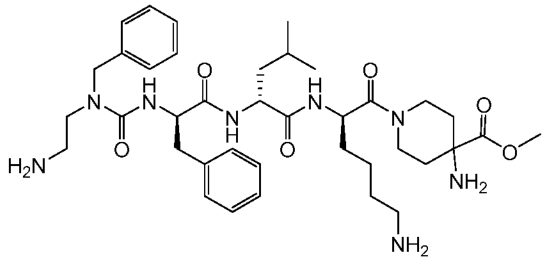

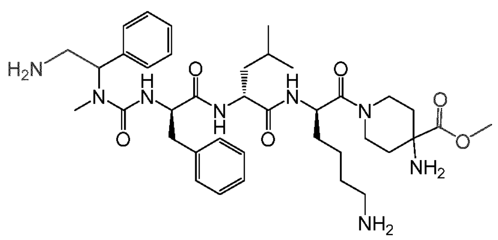

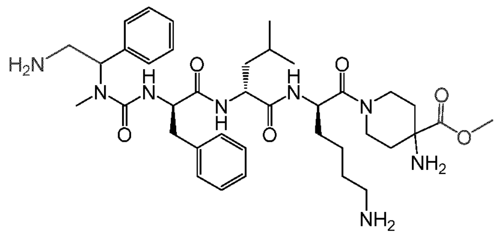

•(HCOOH)3

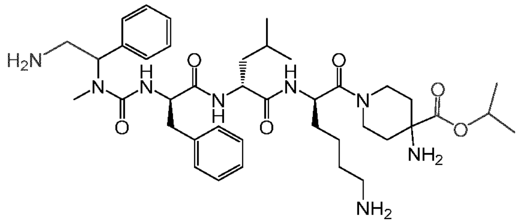

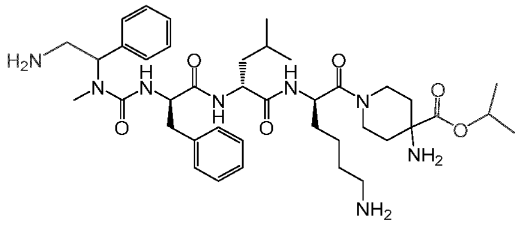

•(HCOOH)2

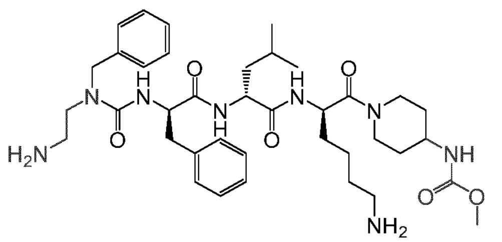

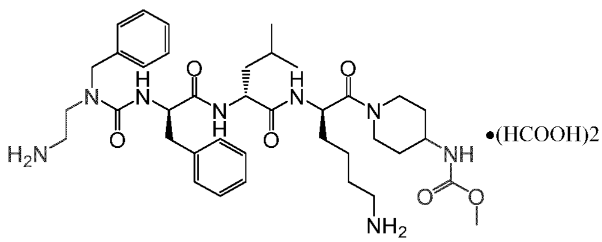

•(HCOOH)2

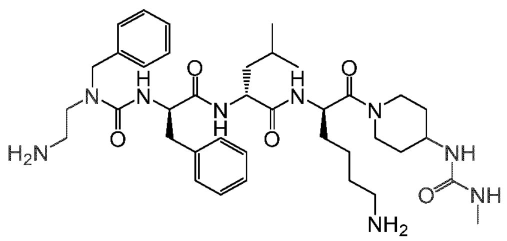

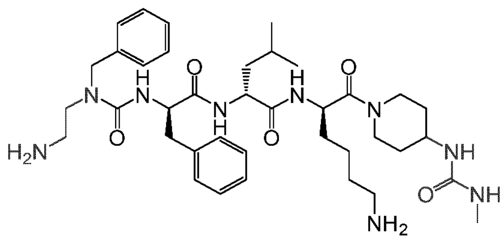

•(HCOOH)2

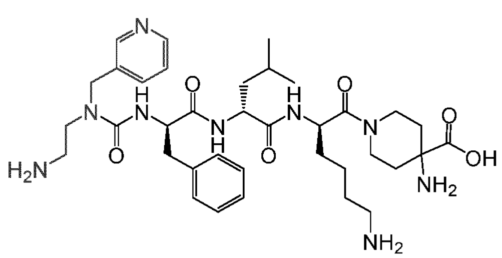

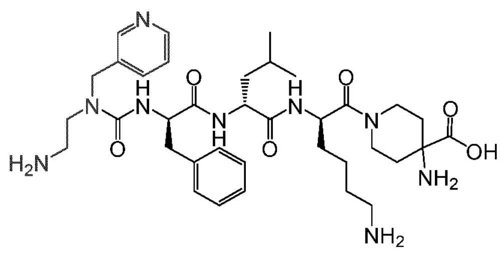

•HCOOH

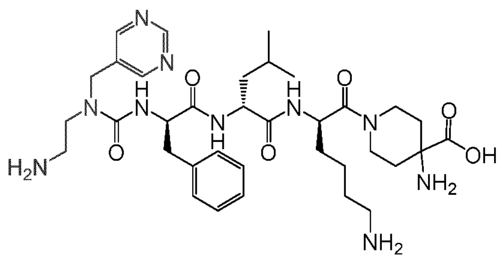

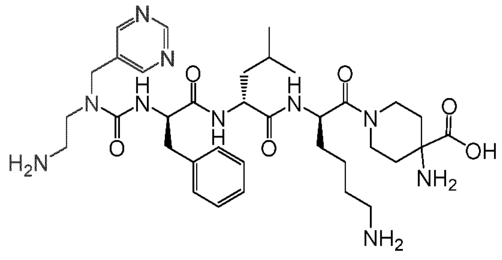

•HCOOH

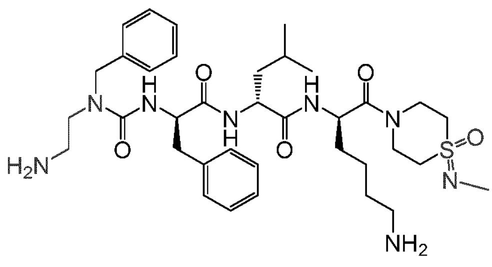

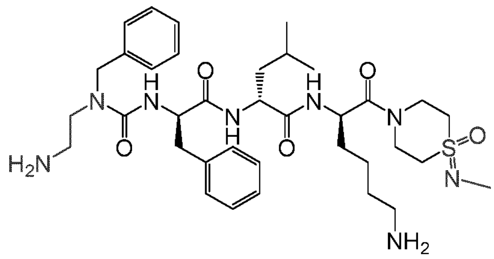

•(HCOOH)2

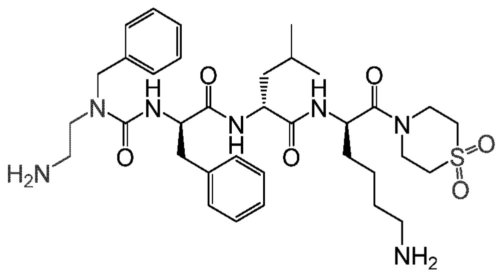

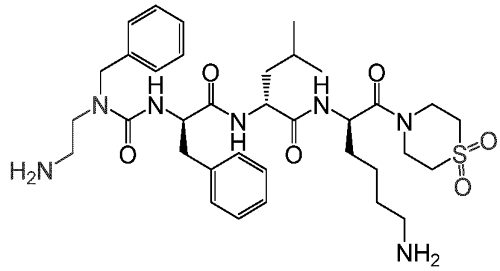

•(HCOOH)2

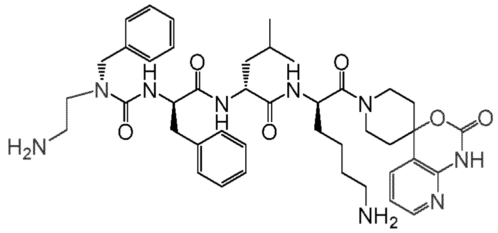

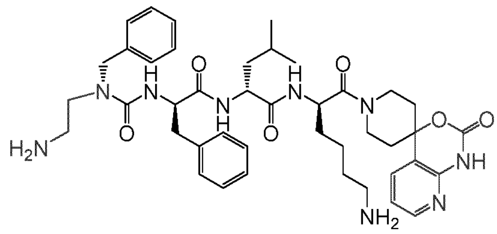

•HCOOH

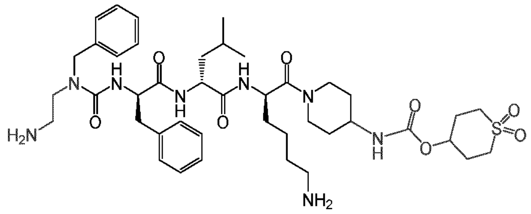

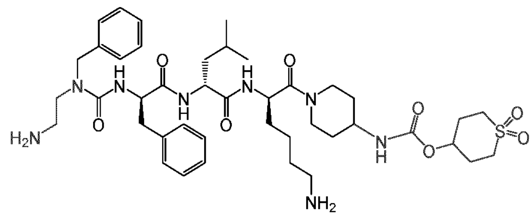

•(HCOOH)2

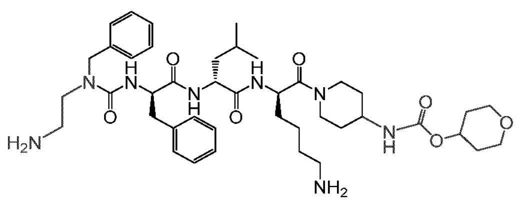

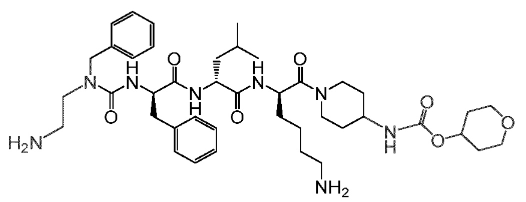

•(HCOOH)2

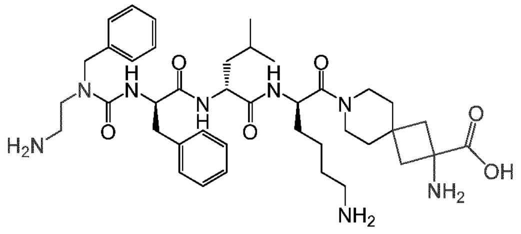

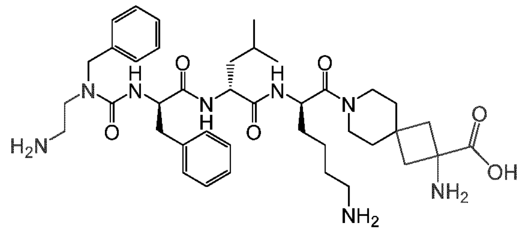

•(HCOOH)2

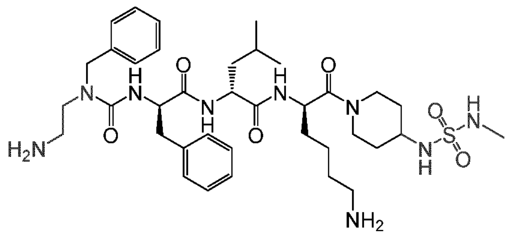

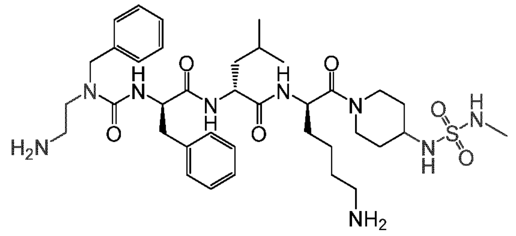

•(HCOOH)2

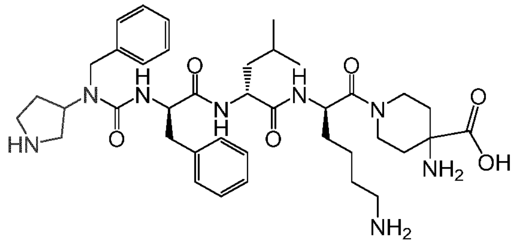

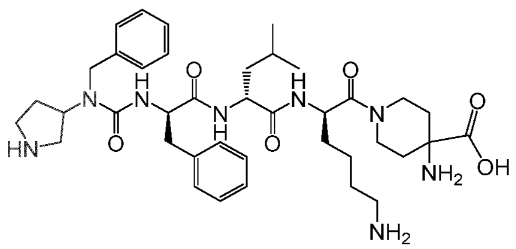

•HCOOH

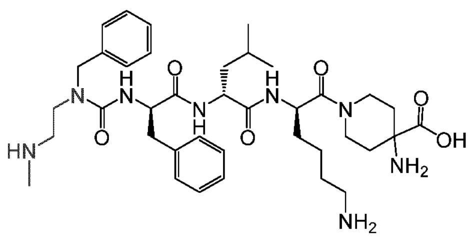

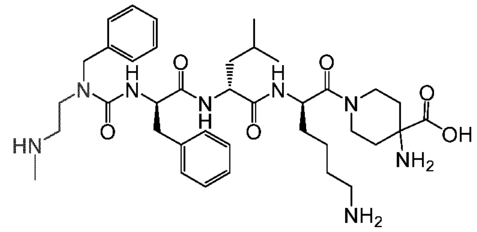

•(HCOOH)2

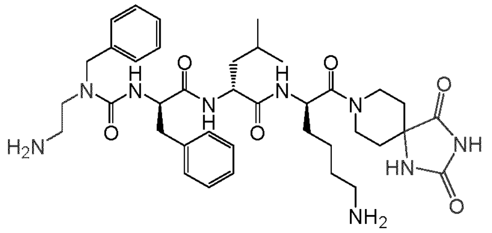

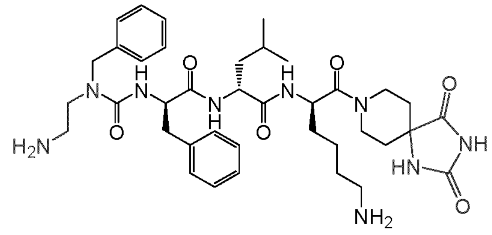

•(HCOOH)2

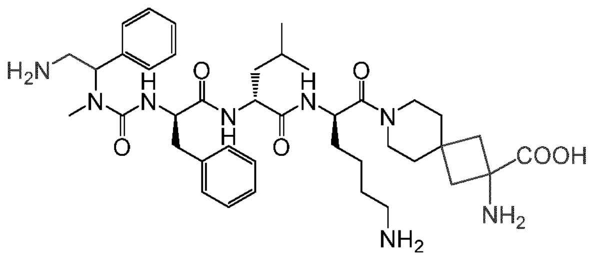

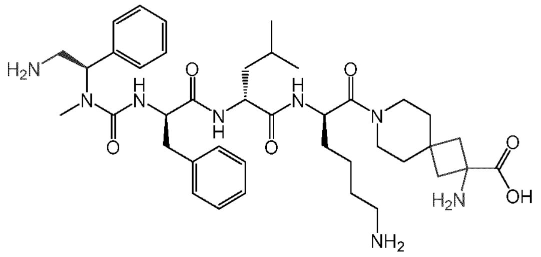

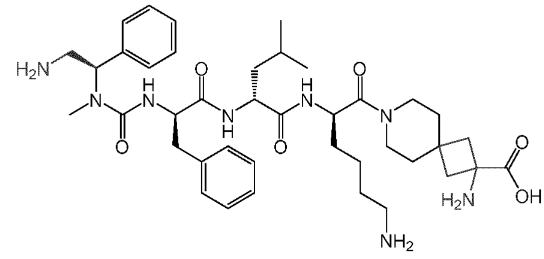

•HCOOH

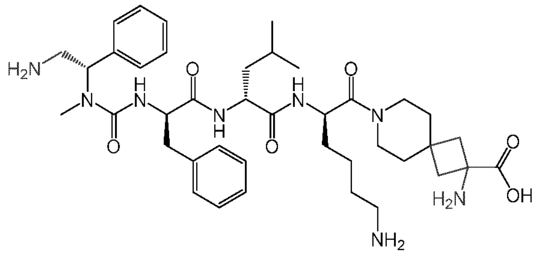

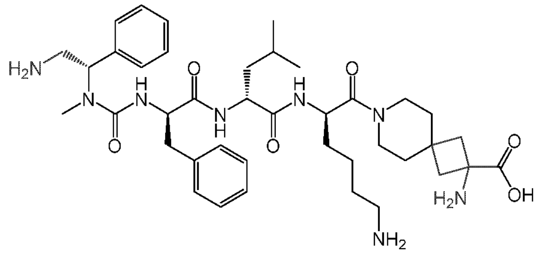

•HCOOH

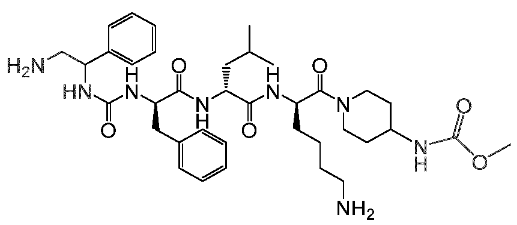

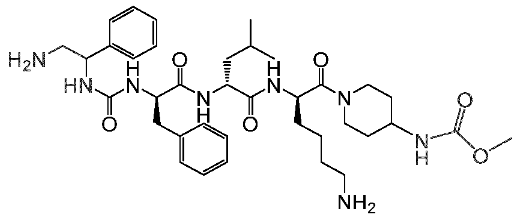

•(HCOOH)2

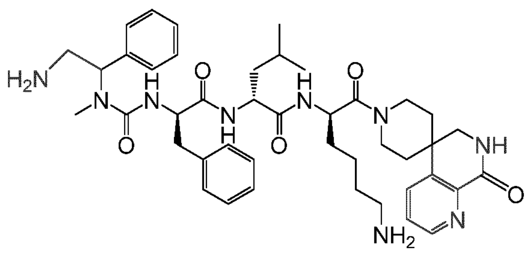

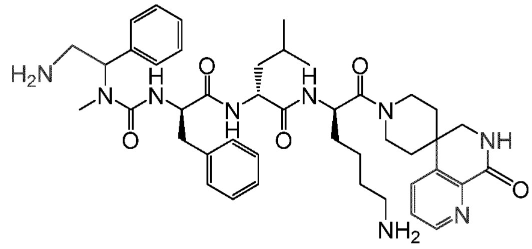

•HCOOH

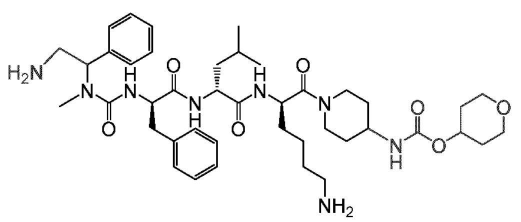

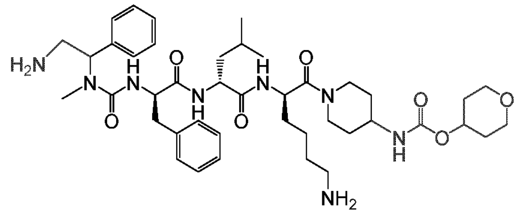

•(HCOOH)2

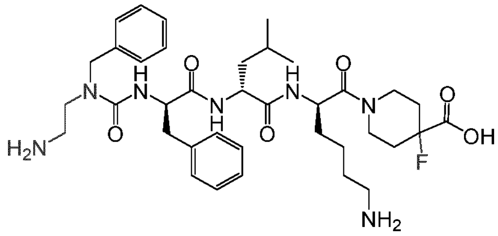

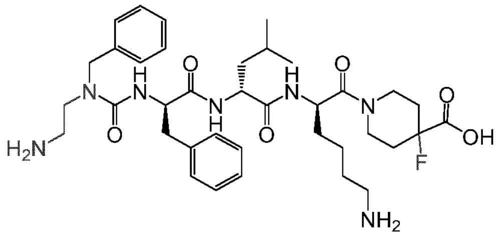

•HCOOH

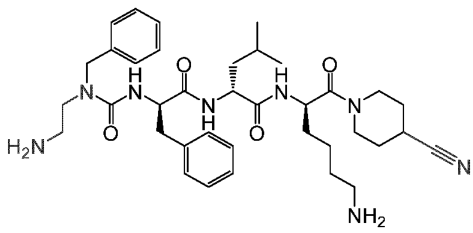

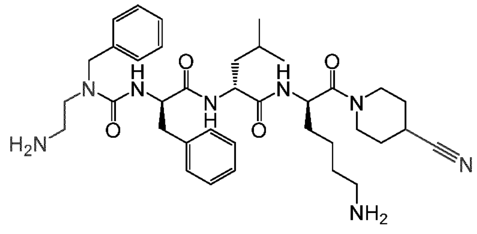

•(HCOOH)2

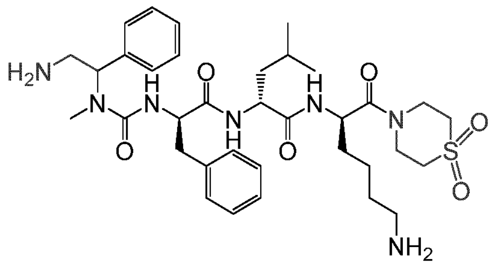

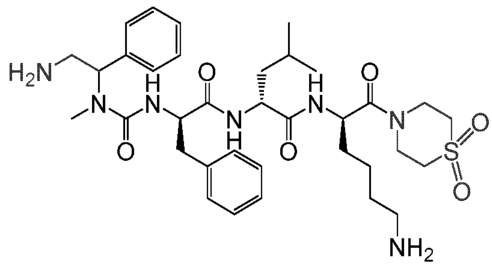

•HCOOH

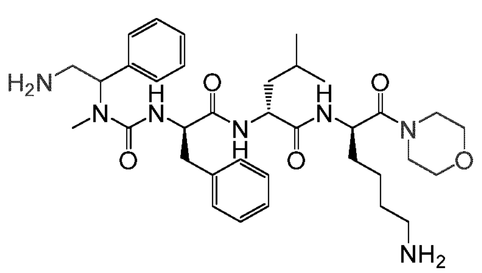

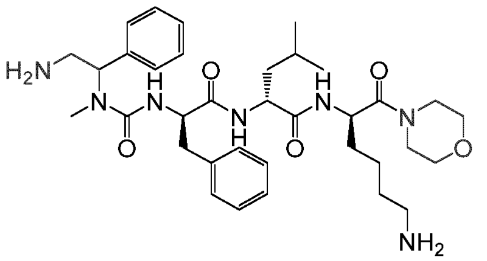

•(HCOOH)2

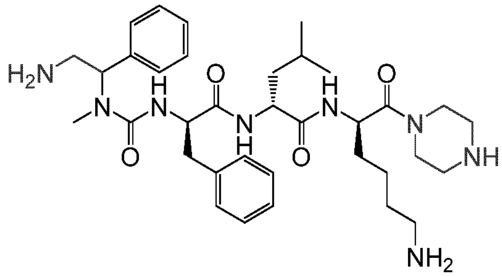

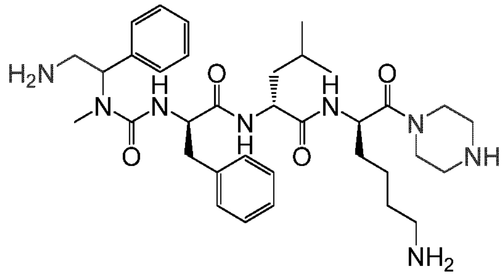

•HCOOH

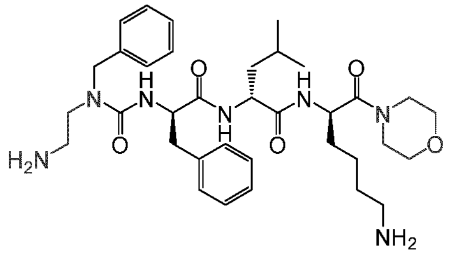

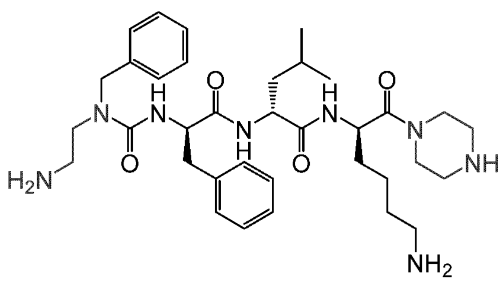

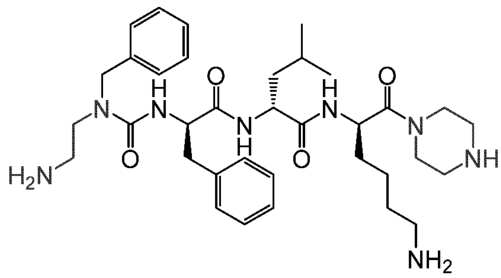

•HCOOH

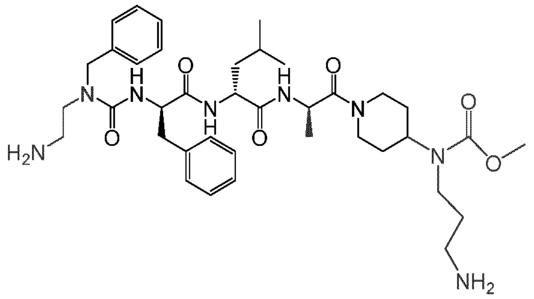

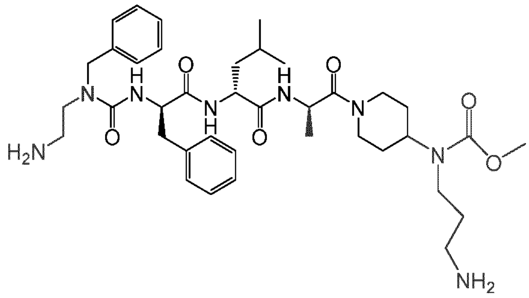

•(HCOOH)2

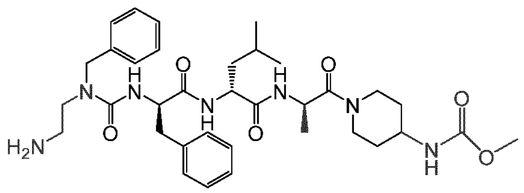

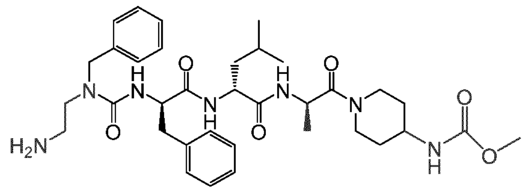

•HCOOH

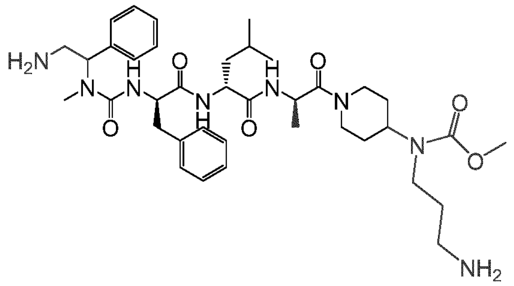

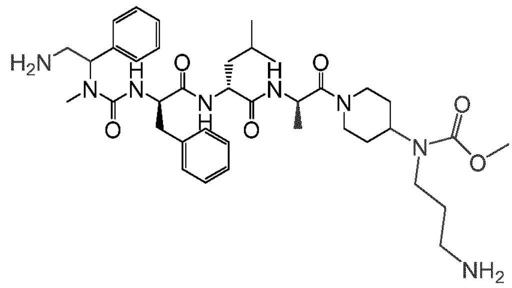

•(HCOOH)2

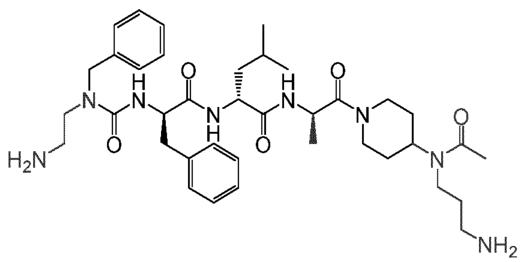

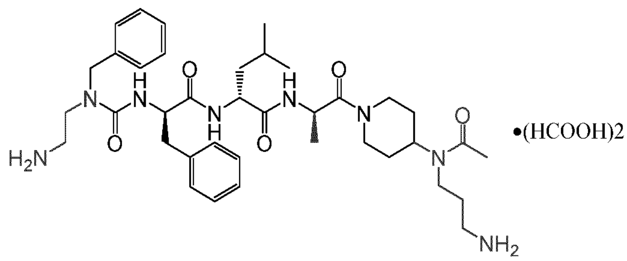

•(HCOOH)2

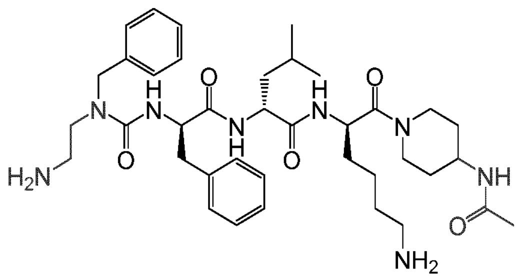

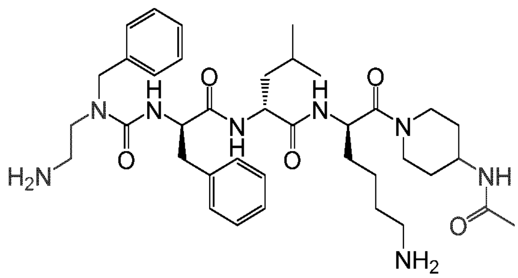

•HCOOH

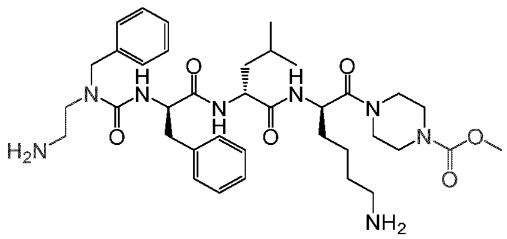

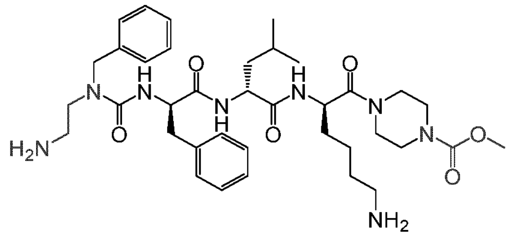

•(HCOOH)2

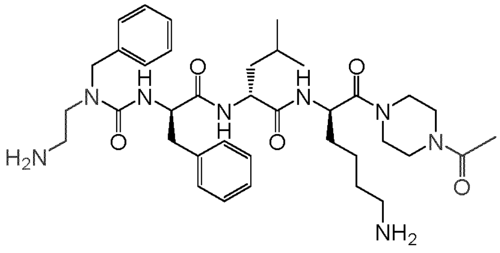

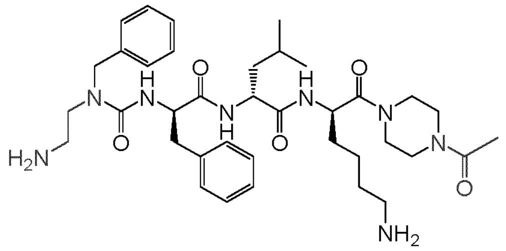

•(HCOOH)2

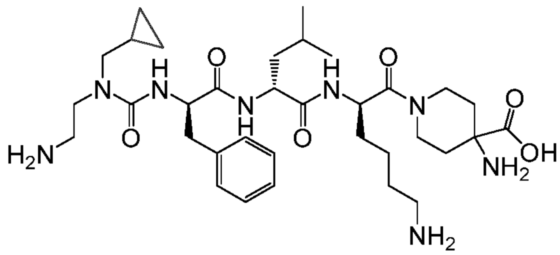

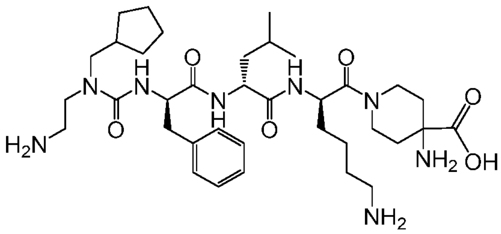

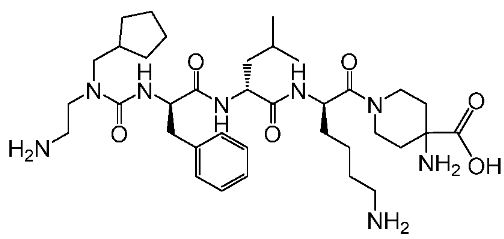

•(HCOOH)2

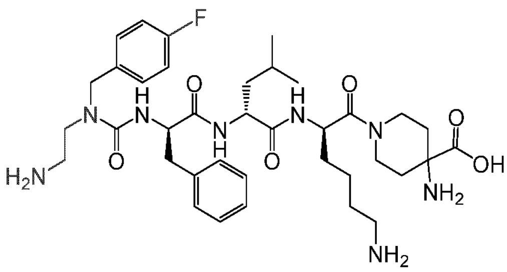

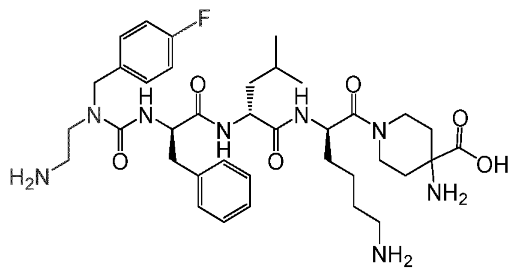

•(HCOOH)2

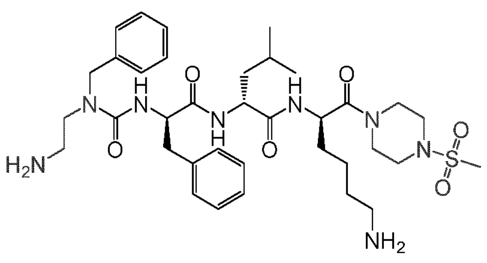

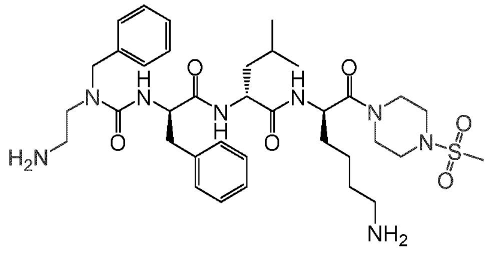

•(HCOOH)2

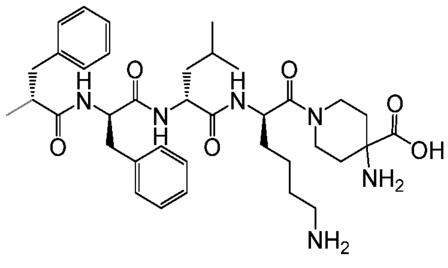

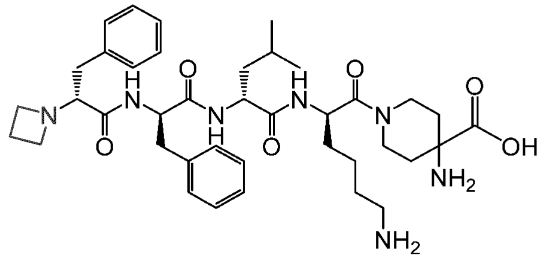

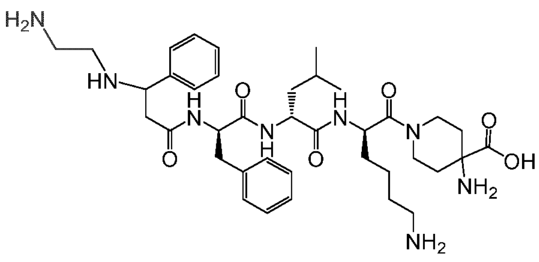

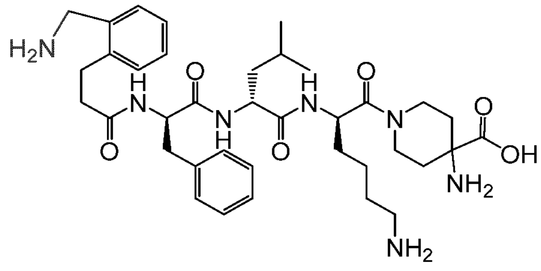

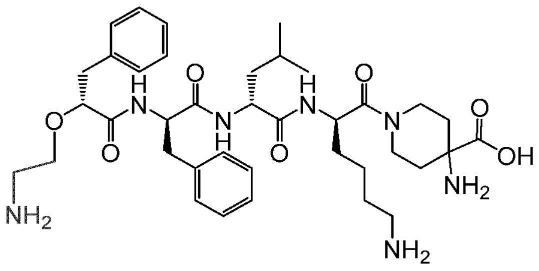

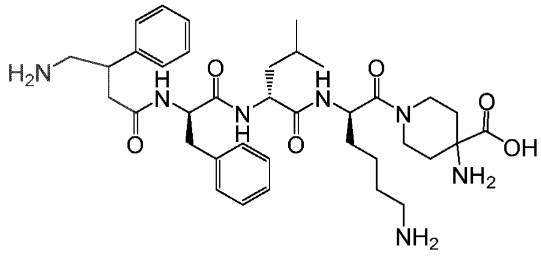

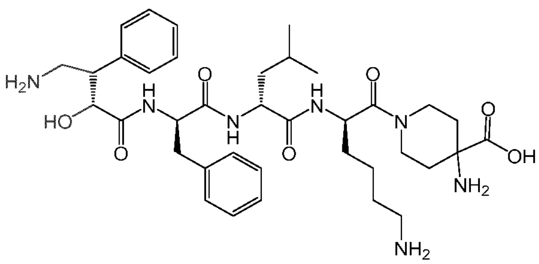

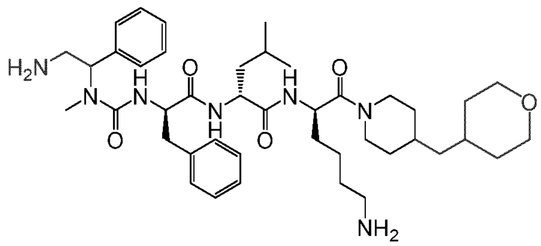

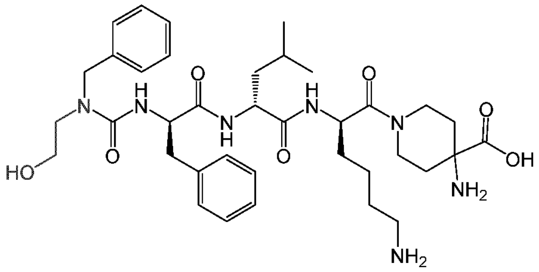

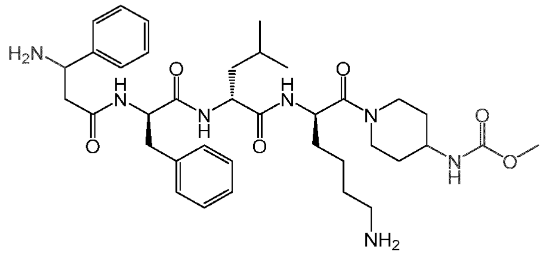

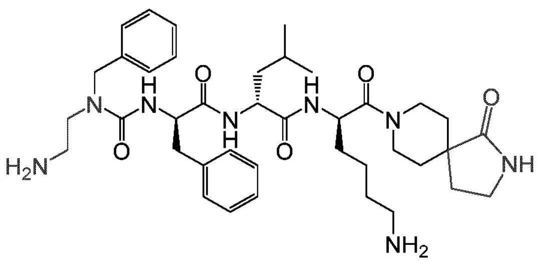

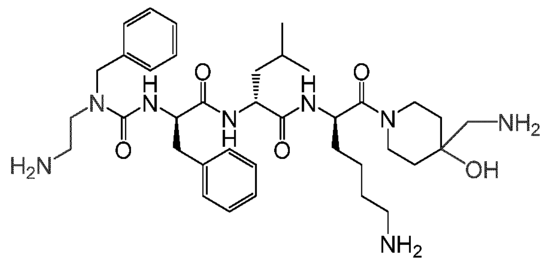

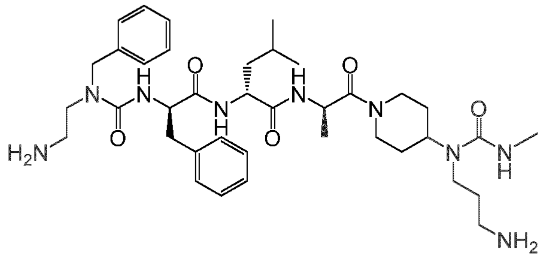

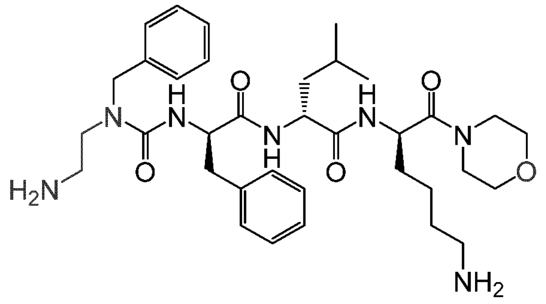

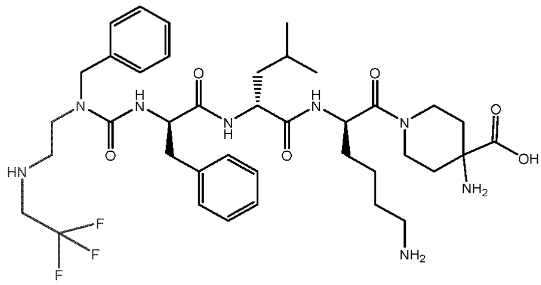

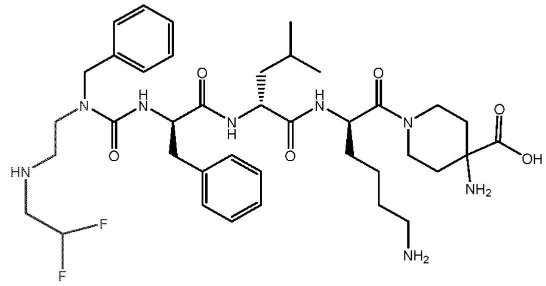

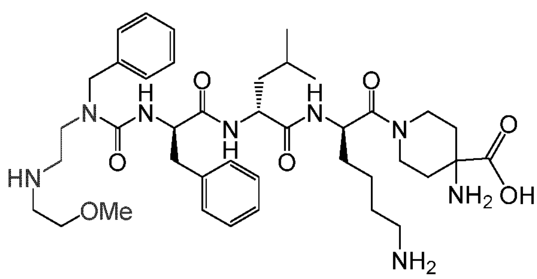

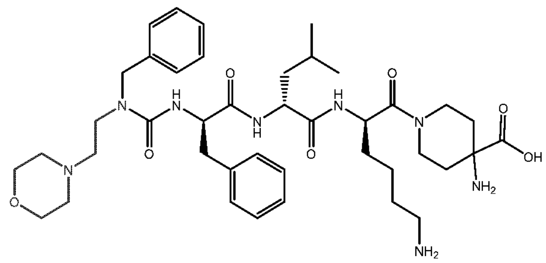

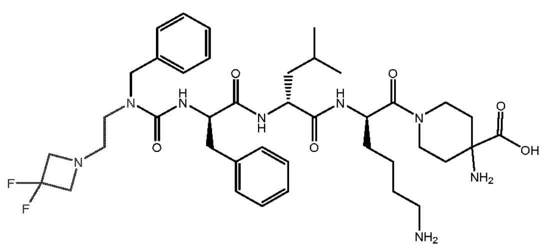

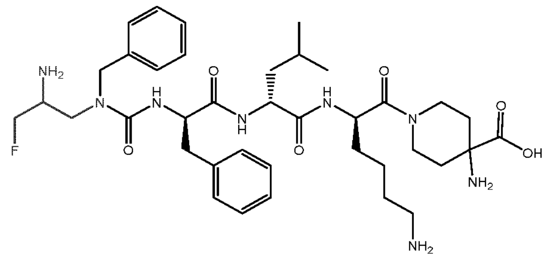

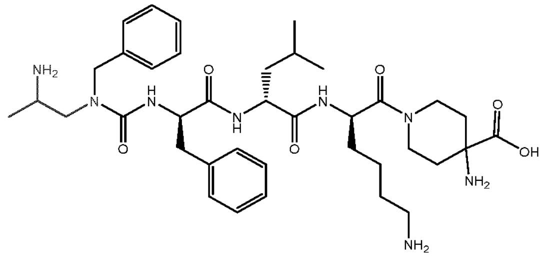

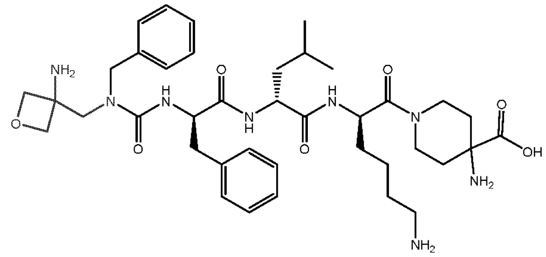

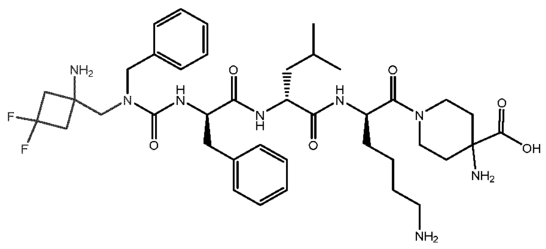

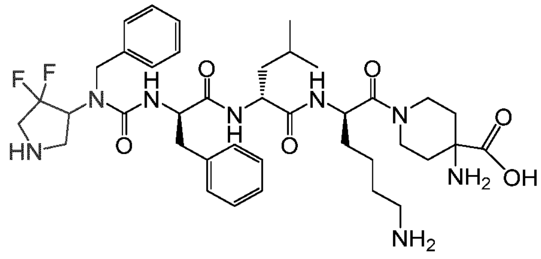

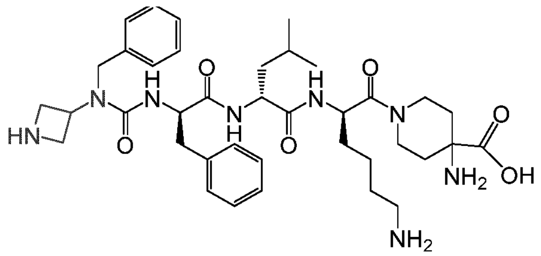

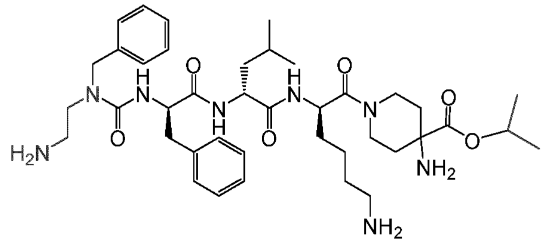

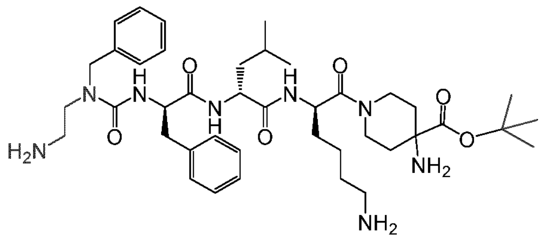

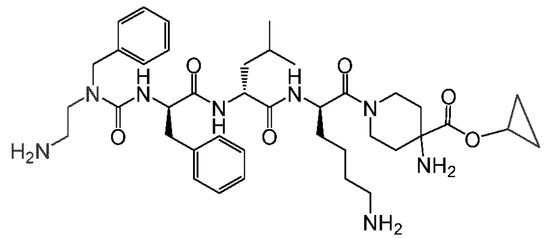

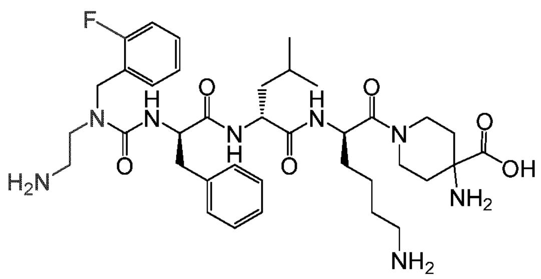

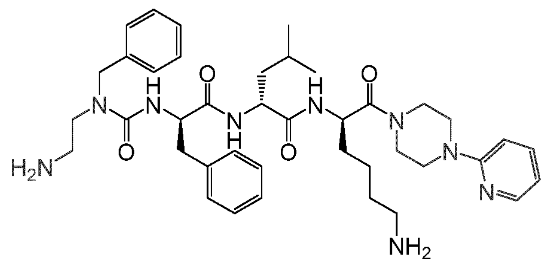

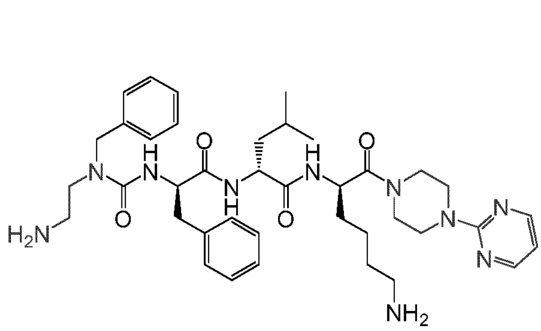

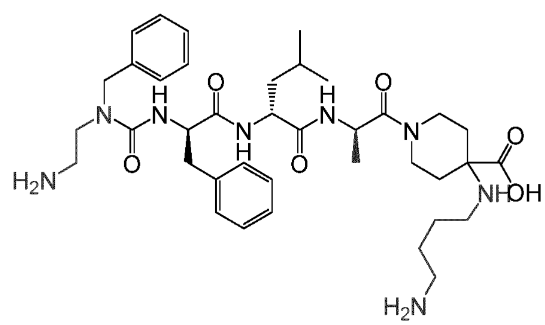

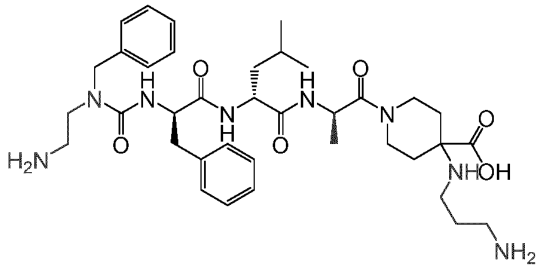

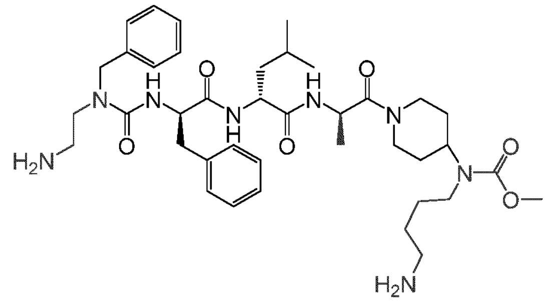

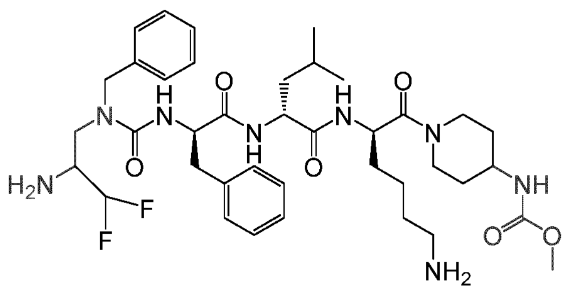

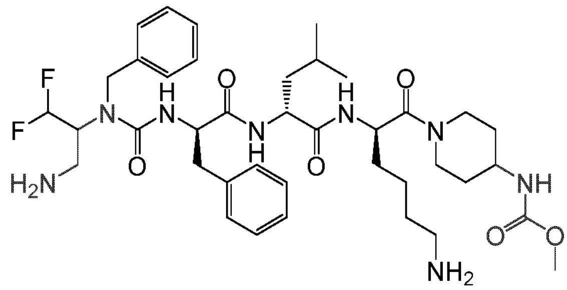

.

**20.** A method for preparing the compound, the stereoisomer thereof, or the pharmaceutically acceptable salt thereof according to any one of claims 14-19, being selected from the following methods:

method I:
method I comprising the following steps:

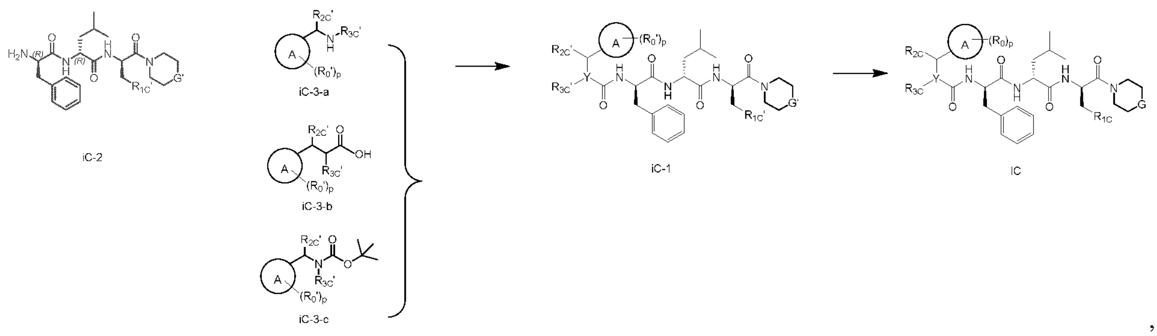

iC-2, iC-3-a, iC-3-b, iC-3-c, iC-1, IC

,

method II:
method II comprising the following steps:

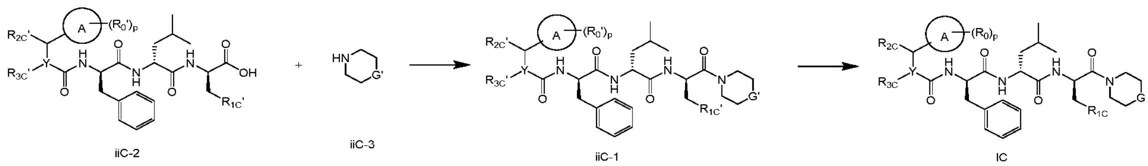

iiC-2, iiC-3, iiC-1, IC

,

wherein, preferably, a compound of formula iiC-2 is prepared by a method selected from:
method III:
method III comprising the following steps: subjecting a compound of formula iiC-4-a and a compound of formula iiC-5-a to a condensation reaction, a hydrolysis reaction, an optional removal reaction of an amino-protecting group, and an amino protection reaction to give the compound of formula iiC-2,

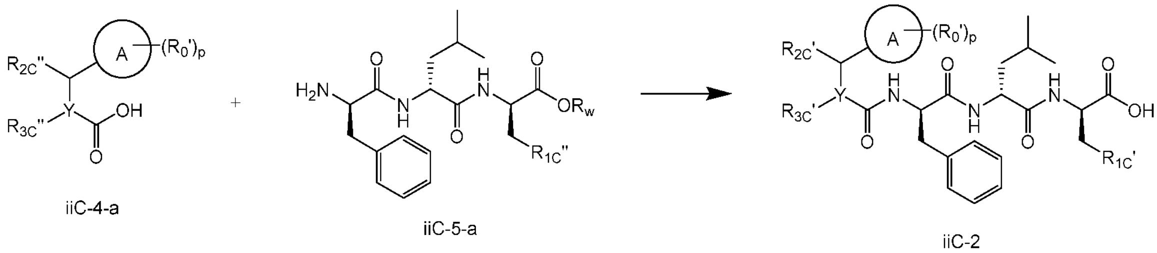

iiC-4-a iiC-5-a iiC-2

method IV:

method IV comprising the following steps: subjecting a compound of formula iiC-4-b and a compound of formula iiC-5-b to a condensation reaction, a hydrolysis reaction, an optional removal reaction of an amino-protecting group, and an amino protection reaction to give the compound of formula iiC-2,

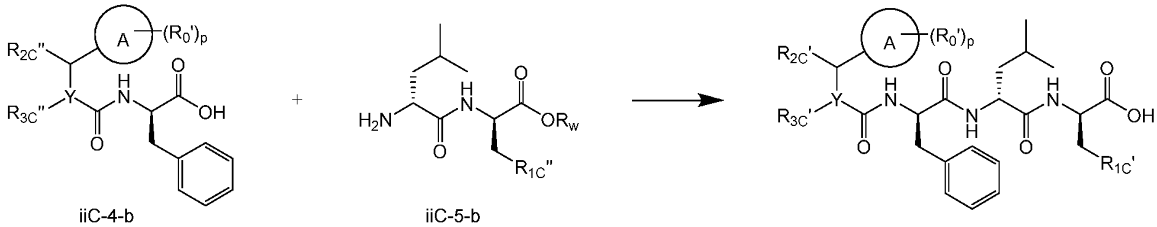

iiC-4-b iiC-5-b iiC-2

method V:

method V comprising the following steps: subjecting a compound of formula iiC-4-c and a compound of formula iiC-5-c to a condensation reaction, a hydrolysis reaction, an optional removal reaction of an amino-protecting group, and an amino protection reaction to give the compound of formula iiC-2,

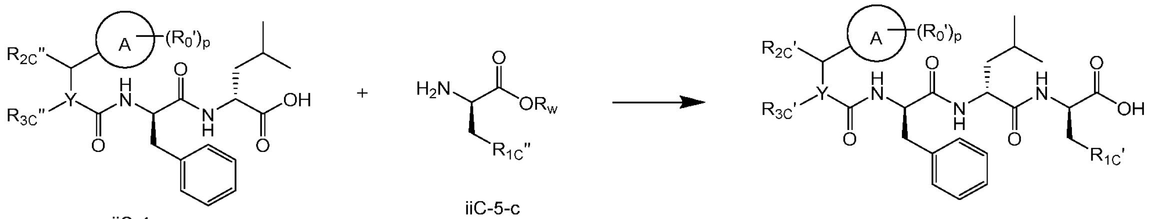

iiC-4-c iiC-5-c iiC-2 ,

method VI:

method VI comprising the following steps: subjecting a compound of formula iiC-4-d and a compound of formula iiC-5-d to a condensation reaction, a hydrolysis reaction, an optional removal reaction of an amino-protecting group, and an amino protection reaction to give the compound of formula iiC-2,

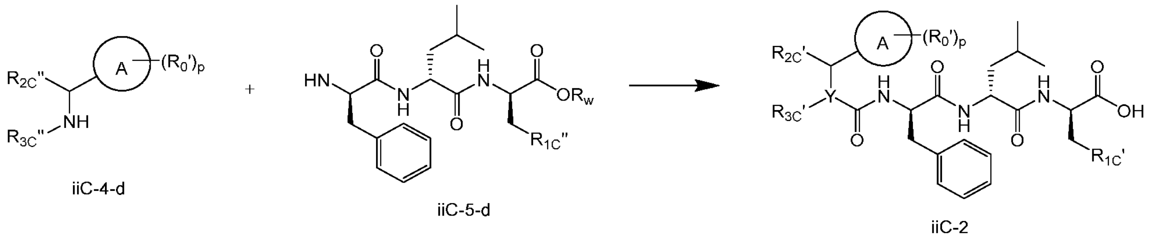

iiC-4-d iiC-5-d iiC-2 ,

preferably, the formula iC-2 is

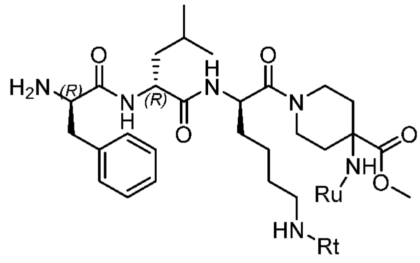

;

preferably, iiC-2 is selected from:

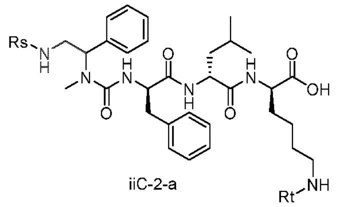

iiC-2-a ,

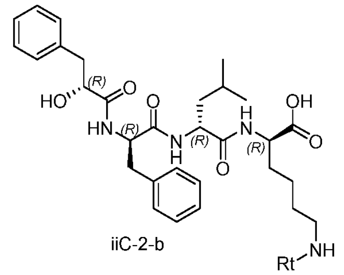

iiC-2-b ,

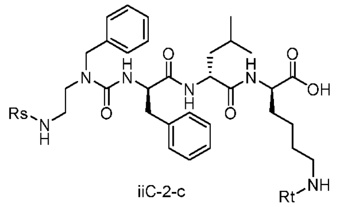

iiC-2-c ,

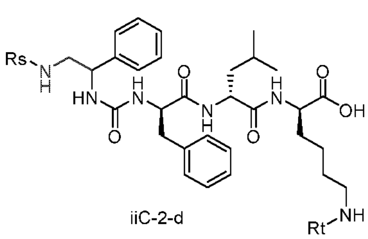

iiC-2-d ,

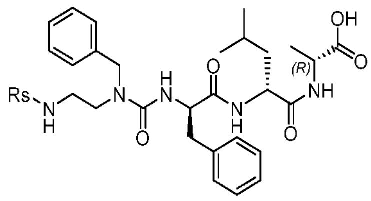

iiC-2-e , and

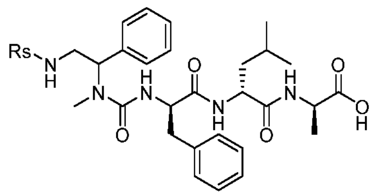

iiC-2-f ,

wherein, $R_0$' is $R_0$ or $R_0$ in which $NH_2$ is protected with an amino-protecting group, $R_{1C}$' and $R_{1C}$" are $R_{1C}$ or $R_{1C}$ in which $NH_2$ is protected with an amino-protecting group, $R_{2C}$' and $R_{2C}$" are $R_{2C}$ or $R_{2C}$ in which $NH_2$ is protected with an amino-protecting group, $R_{3C}$' and $R_{3C}$" are $R_{3C}$ or $R_{3C}$ in which $NH_2$ or the cyclic imino is protected with an amino-protecting group, G' is G or G in which $NH_2$ and/or the cyclic imino is protected with an amino-protecting group and/or the carboxyl is protected with a carboxyl-protecting group, Rw is a carboxyl-protecting group, ring A, Y, $R_0$, $R_{1C}$, $R_{2C}$, $R_{3C}$, G, and p are as defined in any one of claims 14-19, and Rs, Rt, and Ru are each independently selected from H and amino-protecting groups;

preferably, the amino-protecting groups are each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl*,* trifluoroacetyl, methoxycarbonyl, *tert*-butylsulfinyl, 1-phenylethyl, or ethoxycarbonyl; the carboxyl-protecting groups are each independently selected from $C_{1-6}$ alkyl, allyl, benzyl, 2,4-dimethoxybenzyl, *p*-methoxybenzyl, methoxyethoxymethyl, pentafluorophenyl, and 4-*p*-methylbenzyloxybenzyl.

**21.** A compound of the following general formula, a stereoisomer thereof, or a salt thereof,

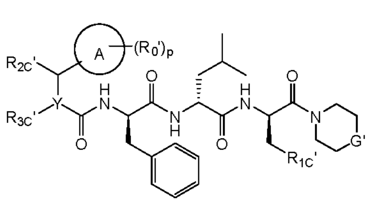

iC-1

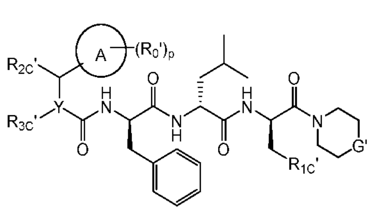

iiC-1

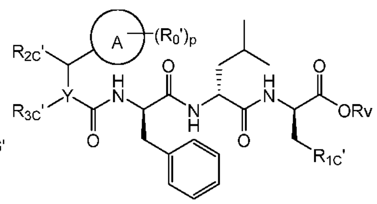

iiC-2-X

wherein preferably, formula iiC-2-X is selected from:

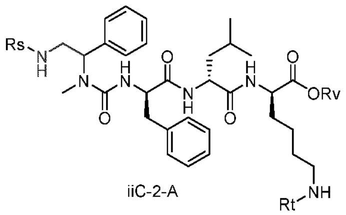
iiC-2-A ,

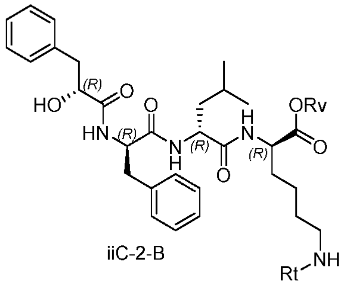
iiC-2-B ,

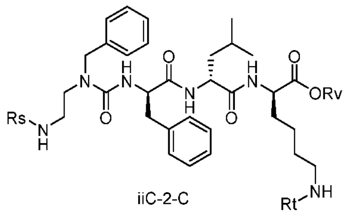
iiC-2-C ,

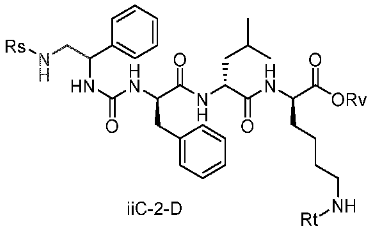
iiC-2-D ,

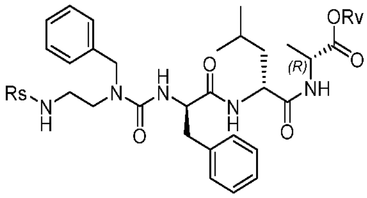
iiC-2-E , and

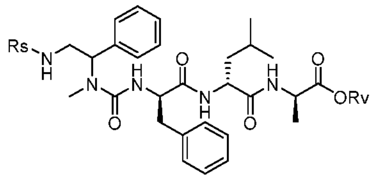
iiC-2-F ,

wherein $R_0$' is $R_0$ or $R_0$ in which $NH_2$ is protected with an amino-protecting group, $R_{1C}$' is $R_{1C}$ or $R_{1C}$ in which $NH_2$ is protected with an amino-protecting group, $R_{2C}$' is $R_{2C}$ or $R_{2C}$ in which $NH_2$ is protected with an amino-protecting group, $R_{3C}$' is $R_{3C}$ or $R_{3C}$ in which $NH_2$ or the cyclic imino is protected with an amino-protecting group, G' is G or G in which $NH_2$ and/or the cyclic imino is protected with an amino-protecting group and/or the carboxyl is protected with a carboxyl-protecting group, ring A, Y, $R_0$, $R_{1C}$, $R_{2C}$, $R_{3C}$, G, and p are as defined in any one of claims 14-19, Rs and Rt are each independently selected from H and amino-protecting groups, and Rv is selected from H and carboxyl-protecting groups;

preferably, the amino-protecting groups are each independently selected from *tert*-butoxycarbonyl, 9-fluorenylmethoxycarbonyl, allyloxycarbonyl, trichloroethoxycarbonyl, trimethylsilylethoxycarbonyl, benzyloxycarbonyl, *p*-toluenesulfonyl, *p*-nitrobenzenesulfonyl, *tert*-butyl, trifluoroacetyl, methoxycarbonyl, *tert*-butylsulfinyl, 1-phenylethyl, or ethoxycarbonyl; the carboxyl-protecting groups are each independently selected from $C_{1-6}$ alkyl, allyl, benzyl, 2,4-dimethoxybenzyl, *p*-methoxybenzyl, methoxyethoxymethyl, pentafluorophenyl, and 4-*p*-methylbenzyloxybenzyl.

**22.** A pharmaceutical composition comprising the compound, the stereoisomer thereof, or the pharmaceutically acceptable salt thereof according to any one of claims 1-5, 8-11, and 14-19, and a pharmaceutically acceptable carrier or excipient, as well as optionally other therapeutic agents.

**23.** Use of the compound, the stereoisomer thereof, or the pharmaceutically acceptable salt thereof according to any one of claims 1-5, 8-11, and 14-19, or the pharmaceutical composition according to claim 22 in the preparation of a medicament, wherein particularly, the medicament is used for agonizing κ-opioid receptor.

**24.** Use of the compound, the stereoisomer thereof, or the pharmaceutically acceptable salt thereof according to any one of claims 1-5, 8-11, and 14-19, or the pharmaceutical composition according to claim 22 in the preparation of a medicament, wherein particularly, the medicament is used for preventing and/or treating a related disease mediated by κ-opioid receptor; preferably, the disease is selected from pain, inflammation, pruritus, edema, hyponatremia, hypokalemia, ileus, cough, and glaucoma, preferably pain; preferably, the pain selected from neuropathic pain, trunk pain, visceral pain, skin pain, arthritis pain, kidney stone pain, uterine cramp, dysmenorrhea, endometriosis, dyspepsia, post-surgical pain, post-medical treatment pain, ocular pain, otitis pain, breakthrough cancer pain, and pain associated with a GI disorder.

# INTERNATIONAL SEARCH REPORT

International application No.
**PCT/CN2023/083063**

**A. CLASSIFICATION OF SUBJECT MATTER**

C07K5/10(2006.01)i； A61K38/07(2006.01)i； A61P25/04(2006.01)i； A61P29/00(2006.01)i

According to International Patent Classification (IPC) or to both national classification and IPC

**B. FIELDS SEARCHED**

Minimum documentation searched (classification system followed by classification symbols)

IPC: C07K, A61K, A61P

Documentation searched other than minimum documentation to the extent that such documents are included in the fields searched

Electronic data base consulted during the international search (name of data base and, where practicable, search terms used)

CNABS, CNTXT, DWPI, VEN, WPABS, WPABSC, ENTXT, ENTXTC, CNKI, Pubmed, ISI_Web of Science: κ阿片样物质受体, KOR受体, κ-opioid receptor, 激动剂, agonist, polyamide compound, 多酰胺化合物, phenyl propanamide derivative, 苯基丙酰胺类衍生物, peptide amide compound, 肽酰胺类化合物, 等

**C. DOCUMENTS CONSIDERED TO BE RELEVANT**

| Category* | Citation of document, with indication, where appropriate, of the relevant passages | Relevant to claim No. |
|---|---|---|
| X | WO 2018059331 A1 (SICHUAN KELUN BIOTECH BIOPHARMACEUTICAL CO., LTD.) 05 April 2018 (2018-04-05) claims 1-19, description, page 23, line 8-page 26, line 1, page 28, line 17-page 31, line 20, and page 35, embodiment 1 | 1-7, 14-24 |
| A | WO 2018059331 A1 (SICHUAN KELUN BIOTECH BIOPHARMACEUTICAL CO., LTD.) 05 April 2018 (2018-04-05) claims 1-19, description, page 23, line 8-page 26, line 1, page 28, line 17-page 31, line 20, and page 35, embodiment 1 | 8-13 |
| X | CN 107098871 A (JIANGSU HENGRUI MEDICINE CO.; SHANGHAI HENGRUI PHARMACEUTICAL CO., LTD.) 29 August 2017 (2017-08-29) claims 1-16 | 1-7, 14-24 |
| A | CN 107098871 A (JIANGSU HENGRUI MEDICINE CO.; SHANGHAI HENGRUI PHARMACEUTICAL CO., LTD.) 29 August 2017 (2017-08-29) claims 1-16 | 8-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **04 July 2023** | **15 July 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/083063**

**C. DOCUMENTS CONSIDERED TO BE RELEVANT**

| Category* | Citation of document, with indication, where appropriate, of the relevant passages | Relevant to claim No. |
|---|---|---|
| X | CN 107098876 A (JIANGSU HENGRUI MEDICINE CO.; SHANGHAI HENGRUI PHARMACEUTICAL CO., LTD.) 29 August 2017 (2017-08-29)<br>claims 1-15 | 1-7, 14-24 |
| X | WO 2021185265 A1 (SICHUAN HAISCO PHARMACEUTICAL CO., LTD.) 23 September 2021 (2021-09-23)<br>abstract, and claim 1 | 1-7, 14-24 |
| X | WO 2013184794 A2 (CARA THERAPEUTICS INC.) 12 December 2013 (2013-12-12)<br>claims 1-16 | 1-7, 14-24 |

Form PCT/ISA/210 (second sheet) (July 2022)

**INTERNATIONAL SEARCH REPORT**
**Information on patent family members**

International application No.
**PCT/CN2023/083063**

| Patent document cited in search report | Publication date (day/month/year) | Patent family member(s) | Publication date (day/month/year) |
|---|---|---|---|
| WO 2018059331 A1 | 05 April 2018 | PH 12019500199 A1 | 21 October 2019 |
| | | JP 2019526538 A | 19 September 2019 |
| | | JP 6794602 B2 | 02 December 2020 |
| | | US 2020109166 A1 | 09 April 2020 |
| | | US 11084847 B2 | 10 August 2021 |
| | | EP 3521301 A1 | 07 August 2019 |
| | | EP 3521301 A4 | 27 May 2020 |
| CN 107098871 A | 29 August 2017 | None | |
| CN 107098876 A | 29 August 2017 | None | |
| WO 2021185265 A1 | 23 September 2021 | US 2023172866 A1 | 08 June 2023 |
| | | TW 202143997 A | 01 December 2021 |
| | | EP 4122483 A1 | 25 January 2023 |
| WO 2013184794 A2 | 12 December 2013 | WO 2013184794 A3 | 30 January 2014 |
| | | US 2015150935 A1 | 04 June 2015 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 108290926 A
- CN 101627049 A